# EUROPEAN PATENT SPECIFICATION

(11) **EP 0 814 154 B1**
(45) Date of publication and mention of the grant of the patent: **29.07.2009**
(21) Application number: 97113527.2
(22) Date of filing: 15.09.1994
(51) Int. Cl.: C12N 7/01, C12N 15/86

(54) **Recombinant alphavirus vectors**
Rekombinante Alphavirus-Vektoren
Vecteurs composés d'alphavirus recombinants

(30) Priority: 15.09.1993 US 122791; 18.02.1994 US 198450
(43) Date of publication of application: 29.12.1997
(62) Divisional of application: 94929221.3
(73) Proprietor: Novartis Vaccines and Diagnostics, Inc., Emeryville, CA 94608 (US)
(72) Inventor: Dubensky, Thomas W. Jr., Del Mar, CA 92014 (US); Ibanez, Carlos E., San Diego, CA 92129 (US); Chang, Stephen M.W., Poway, California 92064 (US); Jolly, Douglas J., Leucadia, CA 92024 (US); Driver, David A., San Diego, CA 92117 (US); Polo, John M., Encinitas, CA 92024 (US)
(74) Representative: Hallybone, Huw George

(56) References cited:
- WO-A-92/10578
- DD-A- 298 269
- US-A- 5 091 309
- US-A- 5 185 440
- US-A- 5 229 293
- GRAKOUI A. ET AL.: "A cis-acting mutation in the Sindbis Virus junction region which affects subgenomic RNA synthesis" J. VIROLOGY, vol. 63, no. 12, December 1989, USA, pages 5216-5227, XP002010507
- RAJU R. ET AL.: "Analysis of Sindbis Virus promoter recognition in vivo using novel vectors with two subgenomic mRNA promoters" J. VIROLOGY, vol. 65, no. 5, May 1991, USA, pages 2501-2510, XP002010508
- CHENG XIONG ET AL.: "Sindbis virus: an efficient broad host range vector for gene expression in animal cells" SCIENCE, vol. 243, 3 March 1989, WASHINGTON, pages 1188-1191, XP002010510
- HERTZ, J.M. AND HUANG, H.V.: "Utilization of heterologous alphavirus junction sequences as promoters by sindbis virus" J. VIROLOGY, vol. 66, no. 2, February 1992, pages 857-864, XP000600233

## Description

### Technical Field

The present invention relates generally to use of recombinant viruses as vectors, and more specifically, to recombinant alphaviruses such as the Sindbis virus which are capable of expressing a heterologous sequence in target cells.

### Background of the Invention

Alphaviruses comprise a set of serologically related arthropod-borne viruses of the Togavirus family. Briefly, alphaviruses are distributed worldwide, and persist in nature through a mosquito to vertebrate cycle. Birds, rodents, horses, primates, and humans are among the defined alphavirus vertebrate reservoir/hosts.

Twenty-six known viruses and virus subtypes have been classified within the alphavirus genus utilizing the hemagglutination inhibition (HI) assay. Briefly, the HI test segregates the 26 alphaviruses into three major complexes: the Venezuelan encephalitis (VE) complex, the Semliki Forest (SF) complex, and the western encephalitis (WE) complex. In addition, four additional viruses, eastern encephalitis (EE), Barmah Forest, Middelburg, and Ndumu, receive individual classification based on the HI serological assay.

Members of the alphavirus genus are also classified based on their relative clinical features in humans: alphaviruses associated primarily with encephalitis, and alphaviruses associated primarily with fever, rash, and polyarthritis. Included in the former group are the VE and WE complexes, and EE. In general, infection with this group can result in permanent sequelae, including behavior changes and learning disabilities, or death. In the latter group is the SF complex, comprised of the individual alphaviruses Chikungunya, O'nyong-nyong, Sindbis, Ross River, and Mayaro. With respect to this group, although serious epidemics have been reported, infection is in general self-limiting, without permanent sequelae.

Sindbis virus is the prototype member of the alphavirus genus of the Togavirus family. Although not usually apparent, clinical manifestations of Sindbis virus infection include fever, arthritis, and rash. Sindbis virus is distributed over Europe, Africa, Asia, and Australia, with the best epidemiological data coming from South Africa where 20% of the population is seropositive. (For a review, *see* Peters and Dalrymple, Fields Virology (2d ed), Fields et al. (eds.), B.N. Raven Press, New York, NY. chapter 26. pp. 713-762). Infectious Sindbis virus has been isolated from human serum only during an outbreak in Uganda and in a single case from Central Africa.

The morphology and morphogenesis of the alphavirus genus is generally quite uniform. In particular, the enveloped 60-65 nm particles infect most vertebrate cells, where productive infection is cytopathic. On the other hand, infection of invertebrate cells, for example, those derived from mosquitoes, does not result in any overt cytopathology. Typically, alphaviruses are propagated on BHK-21 or vero cells, where growth is rapid, reaching a maximum yield within 24 hours of infection. Field strains are usually isolated on primary avian embryo, for example chick, fibroblast cultures.

The genomic RNA (49S RNA) of alphaviruses is unsegmented, of positive polarity, approximately 11-12 kb in length, and contains a 5' cap and a 3' polyadenylated tail. Infectious enveloped virus is produced by assembly of the viral nucleocapsid proteins onto viral genomic RNA in the cytoplasm, and budding through the cell membrane embedded with viral encoded glycoproteins. Entry of virus into cells occurs by endocytosis through clatherin-coated pits, fusion of the viral membrane with the endosome, release of the nucleocapsid and uncoating of the viral genome. During viral replication, the genomic 49S RNA serves as template for synthesis of the complementary negative strand. The negative strand in turn serves as template for genomic RNA and for an internally initiated 26S subgenomic RNA. The non-structural proteins are translated from the genomic RNA. Alphaviral structural proteins are translated from the subgenomic 26S RNA. All viral genes are expressed as a polyprotein and processed into individual proteins by proteolytic cleavage post translation.

The use of recombinant alphavirus vectors to treat individuals requires that they be able to be transported and stored for long periods at a desired temperature, such that infectivity and viability of the recombinant virus is retained. Current methods for storing recombinant viruses generally involve storage as liquids and at low temperatures. Such methods present problems in Third World countries, which typically do not have adequate refrigeration capabilities. For example, each year in Africa, millions of children die from infectious diseases such as measles. Vaccines necessary for the prevention of these diseases cannot be distributed to the majority of these countries because refrigeration is not readily accessible.

In addition to storage as liquids and at low temperatures, present viral formulations often contain media components that are not desirable for injection into patients. Consequently, there is a need in the art for a method of preserving purified recombinant viral vector (and in particular, alphavirus vectors) in a lyophilized form at elevated temperatures, and for this form to be suitable for injection into patients.

The present invention discloses recombinant alphavirus vectors which are suitable for use in a variety of applications, including for example, gene therapy, and further provides other related advantages.

WO 92/10578 discloses DNA expression systems based on alphaviruses. It discloses a helper RNA molecule transcribed in vitro from a helper DNA vector that comprises the region encoding the viral structural proteins.

Suomalainen et al. (Journal of Virology 1992, 66(8):4737-4747) describes a cDNA that directs the synthesis of an RNA encoding only an alphavirus capsid protein.

Frolov & Schlesinger (Journal of Virology 1994, 68(3):1721-1727) describes cDNAs that encode an alphavirus capsid protein and part of the E3 protein.

### Summary of the Invention

The invention provides DNA alphavirus structural protein expression cassettes as defined in the claims.

In one aspect, the invention provides a DNA alphavirus structural.protein expression cassette which comprises a promoter which directs the synthesis of an RNA molecule in a eukaryotic cell, said RNA molecule comprising the coding sequence for an alphavirus capsid protein, but not coding sequences for alphavirus glycoproteins E1 and E2.

In another aspect, the invention provides a DNA alphavirus structural protein expression cassette which comprises a promoter which directs the synthesis of an RNA molecule in a eukaryotic cell, said RNA molecule comprising coding sequences for alphavirus glycoproteins E1 and E2, but not a coding sequence for an alphavirus capsid protein.

Aspects of the following disclosure that do not relate specifically to the claimed invention are included for comparison and illustration only.

### Summary of the Disclosure

Briefly stated, the present disclosure relates to alphavirus vector constructs and alphavirus particles, as well as methods of making and utilizing the same. Within one aspect of the present disclosure alphavirus vector constructs are provided comprising a 5' promoter which is capable of initiating the synthesis of viral RNA *in vitro* from cDNA. a 5' sequence which is capable of initiating transcription of an alphavirus, a nucleotide sequence encoding alphavirus non-structural proteins, a viral junction region which has been inactivated such that viral transcription of the subgenomic fragment is prevented, and an alphavirus RNA polymerase recognition sequence. Within other aspects of the present disclosure, the viral junction region has been modified such that viral transcription of the subgenomic fragment is reduced.

Within yet other aspects of the present disclosure, alphavirus vector constructs are disclosed comprising a 5' promoter which is capable of initiating the synthesis of viral RNA *in vitro* from cDNA, a 5' promoter which is capable of initiating the synthesis of viral RNA *in vitro* from cDNA 5' sequence which is capable of initiating transcription of an alphavirus, a nucleotide sequence encoding alphavirus non-structural proteins, a first viral junction region which has been inactivated such that viral transcription of the subgenomic fragment is prevented, a second viral junction region which has been modified such that viral transcription of the subgenomic fragment is reduced, and an alphavirus RNA polymerase recognition sequence.

Within still other aspects of the present disclosure, alphavirus cDNA vector constructs are disclosed comprising a 5' promoter which is capable of initiating the synthesis of viral RNA *in vitro* from cDNA, a 5' promoter which is capable of initiating the synthesis of viral RNA from cDNA followed by a 5' sequence which is capable of initiating transcription of an alphavirus, a nucleotide sequence encoding alphavirus non-structural proteins, a viral junction region which has been inactivated such that viral transcription of the subgenomic fragment is prevented, an alphavirus RNA polymerase recognition sequence, and a 3' sequence which controls transcription termination.

Within another aspect of the present disclosure, alphavirus cDNA vector constructs are disclosed, comprising a 5' promoter which is capable of initiating the synthesis of viral RNA from cDNA followed by a 5' sequence which is capable of initiating transcription of an alphavirus, a nucleotide sequence encoding alphavirus non-structural proteins, a viral junction region which has been modified such that viral transcription of the subgenomic fragment is reduced, an alphavirus RNA polymerase recognition sequence, and a 3' sequence which controls transcription termination.

Within another aspect of the present disclosure, alphavirus cDNA vector constructs are disclosed comprising a promoter which is capable of initiating the synthesis of viral RNA from cDNA followed by a 5' sequence which is capable of initiating transcription of an alphavirus, a nucleotide sequence encoding alphavirus non-structural proteins, a first viral junction region which has been inactivated such that viral transcription of the subgenomic fragment is prevented, followed by a second viral junction region which has been modified such that viral transcription of the subgenomic fragment is reduced, an alphavirus RNA polymerase recognition sequence, and a 3' sequence which controls transcription termination.

In one embodiment, the vector constructs described above contain a heterologous sequence. Typically, such a vector construct contains a heterologous nucleotide sequence of greater than 100 bases, generally the heterologous nucleotide sequence is greater than 3 kb, and preferably the heterologous nucleotide sequence is greater than 5 kb, and more preferably the heterologous nucleotide sequence is greater than 8 kb. In various embodiments, the heterologous sequence is a sequence encoding a protein selected from the group consisting of IL-1, IL-2, IL-3, IL-4, IL-5, IL-6, IL-7, IL-8, IL-9, IL-10, IL-11, IL-12, IL-13, IL-14, IL-15, α, β, and γ-IFN, G-CSF, and GM-CSF.

In still other embodiments, the vector constructs described above include a selected heterologous sequence which may be from a virus selected from the group consisting of influenza virus, HPV, HBV, HCV, EBV, HIV, HSV, FeLV, FIV, Hanta virus, HTLV I, HTLV II and CMV. Within one preferred embodiment, the heterologous sequence obtained from HPV encodes a protein selected from the group consisting of E5, E6, E7 and L1.

In yet other embodiments, the vector constructs described above include a selected heterologous sequence encoding an HIV protein selected from the group consisting of HIV gp 120 and *gag.*

The selected heterologous sequences described above may be antisense sequences. In preferred embodiments, the antisense sequence is selected from the group consisting of sequences which encode influenza virus, HPV, HBV, HCV, EBV, HIV, HSV. FeLV, FIV, Hanta virus, HTLV I, HTLV II, and CMV.

In another embodiment, the vector constructs described above contain no alphavirus structural proteins. Within other embodiments, the selected heterologous sequence is located downstream from the viral junction region. In the vector constructs described above having a second viral junction, the selected heterologous sequence may, within certain embodiments, be located downstream from the second viral junction region. Where the heterologous sequence is located downstream from the viral junction region, the vector construct may further comprise a polylinker located subsequent to the viral junction region. Within preferred embodiments, such polylinkers do not contain a wild-type alphavirus virus restriction endonuclease recognition sequence.

In yet another embodiment, in the vector constructs described above the selected heterologous sequence may be located within the nucleotide sequence encoding alphavirus non-structural proteins.

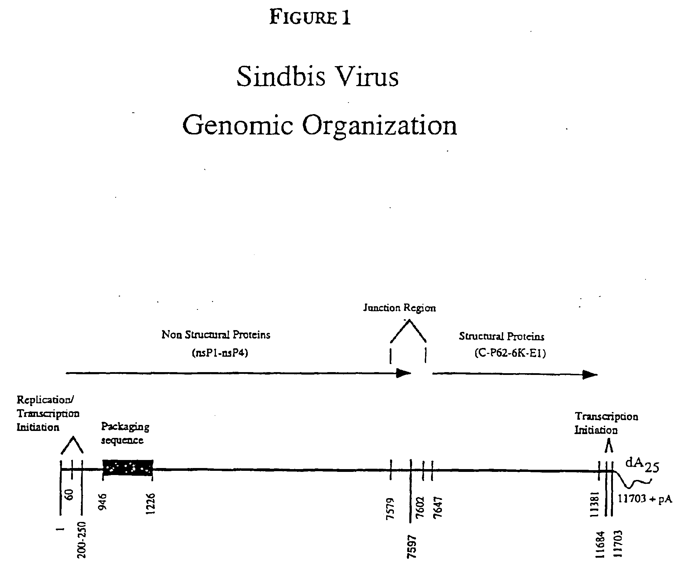

In particular embodiments, the vector constructs described above include a viral junction region consisting of the nucleotide sequence as shown in Figure 1, from nucleotide number 7579, to nucleotide number 7597 (SEQ. ID NO. 1). In alternative embodiments, where the vector construct includes a second viral junction, the vector constructs include an E3 adenovirus gene located downstream from the second viral junction region, and may also further comprise a retroviral packaging sequence located between the first viral junction region and the second viral junction region.

In further aspects, there is disclosed herein an isolated recombinant alphavirus vector which does not contain a functional viral junction region, and which in preferred embodiments produces reduced viral transcription of the subgenomic fragment.

In still a further aspect, there is disclosed herein an expression cassette, comprising a promoter and one or more alphavirus structural proteins, the promoter being capable of directing the expression of the alphavirus structural proteins.

In various embodiments, the expression cassette is capable of expressing the alphavirus capsid protein, such as a Sindbis structural protein selected from the group consisting of 6K, E3, E2, and E1.

In yet another aspect, there is disclosed herein an expression cassette, comprising a promoter, one or more alphavirus structural proteins, and a heterologous ligand sequence, the promoter being capable of directing the expression of the alphavirus structural proteins and the heterologous sequence. In various embodiments, the heterologous ligand sequence is selected from the group consisting of VSVG, HIV gp120. antibody, insulin, and CD4.

In certain embodiments, the expression cassettes described above include a promoter selected from the group consisting of MuL V, MMTV, alphavirus junction region, CMV and VA1RNA.

In still yet another aspect, there is disclosed herein an alphavirus particle which, upon introduction into a target cell, produces an infected cell which is viable at least 72 hours after infection. Also disclosed are target cells which, after infection by an alphavirus particle of the present invention, are viable at least 72 hours after infection.

In another aspect, there is disclosed herein a recombinant alphavirus particle which, upon introduction into a target cell, produces an infected cell which is viable at least 72 hours after infection, the particle also carrying a vector construct which directs the expression of at least one antigen or modified form thereof in target cells infected with the alphavirus particle, the antigen or modified form thereof being capable of stimulating an immune response within an animal. In various embodiments, the expressed antigen or modified form thereof elicits a cell-mediated immune response, preferably an HLA class I-restricted immune response.

In still another aspect, there is disclosed herein a recombinant alphavirus particle which carries a vector construct capable of directing the expression of a palliative in cells infected with the alphavirus particle, the palliative being capable of inhibiting a function of a pathogenic agent necessary for pathogenicity. In various embodiments, the pathogenic agent is a virus, fungi, protozoa, or bacteria, and the inhibited function is selected from the group consisting of adsorption, replication, gene expression, assembly, and exit of the pathogenic agent from infected cells. In other embodiments, the pathogenic agent is a cancerous cell, cancer-promoting growth factor, autoimmune disorder, cardiovascular disorders such as restenosis, osteoporosis and male pattern baldness, and the inhibited function is selected from the group consisting of cell viability and cell replication. In further embodiments, the vector construct directs the expression of a toxic palliative in infected target cells in response to the presence in such cells of an entity associated with the pathogenic agent; preferably the palliative is capable of selectively inhibiting the expression of a pathogenic gene or inhibiting the activity of a protein produced by the pathogenic agent. In still further embodiments, the palliative comprises an inhibiting peptide specific for viral protease, an antisense RNA complementary to RNA sequences necessary for pathogenicity, a sense RNA complementary to RNA sequences necessary for pathogenicity, or a defective structural protein of a pathogenic agent, such protein being capable of inhibiting assembly of the pathogenic agent.

In yet further embodiments, the alphavirus particle described above directing the expression of a palliative, more particularly, directs the expression of a gene product capable of activating an otherwise inactive precursor into an active inhibitor of the pathogenic agent, for example, the herpes thymidine kinase gene product, a tumor suppressor gene, or a protein that activates a compound with little or no cytotoxicity into a toxic product in the presence of a pathogenic agent, thereby effecting localized therapy to the pathogenic agent. Alternatively, the alphavirus particle directs the expression of a protein that is toxic upon processing or modification by a protein derived from a pathogenic agent, a reporting product on the surface of target cells infected with the alphavirus and containing the pathogenic agent, or an RNA molecule which functions as an antisense or ribozyme specific for a pathogenic RNA molecule.

In certain embodiments, in the alphavirus particle described above, the protein is herpes thymidine kinase or CD4.

In yet further aspects, there is disclosed herein an alphavirus particle which directs the expression of a gene capable of suppressing one or more elements of the immune system in target cells infected with the alphavirus, and an alphavirus particle which directs the expression of a blocking element in cells infected with the Sindbis virus, the blocking element being capable of binding to either a receptor or an agent such that the receptor/agent interaction is blocked.

In further aspects, there is disclosed herein a method of stimulating an immune response to an antigen, comprising infecting susceptible target cells with an alphavirus particle which directs the expression of at least one antigen or modified form thereof in target cells infected with the alphavirus, the antigen or modified form thereof being capable of stimulating an immune response within an animal. In a preferred embodiment, the target cells are infected *in vivo.*

In still further aspects of the present disclosure, a method of stimulating an immune response to a pathogenic antigen is disclosed, comprising infecting susceptible target cells with an alphavirus particle which directs the expression of a modified form of a pathogenic antigen in target cells infected with the alphavirus, the modified antigen being capable of stimulating an immune response within an animal but having reduced pathogenicity relative to the pathogenic antigen.

In even further aspects of the present disclosure a method of stimulating an immune response to an antigen is disclosed, comprising infecting susceptible target cells with a alphavirus particle which directs the expression of a peptide having multiple epitopes, one or more of the epitopes derived from different proteins.

In yet another aspect of the disclosure of stimulating an immune response within a warm-blooded animal is disclosed, comprising infecting susceptible target cells associated with a warm-blooded animal with nucleic acid sequences coding for either individual class I or class II MHC protein, or combinations thereof, and infecting the cells with an alphavirus particle which directs the expression of at least one antigen or modified form thereof in target cells infected with the alphavirus particle, the antigen or modified form thereof being capable of stimulating an immune response within the animal.

In another aspect of the present disclosure, a method of inhibiting a pathogenic agent is disclosed, comprising infecting susceptible target cells with an alphavirus particle which directs the expression of a palliative in cells infected with the alphavirus particle, the palliative being capable of inhibiting a function of a pathogenic agent necessary for pathogenicity.

In other aspects of the present disclosure, eukaryotic layered vector initiation systems are disclosed. Briefly, within one embodiment of the disclosure, the eukaryotic layered vector initiation system comprises a 5' promoter, a construct which is capable of expressing a heterologous nucleotide sequence that is capable of replication in a cell either autonomously, or in response to one or more factors, and a transcription termination sequence. Within another aspect, a DNA eukaryotic layered vector initiation system is disclosed, comprising a 5' promoter, a construct which is capable of expressing a heterologous RNA sequence that is capable of replication in a cell either autonomously or in response to one or more factors, and a transcription termination sequence. Within a preferred embodiment, the construct which is capable of expressing one or more heterologous nucleotide sequences is a Sindbis cDNA vector construct. Within other embodiments, the construct is a viral vector selected from the group consisting of poliovirus, rhinovirus, pox virus, retrovirus, influenza virus, adenovirus, adeno-associated virus, herpes virus, SV40 virus, HIV, measles, astrovirus, Semliki Forest Virus and coronavirus. Within a further embodiment, the eukaryotic layered vector initiation system further comprises a polyadenylation sequence.

In still another aspect, there is disclosed herein an alphavirus RNA vector molecule described above, capable of directing the expression of a palliative in a target cell. The palliative in this alphavirus RNA expression vector configuration, when expressed, has the same effect as the aspects described above for an alphavirus particle. The alphavirus RNA expression vector includes, in order, a 5' sequence which is capable of initiating transcription of an alphavirus, a nucleotide sequence encoding alphavirus non-structural proteins, a viral junction region, a heterologous sequence, an alphavirus RNA Polymerase recognition sequence, and a stretch of 25 consecutive polyadenylate residues, and is introduced into the target cells directly by physical means as an RNA molecule, as a complex with various liposome formulations, or as an RNA ligand complex including the alphavirus RNA vector molecule, a polycation compound such as polylysine, a receptor specific ligand, and, optionally, a psoralen inactivated virus such as Sendai or Adenovirus.

There are also disclosed herein packaging cell lines and producer cell lines suitable for producing recombinant alphavirus particles. Such packaging or producer cell lines may be either mammalian or non-mammalian (*e.g.*, insect cells such as mosquito cells).

A wide variety of alphaviruses may be utilized within the context of the present invention. Representative examples include Aura, Venezuelan Equine Encephalitis, Fort Morgan, Ross River, Semliki Forest Virus and Mayaro.

These and other aspects of the present invention will become evident upon reference to the following detailed description and attached drawings. In addition, various references are set forth below which describe in more detail certain procedures or compositions (*e.g.*, plasmids, etc.).

### Brief Description of the Drawings

Figures that do not relate specifically to the claimed invention are for comparison and illustration only.
Figure 1 is a schematic illustration of Sindbis virus genomic organization.

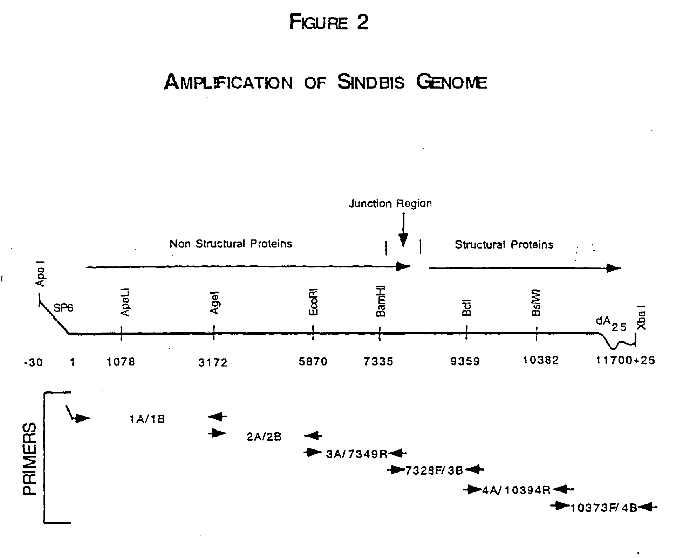

Figure 2 is an illustration which depicts a method for amplification of a Sindbis RNA genome by RT-PCR.
Figures 3A-H sets forth the sequence of a representative Eukaryotic Layered Vector Initiation System derived from Sindbis (see also SEQ. ID NO. 89).
Figure 4 is a schematic illustration of a Sindbis Basic Vector and a Sindbis-luciferase Vector.
Figure 5 is an illustration of Sindbis Helper Vector Construction.
Figure 6 is a graph which illustrates expression and rescue of a Sindbis-luciferase Vector.
Figure 7 is an illustration of one method for modifying a Sindbis junction region.
Figure 8 is a schematic illustration of Sindbis Packaging Expression Cassettes.

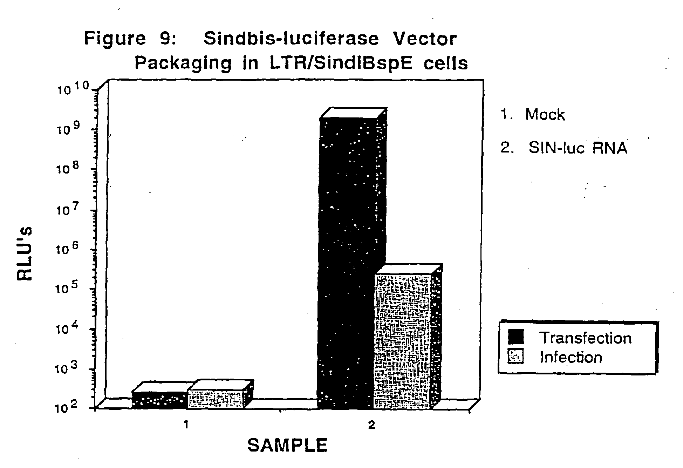

Figure 9 is a bar graph which illustrates Sindbis-luciferase Vector Packing in LTR/SindIBspE cells.

The 20 3' terminal bases of the first primary PCR amplicon product overlaps with the 20 5' terminal bases of the second primary PCR amplicon product; the resultant 2,742 bp overlapping secondary PCR amplicon product is purified by 0.8% agarose/TBE electrophoresis, digested with Bgl II, and the 2,734 bp product is ligated into pcDNASINbgl/xba (see Example 3) treated with Bgl II and CIAP. The resulting construction is 16,641 bps and is known as ELVIS-PySIN. In order to construct a structural protein expression vector similar to pLTR/Sind1Bsp for the derivation of vector packaging cell lines, the EL VIS-PySIN construction is digested to completion with Bsp EI, and religated under dilute conditions, in order to accomplish deletion of the nonstructural proteins between bases 422-7054. This construction is known as ELVIS-PySINdlBspE.

ELVIS-PySIN plasmid DNA is complexed with Lipofectamine (GIBCO-BRL, Gaithersbery, MD) according to the conditions suggested by the supplier (ca. 5 µg DNA/8 mg lipid reagent) and added to 35 mm wells containing undifferentiated PCC4 or F9 cells at approximately 75% confluency. The development of cytopathic effects (CPE), and the level of Sindbis productive infection, quantitated by plaque assay of media supernatant, is determined at regular intervals of 5 days in undifferentiated and differentiated PCC4 or F9 cells. Differentiation of F9 and PCC4 cells is accomplished by addition of retinoic acid (Sigma Chemical Co., St. Louis, Mo), at a final concentration of 1 µM.

If the undifferentiated EC cells demonstrate a heterologous response to transfection with EL VIS-PySIN, remaining cells not lysed by Sindbis virus propagation following G418 selection of pVGELVIS transfected undifferentiated EC cells are cloned and expanded. The cell clones are then tested for the production of Sindbis virus after differentiation, by addition of retinoic acid (Sigma Chemical Co., St. Louis, Mo), at a final concentration of 1 µM.

Isolation of vector packaging cell lines stably transfected with ELVIS-PySINd1BspE, having a cell differentiation state dependent pattern of expression of structural proteins in the presence of Sindbis NSP, is accomplished as described above for the pLTR/SindlBspE plasmid.

### 2. Use of cellular promoters.

The third example of this strategy uses the β-globin locus control region. The β-globin multigene cluster contains five developmentally regulated genes. In the early stages of human development, the embryonic yolk sac is the hematopoietic tissue and expresses the ε-globin gene. This is followed by a switch to the γ-globin gene in

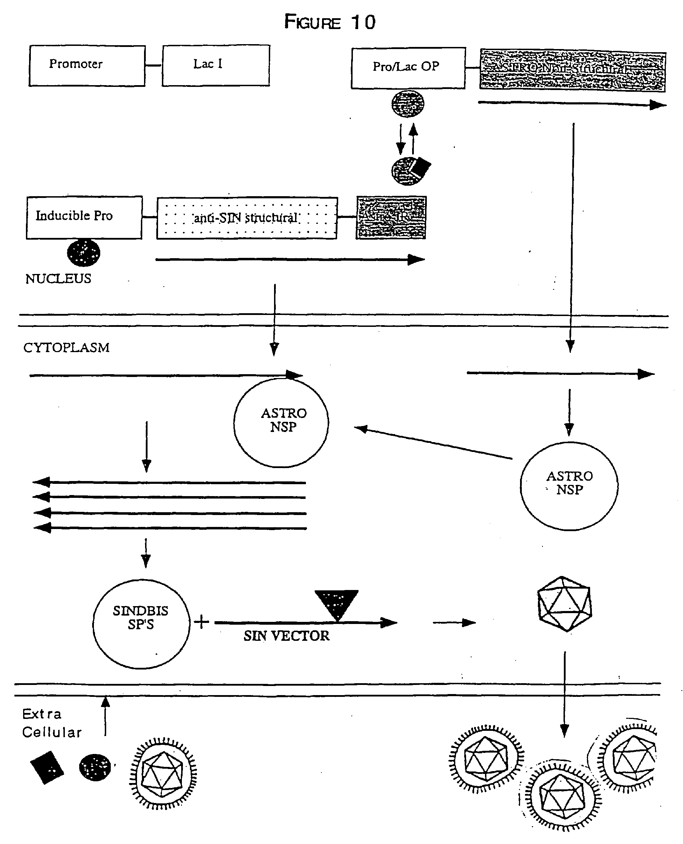

Figure 10 is a schematic illustration of how Astroviruses or other heterologous viruses may be used to express Sindbis structural proteins.

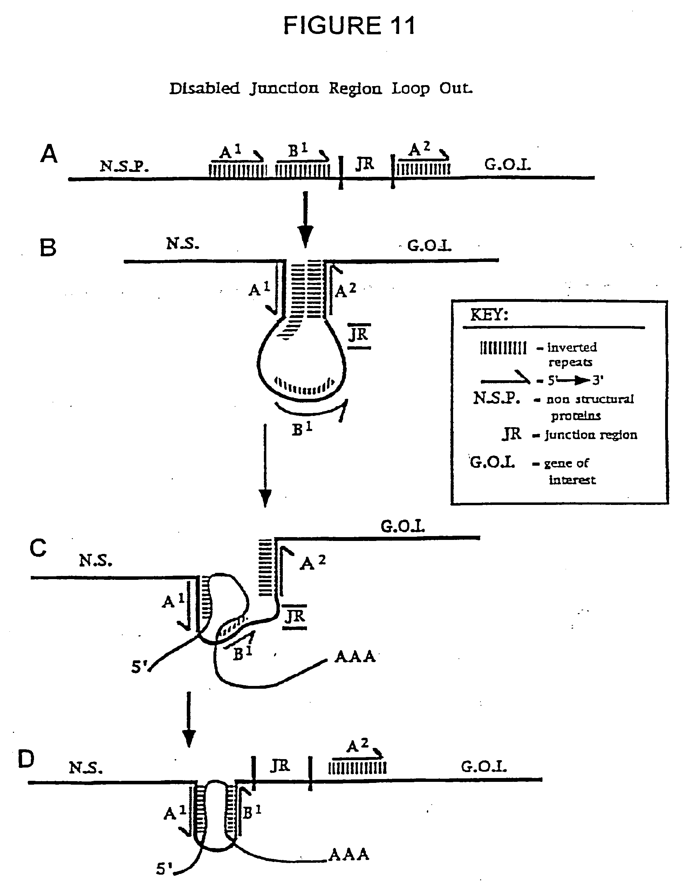

Figure 11 is a schematic illustration of the mechanism for activating a disabled viral junction region by "RNA loop-out."

### Detailed Description of the Invention

Prior to setting forth the invention, it may be helpful to an understanding thereof to first set forth definitions of certain terms that will be used hereinafter.

"Alphavirus vector construct" refers to an assembly which is capable of directing the expression of a sequence(s) or gene(s) of interest. The vector construct should include a 5' promoter which is capable of initiating the synthesis of viral RNA *in vitro* from cDNA, a 5' sequence which is capable of initiating transcription of an alphavirus, as well as sequence(s) which, when expressed, code for biologically active alphavirus non-structural proteins (*i.e.*, NSP1, NSP2, NSP3, and NSP4). In addition, the vector construct should include a viral junction region which may, in certain embodiments, be modified in order to prevent, inhibit or reduce viral transcription of the subgenomic fragment, and an alphavirus RNA polymerase recognition sequence. The vector construct may also include nucleic acid molecule(s) which are of a size sufficient to allow production of viable virus, as well as one or more restriction sites. When the alphavirus vector construct is a cDNA vector construct, it should additionally include a 5' promoter which is capable of initiating the synthesis of viral RNA from cDNA, and a 3' sequence which controls transcription termination and splice recognition.

"Expression cassette" refers to a recombinantly produced molecule which is capable of expressing alphavirus structural protein(s). The expression cassette must include a promoter and a sequence encoding alphavirus structural protein(s). Optionally, the expression cassette may include transcription termination, splice recognition, and polyadenylation addition sites. Preferred promoters include the CMV and adenovirus VAIRNA promoters. In addition, the expression cassette may contain selectable markers such as Neo, SV2 Neo, hygromycin, phleomycin, histidinol, and DHFR.

"Alphavirus particle" refers to a capsid which contains an alphavirus vector. A variety of vectors may be contained within the alphavirus particle, including the alphavirus vector constructs of the present invention. Preferably, the alphavirus capsid is contained within a lipid bilayer, such as a cell membrane, in which viral encoded proteins are embedded.

### A. SOURCES OF ALPHAVIRUS

As noted above, the present invention relates to alphavirus vector constructs, alphavirus particles containing such constructs, as well as methods for utilizing such vector constructs and particles. Briefly, sequences encoding wild-type alphavirus suitable for use in preparing the above-described vector constructs and particles may be readily obtained given the disclosure provided herein from naturally-occurring sources, or from depositories (*e.g.*, the American Type Culture Collection, Rockville, Maryland).

Representative examples of suitable alphaviruses include Aura (ATCC VR-368), Bebaru virus (ATCC VR-600, ATCC VR-1240), Cabassou (ATCC VR-922), Chikungunya virus (ATCC VR-64, ATCC VR-1241), Eastern equine encephalomyelitis virus (ATCC VR-65, ATCC VR-1242), Fort Morgan (ATCC VR-924), Getah virus (ATCC VR-369, ATCC VR-1243), Kyzylagach (ATCC VR-927), Mayaro (ATCC VR-66), Mayaro virus (ATCC VR-1277), Middleburg (ATCC VR-370), Mucambo virus (ATCC VR-580, ATCC VR-1244), Ndumu (ATCC VR-371), Pixuna virus (ATCC VR-372, ATCC VR-1245), Ross River virus (ATCC VR-373, ATCC VR-1246), Semliki Forest virus (ATCC VR-67, ATCC VR-1247), Sindbis virus (ATCC VR-68, ATCC VR-1248), Tonate (ATCC VR-925), Triniti (ATCC VR-469), Una (ATCC VR-374), Venezuelan equine encephalomyelitis (ATCC VR-69), Venezuelan equine encephalomyelitis virus (ATCC VR-923, ATCC VR-1250 ATCC VR-1249, ATCC VR-532), Western equine encephalomyelitis (ATCC VR-70, ATCC VR-1251, ATCC VR-622, ATCC VR-1252), Whataroa (ATCC VR-926), and Y-62-33 (ATCC VR-375).

### B. SEQUENCES WHICH ENCODE WILD-TYPE SINDBIS VIRUS

Within one particularly preferred aspect of the present disclosure, the sequences which encode wild-type alphavirus may be obtained from the Sindbis virus. In particular, within one embodiment of the disclosure (and as described in more detail below in Example 1), a Sindbis cDNA clone may be obtained by linking the 5' end of a Sindbis virus cDNA clone to a bacteriophage RNA polymerase promoter, and the 3' end of the cDNA clone to a poly-adenosine (poly A) tract of at least 25 nucleotides. In particular, synthesis of the first cDNA strand from the viral RNA template may be accomplished with a 3' oligonucleotide primer having a consecutive sequence comprising an enzyme recognition sequence, a sequence of 25 deoxythymidine nucleotides, and a stretch of approximately 18 nucleotides which is complementary to the viral 3' end and with a 5' primer containing buffer nucleotides, an enzyme recognition sequence, a bacteriophage promoter, and a sequence complimentary to the viral 5' end. The enzyme recognition sites present on each of these primers should be different from each other, and not found in the Sindbis virus. Further, the first nucleotide linked to the 3' end of the bacteriophage RNA polymerase promoter should be the authentic first nucleotide of the RNA virus. RNA transcribed *in vitro* from the viral cDNA clone, having the construction described above and linearized by digestion with the unique dT:dA 3' distal restriction enzyme will, after introduction into the appropriate eukaryotic cell, initiate the same infection cycle which is characteristic of infection by the wild-type virus from which the cDNA was cloned. This viral cDNA clone, which yields RNA able to initiate infection after *in vitro* transcription, is referred to below as an "infectious cDNA clone."

### C. PRODUCTION OF RECOMBINANT ALPHAVIRUS VECTOR CONSTRUCTS WITH INACTIVATED VIRAL JUNCTION REGIONS

An infectious cDNA clone prepared as described above (or utilizing sequences encoding an alphavirus obtained, from other sources) may be readily utilized to prepare alphavirus vector constructs as disclosed herein. Briefly, within one aspect of the present disclosure recombinant alphavirus vector constructs are provided, comprising a 5' sequence which is capable of initiating transcription of an alphavirus, a nucleotide sequence encoding alphavirus non-structural proteins, a viral junction region which has been inactivated such that viral transcription of the subgenomic fragment is prevented, and an alphavirus RNA polymerase recognition sequence. As will be discussed in greater detail below, alphavirus vector constructs which have inactivated viral junction regions do not transcribe the subgenomic fragment, making them suitable for a wide variety of applications.

### 1. RNA Polymerase Promoter

As noted above, within certain embodiments of the disclosure alphavirus vector constructs are disclosed which contain a 5' promoter which is capable of initiating the synthesis of viral RNA *in vitro* from cDNA. Particularly, preferred 5' promoters include RNA polymerase promoters such as T7, T3 and SP6.

### 2. Sequences Which Initiate Transcription

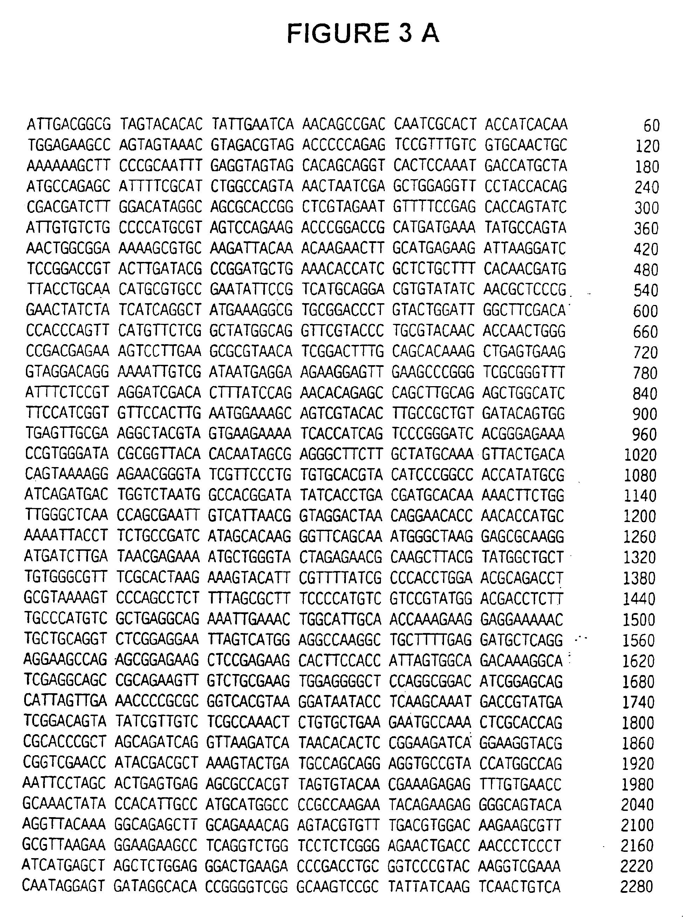

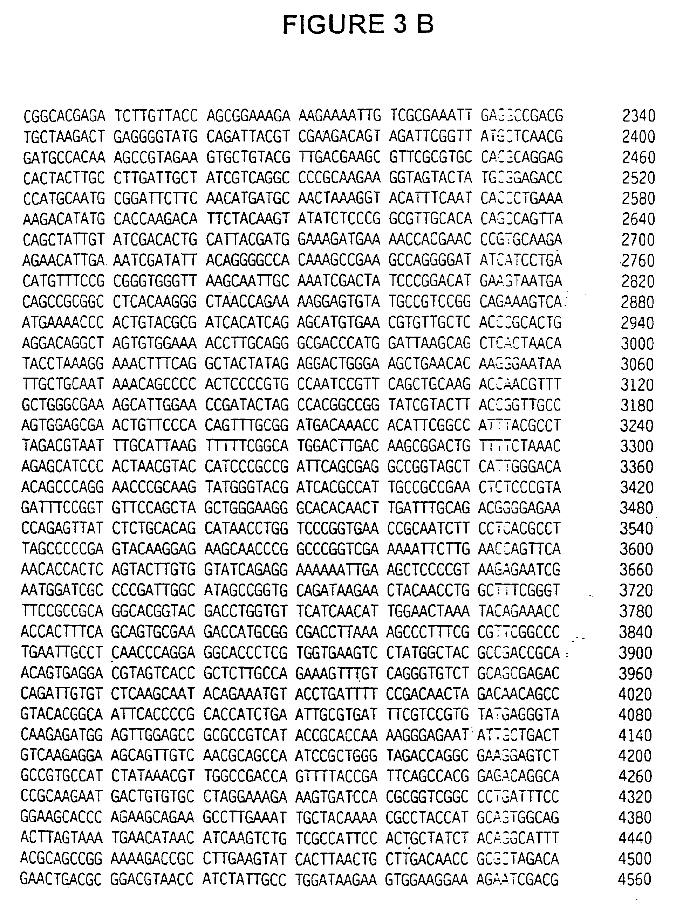

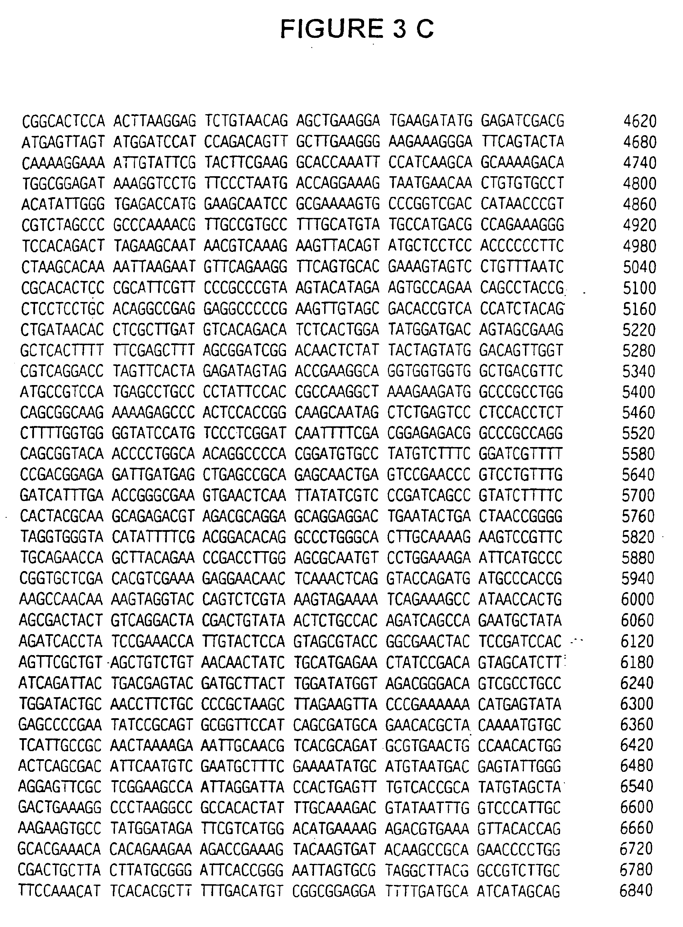

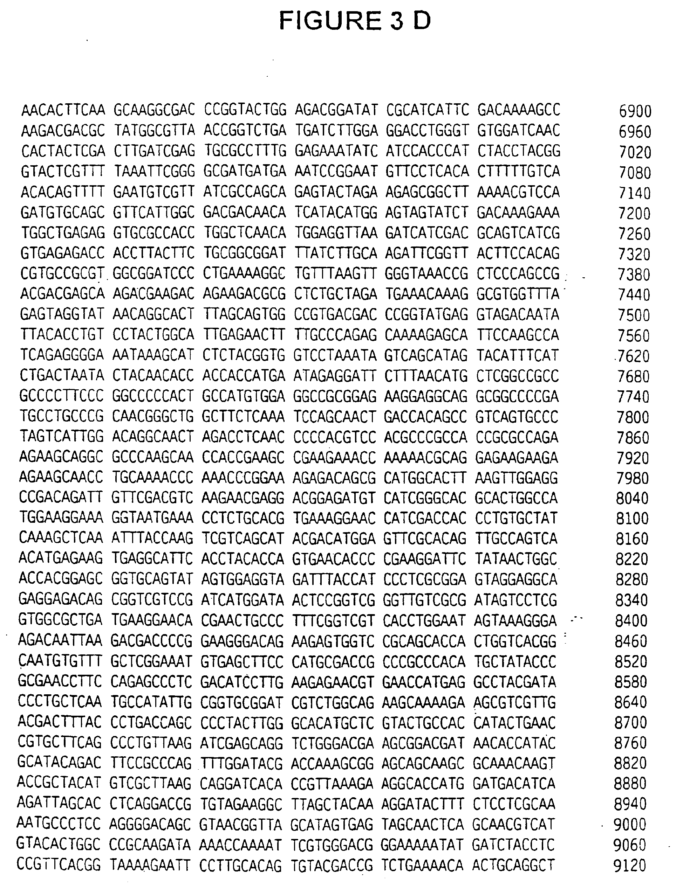

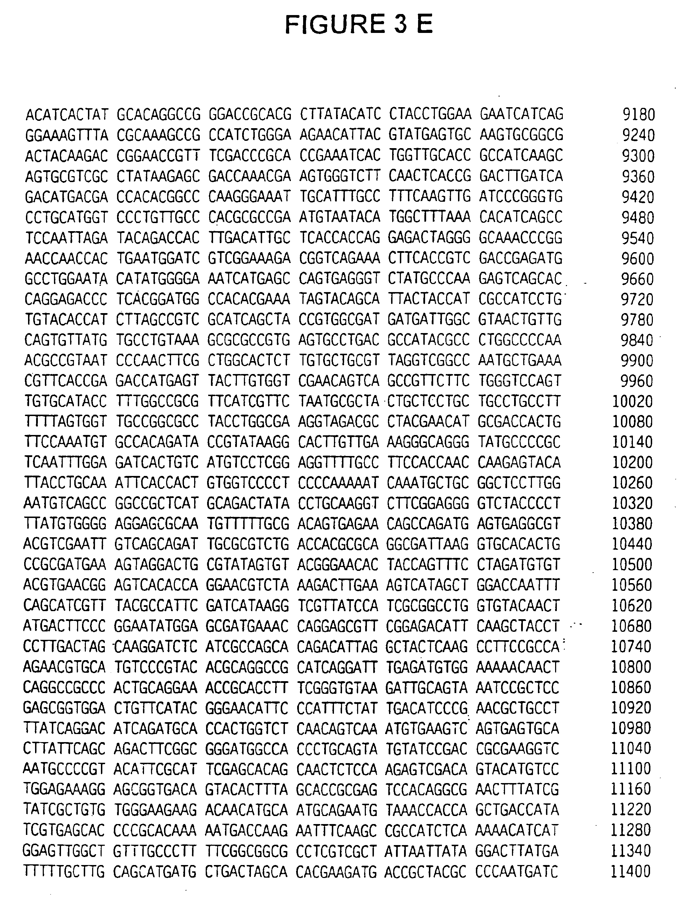

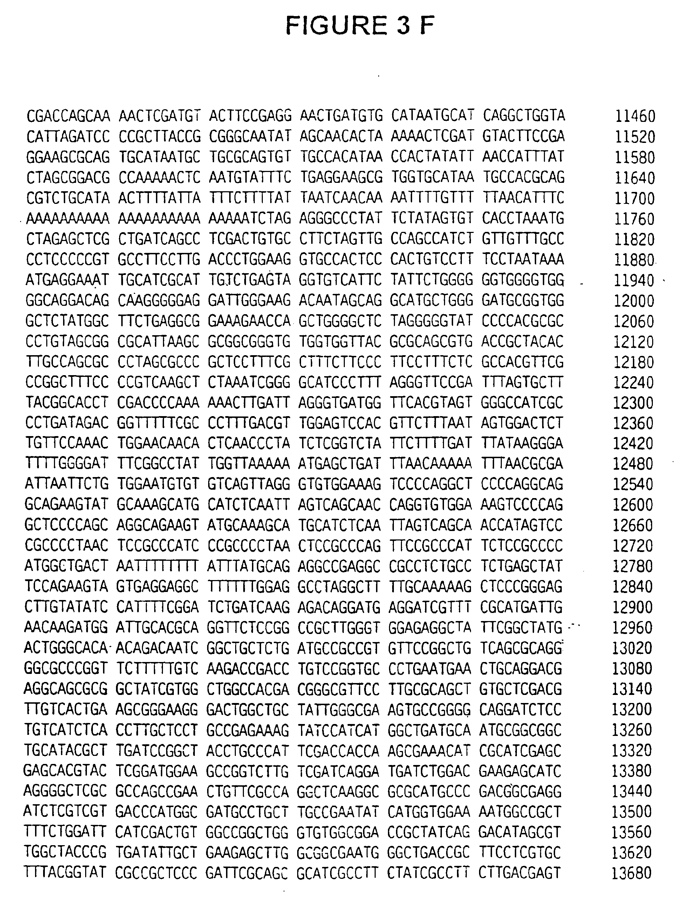

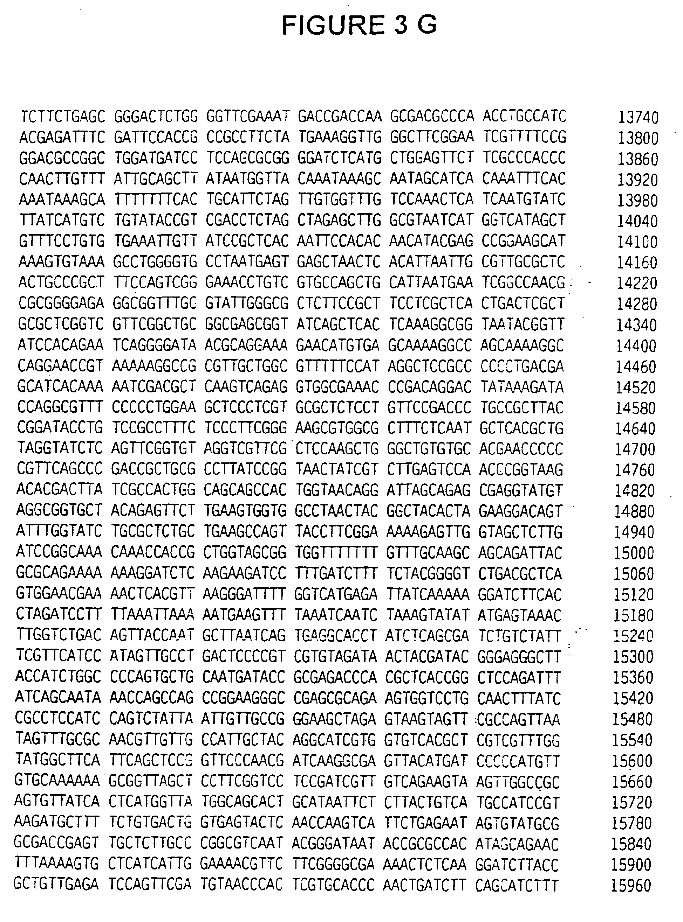

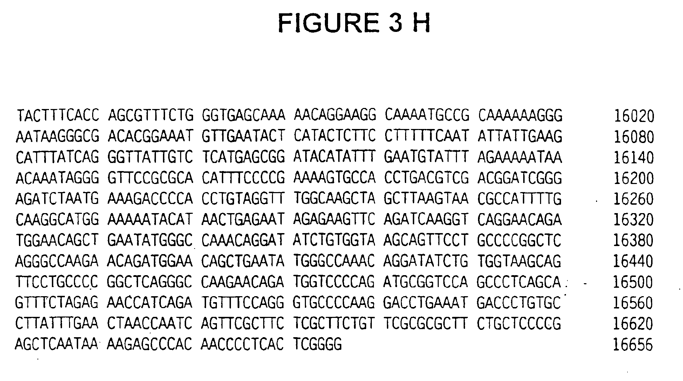

As noted above within preferred embodiments the alphavirus vector constructs of the present disclosure contain a 5' sequence which is capable of initiating transcription of an alphavirus. Representative examples of such sequences include nucleotides 1-60 of the wild-type Sindbis virus (*see* Figure 3), nucleotides 10-75 for tRNA Asparagine (Schlesinger et al., U.S. Patent No. 5,091,309), and 5' sequences from other Togaviruses which initiate transcription.

### 3. Alphavirus Non-Structural Proteins

Alphavirus vector constructs of the present disclosure should also contain sequences which encode Alphavirus Non-Structural Proteins (NSP). As an example, for the Sindbis virus there are four Sindbis non-structural proteins, NSP1, NSP2, NSP3 and NSP4, which encode proteins that enable the virus to self-replicate. Non-structural proteins 1 through 3 (NSP1-NSP3) are, within one embodiment of the disclosure encoded by nucleotides 60 to 5750 of the wild-type Sindbis virus (*see* Figure 3). These proteins are produced as a polyprotein and later cleaved into non-structural proteins NSP1, NSP2, and NSP3. NSP4 is, within one embodiment, encoded by nucleotides 5928 to 7579 (*see* Figure 3).

It will be evident to one of ordinary skill in the art that a wide variety of sequences which encode alphavirus non-structural proteins in addition to those discussed above may be utilized, and are therefore deemed to fall within the scope of the phrase "Alphavirus Non-Structural Proteins." For example, within one embodiment of the disclosure, due to the degeneracy of the genetic code, more than one codon may code for a given amino acid. Therefore, a wide variety of nucleic acid sequences which encode alphavirus non-structural proteins may be generated. Within other embodiments of the disclosure, a variety of other non-structural protein derivatives may be made, including for example, various substitutions, insertions, or deletions, the net result of which do not alter the biological activity of the alphavirus non-structural proteins. Within the context of the present disclosure alphavirus non-structural proteins are deemed to be "biologically active" *in toto* if they promote the self-replication of the vector construct. Self-replication, which refers to replication of viral nucleic acids and not the production of infectious virus, may be readily determined by RNase protection assays performed over a course of time. Methods for making such derivatives may be readily accomplished by one of ordinary skill in the art given the disclosure provided herein (*see also,* Molecular Cloning: A Laboratory Manual (2d. ed.), Cold Spring Harbor Laboratory Press).

### 4. Viral Junction Regions

Within this aspect of the disclosure, the alphavirus vector constructs also include a viral junction region which has been inactivated, such that viral transcription of the subgenomic fragment is prevented. Briefly, the alphavirus viral junction region normally controls transcription initiation of the subgenomic fragment. In the case of the Sindbis virus, the normal viral junction region typically begins at approximately nucleotide number 7579 and continues up through at least nucleotide number 7612 (and possibly beyond). At a minimum, nucleotides 7579 to 7602 (5'- ATC TCT ACG GTG GTC CTA AAT AGT - SEQ. ID NO. I) are believed necessary for transcription of the subgenomic fragment. This region (nucleotides 7579 to 7602) is hereinafter referred to as the "minimal junction region core."

Within preferred embodiments of the disclosure (and as described in more detail below), the viral junction region is inactivated in order to prevent viral transcription of the subgenomic fragment. As utilized within the context of the present disclosure, "inactivated" means that the fragment corresponding to the initiation point of the subgenomic fragment, as measured by a RNase protection assay, is not detected. (Representative assays are described by Melton et al., Nuc. Acids Res. 12:7035-7056, 1984; Calzon et al., Methods in Enz. 152:611-632, 1987; and Kekule et al., Nature 343:457-461, 1990.)

Within one embodiment of the disclosure, the viral junction region is inactivated by truncating the viral junction region at nucleotide 7597 (*i.e.*, the viral junction region will then consist of the sequence as shown in Figure 3, from nucleotide 7579 to nucleotide 7597). This truncation prevents transcription of the subgenomic fragment, and additionally permits synthesis of the complete NSP4 region (which is encoded by nucleotides 5928 to 7579).

As will be evident to one of ordinary skill in the art given the disclosure provided herein, a wide variety of other deletions, substitutions or insertions may also be made in order to inactivate the viral junction region. For example, within other embodiments of the disclosure the viral junction region may be further truncated into the region which encodes NSP4, thereby preventing viral transcription from the subgenomic fragment while retaining the biological activity of NSP4. Alternatively, within other embodiments, due to the redundancy of the genetic code, nucleotide substitutions may be made in the sequence encoding NSP4, the net effect of which does not alter the biological activity of NSP4 yet, nevertheless, prevents transcription of the subgenomic fragment.

### 5. Alphavirus RNA polymerase recognition sequence, and poly-A tail

As noted above, alphavirus vector constructs of the present disclosure should also include an alphavirus RNA polymerase recognition sequence (also termed "alphavirus replicase recognition sequence"). Briefly, the alphavirus RNA polymerase recognition sequence provides a recognition site at which the virus begins replication of the negative strand. A wide variety of sequences may be utilized as an alphavirus RNA polymerase recognition sequence. For example, within one embodiment, Sindbis vector constructs of the present disclosure include a Sindbis polymerase recognition sequence which is encoded by nucleotides 11,647 to 11,703 (*see* Figure 3). Within other embodiments, the Sindbis polymerase recognition is truncated to the smallest region which can still function as a recognition sequence (*e.g.*, nucleotides 11,684 to 11,703 of Figure 3).

Within preferred embodiments of the disclosure, the vector construct may additionally contain a poly-A tail. Briefly, the poly-A tail may be of any size which is sufficient to promote stability in the cytoplasm, thereby increasing the efficiency of initiating the viral life cycle. Within various embodiments of the disclosure, the poly-A tail comprises at least 10 adenosine nucleotides, and most preferably, at least 25 adenosine nucleotides.

### D. OTHER ALPHAVIRUS VECTOR CONSTRUCTS

In addition to the vector constructs which are generally described above, a wide variety of other alphavirus vector constructs may also be prepared utilizing the disclosure provided herein.

### 1. Modified Viral Junction Regions

Within one aspect of the present disclosure, alphavirus vector constructs are disclosure wherein the viral junction region has been modified, such that viral transcription of the subgenomic fragment is reduced. Briefly, infection of cells with wild-type alphavirus normally results in cell death as a result of abundant viral transcription of the subgenomic fragment initiated from the viral junction region. This large abundance of RNA molecules can overwhelm the transcriptional machinery of the infected cell, ultimately resulting in death of the cell. In applications where it is desired that infection of a target cell should result in a therapeutic effect (*e.g.*, strand scission of a target nucleic acid or prolonged expression of a heterologous protein) rather than cell death, several modifications to the alphavirus vector construct (in addition to inactivating the vector construct, as described above) may be made in order to reduce the level of viral transcription of the subgenomic fragment, and thereby prolong the life of the vector infected target cell. Within the context of the present disclosure, viral transcription of the subgenomic fragment is considered to be "reduced" if it produces less subgenomic fragment than a standard wild-type alphavirus (*e.g.*, Sindbis virus ATCC No. VR-1248) as determined by a RNase protection assay.

Viral junction regions may be modified by a variety of methods in order to reduce the level of viral transcription of the subgenomic fragment. For example, within one embodiment of the disclosure, due to the redundancy of the genetic code nucleotide substitutions may be made in the viral junction region 7579 to 7597, the net effect of which does not alter the amino acid sequence NSP4 (or, within other embodiments, the biological activity of NSP4), and yet reduces the level of viral transcription of the subgenomic fragment. If the modified vector construct includes nucleotides beyond 7597 (*e.g.*, to 7602 or 7612), further nucleotide substitutions may likewise be made, although, since NSP4 terminates at 7597, such substitutions need not be based upon genetic redundancy. Representative examples of modified viral junction regions are described in more detail below in Example 3.

### 2. Tandem Viral Junction Regions

Within other aspects of the disclosure, alphavirus vector constructs are disclosed, which comprise a 5' sequence which is capable of initiating transcription of an alphavirus, a nucleotide sequence encoding alphavirus non-structural proteins, a first viral junction region which has been inactivated such that viral transcription of the subgenomic fragment is prevented, a second viral junction region which has been modified such that viral transcription of the subgenomic fragment is reduced, and an alphavirus RNA polymerase recognition sequence. Such vector constructs are referred to as "tandem" vector constructs because they comprise a first inactivated (or "disabled") viral junction region, as well as a second modified (or "synthetic") viral junction region. Within preferred embodiments of the disclosure, the inactivated junction region is followed directly by the second modified viral junction region.

In applications where a low level of subgenomic transcription is required, a minimal junction region core may be inserted downstream in tandem to the inactivated junction region. In order to gradually increase the level of subgenomic transcription for the desired effect, sequences corresponding to the entire junction region may be added to the in-tandem junction region, in increments.

### 3. The Adenovirus E3 gene

Within another aspect of the disclosure, an adenovirus E3 gene is inserted into a tandem vector construct following the second viral junction region, in order to down-regulate HLA expression in alphavirus infected cells. Briefly, within various embodiments of the disclosure, repeated inoculations of a gene therapeutic into the same individual is desirable. However, repeated inoculations of alphaviruses such as the Sindbis virus may lead to the development of specific antibodies or cell-mediated immune response against Sindbis viral non-structural protein (NSP). Thus, it may be necessary to mitigate the host immune response targeted to vector specific proteins in order to administer repeated doses to the same individual.

Therefore, within one embodiment of the disclosure, products of the Adenovirus type 2 early region gene 3 are utilized in order to down-regulate the expression of integral histocompatibility antigens expressed on the surface of infected cells. Briefly, the E3 19,000 dalton (E3/19K) protein binds to, and forms a molecular complex with, class I H-2/HLA antigens in the endoplasmic reticulum, preventing terminal glycosylation pathways necessary for the full maturation and subsequent transport of the class I H-2/HLA antigens to the cell membrane. In target cells infected with an alphavirus vector encoding the Ad 2 E3 protein, co-expression of the viral non-structural proteins in the context of class I antigens will not occur. Thus, it is possible to administer repeated doses of an alphavirus vector which expresses the Ad 2 E3 protein as a component of its therapeutic palliative to the same individual. A representative example of the use of the Adenovirus E3 gene is set forth in more detail below in Example 4A.

### 4. The CMV H301 Gene

Other methods may also be utilized in order to mitigate a host's immune response against viral NSPs. For example, within another aspect of the disclosure, the Human Cytomegalovirus ("HCMV") H301 gene is cloned into an alphavirus vector construct, preferably immediately following the second viral junction region in a tandem vector, in order to inhibit host CTL response directed against viral specific proteins expressed in vector infected cells.

Briefly, β2-Microglobulin (β2m) protein binds to the α1, α2 and α3 domains of the a-chain of the class I major histocompatibility molecules of higher eukaryotes. Preventing the interaction between β2m and MHC class I products renders infected cells unrecognizable by cytotoxic T cells. Therefore, as described in greater detail below in Example 4B, expression of the HCMV H301 gene product as a component of a therapeutic palliative may be utilized in order to mitigate the host immune response to viral NSP.

### 5. Retroviral Packaging Sequence

Within another aspect of the disclosure, a retroviral packaging sequence is inserted into a tandem vector and positioned between the first (inactivated) viral junction region and the second, modified viral junction region. Briefly, retroviral packaging sequences signal the packaging of an RNA genome into a retroviral particle. As described in more detail below, a retroviral packaging sequence may be utilized in order to package an alphavirus vector into a retroviral particle using a retroviral packaging cell line. This is performed in order to increase the efficiency of alphavirus vector transfer into an alphavirus packaging cell line.

### 6. Expression of multiple heterologous genes

The genomic length and subgenomic length of mRNAs transcribed in wild-type alphavirus infected cells are polycistronic, coding for, respectively, the viral four non-structural proteins (NSPs) and four structural proteins (SPs). The genomic and subgenomic mRNAs are translated as polyproteins, and processing into the individual non-structural and structural proteins is accomplished by post translational proteolytic cleavage, catalyzed by viral encoded NSP- and SP- specific proteases.

In certain applications of the alphavirus vectors described herein, the expression of more than one heterologous gene is desired. For example, in order to treat metabolic disorders such as Gaucher's syndrome, multiple administrations of alphavirus vectors or particles may be required, since duration of the therapeutic palliative may be limited. Therefore, with certain embodiments of the disclosure it may be desirable to co-express in a target cell the Ad 2 E3 gene (*see* Example 4), along with a therapeutic palliative, such as the glucocerbrocidase gene (*see* Example 11). In wild-type virus, however, the structural protein ("SP") polycistronic message is translated into a single polyprotein which is subsequently processed into individual proteins by cleavage with SP encoded proteases. Thus, expression of multiple heterologous genes from a polycistronic message requires a mechanism different from the wild-type virus, since the SP protease gene, or the peptides recognized for cleavage, are not present in the replacement region of the alphavirus vectors.

Therefore, within one embodiment of the disclosure alphavirus vectors may be constructed by placing appropriate signals either ribosome readthrough or internal ribosome entry between cistrons. One such representative method of expressing multiple heterologous genes is set forth below in Example 5.

In yet another embodiment of the disclosure, the placement of signals promoting either ribosome readthrough or internal ribosome entry immediately downstream of the disabled junction region vector pKSSINBVdIJR is described (*see* Example 3). In this vector configuration, synthesis of subgenomic message cannot occur; however, the heterologous proteins are expressed from genomic length mRNA by either ribosomal readthrough (scanning) or internal ribosome entry. Relative to wild-type, the low level of viral transcription with this alphavirus vector would prolong the life of the infected target cell.

In still another embodiment of the disclosure, placement of signals promoting either ribosome readthrough or internal ribosome entry immediately downstream of the pKSSINBVdIJRsjr or pKSSINBV vectors is described. Briefly, since synthesis of subgenomic mRNA occurs in cells infected with the pKSSINBVdIJRsjr and pKSSINBV vectors, placement of either a ribosome readthrough sequence or an internal ribosome entry sequence between the two heterologous genes permits translation of both proteins encoded by the subgenomic mRNA polycistronic message. Further, additional heterologous genes can be placed in the subgenomic mRNA region, provided that a suitable translation initiation signal resides at the 5' end of the translational AUG start codon. The number of heterologous gene(s) which can be inserted into the subgenomic mRNA region, as described here, is limited only by the packaging constraints of the vector.

Different sequences which allow either ribosome readthrough, cap-independent translation, or internal ribosome entry may be placed into Sindbis vectors pKSSINBVdIR, pKSSINBV, or pKSSrNBVdIRsjrc in the configurations as discussed above. The source of these translation control sequences are the picornaviruses polio and EMCV, the 5' noncoding region of the human immunoglobulin heavy-chain binding protein, and a synthetic sequence of at least 15 bps corresponding in part to the Kozak consensus sequence for efficient translational initiation. Although not described in detail here, these signals which affect translation initiation can also be placed downstream of the junction region and between heterologous genes in all of the modified junction region vectors described in Example 3.

### 7. Alphavirus cDNA Vector Constructs

As noted above, there are disclosed herein alphavirus cDNA vector constructs. For example, within one aspect of the disclosure alphavirus cDNA vector constructs are disclosed which comprise a 5' promoter which is capable of initiating the synthesis of viral RNA from cDNA, followed by a 5' sequence which is capable of initiating transcription of an alphavirus, a nucleotide sequence encoding alphavirus non-structural proteins, a viral junction region which is either active or which has been inactivated such that viral transcription of the subgenomic fragment is prevented, an alphavirus RNA polymerase recognition sequence, and a 3' sequence which controls transcription termination. Within various embodiments, the viral junction region may be modified, such that viral transcription of the subgenomic fragment is merely reduced, rather than inactivated. Within other embodiments, a second viral junction region may be inserted following the first inactivated viral junction region, the second viral junction region being modified such that viral transcription of the subgenomic fragment is reduced.

Various aspects of the alphavirus cDNA vector constructs have been discussed above, including the 5' sequence which is capable of initiating transcription of an alphavirus, the nucleotide sequence encoding alphavirus non-structural proteins, the viral junction region which has been inactivated such that viral transcription of the subgenomic fragment is prevented, and the alphavirus RNA polymerase recognition sequence. In addition, modified junction regions and tandem junction regions have also been discussed above. Alphavirus cDNA vector constructs however, differ by the addition of a 5' promoter which is capable of initiating the synthesis of viral RNA from cDNA. Representative examples of suitable promoters include the *lac* promoter, metallathione promoter, CMV promoter and heat shock promoter.

As noted above, the alphavirus cDNA vector construct also includes a 3' sequence which controls transcription termination. A representative example of such a sequence is set forth in more detail below in Example 2.

### 8. Tissue specific expression

Within other, aspects of the present disclosure, alphavirus vector constructs are disclosed which are capable of expressing a desired heterologous sequence only in a selected tissue. One such representative example is shown in Figure 11. Briefly, as shown in Figure 11A a, recombinant Alphavirus vector is constructed such that upon introduction of the vector (Figure 11 A) into a target cell, internal inverted repeat sequences which flank the transcriptional control regions (*e.g.*, modified junction region) loop out (*see* Figure 11B), thereby preventing viral transcription of subgenomic sequences ("G.O.I.") from the synthetic junction region.

On the other hand, activation of the vector can be attained if the inverted repeats are designed to also hybridize to a specific cellular RNA sequence which is characteristic of a selected tissue or cell type. Such cellular RNA disrupts the disabling stem loop structure, thereby allowing the formation of a more stable secondary stem loop structure (Figures 11C and 11D). This secondary stem loop structure allows transcription of the sub-genomic message by placing the junction region back into its correct positional configuration.

Full-length alphavirus vectors can also be transcribed using the secondary stem loop structure by taking advantage of the ability of the viral polymerase to switch templates during synthesis of the negative strand using a strand hopping mechanism termed copy choice (King. RNA genetics II, CRC Press, Inc., Boca Raton Fla.. Domingo et al. (ed.), pp. 150-185, 1988). Once a single successful round of transcription has occurred, the resulting RNA transcript does not contain inverted repeats because they are deleted as a result of the polymerase copy choice event. This newly synthesized RNA molecule now functions as the primary RNA vector transcript which will transcribe and express as any other non-disabled genomic alphavirus vector previously described. In this RNA vector configuration, tissue or cell-specific activation of the disabled Sindbis vector can be achieved if specific RNA sequences, present only in the targeted cell or tissue types, are used in the design of the inverted repeats. In this fashion alphaviruses such as Sindbis can be engineered to be tissue-specific expression vectors using similar inverted sequences described above.

Using this vector system to achieve tissue specific expression enables a therapeutic alphavirus vector or particle to be delivered systemically into a patient. If the vector should infect a cell which does not express the appropriate RNA species, the vector will only be capable of expressing non-structural proteins and not the gene of interest. Eventually, the vector will be harmlessly degraded.

Use of the above-described vectors enables virtual tissue-specific expression possible for a variety of therapeutic applications, including for example, targeting vectors for the treatment for various types of cancers. This rationale relies on specific expression of tumor-specific markers such as the carcinoembryonic tumor specific antigen (CEA) and the alpha-fetoprotein tumor marker. Briefly, utilizing such tumor-specific RNA to target specific tumors allows for the tumor-specific expression of toxic molecules, lymphokines or pro-drugs discussed below. Such methods may be utilized for a wide variety of tumors, including for example, colorectal, lung, breast, ovary, bladder and prostate cancers because all these tumors express the CEA. One representative illustration of vectors suitable for use within this aspect of the present disclosure is set forth in more detail below in Example 15.

Briefly, CEA was one of the first tumor-specific markers to be described, along with the alpha-fetoprotein tumor marker. CEA is a normal glycoprotein in the embryonic tissue of the gut, pancreas and liver during the first two trimesters of fetal development (*Pathologic Basis of Disease,* 3rd edition 1984, Robbins et al. editor). Previously, CEA was believed to be specific for adenocarcinomas of the colon, however, with the subsequent development of more sensitive radioimmunoassays it became apparent that CEA was presented in the plasma with many endodermally derived cancers, particularly pancreatic, gastric and broncogenic.

Within related aspects of the present disclosure, alphavirus cell-specific expression vectors may be constructed to express viral antigens, ribozyme, antisense sequences or immunostimulatory factors such as gamma-interferon (γ-IFN) or IL-2 for the targeted treatment of virus infected cell types. In particular, in order to target alphavirus vectors to specific foreign organism or pathogen-infected cells, inverted repeats of the alphavirus vector may be selected to hybridize to any pathogen-specific RNA, for instance target cells infected by pathogens such as HIV, CMV, HBV, HPV and HSV.

Within yet other aspects of the disclosure, specific organ tissues may be targetted for the treatment of tissue-specific metabolic diseases utilizing gene replacement therapies. For example, the liver is an important target tissue because it is responsible for many of the body's metabolic functions and is associated with many metabolic genetic disorders. Such diseases include many of the glycogen storage diseases, phenylketonuria, Gaucher's disease and familial hypercholesterolemia. Presently there are many liver-specific enzymes and markers which have been sequenced which may be used to engineer appropriate inverted repeats for alphavirus vectors. Such liver-specific cDNAs include sequences encoding for S-adenosylmethione synthetase (Horikawa et al., Biochem. Int. 25:81, 1991); lecithin: cholesterolacyl transferase (Rogne et al., Biochem. Biophys. Res. Commun. 148:161, 1987); as well as other liver-specific cDNAs (Chin et al., Ann. N.Y. Acad. Sci. 478:120, 1986). Such a liver-specific alphavirus vector could be used to deliver the low density lipoprotein receptor (Yamamoto et al., Cell 39:27, 1984) to liver cells for the treatment of familial hypercholesterolemia (Wilson et al., Mol. Biol. Med. 7:223, 1990).

### E. HETEROLOGOUS SEQUENCE

As noted above, a wide variety of nucleotide sequences may be carried by the alphavirus vector constructs of the present disclosure. Preferably, the nucleotide sequences should be of a size sufficient to allow production of viable virus. Within the context of the present disclosure, the production of any measurable titer of infectious virus on susceptible monolayers is considered to be "production of viable virus." This may be, at a minimum, an alphavirus vector construct which does not contain any additional heterologous sequence. However, within other embodiments, the vector construct may contain additional heterologous or foreign sequences. Within preferred embodiments, the heterologous sequence will comprise a heterologous sequence of at least about 100 bases, 2 kb, 3.5 kb, 5 kb, 7 kb, or even a heterologous sequence of at least about 8 kb.

As will be evident to one of ordinary skill in the art given the disclosure provided herein, the efficiency of packaging and hence, viral titer, is to some degree dependent upon the size of the sequence to be packaged. Thus, in order to increase the efficiency of packaging and the production of viable virus, additional non-coding sequences may be added to the vector construct. Moreover, within certain embodiments of the disclosure it may be desired to increase or decrease viral titer. This increase or decrease may be accomplished by increasing or decreasing the size of the heterologous sequence, and hence the efficiency of packaging.

A wide variety of heterologous sequences may be included in the vector construct, including for example sequences which encode palliatives such as lymphokines, toxins, prodrugs, antigens which stimulate an immune response, ribozymes, and proteins which assist or inhibit an immune response, as well as antisense sequences (or sense sequences for "antisense applications"). As noted above, within various embodiments of the disclosure the alphavirus vector constructs disclosed herein may contain (and express, within certain embodiments) two or more heterologous sequences.

### 1. Lymphokines

Within one embodiment of the disclosure, the heterologous sequence encodes a lymphokine. Briefly, lymphokines act to proliferate, activate, or differentiate immune effectors cells. Representative examples of lymphokines include gamma interferon, tumor necrosis factor, IL-1, IL-2, IL-3, IL-4, IL-5, IL-6, IL-7, IL-8, IL-9, IL-10, IL-11, IL-12, IL-13, IL-14, IL-15, GM-CSF, CSF-1 and G-CSF.

Within related embodiments of the disclosure, the heterologous sequence encodes an immunomodulatory cofactor. Briefly, as utilized within the context of the present disclosure, "immunomodulatory cofactor" refers to factors which, when manufactured by one or more of the cells involved in an immune response, or when added exogenously to the cells, causes the immune response to be different in quality or potency from that which would have occurred in the absence of the cofactor. The quality or potency of a response may be measured by a variety of assays known to one of skill in the art including, for example, *in vitro* assays which measure cellular proliferation (*e.g.,* ³H thymidine uptake), and *in vitro* cytotoxic assays (*e.g.,* which measure ⁵¹Cr release) (*see* Warner et al., AIDS Res. and Human Retroviruses 7:645-655, 1991).

Representative examples of immunomodulatory co-factors include alpha interferon (Finter et al., Drugs 42(5):749-765, 1991; U.S. Patent No. 4,892,743; U.S. Patent No. 4,966,843; WO 85/02862; Nagata et al., Nature 284:316-320, 1980; Familletti et al., Methods in Enz. 78:387-394, 1981; Twu et al., Proc. Natl. Acad. Sci. USA 86:2046-2050, 1989; Faktor et al., Oncogene 5:867-872, 1990), beta interferon (Seif et al., J. Virol. 65:664-671, 1991), gamma interferons (Radford et al., American Society of Hepatology:2008.2015, 1991; Watanabe et al.. PNAS 86:9456-9460, 1989; Gansbacher et al., Cancer Research 50:7820-7825. 1990. Maio et al., Can. Immuno/. Immunother. 30:34-42, 1989; U.S. Patent Nos. 4,762,791 and 4,727,138), G-CSF (U.S. Patent Nos. 4,999,291 and 4,810,643), GM-CSF (WO 85/04188), TNFs (Jayaraman et al., J. Immunology 144:942-951, 1990), Interleukin- 2 (IL-2) (Karupiah et al., J.. Immunology 144:290-298, 1990; Weber et al., J Exp. Med 166:1716-1733, 1987; Gansbacher et al., J. Exp. Med. 172:1217-1224, 1990; U.S. Patent No. 4,738,927), IL-4 (Tepper et al., Cell 57:503-512, 1989; Golumbek et al., Science 254:713-716, 1991; U.S. Patent No. 5,017,691), IL-6 (Brakenhof et al., J. Immuno/. 139:4116-4121, 1987; WO 90/06370), IL-12, IL-15, ICAM-1 (Altman etal., Nature 338:512-514, 1989), ICAM-2, LFA-1, LFA-3, MHC class I molecules, MHC class II molecules, β ₂-microglobulin, chaperones, CD3, B7/BB1, MHC linked transporter proteins or analogs thereof.

The choice of which immunomodulatory cofactor to include within a alphavirus vector construct may be based upon known therapeutic effects of the cofactor, or experimentally determined. For example, in chronic hepatitis B infections alpha interferon has been found to be efficacious in compensating a patient's immunological deficit and thereby assisting recovery from the disease. Alternatively, a suitable immunomodulatory cofactor may be experimentally determined. Briefly, blood samples are first taken from patients with a hepatic disease. Peripheral blood lymphocytes (PBLs) are restimulated *in vitro* with autologous or HLA-matched cells (*e.g.*, EBV transformed cells), and transduced with an alphavirus vector construct which directs the expression of an immunogenic portion of a hepatitis antigen and the immunomodulatory cofactor. Stimulated PBLs are used as effectors in a CTL assay with the HLA-matched transduced cells as targets. An increase in CTL response over that seen in the same assay performed using HLA-matched stimulator and target cells transduced with a vector encoding the antigen alone, indicates a useful immunomodulatory cofactor. Within one embodiment of the disclosure, the immunomodulatory cofactor gamma interferon is particularly preferred.

Another example of an immunomodulatory cofactor is the B7/BB1 costimulatory factor. Briefly, activation of the full functional activity of T cells requires two signals. One signal is provided by interaction of the antigen-specific T cell receptor with peptides which are bound to major histocompatibility complex (MHC) molecules, and the second signal, referred to as costimulation, is delivered to the T cell by antigen-presenting cells. Briefly, the second signal is required for interleukin-2 (IL-2) production by T cells and appears to involve interaction of the B7/BB1 molecule on antigen-presenting cells with CD28 and CTLA-4 receptors on T lymphocytes (Linsley et al., J Exp. Med. 173:721-730. 1991a. and J. Exp Med., 174:561-570, 1991). Within one embodiment of the disclosure, B7/BB1 may be introduced into tumor cells in order to cause costimulation of CD8⁺ T cells, such that the CD8⁺ T cells produce enough IL-2 to expand and become fully activated. These CD8⁺ T cells can kill tumor cells that are not expressing B7 because costimulation is no longer required for further CTL function. Vectors that express both the costimulatory B7/BB1 factor and, for example, an immunogenic HBV core protein, may be made utilizing methods which are described herein. Cells transduced with these vectors will become more effective antigen-presenting cells. The HBV core-specific CTL response will be augmented from the fully activated CD8⁺ T cell via the costimulatory ligand B7/BB1.

### 2. Toxins

Within another embodiment of the disclosure, the heterologous sequence encodes a toxin. Briefly, toxins act to directly inhibit the growth of a cell. Representative examples of toxins include ricin (Lamb et al., Eur. J. Biochem. 148:265-270, 1985), abrin (Wood et al., Eur. J. Biochem. 198:723-732, 1991; Evensen et al., J. of Biol. Chem. 266:6848-6852, 1991; Collins et al., J. of Biol. Chem. 265:8665-8669, 1990; Chen et al., Fed. of Eur. Biochem Soc. 309:115-118, 1992), diphtheria toxin (Tweten et al., J. Biol. Chem. 260:10392-10394, 1985), cholera toxin (Mekalanos et al., Nature 306:551-557, 1983; Sanchez and Holmgren, PNAS 86:481-485, 1989), gelonin (Stirpe et al., J. Biol. Chem. 255:6947-6953, 1980), pokeweed (Irvin, Pharmac. Ther. 21:371-387, 1983), antiviral protein (Barbieri et al., Biochem. J. 203:55-59, 1982; Irvin et al., Arch. Biochem. & Biophys. 200:418-425, 1980; Irvin, Arch. Biochem. & Biophys. 169:522-528, 1975), tritin, Shigella toxin (Calderwood et al., PNAS 84:4364-4368, 1987; Jackson et al., Microb. Path. 2:147-153, 1987), Pseudomonas exotoxin A (Carroll and Collier, J. Biol. Chem. 262:8707-8711, 1987), herpes simplex virus thymidine kinase (HSVTK) (Field et al., J. Gen. Virol. 49:115-124, 1980), and *E. coli.* guanine phosphoribosyl transferase.

### 3. Pro-drugs

Within other embodiments of the disclosure, the heterologous sequence encodes a "pro-drugs". Briefly, as utilized within the context of the present disclosure, "pro-drugs" refers to a gene product that activates a compound with little or no cytotoxicity into a toxic product. Representative examples of such gene products include HSVTK and VZVTK, which selectively monophosphorylate certain purine arabinosides and substituted pyrimidine compounds, converting them to cytotoxic or cytostatic metabolites. More specifically, exposure of the drugs ganciclovir, acyclovir. or any of their analogues (*e.g.*. FIAU, FIAC, DHPG) to HSVTK phosphorylates the drug into its corresponding active nucleotide triphosphate form.

Representative examples of other pro-drugs which may be utilized within the context of the present disclosure include: *E. coli* guanine phosphoribosyl transferase which converts thioxanthine into toxic thioxanthine monophosphate (Besnard et al., Mol. Cell. Biol. 7:4139-4141, 1987); alkaline phosphatase, which will convert inactive phosphorylated compounds such as mitomycin phosphate and doxorubicin-phosphate to toxic dephosphorylated compounds; fungal (*e.g., Fusarium oxysporum*) or bacterial cytosine deaminase, which will convert 5-fluorocytosine to the toxic compound 5-fluorouracil (Mullen, PNAS 89:33, 1992); carboxypeptidase G2, which will cleave the glutamic acid from para-N-bis (2-chloroethyl) aminobenzoyl glutamic acid, thereby creating a toxic benzoic acid mustard; and Penicillin-V amidase, which will convert phenoxyacetabide derivatives of doxorubicin and melphalan to toxic compounds (*see* generally, Vrudhula et al., J. of Med. Chem. 36(7):919-923, 1993; Kern et al., Canc. Immun. Immunother. 31(4):202-206, 1990).

### 4. Antisense Sequences

Within another embodiment of the disclosure, the heterologous sequence is an antisense sequence. Briefly, antisense sequences are designed to bind to RNA transcripts, and thereby prevent cellular synthesis of a particular protein or prevent use of that RNA sequence by the cell. Representative examples of such sequences include antisense thymidine kinase, antisense dihydrofolate reductase (Maher and Dolnick, Arch. Biochem. & Biophys. 253:214-220, 1987; Bzik et al., PNAS 84:8360-8364, 1987), antisense HER2 (Coussens et al., Science 230:1132-1139, 1985), antisense ABL (Fainstein et al., Oncogene 4:1477-1481, 1989), antisense Myc (Stanton et al., Nature 310:423-425, 1984) and antisense *ras,* as well as antisense sequences which block any of the enzymes in the nucleotide biosynthetic pathway. In addition, within other embodiments of the disclosure antisense sequences to γ-interferon and β-2 microglobulin may be utilized in order to decrease immune response.

In addition, within a further embodiment of the disclosure, antisense RNA may be utilized as an anti-tumor agent in order to induce a potent Class I restricted response. Briefly, in addition to binding RNA and thereby preventing translation of a specific mRNA, high levels of specific antisense sequences are believed to induce the increased expression of interferons (including gamma-interferon) due to the formation of large quantities of double-stranded RNA. The increased expression of gamma interferon, in turn, boosts the expression of MHC Class I antigens. Preferred antisense sequences for use in this regard include actin RNA, myosin RNA. and histone RNA. Antisense RNA which forms a mismatch with actin RNA is particularly preferred.

### 5. Ribozymes

Within other aspects of the present disclosure, alphavirus vectors are disclosed which produce ribozymes upon infection of a host cell. Briefly, ribozymes are used to cleave specific RNAs and are designed such that it can only affect one specific RNA. Generally, the substrate binding sequence of a ribozyme is between 10 and 20 nucleotides long. The length of this sequence is sufficient to allow a hybridization with target RNA and disassociation of the ribozyme from the cleaved RNA. Representative examples for creating ribozymes include those described in U.S. Patent Nos. 5,116,742; 5,225,337 and 5,246,921. Particularly preferred ribozymes for use within the present disclosure include those disclosed in more detail below in the Examples (*e.g.*, Example 18).

### 6. Proteins and other cellular constituents

Within other aspects of the present disclosure, a wide variety of proteins or other cellular constituents may be carried by the alphavirus vector construct. Representative examples of such proteins include native or altered cellular components; as well as foreign proteins or cellular constituents, found in for example, viruses, bacteria, parasites or fungus.

### (a) Altered Cellular Components

Within one embodiment, alphavirus vector constructs are disclosed which direct the expression of an immunogenic, non-tumorigenic, altered cellular component. As utilized herein, the term "immunogenic" refers to altered cellular components which are capable, under the appropriate conditions, of causing an immune response. This response must be cell-mediated, and may also include a humoral response. The term "non-tumorigenic" refers to altered cellular components which will not cause cellular transformation or induce tumor formation in nude mice. The phrase "altered cellular component" refers to proteins and other cellular constituents which are either associated with rendering a cell tumorigenic, or are associated with tumorigenic cells in general, but are not required or essential for rendering the cell tumorigenic.

Before alteration, the cellular components may be essential to normal cell growth and regulation and include, for example, proteins which regulate intracellular protein degradation, transcriptional regulation, cell-cycle control, and cell-cell interaction. After alteration, the cellular components no longer perform their regulatory functions and, hence, the cell may experience uncontrolled growth. Representative examples of altered cellular components include ras*, p53*, Rb*, altered protein encoded by the Wilms' tumor gene, ubiquitin*, mucin*, protein encoded by the DCC, APC, and MCC genes, the breast cancer gene BRCA1*, as well as receptors or receptor-like structures such as neu, thyroid hormone receptor, platelet derived growth factor (PDGF) receptor, insulin receptor, epidermal growth factor (EGF) receptor, and the colony stimulating factor (CSF) receptor

Within one embodiment of the present disclosure, alphavirus vector constructs are disclosed which direct the expression of a non-tumorigenic, altered ras (ras*) gene. Briefly, the ras* gene is an attractive target because it is causally linked to the neoplastic phenotype, and indeed may be necessary for the induction and maintenance of tumorigenesis in a wide variety of distinct cancers, such as pancreatic carcinoma, colon carcinoma and lung adenocarcinoma. In addition, ras* genes are found in pre-neoplastic tumors and, therefore, immune intervention therapy may be applied prior to detection of a malignant tumor.

Normal ras genes are non-tumorigenic and ubiquitous in all mammals. They are highly conserved in evolution and appear to play an important role in maintenance of the cell cycle and normal growth properties. The normal ras protein is a G-protein which binds GTP and has GTPase activity, and is involved in transmitting signals from the external milieu to the inside of the cell, thereby allowing a cell to respond to its environment. Ras* genes on the other hand alter the normal growth regulation of neoplastic cells by uncoupling cellular behavior from the environment, thus leading to the uncontrolled proliferation of neoplastic cells. Mutation of the ras gene is believed to be an early event in carcinogenesis (Kumar et al., Science 248:1101-1104, 1990) which, if treated early, may prevent tumorigenesis.

Ras* genes occur in a wide variety of cancers, including for example, pancreatic, colon, and lung adenocarcinomas.

The spectrum of mutations occurring in the ras* genes found in a variety of cancers is quite limited. These mutations alter the GTPase activity of the ras protein by converting the normal on/off switch to a constitutive ON position. Tumorigenic mutations in ras* occur primarily (*in vivo*) in only 3 codons 12, 13 and 61. Codon 12 mutations are the most prevalent in both human and animal tumors.

Table 1 below summarizes known *in vivo* mutations (codons 12, 13 and 61) which activate human ras, as well as potential mutations which have *in vitro* transforming activity. Potential mutations with *in vitro* transforming activity were produced by the systematic substitution of amino acids for the normal codon (*e.g.*, other amino acids were substituted for the normal glycine at position 12). *In vitro* mutations, while not presently known to occur in humans or animals, may serve as the basis for an anti-cancer immunotherapeutic if they are eventually found to arise *in vivo.*

**TABLE 1**

| Amino Acid Substitutions that Activate Human ras Proteins | | | | | | | | | |
|---|---|---|---|---|---|---|---|---|---|
| Amino Acid | Gly | Gly | Ala | Gln | Glu | Asn | Lys | Asp | |
| Mutant Codon | 12 | 13 | 59 | 61 | 63 | 116 | 117 | 119 | |
| *In vivo* | | Val | Asp | | Arg | | | | |
| | | Arg | Val | | His | | | | |
| | | Asp | Arg | | Leu | | | | |
| | | Cys | | | | | | | |
| | | Ala | | | | | | | |
| | | Ser | | | | | | | |
| | | Phe | | | | | | | |
| *In vitro* | | Ala | Ser | Thr | Val | Lys | His | Glu | His |
| | | Asn | | | Ala | | Ile | Arg | Glu |
| | | Gln | | | Cys | | | | Ala |
| | | Glu | | | Asn | | | | Asn |
| | | His | | | Ile | | | | |
| | | Ile | | | Met | | | | |
| | | Leu | | | Thr | | | | |
| | | Lys | | | Tyr | | | | |
| | | Met | | | Trp | | | | |
| | | Phe | | | Phe | | | | |
| | | Ser | | | Gly | | | | |
| | | Thr | | | | | | | |
| | | Trp | | | | | | | |
| | | Tyr | | | | | | | |

Alterations as described above result in the production of proteins containing novel coding sequence(s). The novel proteins encoded by these sequence(s) may be used as a marker of tumorigenic cells, and an immune response directed against these novel coding regions may be utilized to destroy tumorigenic cells containing the altered sequences (ras*).

Within another embodiment of the present disclosure, alphavirus vector constructs are disclosed which direct the expression of an altered p53 (p53*) gene. Briefly, p53 is a nuclear phosphoprotein which was originally discovered in extracts of transformed cells and thus was initially classified as an oncogene (Linzer and Levine, Cell 17:43-52, 1979; Lane and Crawford, Nature 278:261-263, 1979). It was later discovered that the original p53 cDNA clones were mutant forms of p53 (Hinds et al., J. Virol. 63:739-746, 1989). It now appears that p53 is a tumor suppressor gene which negatively regulates the cell cycle, and that mutation of this gene may lead to tumor formation. Of colon carcinomas that have been studied, 75%-80% show a loss of both p53 alleles, one through deletion and the other through point mutation. Similar mutations are found in lung cancer, and in brain and breast tumors.

The majority of p53 mutations (*e.g.*, p53*¹, p53*², etc.) are clustered between amino acid residues 130 to 290 (*see* Levine et al., Nature 351:453-456, 1991; *see also* the following references which describe specific mutations in more detail: Baker et al., Science 244:217-221, 1989; Nigro et al., Nature 342:705-708, 1989 (p53 mutations cluster at four "hot spots" which coincide with the four highly conserved regions of the genes and these mutations are observed in human brain, breast, lung and colon tumors); Vogelstein, Nature 348:681-682, 1990; Takahashi et al., Science 246:491-494, 1989; Iggo et al., Lancet 335:675-679, 1990; James et al., Proc. Natl. Acad. Sci. USA 86:2858-2862, 1989; Mackay etal., Lancet 11:1384-1385,1988; Kelman et al., Blood 74:2318-2324, 1989; Malkin et al., Science 250:1233-1238, 1990; Baker et al., Cancer Res. 50:7717-7722, 1991; Chiba et al., Oncogene 5:1603-1610, 1990 (pathogenesis of early stage non-small cell lung cancer is associated with somatic mutations in the p53 gene between codons 132 to 283); Prosser et al., Oncogene 5:1573-1579, 1990 (mutations in the p53 gene coding for amino acids 126 through 224 were identified in primary breast cancer); Cheng and Hass, Mol. Cell. Biol. 10:5502-5509, 1990; Bartek et al.. Oncogene 5:893-899, 1990; Rodrigues et al., Proc. Natl. Acad. Sci. USA 87:7555-7559, 990; Menon et al., Proc. Natl. Acad. Sci. USA 87:5435-5439, 1990; Mulligan et al., Proc. Natl. Acad Sci. USA 87:5863-5867, 1990; and Romano et al.. Oncogene 4:1483-1488, 1990 (identification of a p53 mutation at codon 156 in human osteosarcoma derived cell line HOS-SL)).

Certain alterations of the p53 gene may be due to certain specific toxins. For example, Bressac et al. (Nature 350:429-431, 1991) describes specific G to T mutations in codon 249 in patients affected with hepatocellular carcinoma. One suggested causative agent of this mutation is aflatoxin B₁, a liver carcinogen which is known to be a food contaminant in Africa.

Four regions of the gene that are particularly affected occur at residues 132-145, 171-179, 239-248, and 272-286. Three "hot spots" which are found within these regions that are of particular interest occur at residues 175, 248 and 273 (Levine et al., Nature 351:453-456, 1991). These alterations, as well as others which are described above, result in the production of protein(s) which contain novel coding sequence(s). The novel proteins encoded by these sequences may be used as a marker of tumorigenic cells and an immune response directed against these novel coding regions may be utilized to destroy tumorigenic cells containing the altered sequence (p53*).

Once a sequence encoding the altered cellular component has been obtained, it is necessary to ensure that the sequence encodes a non-tumorigenic protein. Various assays are known and may easily be accomplished which assess the tumorigenicity of a particular cellular component. Representative assays include a rat fibroblast assay, tumor formation in nude mice or rats, colony formation in soft agar, and preparation of transgenic animals, such as transgenic mice.

Tumor formation in nude mice or rats is a particularly important and sensitive method for determining the tumorigenicity of a particular cellular component. Nude mice lack a functional cellular immune system (*i.e.,* do not possess CTLs), and therefore provide a useful *in vivo* model in which to test the tumorigenic potential of cells. Normal non-tumorigenic cells do not display uncontrolled growth properties if infected into nude mice. However, transformed cells will rapidly proliferate and generate tumors in nude mice. Briefly, in one embodiment the alphavirus vector construct is administered to syngeneic murine cells, followed by injection into nude mice. The mice are visually examined for a period of 2 to 8 weeks after injection in order to determine tumor growth. The mice may also be sacrificed and autopsied in order to determine whether tumors are present. (Giovanella et al., J. Natl. Cancer Inst. 48:1531-1533, 1972; Furesz et al., Abnormal Cells, New Products and Risk, Hopps and Petricciani (eds.). Tissue Culture Association, 1985; and Levenbook et al., J. Biol. Std. 13:135-141, 1985.)

Tumorigenicity may also be assessed by visualizing colony formation in soft agar (Macpherson and Montagnier, Vir. 23:291-294, 1964). Briefly, one property of normal non-tumorigenic cells is "contact inhibition" (*i.e.*, cells will stop proliferating when they touch neighboring cells). If cells are plated in a semi-solid agar support medium, normal cells rapidly become contact inhibited and stop proliferating, whereas tumorigenic cells will continue to proliferate and form colonies in soft agar.

Transgenic animals, such as transgenic mice, may also be utilized to assess the tumorigenicity of an altered cellular component. (Stewart et al., Cell 38:627-637, 1984; Quaife et al., Cell 48:1023-1034, 1987; and Koike et al., Proc. Natl. Acad. Sci. USA 86:5615-5619, 1989.) In transgenic animals, the gene of interest may be expressed in all tissues of the animal. This dysregulated expression of the transgene may serve as a model for the tumorigenic potential of the newly introduced gene.

If the altered cellular component is associated with making the cell tumorigenic, then it is necessary to make the altered cellular component non-tumorigenic. For example, within one embodiment the sequence or gene of interest which encodes the altered cellular component is truncated in order to render the gene product non-tumorigenic. The gene encoding the altered cellular component may be truncated to a variety of sizes, although it is preferable to retain as much as possible of the altered cellular component. In addition, it is necessary that any truncation leave intact at least some of the immunogenic sequences of the altered cellular component. Alternatively, multiple translational termination codons may be introduced downstream of the immunogenic region. Insertion of termination codons will prematurely terminate protein expression, thus preventing expression of the transforming portion of the protein.

Within one embodiment, the ras^{*} gene is truncated in order to render the ras^{*} protein non-tumorigenic. Briefly, the carboxy-terminal amino acids of ras^{*} functionally allow the protein to attach to the cell membrane. Truncation of these sequences renders the altered cellular component non-tumorigenic. Preferably, the ras^{*} gene is truncated in the purine ring binding site, for example around the sequence which encodes amino acid number 110. The ras^{*} gene sequence may be truncated such that as little as about 20 amino acids (including the altered amino acid(s)) are encoded by the alphavirus vector construct, although preferably, as many amino acids as possible should be expressed (while maintaining non-tumorigenicity).

Within another embodiment, the p53^{*} protein is modified by truncation in order to render the cellular component non-tumorigenic. As noted above, not all mutations of the p53-protein are tumorigenic, and therefore, not all mutations would have to be truncated. Nevertheless, within a preferred embodiment, p53^{*} is truncated to a sequence which encodes amino acids 100 to 300, thereby including all four major "hot spots."

Other altered cellular components which are oncogenic may also be truncated in order to render them non-tumorigenic. For example, both neu and bcr/abl may be truncated in order to render them non-tumorigenic. Non-tumorigenicity may be confirmed by assaying the truncated altered cellular component as described above.

It should be noted, however, that if the altered cellular component is only associated with non-tumorigenic cells in general, and is not required or essential for making the cell tumorigenic, then it is not necessary to render the cellular component non-tumorigenic. Representative examples of such altered cellular components which are not tumorigenic include Rb*, ubiquitin*, and mucin*.

As noted above, in order to generate an appropriate immune response, the altered cellular component must also be immunogenic. Immunogenicity of a particular sequence is often difficult to predict, although T cell epitopes often possess an immunogenic amphipathic alpha-helix component. In general, however, it is preferable to determine immunogenicity in an assay. Representative assays include an ELISA, which detects the presence of antibodies against the newly introduced vector, as well as assays which test for T helper cells such as gamma-interferon assays, IL-2 production assays, and proliferation assays.

As noted above, within another aspect of the present disclosure, several different altered cellular components may be co-expressed in order to form a general anti-cancer therapeutic. Generally, it will be evident to one of ordinary skill in the art that a variety of combinations can be made. Within preferred embodiments, this therapeutic may be targeted to a particular type of cancer. For example, nearly all colon cancers possess mutations in ras, p53, DCC APC or MCC genes. An alphavirus vector construct which co-expresses a number of these altered cellular components may be administered to a patient with colon cancer in order to treat all possible mutations. This methodology may also be utilized to treat other cancers. Thus, an alphavirus vector construct which co-expresses mucin*, ras*, neu, BRCA1* and p53* may be utilized to treat breast cancer.

### (b) Antigens from foreign organisms or other pathogens

Within other aspects of the present disclosure, alphavirus vector constructs are provided which direct the expression of immunogenic portions of antigens from foreign organisms or other pathogens. Representative examples of foreign antigens include bacterial antigens (*e.g., E. coli,* streptococcal, staphylococcal, mycobacterial. etc.), fungal antigens, parasitic antigens, and viral antigens (*e.g.*, influenza virus. Human Immmunodeficiency Virus ("HIV"), Hepatitis A, B and C Virus ("HAV". "HBV" and "HCV", respectively). Human Papiloma Virus ("HPV"), Epstein-Barr Virus ("EBV"). Herpes Simplex Virus ("HSV"), Hantavirus, TTLV I, HTLV II and Cytomegalovirus ("CMV"). As utilized within the context of the present disclosure. "immunogenic portion" refers to a portion of the respective antigen which is capable, under the appropriate conditions, of causing an immune response, *i.e.*, cell-mediated or humoral). "Portions" may be of variable size, but are preferably at least 9 amino acids long, and may include the entire antigen. Cell-mediated immune responses may be mediated through Major Histocompatability Complex ("MHC") class I presentation, MHC Class II presentation, or both.

Within one aspect of the disclosure, alphavirus vector constructs are provided which direct the expression of immunogenic portions of Hepatitis B antigens. Briefly, the Hepatitis B genome is comprised of circular DNA of about 3.2 kilobases in length and has been well characterized (Tiollais et al., Science 213:406-411, 1981; Tiollais et al., Nature 317:489-495, 1985; and Ganem and Varmus, Ann. Rev. Biochem. 56:651-693, 1987; *see also* EP 0 278,940, EP 0 241,021, WO 88/10301, and U.S. Patent Nos. 4,696,898 and 5,024,938). The Hepatitis B virus presents several different antigens, including among others, three HB "S" antigens (HBsAgs), an HBc antigen (HBcAg), an HBe antigen (HBeAg), and an HBx antigen (HBxAg) (*see* Blum et al., TIG 5(5):154-158, 1989). Briefly, the HBeAg results from proteolytic cleavage of P22 pre-core intermediate and is secreted from the cell. HBeAg is found in serum as a 17 kD protein. The HBcAg is a protein of 183 amino acids, and the HBxAg is a protein of 145 to 154 amino acids, depending on subtype.

The HBsAgs (designated "large," "middle" and "small") are encoded by three regions of the Hepatitis B genome: S, pre-S2 and pre-S1. The large protein, which has a length varying from 389 to 400 amino acids, is encoded by pre-S1, pre-S2, and S regions, and is found in glycosylated and non-glycosylated forms. The middle protein is 281 amino acids long and is encoded by the pre-S2 and S regions. The small protein is 226 amino acids long and is encoded by the S region. It exists in two forms, glycosylated (GP 27^{s}) arid non-glycosylated (P24^{s}). If each of these regions are expressed separately, the pre-S1 region will code for a protein of approximately 119 amino acids, the pre-S2 region will code for a protein of approximately 55 amino acids, and the S region will code for a protein of approximately 226 amino acids.

As will be evident to one of ordinary skill in the art, various immunogenic portions of the above-described S antigens may be combined in order to induce an immune response when administered by one of the alphavirus vector constructs described herein. In addition, due to the large immunological variability that is found in different geographic regions for the S open reading frame of HBV, particular combinations of antigens may be preferred for administration in particular geographic regions. Briefly, epitopes that are found in all human hepatitis B virus S samples are defined as determinant "*a*". Mutually exclusive subtype determinants, however, have also been identified by two-dimensional double immunodiffusion (Ouchterlony, Progr. Allergy 5:1, 1958). These determinants have been designated "*d*" or "*y*" and "*w*" or "*r*" (LeBouvier, J. Infect. 123:671, 1971; Bancroft et al., J. Immunol. 109:842, 1972; and Courouce et al., Bibl. Haematol. 42:1-158, 1976). The immunological variability is due to single nucleotide substitutions in two areas of the hepatitis B virus S open reading frame, resulting in the following amino acid changes: (1) exchange of lysine-122 to arginine in the Hepatitis B virus S open reading frame causes a subtype shift from *d* to *y,* and (2) exchange of arginine-160 to lysine causes the shift from subtype *r* to *w*. In Africans, subtype *ayw* is predominant, whereas in the U.S. and northern Europe the subtype *adw*₂ is more abundant (Molecular Biology of the Hepatitis B Virus, McLachlan (ed.), CRC Press, 1991). As will be evident to one of ordinary skill in the art, it is generally preferred to construct a vector for administration which is appropriate to the particular hepatitis B virus subtype which is prevalent in the geographical region of administration. Subtypes of a particular region may be determined by two-dimensional double immunodiffusion or, preferably, by sequencing the S open reading frame of HBV virus isolated from individuals within that region.

Also presented by HBV are *pol* ("HBV *pol"*), ORF 5, and ORF 6 antigens. Briefly, the polymerase open reading frame of HBV encodes reverse transcriptase activity found in virions and core-like particles in infected livers. The polymerase protein consists of at least two domains: the amino terminal domain which encodes the protein that primes reverse transcription, and the carboxyl terminal domain which encodes reverse transcriptase and RNase H activity. Immunogenic portions of HBV *pol* may be determined utilizing methods described herein (*e.g.,* below and in Examples 12Aii and 13), utilizing alphavirus vector constructs described below, and administered in order to generate an immune response within a warm-blooded animal. Similarly, other HBV antigens, such as ORF 5 and ORF 6 (Miller et al., Hepatology 9:322-327, 1989) may be expressed utilizing alphavirus vector constructs as described herein. Representative examples of alphavirus vector constructs utilizing ORF 5 and ORF 6 are set forth below in the examples.

As noted above, at least one immunogenic portion of a hepatitis B antigen is incorporated into an alphavirus vector construct. The immunogenic portion(s) which are incorporated into the alphavirus vector construct may be of varying length, although it is generally preferred that the portions be at least 9 amino acids long and may include the entire antigen. Immunogenicity of a particular sequence is often difficult to predict, although T cell epitopes may be predicted utilizing computer algorithms such as TSITES (MedImmune, Maryland), in order to scan coding regions for potential T-helper sites and CTL sites. From this analysis, peptides are synthesized and used as targets in an *in vitro* cytotoxic assay. Other assays, however, may also be utilized, including, for example, ELISA, which detects the presence of antibodies against the newly introduced vector, as well as assays which test for T helper cells, such as gamma-interferon assays, IL-2 production assays and proliferation assays.

Immunogenic portions may also be selected by other methods. For example, the HLA A2.1 transgenic mouse has been shown to be useful as a model for human T-cell recognition of viral antigens. Briefly, in the influenza and hepatitis B viral systems, the murine T cell receptor repertoire recognizes the same antigenic determinants recognized by human T cells. In both systems, the CTL response generated in the HLA A2.1 transgenic mouse is directed toward virtually the same epitope as those recognized by human CTLs of the HLA A2.1 haplotype (Vitiello et al., J Exp. Med 173:1007-1015, 1991; Vitiello et al., Abstract of Molecular Biology of Hepatitis B Virus Symposia, 1992).

Particularly preferred immunogenic portions for incorporation into alphavirus vector constructs include HBeAg, HBcAg and HBsAgs, as described in greater detail below in Example 10.

Additional immunogenic portions of the hepatitis B virus may be obtained by truncating the coding sequence at various locations including, for example, the following sites: Bst UI, Ssp I, Ppu M1, and Msp I (Valenzuela et al., Nature 280:815-19, 1979; Valenzuela et al., Animal Virus Genetics: ICN/UCLA Symp. Mol. Cell Biol., 1980, B. N. Fields and R. Jaenisch (eds.), pp. 57-70, New York: Academic). Further methods for determining suitable immunogenic portions as well as methods are also described below in the context of hepatitis C.

As noted above, more than one immunogenic portion may be incorporated into the alphavirus vector construct. For example, an alphavirus vector construct may express (either separately or as one construct) all or immunogenic portions of HBcAg, HBeAg, HBsAgs, HBxAg, as well as immunogenic portions of HCV antigens.

### 7. Sources for Heterologous Sequences

Sequences which encode the above-described proteins may be readily obtained from a variety of sources, including for example, depositories such as the American Type Culture Collection (ATCC, Rockville, Maryland), or from commercial sources such as British Bio-Technology Limited (Cowley, Oxford, England). Representative examples include BBG 12 (containing the GM-CSF gene coding for the mature protein of 127 amino acids); BBG 6 (which contains sequences encoding gamma interferon), ATCC No. 39656 (which contains sequences encoding TNF), ATCC No. 20663 (which contain sequences encoding alpha interferon), ATCC Nos. 31902, 31902 and 39517 (which contains sequences encoding beta interferon), ATCC No 67024 (which contain a sequence which encodes Interleukin-1b); ATCC Nos. 39405, 39452, 39516, 39626 and 39673 (which contains sequences encoding Interleukin-2); ATCC Nos. 59399, 59398, and 67326 (which contain sequences encoding Interleukin-3); ATCC No. 57592 (which contains sequences encoding Interleukin-4), ATCC Nos. 59394 and 59395 (which contain sequences encoding Interleukin-5), and ATCC No. 67153 (which contains sequences encoding Interleukin-6).

Sequences which encode altered cellular components as described above may be readily obtained from a variety of sources. For example, plasmids which contain sequences that encode altered cellular products may be obtained from a depository such as the American Type Culture Collection (ATCC, Rockville, Maryland), or from commercial sources such as Advanced Biotechnologies (Columbia, Maryland). Representative examples of plasmids containing some of the above-described sequences include ATCC No. 41000 (containing a G to T mutation in the 12th codon of ras), and ATCC No. 41049 (containing a G to A mutation in the 12th codon).

Alternatively, plasmids which encode normal cellular components may also be obtained from depositories such as the ATCC (*see,* for example, ATCC No. 41001, which contains a sequence which encodes the normal ras protein; ATCC No. 57103, which encodes abl; and ATCC Nos. 59120 or 59121, which encode the bcr locus) and mutated to form the altered cellular component. Methods for mutagenizing particular sites may readily be accomplished using methods known in the art (*see* Sambrook et al., *supra.,* 15.3 *et seq.*). In particular, point mutations of normal cellular components such as ras may readily be accomplished by site-directed mutagenesis of the particular codon, for example, codons 12, 13 or 61.

Sequences which encode the above-described viral antigens may likewise be obtained from a variety of sources. For example, molecularly cloned genomes which encode the hepatitis B virus may be obtained from sources such as the American Type Culture Collection (ATCC, Rockville, Maryland). For example, ATCC No. 45020 contains the total genomic DNA of hepatitis B (extracted from purified Dane particles) (*see* Figure 3 of Blum et al., TIG 5(5):154-158, 1989) in the Bam HI site of pBR322 (Moriarty et al., Proc. Natl. Acad Sci. USA 78:2606-2610, 1981).

Alternatively, cDNA sequences which encode the above-described heterologous sequences may be obtained from cells which express or contain the sequences. Briefly, within one embodiment, mRNA from a cell which expresses the gene of interest is reverse transcribed with reverse transcriptase using oligonucleotide dT or random primers. The single stranded cDNA may then be amplified by PCR (*see* U.S. Patent Nos. 4,683,202; 4,683,195 and 4,800,159. *See also* PCR Technology: Principles and Applications for DNA Amplification, Erlich (ed.), Stockton Press, 1989) utilizing oligonucleotide primers complementary to sequences on either side of desired sequences. In particular, a double-stranded DNA is denatured by heating in the presence of heat stable Taq polymerase, sequence-specific DNA primers, dATP, dCTP, dGTP and dTTP. Double-stranded DNA is produced when synthesis is complete. This cycle may be repeated many times, resulting in a factorial amplification of the desired DNA.

Sequences which encode the above-described proteins may also be synthesized, for example, on an Applied Biosystems Inc. DNA synthesizer (*e.g.*, APB DNA synthesizer model 392 (Foster City, CA)).

### F. EUKARYOTIC LAYERED VECTOR INITIATION SYSTEMS

As noted above, there are also disclosed herein eukaryotic layered vector initiation systems, which are comprised of a 5' promoter, a construct (*e.g.* , an alphavirus vector construct) which is capable of expressing a heterologous nucleotide sequences that is capable of replication in a cell either autonomously or in response to one or more factors, and a transcription termination sequence. Briefly, eukaryotic layered vector initiation systems provide a two-stage or "layered" mechanism which controls expression of heterologous nucleotide sequences. The first layer initiates transcription of the second layer, and comprises a 5' promoter, transcription termination site, as well as one or more splice sites and a polyadenylation site, if desired. Representative examples of promoters suitable for use in this regard include any viral or cellular promoters such as CMV, retroviral LTRs, SV40, β-actin, immunoglobulin promoters, and inducible promoters such as the metallothionein promoter and glucocorticoid promoter. The second layer comprises a construct which is capable of expressing one or more heterologous nucleotide sequences, and of replication in a cell either autonomously or in response to one or more factors. Within one embodiment of the disclosure, the construct may be a Sindbis cDNA vector construct as described above.

A wide variety of other cDNA and DNA vector constructs may also be utilized in eukaryotic layered vector initiation systems including, for example, viral vector constructs developed from poliovirus (Evans et al., Nature 339:385-388, 1989; and Sabin, J. Biol. Standardization 1:115-118, 1973); rhinovirus; pox viruses, such as canary pox virus or vaccinia virus (Fisher-Hoch et al., PNAS 86:317-321, 1989; Flexner et al., Ann. N. Y. Acad. Sci. 569:86-103, 1989; Flexner et al., Vaccine 8:17-21, 1990; U.S. Patent Nos. 4,603,112, 4,769,330 and 5,017,487; WO 89/01973); SV40 (Mulligan et al., Nature 277:108-114, 1979); retrovirus (U.S. Patent No. 4,777,127, GB 2,200,651, EP 0,345,242 and WO91/02805); influenza virus (Luytjes et al., Cell 59:1107-1113, 1989; McMicheal et al., N. Eng. J. Med. 309:13-17, 1983; and Yap et al., Nature 273:238-239, 1978); adenovirus (Berkner, Biotechniques 6:616-627, 1988; Rosenfeld et al., Science 252:431-434, 1991); parvovirus such as adeno-associated virus (Samulski et al., J Vir. 63:3822-3828, 1989; Mendelson et al., Virol. 166:154-165, 1988; PA 7/222,684); herpes (Kit, Adv. Exp. Med. Biol. 215:219-236, 1989); SV40; HIV (Poznansky, *J. Virol. 65*:532-536, 1991); measles (EP 0,440,219); astrovirus (Munroe et al., J. Vir. 67:3611-3614, 1993); Semliki Forest Virus, and coronavirus, as well as other viral systems (*e.g.*, EP 0,440,219; WO 92/06693; U.S. Patent No. 5,166,057).

As noted above, within other embodiments of the disclosure eukaryotic layered vector initiation systems are disclosed comprising a 5' sequence which is capable of initiating transcription *in vitro* of an alphavirus at the authentic 5' end, a 5' sequence which is capable of initiating transcription of an alphavirus virus, a nucleotide sequence encoding alphavirus non-structural proteins, a viral junction region, a heterologous nucleotide sequence, an alphavirus RNA polymerase recognition sequence, and a polyadenylate sequence. Following *in vitro* transcription of an alphavirus cDNA vector construct, the resulting alphavirus RNA vector molecule is comprised of a 5' sequence which is capable of initiating transcription of an alphavirus, a nucleotide sequence encoding alphavirus non-structural proteins, a viral junction region, a heterologous nucleotide sequence, an alphavirus, RNA polymerase recognition sequence, and a polyadenylate sequence.

Within other aspects of the present disclosure, methods are disclosed for delivering a heterologous nucleotide sequence to a warm blooded animal, comprising the step of administering a eukaryotic layered vector initiation system to a warm-blooded animal. Eukaryotic layered vector initiation systems may be administered to warm-blooded animals either directly (*e.g.*, intravenously, intramuscularly, intraperitoneally, subcutaneously, orally, rectally, intraocularly, intranasally), or by various physical methods such as lipofection (Felgner et al.. Proc. Natl. Acad. Sci. USA 84:7413-7417, 1989), direct DNA injection (Acsadi et al.. Nature 352:815-818. 1991); microprojectile bombardment (Williams et al., PNAS 88:2726-2730, 1991); liposomes of several types (*see, e.g..* Wang et al., PNAS 84:7851-7855, 1987); CaPO₄ (Dubensky et al., PNAS 81:7529-7533, 1984); DNA ligand (Wu et al, J. of Biol. Chem. 264:16985-16987, 1989); administration of nucleic acids alone (WO 90/11092); or administration of DNA linked to killed adenovirus (Curiel et al., Hum. Gene Ther. 3:147-154, 1992); polycation compounds such as polylysine, receptor specific ligands; as well as psoralen inactivated viruses such as Sendai or Adenovirus.

Eukaryotic layered vector initiation systems may also be administered to a warm-blooded animal for the purposes of stimulating a specific immune response; inhibiting the interaction of an agent with a host cell receptor; to express a toxic palliative, including for example, conditional toxic palliatives; to immunologically regulate an immune system: to express markers, and for replacement gene therapy. These and other uses are discussed in more detail below.

### G. SINDBIS PACKAGING CELL LINES

Within further embodiments of the disclosure, alphavirus packing cell lines are disclosed. In particular, within one aspect of the present disclosure, alphavirus packaging cell lines are disclosed wherein viral structural proteins are supplied in trans from a stably integrated expression vector. Subsequent transfection or infection of alphavirus vector RNA transcripts creates an alphavirus vector-producing cell line. For example, within one embodiment of the disclosure, alphavirus RNA vector molecules are initially produced using a T6 *in vitro* RNA polymerase system to transcribe from a cDNA clone encoding the gene of interest and the alphavirus non-structural proteins. The vector RNA may then be transfected into an alphavirus packaging cell line, where vector RNA transcripts replicate to high levels, and are subsequently packaged by the viral structural proteins, yielding infectious viral particles. By nature of the extended length of the alphavirus cDNA molecule, the *in vitro* transcription process is inefficient. In addition, only 1% - 10% of the cells contained on a petri plate can be successfully transfected. Consequently, to optimize vector producer cell line performance and titer, two successive cycles of gene transfer may be performed. To accomplish this, the vector may be first transfected into a primary alphavirus packaging cell line. This transfected cell line then produces low titers of infectious viral particles in the culture supernatants. These infectious supernatants are then used to transduce a fresh monolayer of alphavirus packaging cells. Infection of the alphavirus vector into the packaging cell line is preferred over transfection because of its higher RNA transfer efficiency into cells and optimized biological placement of the vector in the cell. This two-step approach leads to higher expression and higher titer of packaged infectious recombinant Sindbis vector.

Within certain embodiments of the disclosure, alphavirus particles may fail to transduce the same packaging cell line because the cell line produces increased cellular envelope proteins which block cellular receptors for alphavirus vector attachment. In such cases, a second type of alphavirus viral particle may be created which is able to infect the alphavirus packaging cells. This second type of viral particle must be produced by a packaging cell line known as a "hopping cell line," which produces transient vector particles as the result of being transfected with *in vitro* transcribed alphavirus RNA vector transcripts. This hopping cell line is engineered to redirect the envelope tropism of the transiently produced vector particle by providing alternative viral envelope proteins which redirect alphavirus vectors to different cellular receptors in a process termed pseudo-typing. Currently, two approaches for alphavirus vector particle pseudo-typing have been devised. The first approach consists of an alphavirus packaging cell line (described above) which co-expresses the vesicular stomatitis virus-G protein (VSV-G). The VSV-G pseudo-typing has been demonstrated to infect a wide variety of cell types (Marsh, Adv. Virus Res. 36:107-151, 1989). The second approach of producing a pseudo-typed alphavirus vector particle is to utilize a retroviral packaging cell lines (*e.g.,* WO 92/05266) containing retroviral *gag*/*pol* and *env* sequences which are capable of packaging an alphavirus RNA vector containing a retroviral packaging sequence.

Within other aspects of the present disclosure, stably integrated alphavirus DNA expression vectors are used to produce the alphavirus vector RNA molecule which maintains the ability to self-replicate. This approach may prove useful to maintain high levels of expression over long periods of culturing because the integrated DNA vector will constitutively express unaltered RNA vectors. In this configuration, the vectors previously transcribed from a plasmid containing the SP6 RNA polymerase recognition site are replaced with the appropriate promoter sequence defined by the parent cell line used. This plasmid sequence may also contain a selectable marker different from those used to create the packaging cell line. In this configuration, the DNA-based alphavirus vectors are introduced by transfection into the packaging cell line, as previously described, followed by dilution cloning to find the highest titre producing cell lines.

### 1. Suicide Vector

One further aspect of the present disclosure relates to the expression of alphavirus suicide vectors to limit the spread of wild-type alphavirus in the packaging/producer cell lines. Briefly, within one embodiment the alphavirus suicide vector would be comprised of an antisense or ribozyme sequence, specific for the wild-type alphavirus sequence generated from an RNA recombination event between the 3' sequences of the junction region of the vector, and the 5' alphavirus structural sequences of the packaging cell line expression vector. The antisense or riboryme molecule would only be thermostable in the presence of the specific recombination sequence and would not have any other effect in the alphavirus packaging/producer cell line. Alternatively, a toxic molecule (such as those disclosed below), may also be expressed in the context of a vector that would only express in the presence of wild-type alphavirus.

### 2. Alphavirus Vectors to Prevent the Spread of Metastatic Tumors

One further aspect of the present disclosure relates to the use of alphavirus vectors for inhibiting or reducing the invasiveness of malignant neoplasms. Briefly, the extent of malignancy typically relates to vascularization of the tumor. One cause for tumor vascularization is the production of soluble tumor angiogenesis factors (TAF) (Paweletz et al., Crit. Rev. Oncol. Hematol 9:197, 1989) expressed by some tumors. Within one aspect of the present disclosure, tumor vascularization may be slowed by using alphavirus vectors to express antisense or ribozyme RNA molecules specific for TAF. Alternatively, anti-angiogenesis factors (Moses et al., Science 248:1408, 1990; Shapiro et al., PNAS 84:2238, 1987) may be expressed either alone or in combination with the above-described ribozymes or antisense sequences in order to slow or inhibit tumor vascularization. Alternatively, alphavirus vectors can also be used to express an antibody specific for the TAF receptors on surrounding tissues.

### H. METHODS FOR UTILIZING ALPHAVIRUS VECTORS

### 1. Immunostimulation

Within other aspects of the present disclosure, compositions and methods are provided for administering an alphavirus vector construct which is capable of preventing, inhibiting, stabilizing or reversing infectious, cancerous, auto-immune or immune diseases. Representative examples of such diseases include viral infections such as HIV, HBV HTLV I, HTLV II, CMV, EBV and HPV, melanomas, diabetes, graft vs. host disease, Alzheimer's disease and heart disease.

More specifically, within one aspect of the present disclosure, compositions and methods are provided for stimulating an immune response (either humoral or cell-mediated) to a pathogenic agent, such that the pathogenic agent is either killed or inhibited. Representative examples of pathogenic agents include bacteria, fungi, parasites, viruses and cancer cells.

Within one embodiment of the disclosure the pathogenic agent is a virus, and methods are disclosed for stimulating a specific immune response and inhibiting viral spread by using recombinant alphavirus viral particles designed to deliver a vector construct that directs the expression of an antigen or modified form thereof to susceptible target cells capable of either (I) initiating an immune response to the viral antigen or (2) preventing the viral spread by occupying cellular receptors required for viral interactions. Expression of the vector nucleic acid encoded protein may be transient or stable with time. Where an immune response is to be stimulated to a pathogenic antigen, the recombinant alphavirus is preferably designed to express a modified form of the antigen which will stimulate an immune response and which has reduced pathogenicity relative to the native antigen. This immune response is achieved when cells present antigens in the correct manner, *i.e*., in the context of the MHC class I and/or II molecules along with accessory molecules such as CD3, ICAM-1, ICAM-2, LFA-1, or analogs thereof (e.g., Altmann et al., Nature 338:512, 1989). Cells infected with alphavirus vectors are expected to do this efficiently because they closely mimic genuine viral infection and because they: (a) are able to infect non-replicating cells, (b) do not integrate into the host cell genome, (c) are not associated with any life threatening diseases, and (d) express high levels of heterologous protein. Because of these differences, alphavirus vectors can easily be thought of as safe viral vectors which can be used on healthy individuals for vaccine use.

This aspect of the disclosure has a further advantage over other systems that might be expected to function in a similar manner, in that the presenter cells are fully viable and healthy and low levels of viral antigens, relative to heterologous genes, are expressed. This presents a distinct advantage since the antigenic epitopes expressed can be altered by selective cloning of sub-fragments of the gene for the antigen into the recombinant alphavirus, leading to responses against immunogenic epitopes which may otherwise be overshadowed by immunodominant epitopes. Such an approach may be extended to the expression of a peptide having multiple epitopes, one or more of the epitopes being derived from different proteins. Further, this aspect of the disclosure allows efficient stimulation of cytotoxic T lymphocytes (CTL) directed against antigenic epitopes, and peptide fragments of antigens encoded by sub-fragments of genes, through intracellular synthesis and association of these peptide fragments with MHC Class I molecules. This approach may be utilized to map major immunodominant epitopes for CTL induction. An immune response may also be achieved by transferring to an appropriate immune cell (such as a T lymphocyte) the gene for the specific T cell receptor which recognizes the antigen of interest (in the context of an appropriate MHC molecule if necessary), for an immunoglobulin which recognizes the antigen of interest, or for a hybrid of the two which provides a CTL response in the absence of the MHC context: Thus, the recombinant alphavirus infected cells may be used as an immunostimulant, immunomodulator, or vaccine.

In another embodiment of the disclosure, methods are disclosed for producing inhibitor palliatives wherein alphavirus vectors deliver and express defective interfering viral structural proteins, which inhibit viral assembly. Such vectors may encode defective *gag, pol, env* or other viral particle proteins or peptides and these would inhibit in a dominant fashion the assembly of viral particles. This occurs because the interaction of normal subunits of the viral particle is disturbed by interaction with the defective subunits.

In another embodiment of the disclosure, methods are disclosed for the expression of inhibiting peptides or proteins specific for viral protease. Briefly, viral protease cleaves the viral *gag* and *gag*/*pol* proteins into a number of smaller peptides. Failure of this cleavage in all cases leads to complete inhibition of production of infectious retroviral particles. As an example, the HIV protease is known to be an aspartyl protease and these are known to be inhibited by peptides made from amino acids from protein or analogues. Vectors to inhibit HIV will express one or multiple fused copies of such peptide inhibitors.

Another embodiment involves the delivery of suppressor genes which, when deleted, mutated, or not expressed in a cell type, lead to tumorigenesis in that cell type. Reintroduction of the deleted gene by means of a viral vector leads to regression of the tumor phenotype in these cells. Examples of such cancers are retinoblastoma and Wilms Tumor. Since malignancy can be considered to be an inhibition of cellular terminal differentiation compared with cell growth, the alphavirus vector delivery and expression of gene products which lead to differentiation of a tumor should also, in general, lead to regression.

In yet another embodiment, the alphavirus vector provides a therapeutic effect by encoding a ribozyme (an RNA enzyme) (Haseloff and Gerlach, Nature 334:585, 1989) which will cleave and hence inactivate RNA molecules corresponding to a pathogenic function. Since ribozymes function by recognizing a specific sequence in the target RNA and this sequence is normally 12 to 17 bp, this allows specific recognition of a particular RNA species such as a RNA or a retroviral genome. Additional specificity may be achieved in some cases by making this a conditional toxic palliative (*see* below).

One way of increasing the effectiveness of inhibitory palliatives is to express viral inhibitory genes in conjunction with the expression of genes which increase the probability of infection of the resistant cell by the virus in question. The result is a nonproductive "dead-end" event which would compete for productive infection events. In the specific case of HIV, vectors may be delivered which inhibit HIV replication (by expressing anti-sense tat, etc., as described above) and also overexpress proteins required for infection, such as CD4. In this way, a relatively small number of vector-infected HIV-resistant cells act as a "sink" or "magnet" for multiple nonproductive fusion events with free virus or virally infected cells.

### 2. Blocking Agents

Many infectious diseases, cancers, autoimmune diseases, and other diseases involve the interaction of viral particles with cells, cells with cells, or cells with factors. In viral infections, viruses commonly enter cells via receptors on the surface of susceptible cells. In cancers, cells may respond inappropriately or not at all to signals from other cells or factors. In autoimmune disease, there is inappropriate recognition of "self markers. Within the present disclosure, such interactions may be blocked by producing, *in vivo,* an analogue to either of the partners in an interaction.

This blocking action may occur intracellularly, on the cell membrane, or extracellularly. The blocking action of a viral or, in particular, an alphavirus vector carrying a gene for a blocking agent. can be mediated either from inside a susceptible cell or by secreting a version of, the blocking protein to locally block the pathogenic interaction.

In the case of HIV, the two agents of interaction are the gp 120/gp 41 envelope protein and the CD4 receptor molecule. Thus, an appropriate blocker would be a vector construct expressing either an HIV *env* analogue that blocks HIV entry without causing pathogenic effects, or a CD4 receptor analogue. The CD4 analogue would be secreted and would function to protect neighboring cells, while the gp 120/gp 41 is secreted or produced only intracellularly so as to protect only the vector-containing cell. It may be advantageous to add human immunoglobulin heavy chains or other components to CD4 in order to enhance stability or complement lysis. Delivery of an alphavirus vector encoding such a hybrid-soluble CD4 to a host results in a continuous supply of a stable hybrid molecule. Efficacy of treatment can be assayed by measuring the usual indicators of disease progression, including antibody level, viral antigen production, infectious HIV levels, or levels of nonspecific infections.

### 3. Expression of Palliatives

Techniques similar to those described above can be used to produce recombinant alphavirus with vector constructs which direct the expression of an agent (or "palliative") which is capable of inhibiting a function of a pathogenic agent or gene. Within the present disclosure, "capable of inhibiting a function" means that the palliative either directly inhibits the function or indirectly does so, for example, by converting an agent present in the cells from one which would not normally inhibit a function of the pathogenic agent to one which does. Examples of such functions for viral diseases include adsorption, replication, gene expression, assembly, and exit of the virus from infected cells. Examples of such functions for a cancerous cell or cancer-promoting growth factor include viability, cell replication, altered susceptibility to external signals (*e.g*., contact inhibition), and lack of production or production of mutated forms of anti-oncogene proteins.

### (a) Inhibitor Palliatives

In one aspect of the present disclosure, the alphavirus vector construct directs the expression of a gene which can interfere with a function of a pathogenic agent, for instance in viral or malignant diseases. Such expression may either be essentially continuous or in response to the presence in the cell of another agent associated either with the pathogenic condition or with a specific cell type (an "identifying agent"). In addition, vector delivery may be controlled by targeting vector entry specifically to the desired cell type (for instance, a virally infected or malignant cell) as discussed above.

One method of administration is leukophoresis, in which about 20% of an individual's PBLs are removed at any one time and manipulated *in vitro.* Thus, approximately 2 x 10⁹ cells may be treated and replaced. Repeat treatments may also be performed. Alternatively, bone marrow may be treated and allowed to amplify the effect as described above. In addition, packaging cell lines producing a vector may be directly injected into a subject, allowing continuous production of recombinant virions.

In one embodiment, alphavirus vectors which express RNA complementary to key pathogenic gene transcripts (for example, a viral gene product or an activated cellular oncogene) can be used to inhibit translation of that transcript into protein, such as the inhibition of translation of the HIV tat protein. Since expression of this protein is essential for viral replication, cells containing the vector would be resistant to HIV replication.

In a second embodiment, where the pathogenic agent is a single-stranded virus having a packaging signal, RNA complementary to the viral packaging signal *(e.g.,* an HIV packaging signal when the palliative is directed against HIV) is expressed, so that the association of these molecules with the viral packaging signal will, in the case of retroviruses, inhibit stem loop formation or tRNA primer binding required for proper encapsidation or replication of the alphavirus RNA genome.

In a third embodiment, an alphavirus vector may be introduced which expresses a palliative capable of selectively inhibiting the expression of a pathogenic gene, or a palliative capable of inhibiting the activity of a protein produced by the pathogenic agent. In the case of HIV, one example is a mutant tat protein which lacks the ability to transactivate expression from the HIV LTR and interferes (in a transdominant manner) with the normal functioning of tat protein. Such a mutant has been identified for HTLV II tat protein ("XII Leu^{5"} mutant; *see* Wachsman et al., Science 235:674, 1987). A mutant transrepressor tat should inhibit replication much as has been shown for an analogous mutant repressor in HSV-1 (Friedmann et al., Nature 335:452, 1988).

Such a transcriptional repressor protein may be selected for in tissue culture using any viral-specific transcriptional promoter whose expression is stimulated by a virus-specific transactivating protein (as described above). In the specific case of HIV, a cell line expressing HIV tat protein and the HSVTK gene driven by the HIV promoter will die in the presence of ACV. However, if a series of mutated tat genes are introduced to the system, a mutant with the appropriate properties (*i.e*., represses transcription from the HIV promoter in the presence of wild-type tat) will grow and be selected. The mutant gene can then be reisolated from these cells. A cell line containing multiple copies of the conditionally lethal vector/tat system may be used to assure that surviving cell clones are not caused by endogenous mutations in these genes. A battery of randomly mutagenized tat genes are then introduced into these cells using a "rescuable" alphavirus vector (*i.e*., one that expresses the mutant tat protein and contains a bacterial origin of replication and drug resistance marker for growth and selection in bacteria). This allows a large number of random mutations to be evaluated and permits facile subsequent molecular cloning of the desired mutant cell line. This procedure may be used to identify and utilize mutations in a variety of viral transcriptional activator/viral promoter systems for potential antiviral therapies.

### 4. Conditional Toxic Palliatives

Another approach for inhibiting a pathogenic agent is to express a palliative which is toxic for the cell expressing the pathogenic condition. In this case, expression of the palliative from the vector should be limited by the presence of an entity associated with the pathogenic agent, such as a specific viral RNA sequence identifying the pathogenic state, in order to avoid destruction of nonpathogenic cells.

In one embodiment of this method, a recombinant alphavirus vector carries a vector construct containing a toxic gene (as discussed above) expressed from a cell-specific responsive vector. In this manner, rapidly replicating cells, which contain the RNA sequences capable of activating the cell-specific responsive vectors, are preferentially destroyed by the cytotoxic agent produced by the alphavirus vector construct.

In a similar manner to the preceding embodiment, the alphavirus vector construct can carry a gene for phosphorylation, phosphoribosylation, ribosylation, or other metabolism of a purine- or pyrimidine-based drug. This gene may have no equivalent in mammalian cells and might come from organisms such as a virus, bacterium, fungus, or protozoan. An example of this would be the *E. coli* guanine phosphoribosyl transferase gene product, which is lethal in the presence of thioxanthine (*see* Besnard et al., Mol. Cell. Biol. 7:4139-4141, 1987). Conditionally lethal gene products of this type (also referred to as "pro-drugs" above) have application to many presently known purine- or pyrimidine-based anticancer drugs, which often require intracellular ribosylation or phosphorylation in order to become effective cytotoxic agents. The conditionally lethal gene product could also metabolize a nontoxic drug which is not a purine or pyrimidine analogue to a cytotoxic form (*see* Searle et al., Brit. J Cancer 53:377-384, 1986).

Mammalian viruses in general tend to have "immediate early" genes which are necessary for subsequent transcriptional activation from other viral promoter elements. RNA sequences of this nature are excellent candidates for activating alphavirus vectors intracellular signals (or "identifying agents") of viral infection. Thus, conditionally lethal genes expressed from alphavirus cell-specific vectors responsive to these viral "immediate early" gene products could specifically kill cells infected with any particular virus. Additionally, since the human a and β interferon promoter elements are transcriptionally activated in response to infection by a wide variety of nonrelated viruses, the introduction of vectors expressing a conditionally lethal gene product like HSVTK, for example, in response to interferon production could result in the destruction of cells infected with a variety of different viruses.

In another aspect of the present disclosure, the recombinant alphavirus viral vector carries a vector construct that directs the expression of a gene product capable of activating an otherwise inactive precursor into an active inhibitor of the pathogenic agent. For example, the HSVTK gene product may be used to more effectively metabolize potentially antiviral nucleoside analogues such as AZT or ddC. The HSVTK gene may be expressed under the control of a cell-specific responsive vector and introduced into these cell types. AZT (and other nucleoside antivirals) must be metabolized by cellular mechanisms to the nucleotide triphosphate form in order to specifically inhibit retroviral reverse transcriptase, and thus, HIV replication (Furmam et al., Proc. Natl. Acad Sci. USA 83:8333-8337, 1986). Constitutive expression of HSVTK (a nucleoside and nucleoside kinase with very broad substrate specificity) results in more effective metabolism of these drugs to their biologically active nucleotide triphosphate form. AZT or ddC therapy will thereby be more effective, allowing lower doses, less generalized toxicity, and higher potency against productive infection. Additional nucleoside analogues whose nucleotide triphosphate forms show selectivity for retroviral reverse transcriptase but, as a result of the substrate specificity of cellular nucleoside and nucleotide kinases are not phosphorylated, will be made more efficacious.

Administration of these alphavirus vectors to human T cell and macrophage/monocyte cell lines can increase their resistance to HIV in the presence of AZT and ddC compared to the same cells without retroviral vector treatment. Treatment with AZT would be at lower than normal levels to avoid toxic side effects but still efficiently inhibit the spread of HIV. The course of treatment would be as described for the blocker.

In one embodiment, the recombinant alphavirus vector carries a gene specifying a product which is not in itself toxic but, when processed or modified by a protein such as a protease specific to a viral or other pathogen, is converted into a toxic form. For example, the recombinant alphavirus could carry a gene encoding a proprotein for ricin A chain, which becomes toxic upon processing by the HIV protease. More specifically, a synthetic inactive proprotein form of the toxin ricin or diphtheria A chains could be cleaved to the active form by arranging for the HIV virally encoded protease to recognize and cleave off an appropriate "pro" element.

In another embodiment, the alphavirus construct may express a "reporting product" on the surface of the target cells in response to the presence of an identifying agent in the cells (such as expression of a viral gene). This surface protein can be recognized by a cytotoxic agent, such as antibodies for the reporting protein, or by cytotoxic T cells. In a similar manner, such a system can be used as a detection system (*see* below) to simply identify those cells having a particular gene which expresses an identifying protein.

Similarly, in another embodiment, a surface protein could be expressed which would itself be therapeutically beneficial. In the particular case of HIV, expression of the human CD4 protein specifically in HIV-infected cells may be beneficial in two ways:
1. Binding of CD4 to HIV *env* intracellularly could inhibit the formation of viable viral particles, much as soluble CD4 has been shown to do for free virus, but without the problem of systematic clearance and possible immunogenicity, since the protein will remain membrane bound and is structurally identical to endogenous CD4 (to which the patient should be immunologically tolerant).
2. Since the CD4/HIV *env* complex has been implicated as a cause of cell death, additional expression of CD4 (in the presence of excess HIV-env present in HIV-infected cells) leads to more rapid cell death and thus inhibits viral dissemination. This may be particularly applicable to monocytes and macrophages, which act as a reservoir for virus production as a result of their relative refractility to HIV-induced cytotoxicity (which, in turn, is apparently due to the relative lack of CD4 on their cell surfaces).

In another embodiment, the alphavirus vector codes for a ribozyme which will cleave and inactivate RNA molecules essential for viability of the vector infected cell. By making ribozyme production dependent on a specific RNA sequence corresponding to the pathogenic state, such as HIV tat, toxicity is specific to the pathogenic state.

### 5. Expression of Markers

The above-described technique of expressing a palliative in a cell in response to a specific RNA sequence can also be modified to enable detection of a particular gene in a cell which expresses an identifying protein (for example, a gene carried by a particular virus), and hence enable detection of cells carrying that virus. In addition, this technique enables the detection of viruses (such as HIV) in a clinical sample of cells carrying an identifying protein associated with the virus.

This modification can be accomplished by providing a genome coding for a product, the presence of which can be readily identified (the "marker product"), in an alphavirus vector which responds to the presence of the identifying protein in the infected cells. For example, HIV, when it infects suitable cells, makes tat and rev. The indicator cells can thus be provided with a genome (such as by infection with an appropriate recombinant alphavirus) which codes for a marker gene, such as the alkaline phosphatase gene, β-galactosidase gene, or the luciferase gene which is expressed by the recombinant alphavirus upon activation by the tat and/or rev RNA transcript. In the case of β-galactosidase or alkaline phosphatase, exposing the cells to substrate analogues results in a color or fluorescence change if the sample is positive for HIV. In the case of luciferase, exposing the sample to luciferin will result in luminescence if the sample is positive for HIV. For intracellular enzymes such as β-galactosidase, the viral titre can be measured directly by counting colored or fluorescent cells, or by making cell extracts and performing a suitable assay. For the membrane bond form of alkaline phosphatase, virus titre can also be measured by performing enzyme assays on the cell surface using a fluorescent substrate. For secreted enzymes, such as an engineered form of alkaline phosphatase, small samples of culture supernatant are assayed for activity, allowing continuous monitoring of a single culture over time. Thus, different forms of this marker system can be used for different purposes. These include counting active virus, or sensitively and simply measuring viral spread in a culture and the inhibition of this spread by various drugs.

Further specificity can be incorporated into the preceding system by testing for the presence of the virus either with or without neutralizing antibodies to that virus. For example, in one portion of the clinical sample being tested, neutralizing antibodies to HIV may be present; whereas in another portion there would be no neutralizing antibodies. If the tests were negative in the system where there were antibodies and positive where there were no antibodies, this would assist in confirming the presence of HIV.

Within an analogous system for an *in vitro* assay, the presence of a particular gene, such as a viral gene, may be determined in a cell sample. In this case, the cells of the sample are infected with a suitable alphavirus vector which carries the reporter gene which is only expressed in the presence of the appropriate viral RNA transcript. The reporter gene, after entering the sample cells, will express its reporting product (such as β-galactosidase or luciferase) only if the host cell expresses the appropriate viral proteins.

These assays are more rapid and sensitive, since the reporter gene can express a greater amount of reporting product than identifying agent present, which results in an amplification effect.

### 6. Immune Down-Regulation

As briefly described above, there are also disclosed herein recombinant alphavirus which carry a vector construct capable of suppressing one or more elements of the immune system in target cells infected with the alphavirus.

Briefly, specific down-regulation of inappropriate or unwanted immune responses, such as in chronic hepatitis or in transplants of heterologous tissue such as bone marrow, can be engineered using immune-suppressive viral gene products which suppress surface expression of transplantation (MHC) antigen. Group C adenoviruses Ad2 and Ad5 possess a 19 kd glycoprotein (gp 19) encoded in the E3 region of the virus. This gp 19 molecule binds to class I MHC molecules in the endoplasmic reticulum of cells, and prevents terminal glycosylation and translocation of class I MHC to the cell surface. For example, prior to bone marrow transplantation, donor bone marrow cells may be infected with gp 19-encoding vector constructs which, upon expression of the gp 19, inhibit the surface expression of MHC class I transplantation antigens. These donor cells may be transplanted with low risk of graft rejection and may require a minimal immunosuppressive regimen for the transplant patient. This may allow an acceptable donor-recipient chimeric state to exist with fewer complications. Similar treatments may be used to treat the range of so-called autoimmune diseases, including lupus erythromiatis, multiple sclerosis, rheumatoid arthritis or chronic hepatitis B infection.

An alternative method involves the use of anti-sense message, ribozyme, or other specific gene expression inhibitor specific for T cell clones which are autoreactive in nature. These block the expression of the T cell receptor of particular unwanted clones responsible for an autoimmune response. The anti-sense, ribozyme, or other gene may be introduced using the viral vector delivery system.

### 7. Replacement or Augmentation Gene Therapy

One further aspect of the present disclosure relates to transforming cells of an animal with recombinant alphavirus vectors which serve as gene transfer vehicles to supply genetic sequences capable of expressing a therapeutic protein. Within one embodiment of the present disclosure, the viral vector construct is designed to express a therapeutic protein capable of preventing, inhibiting, stabilizing or reversing an inherited or noninherited genetic defect in metabolism, immune regulation, hormonal regulation, enzymatic or membrane associated structural function. This embodiment also describes said viral vector capable of transducing individual cells, whereby the therapeutic protein is able to be expressed systemically or locally from a specific cell or tissue, whereby the therapeutic protein is capable of (a) the replacement of an absent or defective cellular protein or enzyme, or (b) supplement production of a defective of low expressed cellular protein or enzyme. Such diseases may include cystic fibrosis, Parkinson's disease, hypercholesterolemia, adenosine deaminase deficiency, β-globin disorders. Hemophilia A & B. Gaucher's disease, diabetes and leukemia.

As an example, a recombinant alphavirus viral vector can be used to treat Gaucher disease. Briefly, Gaucher disease is a genetic disorder that is characterized by the deficiency of the enzyme glucocerebrosidase. This type of therapy is an example of a single gene replacement therapy by providing a functional cellular enzyme. This enzyme deficiency leads to the accumulation of glucocerebroside in the lysosomes of all cells in the body. However, the disease phenotype is manifested only in the macrophages, except in the very rare neuronpathic forms of the disease. The disease usually leads to enlargement of the liver and spleen and lesions in the bones. (For a review, *see* Science 256:794, 1992 and The Metabolic Basis of Inherited Disease, 6th ed., Scriver et al., vol. 2, p. 1677).

### 8. Administration of Alphavirus Particles

Within other aspects of the present disclosure, methods are disclosed for administering recombinant alphavirus vectors or particles. Briefly, the final mode of viral vector administration usually relies on the specific therapeutic application, the best mode of increasing vector potency, and the most convenient route of administration. Generally, this embodiment includes recombinant alphavirus vectors which can be designed to be delivered by, for example, (1) direct injection into the blood stream; (2) direct injection into a specific tissue or tumor; (3) oral administration; (4) nasal inhalation; (5) direct application to mucosal tissues; or (6) *ex vivo* administration of transduced autologous cells into the animal. Thus the therapeutic alphavirus vector can be administered in such a fashion such that the vector can (a) transduce a normal healthy cell and, transform the cell into a producer of a therapeutic protein or agent which is secreted systemically or locally, (b) transform an abnormal or defective cell, transforming the cell into a normal functioning phenotype, (c) transform an abnormal cell so that it is destroyed, and/or (d) transduce cells to manipulate the immune response.

### I. MODULATION OF TRANSCRIPTION FACTOR ACTIVITY

In yet another embodiment, alphavirus vectors may be utilized in order to regulate the growth control activity of transcription factors in the infected cell. Briefly, transcription factors directly influence the pattern of gene expression through sequence-specific *trans*-activation or repression (Karin, New Biologist 21:126-131, 1990). Thus, it is not surprising that mutated transcription factors represent a family of oncogenes. Alphavirus gene transfer therapy can be used, for example, to return control to tumor cells whose unregulated growth is activated by oncogenic transcription factors, and proteins which promote or inhibit the binding cooperatively in the formation of homo- and heterodimer *trans*-activating or repressing transcription factor complexes.

One method for reversing cell proliferation would be to inhibit the *trans*-activating potential of the *c-myc*/Max heterodimer transcription factor complex. Briefly, the nuclear oncogene *c-myc* is expressed by proliferating cells and can be activated by several distinct mechanisms, including retroviral insertion, amplification, and chromosomal translocation. The Max protein is expressed in quiescent cells and, independently of *c-myc,* either alone or in conjunction with an unidentified factor, functions to repress expression of the same genes activated by the myc/Max heterodimer (Cole, Cell 65:715-716, 1991).

Inhibition of *c-myc* or *c-myc*/Max proliferation of tumor cells may be accomplished by the overexpression of Max in target cells controlled by alphavirus vectors. The Max protein is only 160 amino acids (corresponding to 480 nucleotide RNA length) and is easily incorporated into an alphavirus vector either independently, or in combination with other genes and/or antisense/ribozyme moieties targeted to factors which release growth control of the cell.

Modulation of homo/hetero-complex association is another approach to control transcription factor activated gene expression. For example, transport from the cytoplasm to the nucleus of the *trans*-activating transcription factor NF-κB is prevented while in a heterodimer complex with the inhibitor protein IκB. Upon induction by a variety of agents, including certain cytokines, IκB becomes phosphorylated and NF-κB is released and transported to the nucleus, where it can exert its sequence-specific *trans*-activating function (Baeuerle and Baltimore, Science 242:540-546, 1988). The dissociation of the NF-κB/IκB complex can be prevented by masking with an antibody the phosphorylation site of IκB. This approach would effectively inhibit the trans-activation activity of the NF-IκB transcription factor by preventing its transport to the nucleus. Expression of the IκB phosphorylation site specific antibody or protein in target cells may be accomplished with an alphavirus gene transfer vector. An approach similar to the one described here could be used to prevent the formation of the *trans*-activating transcription heterodimer factor AP-1 (Turner and Tijan, Science 243:1689-1694, 1989), by inhibiting the association between the *jun* and *fos* proteins.

### J. PHARMACEUTICAL COMPOSITIONS

As noted above, there are also disclosed herein pharmaceutical compositions comprising a recombinant Sindbis particle or virus, or Sindbis vector construct, in combination with a pharmaceutically acceptable carrier, diluent, or recipient.

Briefly, infectious recombinant virus (also referred to above as particles) may be preserved either in crude or purified forms. In order to produce virus in a crude form, virus-producing cells may first be cultivated in a bioreactor, wherein viral particles are released from the cells into the culture media. Virus may then be preserved in crude form by first adding a sufficient amount of a formulation buffer to the culture media containing the recombinant virus to form an aqueous suspension. Within certain preferred embodiments, the formulation buffer is an aqueous solution that contains a saccharide, a high molecular weight structural additive, and a buffering component in water. The aqueous solution may also contain one or more amino acids.

The recombinant virus can also be preserved in a purified form. More specifically, prior to the addition of the formulation buffer, the crude recombinant virus described above may be clarified by passing it through a filter and then concentrated, such as by a cross flow concentrating system (Filtron Technology Corp., Nortborough, Mass.). Within one embodiment, DNase is added to the concentrate to digest exogenous DNA. The digest is then diafiltrated in order to remove excess media components and to establish the recombinant virus in a more desirable buffered solution. The diafiltrate is then passed over a Sephadex S-500 gel column and a purified recombinant virus is eluted. A sufficient amount of formulation buffer is then added to this eluate in order to reach a desired final concentration of the constituents and to minimally dilute the recombinant virus. The aqueous suspension may then be stored, preferably at -70°C, or immediately dried. As above, the formulation buffer may be an aqueous solution that contains a saccharide, a high molecular weight structural additive, and a buffering component in water. The aqueous solution may also contain one or more amino acids.

Crude recombinant virus may also be purified by ion exchange column chromatography. Briefly, crude recombinant virus may be clarified by first passing it through a filter, followed by loading the filtrate onto a column containing a highly sulfonated cellulose matrix. The recombinant virus may then be eluted from the column in purified form by using a high salt buffer, and the high salt buffer exchanged for a more desirable buffer by passing the eluate over a molecular exclusion column. A sufficient amount of formulation buffer is then added, as discussed above, to the purified recombinant virus and the aqueous suspension is either dried immediately or stored, preferably at -70°C.

The aqueous suspension in crude or purified form can be dried by lyophilization or evaporation at ambient temperature. Briefly, lyophilization involves the steps of cooling the aqueous suspension below the glass transition temperature or below the eutectic point temperature of the aqueous suspension, and removing water from the cooled suspension by sublimation to form a lyophilized virus. Within one embodiment, aliquots of the formulated recombinant virus are placed into an Edwards Refrigerated Chamber (3 shelf RC3S unit) attached to a freeze dryer (Supermodulyo 12K). A multistep freeze drying procedure as described by Phillips et al. (Cryobiology 18:414, 1981) is used to lyophilize the formulated recombinant virus, preferably from a temperature of -40°C to -45°C. The resulting composition contains less than 10% water by weight of the lyophilized virus. Once lyophilized, the recombinant virus is stable and may be stored at -20°C to 25°C, as discussed in more detail below.

Within the evaporative method, water is removed from the aqueous suspension at ambient temperature by evaporation. Within one embodiment, water is removed through spray-drying (EP 520,748). Within the spray-drying process, the aqueous suspension is delivered into a flow of preheated gas, usually air, whereupon water rapidly evaporates from droplets of the suspension. Spray-drying apparatus are available from a number of manufacturers (*e.g*., Drytec, Ltd., Tonbridge, England; Lab-Plant, Ltd., Huddersfield, England). Once dehydrated, the recombinant virus is stable and may be stored at -20°C to 25°C. Within the methods described herein, the resulting moisture content of the dried or lyophilized virus may be determined through use of a Karl-Fischer apparatus (EM Science Aquastar™ V 1 B volumetric titrator, Cherry Hill, NJ), or through a gravimetric method.

The aqueous solutions used for formulation, as previously described, are preferably composed of a saccharide, high molecular weight structural additive, a buffering component, and water. The solution may also include one or more amino acids. The combination of these components act to preserve the activity of the recombinant virus upon freezing and lyophilization or drying through evaporation. Although a preferred saccharide is lactose, other saccharides may be used, such as sucrose, mannitol, glucose, trehalose, inositol, fructose, maltose or galactose. In addition, combinations of saccharides can be used, for example, lactose and mannitol, or sucrose and mannitol. A particularly preferred concentration of lactose is 3%-4% by weight. Preferably, the concentration of the saccharide ranges from 1% to 12% by weight.

The high molecular weight structural additive aids in preventing viral aggregation during freezing and provides structural support in the lyophilized or dried state. Within the context of the present disclosure, structural additives are considered to be of "high molecular weight" if they are greater than 5000 m.w. A preferred high molecular weight structural additive is human serum albumin. However, other substances may also be used, such as hydroxyethyl-cellulose, hydroxymethyl-cellulose, , dextran, cellulose, gelatin, or povidone. A particularly preferred concentration of human serum albumin is 0.1% by weight. Preferably, the concentration of the high molecular weight structural additive ranges from 0.1 % to 10% by weight.

The amino acids, if present, function to further preserve viral infectivity upon cooling and thawing of the aqueous suspension. In addition, amino acids function to further preserve viral infectivity during sublimation of the cooled aqueous suspension and while in the lyophilized state. A preferred amino acid is arginine, but other amino acids such as lysine, ornithine, serine, glycine, glutamine, asparagine, glutamic acid or aspartic acid can also be used. A particularly preferred arginine concentration is 0.1 % by weight. Preferably, the amino acid concentration ranges from 0.1 % to 10% by weight.

The buffering component acts to buffer the solution by maintaining a relatively constant pH. A variety of buffers may be used, depending on the pH range desired, preferably between 7.0 and 7.8. Suitable buffers include phosphate buffer and citrate buffer. A particularly preferred pH of the recombinant virus formulation is 7.4, and a preferred buffer is tromethamine.

In addition, it is preferable that the aqueous solution contain a neutral salt which is used to adjust the final formulated recombinant alphavirus to an appropriate iso-osmotic salt concentration. Suitable neutral salts include sodium chloride, potassium chloride or magnesium chloride. A preferred salt is sodium chloride.

Aqueous solutions containing the desired concentration of the components described above may be prepared as concentrated stock solutions.

It will be evident to those skilled in the art, given the disclosure provided herein, that it may be preferable to utilize certain saccharides within the aqueous solution when the lyophilized virus is intended for storage at room temperature. More specifically, it is preferable to utilize disaccharides, such as lactose or trehalose, particularly for storage at room temperature.

The lyophilized or dehydrated viruses disclosed herein may be reconstituted using a variety of substances, but are preferably reconstituted using water. In certain instances, dilute salt solutions which bring the final formulation to isotonicity may also be used. In addition, it may be advantageous to use aqueous solutions containing components known to enhance the activity of the reconstituted virus. Such components include cytokines, such as IL-2, polycations, such as protamine sulfate, or other components which enhance the transduction efficiency of the reconstitute virus. Lyophilized or dehydrated recombinant virus may be reconstituted with any convenient volume of water or the reconstituting agents noted above that allow substantial and preferably total solubilization of the lyophilized or dehydrated sample.

The following examples are offered by way of illustration, and not by way of limitation. Aspects of the examples that do not relate specifically to the claimed invention are for illustration and comparison only.

### EXAMPLES

### EXAMPLE 1

### CLONING OF A SINDBIS GENOMIC LENGTH cDNA

The nature of viruses having an RNA genome with positive polarity is such that when introduced into a eukaryotic cell which serves as a permissive host, the purified genomic nucleic acid serves as a functional message RNA (mRNA) molecule. Thus, this genomic RNA, purified from the virus, can initiate the same infection cycle which is characteristic of infection by the wild type virus from which the RNA was purified.

Sindbis virus strain Ar-339 (ATCC #VR-1248, Taylor et al., Am. J. Trop. Med Hyg. 4:844 1955), isolated from the mosquito *Culexus univittatus* is propagated on baby hamster kidney (BHK) cells, infected at low multiplicity (0.1 PFU/cell). Alternatively, Sindbis virus strain hr (Lee Biomolecular, San Diego, CA) can be used and propagated by the same methods. Sindbis virions are precipitated from a clarified lysate at 48 hours post infection with 10% (w/v) of polyethylene glycol (PEG-8000) at 0°C, as described previously. The Sindbis virions contained in the PEG pellet are lysed with 2% SDS, and the poly-adenylated mRNA is isolated by chromatography using commercially available oligo dT columns (Invitrogen). Two rounds of first strand cDNA synthesis are performed on the polyA selected mRNA, using an oligonucleotide primer with the sequence shown below:
5'- TATATTCTAGA(dT)25-GAAATG-3' (SEQ. ID NO. 2)

The primer contains at its 5' end a five nucleotide 'buffer sequence' for efficient restriction endonuclease digestion, followed by the Xba I recognition sequence, 25 consecutive dT nucleotides and six nucleotides which are precisely complementary to the extreme Sindbis 3' end. Thus, selection for first round cDNA synthesis is at two levels: (1) polyadenylated molecules, a prerequisite for functional mRNA, and (2) selective priming from Sindbis mRNA molecules, in a pool containing multiple mRNA species. Further, the reverse transcription is performed in the presence of 10 mM MeHgOH to mitigate the frequency of artificial stops during reverse transcription.

The primary genomic length Sindbis cDNA is amplified by PCR in six distinct segments using six pairs of overlapping primers. In addition to viral complementary sequences, the Sindbis 5' end forward primer contains the 19 nucleotide sequence corresponding to the bacterial SP6 RNA polymerase promoter and the Apa I restriction endonuclease recognition sequence linked to its 5' end A five nucleotide 'buffer sequence' for efficient digestion precedes the Apa I recognition sequence. The bacterial SP6 RNA polymerase is poised such that *transcription in vitro* results in the inclusion of only a single non-viral G ribonucleotide linked to the A ribonucleotide, which corresponds to the authentic Sindbis 5' end. Inclusion of the Apa I recognition sequence facilitates insertion of the PCR amplicon into the plasmid vector (pKS II⁺, Strategene) polylinker sequence. The sequence of the SP6-5' Sindbis forward primer and all of the primer pairs necessary to amplify the entire Sindbis genome are shown below. The reference sequence (GenBank accession no. SINCG) is from Strauss et al., Virology 133:92-110*.*

| Primer | Location | Seq. ID No. | Sequence | Recognition Sequence (5'->3') |
|---|---|---|---|---|
| SP6-1A | ApaI/SP6/+ SIN nts. 1-18 | 3 | | Apa I |
| | | | | |
| 1B | 3182-3160 | 4 | CTGGCAACCGGTAAGTACGATAC | Age I |
| | | | | |
| 2A | 3144-3164 | 5 | ATACTAGCCACGGCCGGTATC | Age I |
| 2B | 5905-5885 | 6 | TCCTCTTTCGAGCGTGTCGAGC | Eco RI |
| | | | | |
| 3A | 5844-5864 | 7 | ACCTTGGAGCGCAATGTCCTG | Eco RI |
| 7349R | 7349-7328 | 8 | CCTTTTCAGGGGATCCGCCAC | Bam HI |
| | | | | |
| 7328F | 7328-7349 | 9 | GTGGCGGATCCCCTGAAAAGG | Bam HI |
| 3B | 9385-9366 | 10 | TGGGCCGTGTGGTCGTATG | Bcl I |
| | | | | |
| 4A | 9336-9356 | 11 | TGGGTCTTCAACTCACCGGAC | Bcl I |
| 10394R | 10394-10372 | 12 | CAATTCGACGTACGCCTCACTC | Bsi W_{I} |
| | | | | |
| 10373F | 10373-10394 | 13 | GAGTGAGGCGTACGTCGAATTG | Bsi W_{I} |
| 4B | XbaI/dT₂₅/ | | | |
| | | | | |
| | 11703-11698 | | TATATTCTAGA(dT)₂₅-GAAATG | Xba I |

PCR amplification of Sindbis cDNA with the six primer sets shown above is performed in separate reactions, using the Thermalase thermostable DNA polymerase (Amresco Inc., Solon, Ohio) and the buffer containing 1.5 mM MgCl₂, provided by the supplier. Additionally, the reactions contain 5% DMSO, and the Hot Start Wax beads (Perkin-Elmer), using the following PCR amplification protocol shown below:

| Temperature (°C) | Time (Min.) | No. Cycles |
|---|---|---|
| 94 | 2 | 1 |
| 94 | 0.5 | |
| 55 | 0.5 | 35 |
| 72 | 3.5 | |
| 72 | 10 | 10 |

Following amplification, the six reaction products are inserted first into the pCR II vector (Invitrogen), then using the appropriate enzymes shown above, are inserted, stepwise, into the pKS II⁺ (Stratagene) vector, between the Apa I and Xba I sites. This clone is designated as pVGSP6GEN.

The Sindbis genomic cDNA clone pVGSP6GEN is linearized by digestion with Xba I, which cuts pVGSP6GEN once, immediately adjacent and downstream of the 25 nucleotide long poly dA:dT stretch. The linearized pVGSP6GEN clone is purified with Gene Clean (BIO 101, La Jolla, CA), and is adjusted to a concentration of 0.5 mg/ml. Transcription of the linearized pVGSP6GEN clone is performed *in vitro* at 40°C for 90 min according to the following reaction conditions: 2 ml DNA/4.25 ml H₂0/10 ml 2.5 mM NTPs (UTP, ATP, GTP, CTP)/1.25 ml 20 mM m⁷G(5')ppp(5')G cap analog/1.25 ml 100 mM DTT/5 ml 5X transcription buffer (Promega)/0.5 ml RNasin (Promega)/0.25 ml 10 mg/ml bovine serum albumin/0.5 ml SP6 RNA polymerase (Promega). The *in vitro* transcription reaction products can be digested with DNase I (Promega) and purified by sequential phenol/CHCl₃ and ether extraction, followed by ethanol precipitation, or alternatively, can be used directly for transfection. The *in vitro* transcription reaction products or purified RNA are complexed with a commercial cationic lipid compound (Lipofectin, GIBCO-BRL, Gaithersburg, MD), and applied to Baby Hamster Kidney-21 (BHK-21) cells maintained in a 60 mM petri dish at 75% confluency. The transfected cells are incubated at 30°C. After 94 hours post transfection, extensive cytopathologic effects (CPE) are observed. No obvious CPE is observed in plates not receiving RNA transcribed from the Sindbis cDNA clone. Further, 1 ml of supernatant taken from transfected cells, added to fresh monolayers of BHK-21 cells, and incubated at 30°C or 37°C results in obvious CPE within 18 hrs. This demonstrates that the Sindbis cDNA clone pVGSP6GEN is indeed infectious.

Sequence analysis of pVGSP6GEN, shown in Table 1, reveals multiple sequence differences between the Sindbis genomic clone described herein, and the viral clone contained in Genbank. Many sequence differences result in the substitution of non-conservative amino acids changes in the Sindbis proteins. To address which sequence changes are unique to the clone described herein, or are a result of cloning artifact, virion RNA is amplified by RT-PCR as described above, and sequence relating to the nucleotides in question is determined by direct sequencing of the RT-PCR amplicon product, using a commercially available kit (Promega, Madison WI), and compared to the corresponding pVGSP6GEN sequence. The results of this study are given in Table 2. Briefly, three non conservative amino acid changes, Gly ->Glu, Asp->Gly, and Tyr->Cys, which are a result of cloning artifact are observed respectively at viral nucleotides 2245, 6193, and 6730. These nucleotide changes resulting in non conservative amino acid changes all map to the viral non-structural protein (NSP) gene, nt 2245 to NSP 2, and nts 6193 and 6730 to NSP4.

Repair of the NSP 2 and NSP 4 genes is accomplished by RT-PCR, as described above, using virion RNA from a 5 times plaque purified stock. The SP6-1A/1B primer pair described above is used to repair the nt 2245 change. The RT-PCR amplicon product is digested with Eco 47III and Bgl II, and the 882 bp fragment is purified by 1% agarose/TBE gel electrophoresis, and exchanged into the corresponding region of the pVGSP6GEN clone, prepared by digestion with Eco 47III and Bgl II, and treatment with CIAP. The 3A/7349R primer pair described above is used to repair the nt 6193 and nt 6730 changes. The RT-PCR amplicon product is digested with Eco RI and Hpa I, and the 1,050 bp fragment is purified by 1% agarose/TBE gel electrophoresis, and exchanged into the corresponding region of the pVGSP6GEN clone. This clone is known as pVGSP6GENrep. Transfection of BHK cells with *in vitro* transcribed RNA from pVGSP6GENrep DNA linearized by digestion with Xba I, as described above results in extensive CPE 18 hrs post transfection.

**Table 1**

| Sindbis Genomic Clone Differences between Viagene and GenBank Sequences | | | | |
|---|---|---|---|---|
| SIN nt. # | Change | Codon Change | Location in Codon | amino acid change |
| | | | | |
| Noncoding Region: | | | | |
| 45 | T->C | N.A. | N.A. | N.A. |
| Non-structural Proteins: | | | | |
| 353 | C->T | UAU->UAC | 3' | Tyr->Tyr |
| 1095 | A->C | AUA->CUA | 1' | Ile->Leu |
| 1412 | T->C | UUU->UUC | 3' | Phe->Phe |
| 2032 | A->G | GAG->GGG | 2' | Glu->Gly |
| 2245 | G->A | GGG->GAG | 2' | Gly->Glu |
| 2258 | A->C | UCA->UCC | 3' | Ser->Ser |
| 2873 | A->G | CAA->CAG | 3' | Gln->Gln |
| 2992 | C->T | CCC->CUC | 2' | Pro->Leu |
| 3544 | T->C | GUC->GCC | 2 | Val->Ala |
| 3579 | A->G | AAA->GAA | 1' | Lys->Glu |
| 3822 | A→G | ACC->GCC | 1' | Thr->Ala |
| 3851 | T->C | CUU->CUC | 3' | Leu->Leu |
| 5351 | A->T | CAA->CAU | 3' | Gln->His |
| 5466 | G->A | GGU->AGU | 1' | Gly->Ser |
| 5495 | T->C | AUU→AUC | 3' | Ile->Ile |
| 5543 | A->T | ACA->ACU | 3' | Thr->Thr |
| 5614 | T->C | GUA->GCA | 2' | Val->Ala |
| 6193 | A->G | GAC->GGC | 2' | Asp->Gly |
| 6564 | G->A | GCA->ACA | 1' | Ala->Thr |
| 6730 | A->G | UAC->UGC | 2' | Tyr->Cys |

| Structural Proteins: | | | | |
|---|---|---|---|---|
| 8637 | A->G | AUU->GUU | 1' | Ile->Val |
| 8698 | T->A | GUA->GAA | 2' | Val→Glu |
| 9108 | AAG del | AAG->del | 1'-3' | Glu→del |
| 9144 | A->G | AGA->GGA | 1' | Arg->Gly |
| 9420 | A->G | AGU->GGU | 1' | Ser->Gly |
| 9983 | T->G | GCU->GCG | 3' | Ala->Ala |
| 10469 | T->A | AUU->AUA | 3' | Ile->Ile |
| 10664 | T->C | UUU->UUC | 3' | Phe->Phe |
| 10773 | T->G | UCA->GCA | 1' | Ser->Ala |

**Table 2**

| Sindbis Genomic Clone Artifact Analysis | | | |
|---|---|---|---|
| SIN nt. # | Amino Acid change | Viagene Unique | Cloning Artifact |
| Nonstructural Proteins: | | | |
| 2032 | Glu->Gly | +* | |
| 2245 | Gly->Glu | | + |
| 2258 | Ser->Ser | +* | |
| 2873 | Gln→Gln | + | |
| 2992 | Pro->Leu | + | |
| 3544 | Val→Ala | | + |
| 3579 | Lys->Glu | + | |
| 3822 | Thr→Ala | | + |
| 3851 | Leu->Leu | | + |
| 5351 | Gln->His | + | |
| 5466 | Gly->Ser | | + |
| 5495 | Ile->Ile | | + |
| 5543 | Thr->Thr | | + |
| 6193 | Asp->Gly | | + |
| 6730 | Tyr->Cys | | + |

| Structural Proteins: | | | |
|---|---|---|---|
| 8637 | Ile->Val | + | |
| 8698 | Val→Glu | + | |
| 9108 | Glu→del | + | |
| 9144 | Arg->Gly | + | |

| | | | |
|---|---|---|---|
| *Mixture: Both Genbank and Viagene Sindbis strains present at this nucleotide. | | | |

### EXAMPLE 2

### GENERATION OF PLASMID DNA VECTORS WHICH INITIATE SINDBIS INFECTION

Because of the size of the full length genomic Sindbis cDNA clone, *in vitro* transcription of full length molecules is rather inefficient. This results in a lowered transfection efficiency, in terms of infectious centers of virus, as measured by plaque formation, relative to the amount of *in vitro* transcribed RNA transfected. This concern is also relevant to the *in vitro* transcription of Sindbis expression vectors. Testing of candidate cDNA clones and other Sindbis cDNA expression vectors for their ability to initiate an infectious cycle or to direct the expression of a heterologous sequence would be greatly facilitated if the cDNA clone was transfected into susceptible cells as a DNA molecule which then directed the synthesis of viral RNA *in vivo.* Transfection efficiencies as high as 100% have been reported in cells transfected with DNA complexed with various commercial synthetic lipid preparations or by electroporation. Also, it has been demonstrated that cDNA from picornaviruses is able to initiate an infectious cycle when transfected into susceptible cells (van der Werf et al., PNAS 83:2330-2334, 1986). It has not been described in the literature, however, if genomic Sindbis cDNA, when placed proximal to a mammalian promoter and transfected into cells, yields RNA which is able to initiate the infectious cycle characteristic of wild type virus.

It is well known that DNA molecules having the appropriate signals can replicate *in vivo* when introduced directly into animals (Dubensky et al., PNAS 81:7429-7533, 1984). Administration of Sindbis cDNA vectors directly as DNA molecules is feasible in some applications in which the Sindbis directed expression of palliatives have a *trans* effect. These applications include immunomodulation and expression of cytokines or other therapeutic proteins. Within the scope of Sindbis, the direct administration of Sindbis cDNA expression vectors offers a utility which is more simple than generation of a Sindbis particle.

Sindbis cDNA vector constructs containing a heterologous therapeutic palliative sequence are inserted within a eukaryotic RNA polymerase II expression cassette. This construction is known as a Eukaryotic Layered Vector Initiation System (ELVIS) and is comprised of the following ordered elements: a 5' eukaryotic promoter capable of initiating the synthesis of viral RNA at the authentic Sindbis 5' end, a 5' sequence which is capable of initiating transcription of a Sindbis virus, a nucleotide sequence encoding Sindbis non structural proteins, a viral junction region, a heterologous sequence, a Sindbis RNA polymerase recognition sequence, a transcription termination sequence and a 3' polyadenylation signal. The eukaryotic Sindbis cDNA expression vector described herein may include also the appropriate splicing signals located, for example, between Sindbis and heterologous gene regions.

The ability of the Sindbis cDNA clone pVGSP6GENrep to initiate an infectious cycle characteristic of wild type virus is first determined by placement of the genomic viral cDNA within a mammalian RNA polymerase II expression cassette. This construction is accomplished as described in the following detail. The clone pVGSP6GENrep is digested with Bgl II and Xba I, the reaction products are electrophoresed on a 0.8% agarose/TBE gel, and the resulting 9,438 bp fragment is excised, purified with Gene Clean (BIO 101, Vista, CA), and ligated into the 4,475 bp vector fragment resulting from treatment of pcDNA3 (Invitrogen, San Diego, CA) with Bgl II, Xba I, and CIAP. This construction is known as pcDNASINbgl/xba.

The long terminal repeat (LTR) from Moloney murine leukemia virus (Mo-MLV) is positioned at the 5' viral end such that the first transcribed nucleotide is a single G residue, which is capped *in vivo,* followed by the Sindbis 5' end. It is known that *in vitro* transcribed RNAs containing three non-viral nucleotides at their 5' end are infectious (Rice et al., J. Virol. 61:3809-3819, 1987). Juxtaposition of the Mo-MLV LTR and the Sindbis 5' end is accomplished by overlapping PCR as described in the following detail. Amplification of the Mo-MLV LTR in the first primary PCR reaction is accomplished in a reaction containing the BAG vector (Price et al., PNAS 84:156-160, 1987) and the following primer pair:
Forward primer: BAGBg12F1 (buffer sequence/Bgl II recoginition sequence/Mo-MLV LTR nts 1-22):
   5'-TATATAGATCTAATGAAAGACCCCACCTGTAGG
Reverse primer: BAGwt441 R2 (SIN nts 5-1/Mo-MLV LTR nts 441-406):
   5'-TCAATCCCCGAGTGAGGGGTTGTGGGCTCTTTTATTGAGC

PCR amplification of the Mo-MLV LTR with the primer pair shown above is performed using the Thermalase thermostable DNA polymerase (Amresco Inc., Solon, Ohio) and the buffer containing 1.5 mM MgCl₂, provided by the supplier. Additionally, the reaction contains 5% DMSO, and the Hot Start Wax beads (Perkin-Elmer), using the following PCR amplification protocol shown below:

| Temperature (°C) | Time (Min.) | No. Cycles |
|---|---|---|
| 94 | 2 | 1 |
| 94 | 0.5 | |
| 55 | 0.5 | 35 |
| 72 | 0.5 | |
| 72 | 10 | 1 |

Amplification of the Sindbis 5' end in the second primary PCR reaction is accomplished in a reaction containing the pVGSP6GENrep clone and the following primer pair:
Forward primer: (Mo-MLV LTR nts 421-441/SIN nts 1-16):
   5'-CCACAACCCCTCACTCGGGGATTGACGGCGTAGTAC
Reverse primer: (SIN nts 3182-3160):
   5'-CTGGCAACCGGTAAGTACGATAC

PCR amplification of the Mo-MLV LTR is with the primer pair and amplification reaction conditions shown above, and using the following PCR amplification protocol shown below:

| Temperature (°C) | Time (Min.) | No. Cycles |
|---|---|---|
| 94 | 2 | 1 |
| 94 | 0.5 | |
| 55 | 0.5 | 35 |
| 72 | 3.0 | |
| 72 | 10 | 1 |

The 457 bp and 3202 bp products from the primary PCR reactions are purified with Gene Clean, and used together in a PCR reaction with the following primer pair:
Forward primer: BAGBgI2F (buffer sequence/Bgl II recoginition sequence/Mo-MLV LTR nts 1-22):
   5'-TATATAGATCTAATGAAAGACCCCACCTGTAGG
Reverse primer: (SIN nts 2300-2278):
   5'-GGTAACAAGATCTCGTGCCGTG

PCR amplification of the primer PCR amplicon products is with the primer pair and amplification reaction conditions shown above, and using the following PCR amplification protocol shown below:

| Temperature (°C) | Time (Min.) | No. Cycles |
|---|---|---|
| 94 | 2 | 1 |
| 94 | 0.5 | |
| 55 | 0.5 | 35 |
| 72 | 3.0 | |
| 72 | 10 | 1 |

The 25 3' terminal bases of the first primary PCR amplicon product overlaps with the 25 5' terminal bases of the second primary PCR amplicon product; the resultant 2,752 bp overlapping secondary PCR amplicon product is purified by 0.8% agarose/TBE electrophoresis, digested with Bgl II, and the 2,734 bp product is ligated into pcDNASINbgl/xba treated with Bgl II and CIAP. The resulting construction is 16,656 bps and is known as pVGELVIS. The sequence of pVGELVIS is given in Figure 3. Sindbis nucleotides are contained within bases 1-11,700 of the sequence.

pVGELVIS plasmid DNA is complexed with Lipofectamine (GIBCO-BRL, Gaithersburg, MD) according to the conditions suggested by the supplier (ca. 5 µg DNA/8 mg lipid reagent) and added to 35 mm wells containing BHK-21 cells at approximately 75% confluency. Cytopathic effects (CPE), characteristic of wild type Sindbis virus infection are observed within 48 hrs post infection. Addition of 1 ml of transfection supernatant to fresh BHK-21 monolayers results in CPE within 16 hrs. This data demonstrates the correct juxtaposition of viral cDNA and RNA polymerase II expression cassette signals in the pVGELVIS construct, resulting in the *de novo* initiation of an RNA virus from a DNA expression module. This inovation not only enhances the utility of the Sindbis system in general, but also provides another method for a Physical Gene Transfer vector.

The efficiency of vector replication following initial transcription of genome-length RNA from pVGELVIS is also enhanced by modifications that result in a more authentic 3' end. For this purpose, two different modifications are made to the ELVIS vector construct. The first utilizes the antigenomic ribozyme sequence of hepatitis delta virus (HDV), positioned adjacent to the Sindbis polyadenylate tract. The second involves a deletion of the Sindbis polyadenylate tract and downstream vector sequences, resulting in the fusion of Sindbis nucleotide 11,700 to the bovine growth hormone gene transcription termination/ polyadenylation sequence.

The HDV ribozyme-containing construct is generated by using PCR techniques and overlapping oligonucleotide primers which contain the entire 84 nucleotide antigenomic ribozyme sequence (Perotta and Been, Nature 350:434-6, 1991). In addition to the HDV sequence, the two primers contain flanking restriction enzyme sites for purposes of insertion into the ELVIS vector. Two rounds of PCR are performed: first by using oligonucleotide primers HDV49-XC and HDV 17-68 (shown below).
HDV17-68:
HDV49-XC:

followed by dilution of the first PCR reaction, and its subsequent use a template in a second round of PCR with primers HDV49-XC (from first reaction) and HDVX-36 (shown below).
HDVX-36:
   5'-ACGTCTAGATCTGGGTCGGCATGGCATCTCCACCTCCTCGCGGTCCGA-3'
Following synthesis, the HDV ribozyme fragment is digested with Xba I, which cleaves in the flanking sequences (italics), and ligated into ELVIS vector DNA which has been partially digested with Xba I to cleave only one of its two sites. Screening for insertion into the proper site and correct orientation is performed by restriction site and sequence analysis. This construction is known as pVGELVISHDV.

In the second method, fusion of Sindbis nucleotide 11,700 to the bovine growth hormone gene transcription termination/ polyadenylation sequence by deletion of the Sindbis polyadenylate tract and downstream vector sequencesin pVGELVIS is accomplished by overlapping PCR. Amplification of the Sindbis 3' end in the first primary PCR reaction is accomplished in a reaction containing pVGELVIS and the following primer pair :
Forward primer: SIN10349F (SIN nts 10394-10372):
   5'-GACAGTGAGAACAGCCAGATGAG
Reverse primer: SINBGH11700R (BGH nts 13-1/SIN nts 11700-11675):
   5'-CAGCGAGCTCTAGGAAATGTTAAAAACAAAATTTTGTTG

PCR amplification of pVGELVIS with the primer pair shown above is performed using the Thermalase thermostable DNA polymerase (Amresco Inc., Solon, Ohio) and the buffer containing 1.5 mM MgCl₂, provided by the supplier. Additionally, the reaction contains 5% DMSO, and the Hot Start Wax beads (Perkin-Elmer), using the following PCR amplification protocol shown below:

| Temperature (°C) | Time (Min.) | No. Cycles |
|---|---|---|
| 94 | 2 | 1 |
| 94 | 0.5 | |
| 55 | 0.5 | 35 |
| 72 | 1.5 | |
| 72 | 10 | 1 |

Amplification of the BGH gene transcription termination/polyadenylation sequence in the second primary PCR reaction is accomplished in a reaction containing the pVGELVIS clone and the following primer pair:
Forward primer: pCSIN1018F (SIN nts 11,687-11,700/PCDNA3 nts 1018-1039):
   5'-CCACAACCCCTCACTCGGGGATTGACGGCGTAGTAC
Reverse primer: pCDNA1633R (pCDNA3 nts 1633-1608):
   5'-GTTCCAGTTTGGAACAAGAGTCCAC

PCR amplification of the 5' end of the BGH gene is with the primer pair and amplification reaction conditions shown above, and using the following PCR amplification protocol shown below:

| Temperature (°C) | Time (Min.) | No. Cycles |
|---|---|---|
| 94 | 2 | 1 |
| 94 | 0.5 | |
| 55 | 0.5 | 35 |
| 72 | 1.0 | |
| 72 | 10 | 1 |

The 1,364 bp and 629 bp products from the primary PCR reactions are purified with Gene Clean, and used together in a PCR reaction with the following primer pair:
Forward primer: SIND310374F (5 nt buffer/Hind III recognition site/SIN nts 10374-10394):
   5'-TATATAAGCTTGAGGCGTACGTCGAATTGTCAG
Reverse primer: pCDNA1575R (pCDNA3 nts 1575-1552):
   5'-GAAAAACCGTCTATCAGGGCGATG

PCR amplification of the primer PCR amplicon products is with the primer pair and amplification reaction conditions shown above, and using the following PCR amplification protocol shown below:

| Temperature (°C) | Time (Min.) | No. Cycles |
|---|---|---|
| 94 | 2 | 1 |
| 94 | 0.5 | |
| 55 | 0.5 | 35 |
| 72 | 2.0 | |
| 72 | 10 | 1 |

The 27 3' terminal bases of the first primary PCR amplicon product overlaps with the 27 5' terminal bases of the second primary PCR amplicon product; the resultant 1,976 bp overlapping secondary PCR amplicon product is purified by 0.8% agarose/TBE electrophoresis, digested with Hind III and Dra III, and the 1,941 bp product is ligated into pcDNA3 digested with Hind III and Dra III, and treated with CIAP. This construction is known as pCDNAELVIS3'. The pCDNAELVIS3' construction is digested with Bsi WI and Pvu I, and the resultant 5197 bp fragment is purified by 0.8% agarose/TBE electrophoresis, and ligated with the 11,398 bp fragment isolated by digestion of pVGELVIS with Bsi WI and Pvu I, and treatment with CIAP, followed by 0.8% agarose/TBE electrophoresis and Gene Clean. The resulting construction is 16,595 bps and is known as pVGELVISd13'.

To test the ability of the pVGELVISHDV and pVGELVISd13' plasmids to initiate infection characteristic of wild type Sindbis virus, the DNA clones are complexed with Lipofectamine (GIBCO-BRL, Gaithersburg, MD) according to the conditions suggested by the supplier (ca. 5 µg DNA/8 mg lipid reagent) and added to 35 mm wells containing BHK-21 cells at approximately 75% confluency. If CPE is observed by this method, 1 ml of transfection supernatant can be used to infect fresh BHK-21 monolayers, or alternatively, the level of Sindbis virus in the supernatants can be quantitated directly by plaque assay.

In other applications, it is desireable to position the CMV promoter contained in the pCDNA3 plasmid next to the Sindbis genomic cDNA, such that transcription initiation *in vivo* corresponds to the authentic 5' end viral nucleotide. The CMV promoter start site on the plasmid pcDNA3 is determined by mapping the transcription initiation point *in vitro.* This is accomplished by first digesting the pcDNA3 plasmid with Dra III restriction endonuclease (New England Biolabs Inc., Beverly, MA) according to the manufacturer's instructions. Dra III cleaves the plasmid once at nucleotide 1546 resulting in a linear 5446 base pair DNA molecule. The DNA is purified using the Geneclean II kit (BIO 10 San Diego, CA) exactly as stated in the instructions. Linearized pcDNA3 is transcribed with the HeLa Nuclear Extract *in vitro* Transcription System (Promega Corp., Madison, WI) in a reaction containing 3µg linearized pcDNA3, 400 µM each ribonucleotide 3mM MgCl₂, and 8 units of HeLa cell nuclear extract. The 25 µL reaction is incubated for one hour at 35°C. One unit of RQ1 DNAse (Promega Corp. Madison, WI) and 40 units of RNasin (Promega Corp. Madison, WI) are added to the reaction and incubated at 37°C for 30 minutes to eliminate the DNA template. The reaction is terminated by the addition of 175 µL of Stop Mix (supplied with the HeLa extract). The reaction is extracted with an equal volume of phenolchlaroformisoarnyl alcohol (25:24:1) followed by an extraction with chloroformlisoarnyl alcohol (24:1). The reaction is precipitated with 500 µL absolute ethanol. The RNA is pelleted by centrifugation at 12,000 x G for 15 minutes. The pellet is rinsed with 80% ethanol and resuspended in 20 µL of water.

A primer complementary to the SP6 promoter (ATTTAGGTGACACTATAG, BRL Corp., Bethesda, MD) is labeled at the 5' end with ³²P by mixing 10 pmol primer with a twofold excess of gamma ³²P ATP (ICN Corp. Irvine, CA), ten units of T4 Polynucleotide Kinase (Promega Corp., Madison, WI), and 1X Kinase Buffer (supplied with the T4 Kinase). The reaction is incubated for 30 minutes at 37°C then for 5 minutes at 95°C.

The *in vitro* transcribed RNA is annealed to the labeled primer by mixing 10 µL of the RNA with 1.5 pmol primer. The mixture is heated to 90°C and allowed to cool slowly. The mixture is brought to 50 mM Tris-HCL pH 8.3, 10 mM MgCl₂, 50 mM NaCl, 1 mM DTT, 40 µM each dNTP, and 15 units of AMV Reverse Transcriptase (USB Corp., Cleveland, OH). The reaction mixture is incubated at 42°C for 30 minutes. The reaction is terminated by the addition of one third volume of 95% Formamide, 20 mMEDTA, 0.05% Bromophenol Blue, and 0.05% Xylene Cyanol FF.

The pcDNA3 plasmid is sequenced using the same ³²P labeled primer described above, by utilizing the fmol DNA Sequencing System (Promega Corp., Madison, WI) exactly as described in the instructions. The sequencing reactions are loaded on a 6% denaturing gel adjacent to 2 µL of the primer extended *in vitro* RNA reaction. The gel is electrophoresed for one hour at 1600V, dried, and exposed to film overnight. The full length cDNA band can be compared to the pcDNA3 sequence lanes to determine the transcription initiation site. Using this method, the transcription initiation site of the CMV promoter can be determined to be exactly 39 bases downstream of the CMV promoter at the G residue of nucleotide number 858 (according to the numbering of Invitrogen pcDNA3 vector).

The CMV promoter from the pCDNA3 plasmid is then juxtaposed to Sindbis cDNA by overlapping PCR, using the methods described above for the construction of pVGELVIS The precise transcription initiation point of any RNA polymerase 11 promoter can be determined by the methods described above, allowing appropriate juxtapositioning of the promoter in the ELVIS configuration. The use of inducible promoters in the ELVIS configuration allows the initiation of infection in transfected cell lines to be controlled.

### EXAMPLE 3

### PREPARATION OF RNA AND PLASMID DNA SINDBIS VECTORS

### A. Construction of SINDBIS Basic Vector

The first step in the construction of the basic Sindbis vector is the generation of two plamid subclones containing separate elements from the viral 5' and 3' ends which are used subsequently to assemble the basic gene transfer vector. The first plasmid subclone contains the 40 terminal nucleotides at the viral 3' end and a 25 base stretch of dA:dT nucleotides. The vector 3' end is synthesized chemically to have the sequence of the primer pair shown below.
Forward Primer: SIN11664F: (buffer sequence/Not I site/ SIN nts 11664-11698):
   5'-TATATGCGGCCGCTTTCTTTTATTAATCAACAAAATTTTGTTTTTAA
Reverse Primer: SINXba11700R (buffer sequence/Sac I site dT25/SIN nts 11700-11692):
   5'-TATATGAGCTCTTTTTTTTTTTTTTTTTTTTTTTTTGAAATGTTAAAA

The above oligonucleotides are mixed together at equal molar concentrations in the presence of 10 mM Mg Cl₂, heated to 100°C for 5 min. and cooled slowly to room temperature. The partially double stranded molecule is then filled in using Klenow DNA polymerase and 50 uM dNTPs. The 89 bp molecule is then digested with Not I and Sac I, purified on a 2% NuSieve/1% agarose gel, ligated into pKS II+ plasmid, digested with Not I and Sac I, and treated with CIAP at a 10:1 molar excess of insert:vector ratio. This construction is known as pKSII3'SIN.

The second plasmid subclone contains the 5' 7,643 bps including a 5' terminal RNA polymerase promoter of Sindbis. The 3' end of this clone is derived by PCR amplification with a reverse primer having the sequence shown below.
Reverse Primer: SINXho7643R (buffer sequence/Xho I site/SIN nts 7643-7621):
   5'TATATCTCGAGGGTGGTGTTGTAGTATTAGTCAG

The reverse primer maps from viral nucleotides 7643 -762 and is 41 bps downstream from the junction core element 3' end. Additionally, viral nucleotide 7643 is 4 nucleotides upstream from the structural proteins translation initiation point. The first five 5' nucleotides in this primer, which are followed by 6 nucleotides comprising the Xho I recognition sequence, are included to serve as a 'buffer sequence' for the efficient digestion of the PCR amplicon products.

The forward primer in this reaction is primer 2A (described in Example 1), having the following sequence:
ATACTAGCCACGGCCGGTATC

The 4510 bp amplicon product, resulting from the PCR experiment using the primers shown above with the pVGSP6GENrep plasmid, is digested with the enzymes Sfi I and Xho 1. The resultant 2526 bp fragment is gel purified. The Sindbis cDNA clone pVGSP6GENrep is digested with Apa I and Sfi I. The 5144 bp fragment is gel purified and ligated together with the 2526 bp Sfi I/Xho I digested fragment and the Apa I and the Xho I digested CIAP treated pKS II+ plasmids. A clone is isolated having the Sindbis nucleotides I - 7643 including the RNA polymerase promoter at the 5' end contained in the pKSII+ plasmid. This construction is known as pKSII5'SIN.

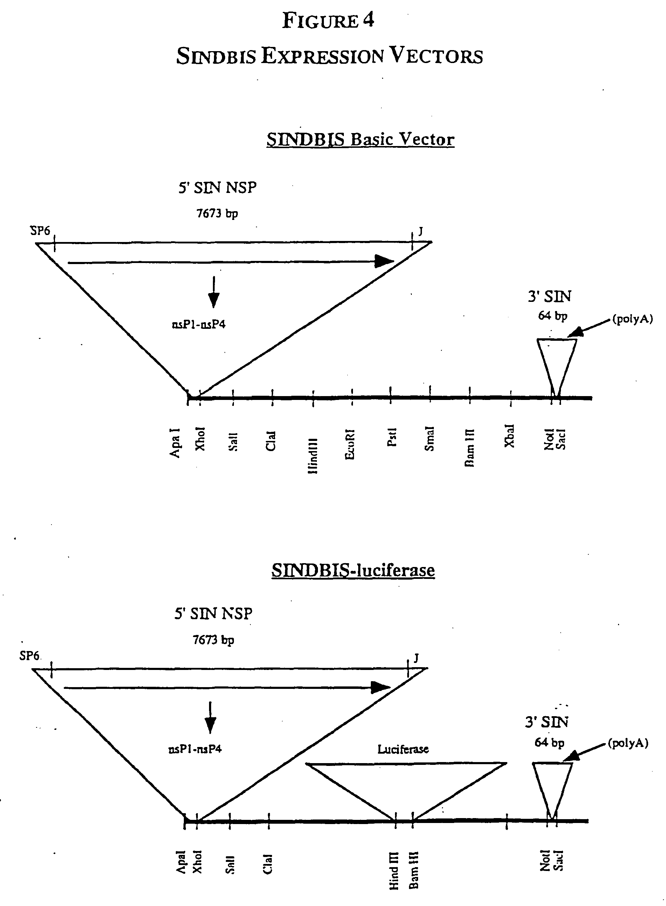

Assembly of the complete basic vector is accomplished by digesting pKS5'SIN with Xho I and Sac I, treating with CIAP, and gel purification of the large 10,533 bp fragment. The pKS5'SIN 10,533 bp fragment is ligated together with the 168 bp small fragment resulting from digestion of pKSII3'SIN with Xho I and Sac I. This construction is known as pKSSINBV, and is shown schematically in Figure 4.

The firefly luciferase reporter gene is inserted into the Sindbis basic vector in order to demonstrate the expression of a heterologous gene in cells transfected with RNA resulting from *in vitro* transcription of the Sindbis vector clone. This experiment demonstrates the overall functionality of the Sindbis vector.

### B. Construction of SINDBIS luciferase Vector

Construction of the Sindbis luciferase vector is performed by assembling together components of 3 independent plasmids: pKSII5'SIN, pKSII3'SIN, and pGL2-basic vector. The pGL2-basic vector plasmid (Promega, Madison, WI) contains the entire firefly luciferase gene. The luciferase gene is first inserted into the pKSII3'SIN plasmid. This is done by gel purification of the 2689 bp luciferase containing fragment resulting from digestion of pGL2-basic vector with Bam HI and Hind III, and ligation with the gel purified 3008 bp large fragment resulting from digestion with Bam HI and Hind III and treatment with CIAP of pKSII3'SIN. This construction is known as pKSII3'SIN-luc.

Final assembly of the Sindbis luciferase vector is accomplished by digesting pKS5'SIN with Xho I and Sac I, treating with CIAP, and gel purification of the large 10,533 bp fragment. The pKS5'SIN 10,533 bp fragment is ligated together with the 2854 bp small fragment resulting from digestion of pKSII3'SIN-luc with Xho I and Sac I. This construction contains the entire Sindbis nonstructural gene coding region and 3' viral elements necessary for genome replication. The firefly luciferase gene is placed between these two viral 5' and 3' elements. This vector is known as pKSSINBV-luc, and is shown schematically in Figure 4. The SIN-BV constructs between FfiI and SacI are exchanged into pGVELVIS between FfiI and XbaI sites. Expression of heterologous genes is observed after transfection onto BHK cells.

### C. Expression of luciferase in transfected and infected BHK cells

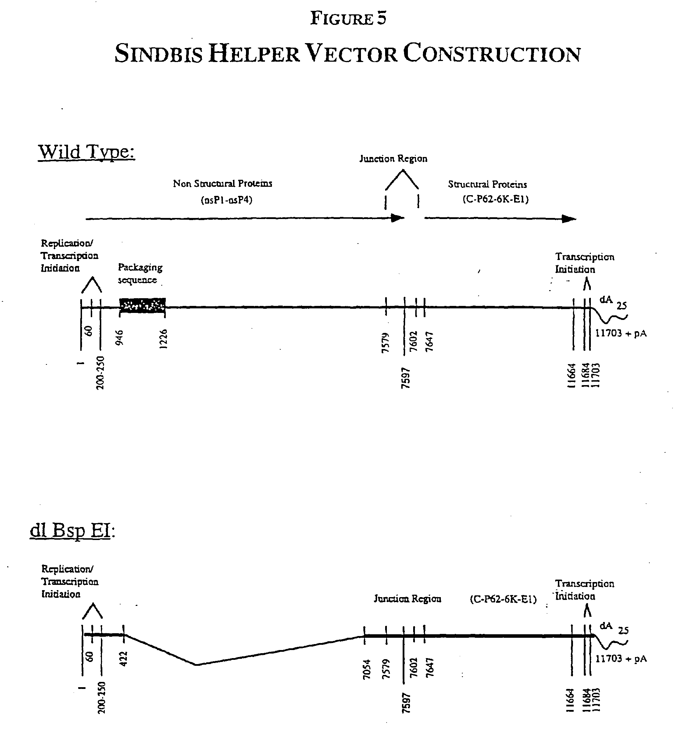

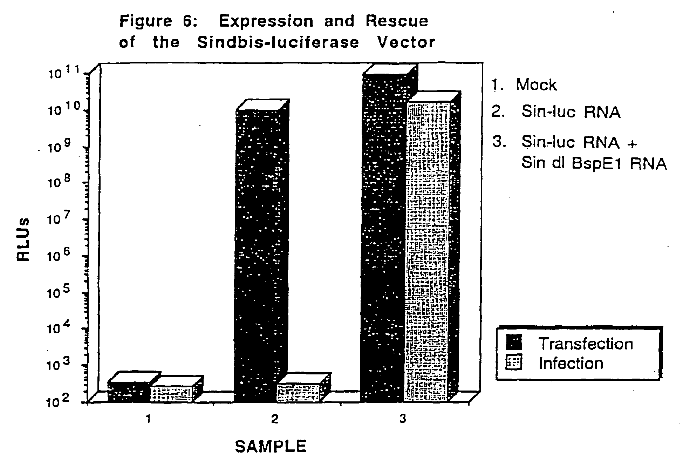

In order to test the functionality of the Sindbis basic vector, the expression of luciferase in cells transfected with RNA transcribed *in vitro,* as described in Example 1, from pKSSINBV-luc, linearized by digestion with Sac I. In addition, a complementary packaging vector, which is deleted of most of the non structural gene region, is constructed by digestion of pVGSP6GENrep with Bsp EI and ligated under dilute conditions. This construction is known as pVGSP6GENd1Bsp and is deleted of nonstructural gene sequences between bases 422-7,054. This clone is 8,008 bps and is shown schematically in Figure 5. Transcription *in vitro* of pVGSP6GENd1Bsp is as described in Example 1; linearization is with Xba I The expression of luciferase in transfected cells is tested at 24 hr. post transfection. Transfections and co-transfections are performed by complexing directly *in vitro* transcription products with lipofectin (Gibco-BRL, Gaithersberg, MD). Additionally, 1 ml of supernatant is used to infect a confluent monolayer of BHK cells and the expression of luciferase is tested at 24 hr. post infection. The results of this experiment are shown in Figure 6. The results demonstrate clearly abundant reporter gene expression after transfection of BHK cells, and transfer (e.g. packaging) of the expression activity when cells are cotransfected with *in vitro* transcribed pVGSP6GENd1Bsp.

### D. Construction of Altered Junction Region SINDBIS Vectors

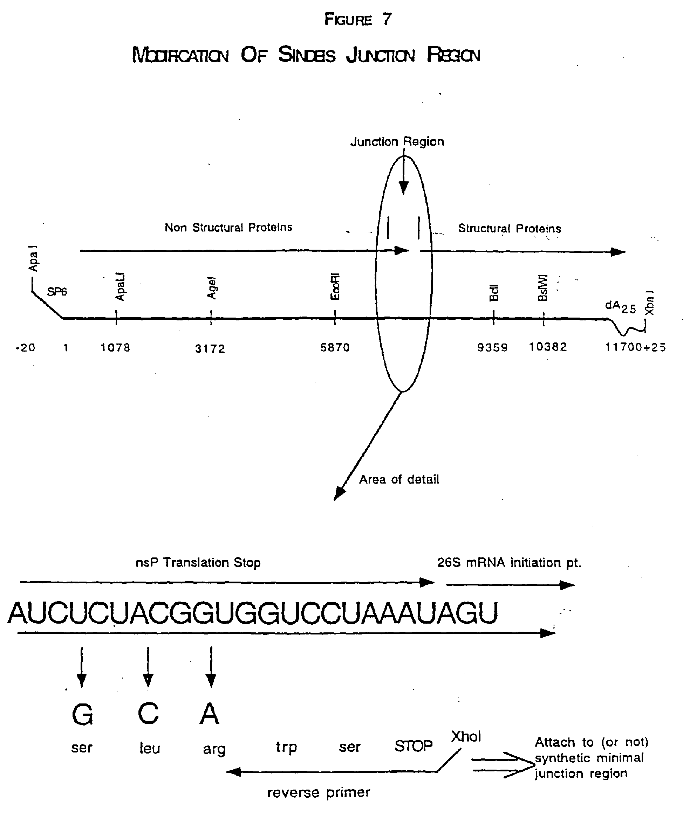

1. In order to inactivate the junction region, nucleotides within the NSP4 carboxy terminus and junction region overlap are changed, and the vector nucleotides corresponding to Sindbis are terminated prior to the subgenomic initiation point at Sindbis nt. 7598. This construction is shown schematically in Figure 7.
   Briefly, a fragment is PCR amplified from the pKSSINBV clone under nonstringent reaction cycle conditions utilizing a reverse primer having the following sequence:
   TATATGGGCCCTTAAGACCATCGGAGCGATGCTTTATTTCCCC
   The underlined bases in the reverse primer relate to nucleotide changes in the junction region without affecting the coded amino acid (see below). All of the nucleotide changes are transversions.
   3' end of NSP 4 (viral nts. 7580-7597):
   TCT CTA CGG TGG TCC TAA
   (resulting nt. changes from reverse primer)
   The reverse primer is complementary to Sindbis nts 7597-7566 (except at nucleotides, as shown, were junction region changes were made), and includes at its 5' end the 6 nucleotide Apa I recognition sequence following a 5' terminal TATAT tail 'buffer sequence' for efficient enzyme digestion.
   The forward primer in this reaction is primer 2A (described in Example 1), having the following sequence:
   ATACTAGCCACGGCCGGTATC

   The 4,464 bp amplicon resulting from a PCR reaction with pKSSfNBV using the primer pair described above is digested with Sfi I and Apa I and the gel purified 2,480 bp fragment is ligated together with the gel purified 5,142 bp fragment resulting from the digestion of pKSSINBV with Apa I and Sfi I, and with the gel purified 2,961 bp fragment resulting from the digestion of pKSII+ with Apa I and from the treatment with CIAP. This construction, comprised of Sindbis nucleotides 1 - 7597, including the changes in the junction region described above, and including the bacterial T7 promoter attached to Sindbis nt. 1 is referred to as pKS5'SINdIJR.
   Final construction of the inactivated junction region vector is accomplished by ligation of the 7,622 bp large Sindbis fragment resulting from digestion of pKS5'SINdIJR with Apa I, with the 3,038 bp fragment resulting from digestion with Apa I and treatment with CIAP of pKSII3'SIN. The positive orientation of the 5' Sindbis element, relative to the 3' Sindbis element, is confirmed by restriction endonuclease analysis. This construction is referred to as pKSSINBVdIJR.
   Initiation and synthesis of subgenomic mRNA can not occur from the pKSSINBVdIJR vector. In order to prove this supposition, comparative RNase protection assays the pKSSINBV and pKSSINBVdIJR vectors are performed. A ³²P end labeled RNA probe complementary in part to the junction region, including the subgenomic RNA initiation point at viral nt 7,598 is used to hybridize with the viral RNA resulting from the transfection of BHK-21 cells with the pKSSINBV and pKSSINBVdIJR vectors. The RNase protection assay demonstrates that cells transfected with pKSSINBV have two fragments, of genomic and subgenomic specificity, while cells transfected with pKSSINBVdIJR have only a single fragment of genomic specificity. These results prove that the junction region in the pKSSINBVdIJR vector is indeed inactivated.
2. In order to test that translation of genomic RNA from the region corresponding to the subgenomic RNA message, the luciferase reporter gene is inserted into the inactivated junction region vector pKSSINBVdIJR described above. This construction is accomplished by digesting with Xho I and Sac I and treating with CIAP the pKSSINBVdIJR plasmid and gel purifying the resulting 10, 197 bp fragment. The pKSSINBVdIJR fragment is ligated together with the 2854 bp small fragment resulting from digestion of pKSII3'SIN-luc with Xho I and Sac I. This construction contains the entire Sindbis nonstructural gene coding region terminating in an inactivated junction region at Sindbis nt. 7597, and 3' viral elements necessary for genome replication; the firefly luciferase gene is placed between these two viral 5' and 3' elements. This vector is known as pKSSINBVdIJR-luc.
   The expression of the reporter gene from the pKSSINBVdIJR-luc vector is tested in transfected BHK-21 cells. Translation of functional luciferase protein is determined by the luciferin luminescent assay, using a luminometer for detection. The sensitivity in this assay is 1 x 10-20 moles of luciferase. Given that the molecular weight of luciferase is 62,000 daltons, this limit of detection transforms to 6,020 molecules. Thus, in a typical experiment if only 0.6% of the 1 x 10⁶ cells contained in a 60 mM petri dish are transfected with the pKSSINBVdIJR-luc vector, and if these transfected cells express only a single functional molecule of luciferase, the enzymatic activity is detected by the assay used. It is important to demonstrate in this experiment that the junction region of the pKSSINBVdIJR-luc vector is inactivated. This is accomplished by an RNase protection assay, comparing the viral RNA's synthesized in cells transfected with the pKSSINBVdIJR-luc and the pKSSINBV-luc vectors, using the probe described above.
3. The minimal -19->+5 junction region core oligonucleotide pair, comprised of Sindbis nts. 7579-7602, as defined previously (Levis et al., J. Virol. 64:1726-1733, 1990), is synthesized *in vitro,* and flanked with Apa I and Xho I recognition sequences as shown:
   oligonucleotide 1:
      CATCTCTACGGTGGTCCTAAATAGTC
   oligonucleotide 2:
      TCGAGACTATTTAGGACCACCGTAGAGATGGGCC
   The oligonucleotides above are mixed together in the presence of 10 mM Mg²⁺, heated to 100°C for 5 min. and cooled slowly to room temperature. The annealed oligonucleotides are ligated at a 25:1 molar ratio of insert to the pKSSINBVdIJR vector, prepared accordingly: complete digestion with Xho I, followed by digestion with Apa I under partial conditions, resulting in one Apa I induced cleavage per molecule (of two cleavages possible), gel purification of the 10,655 bp fragment, and treatment with CIAP. This vector containing the entire NSP coding region which terminates in an inactivated junction region core, attached to a synthetic junction region core and followed by 3' viral elements required for replication, and contained in the pKSII+ plasmid, is known pKSSINdIJRsjrc.

In order to regulate the level of subgenomic mRNA synthesis, further modifications of the tandemly inserted synthetic junction region core in plasmid pKSSINdIJRsjrc are performed. These modifications of the junction region core are accomplished by two approaches; nucleotide changes within the junction region core, or extension at the 5' and 3' junction region core termini of flanking Sindbis nucleotides, according to the authentic viral sequence. The minimal junction region core, spanning viral nts. 7579 - 7602 is shown below:
ATCTCTACGGTGGTCCTAAATAGT

By comparing genomic sequence between eight alphaviruses, it has been shown previously that there is sequence diversity within the junction region core. Shown below, for particular junction region locations, is the Sindbis nucleotide followed by the corresponding nucleotide found in other alphaviruses:

| Nucleotide Number | Sindbis | Permissive Change |
|---|---|---|
| 7579 | A | C |
| 7580 | U | C |
| 7581 | C | U |
| 7583 | C | G |
| 7589 | U | C |
| 7590 | G | U |
| 7591 | G | A |
| 7592 | U | A |
| 7600 | A | U or G |
| 7602 | U | G or A |

Junction region changes at Sindbis nts. 7579, 7580, 7581, 7583, 7589, 7590, 7591, 7592, result in amino acid coding potential changes within all 5 codons of the carboxy terminus of NSP 4 which overlap in the junction region. These changes observed in the junction region between alphaviruses at the level of NSP 4 coding potential and at the level of junction region cis activity may represent either, or both, permissive changes in NSP 4 and the junction region which do not affect functionality, or on the other hand, simply different viruses. In any event, the junction region changes presented herein regard the tandemly inserted junction region core, from which no NSP protein synthesis occurs. Discussed above, translation of the entire NSP region occurs from the pKSSINBVdIJR construct. Junction region changes at Sindbis nts. 7600 and 7602 are downstream of the NSP 4 termination codon and upstream of the structural proteins initiation codon.

Locations of nucleotide differences within the junction region core observed between the several alphavirus strains are referred to here as permissive changes. Locations of nucleotides within the junction region core corresponding to conserved sequences between the several alphavirus strains are referred to here as nonpermissive changes.

To decrease the level of subgenomic mRNA initiation from the synthetic junction region core, changes are made separately within nucleotides corresponding to permissive changes, and within nucleotides corresponding to nonpermissive changes. Junction region nucleotides corresponding to permissive changes are given in the table above. Fourteen junction region nucleotides for which no changes are observed among the eight alphaviruses sequenced (Semliki Forest virus, Middleburg virus, Ross River virus, O'Nyong Nyong virus, Eastern Equine Encephalitis virus, Western Equine Encephalitis virus, and Venezuelan Equine Encephalitis virus) are given below:

| | Nucleotide Number: | |
|---|---|---|
| | 7582 | |
| | 7584 | |
| | 7585 | |
| | 7586 | |
| | 7587 | |
| | 7588 | |
| | 7593 | |
| | 7594 | |
| | 7595 | |
| | 7596 | |
| | 7597 | |
| | 7598 | |
| | 7599 | |
| | 7601 | |

Changes within the junction region observed among alpha viruses may reflect a specific interaction between a given alphaviral RNA polymerase and its cognate junction region. Thus, changes among the "permissive" nucleotides may result in as marked a decrease in the subgenomic mRNA synthesis levels as changes among the "nonpermissive" nucleotides of the junction region. On the other hand, these may indeed be sites of permissive change within the junction region core.

The single authentic nonpermissive change within the junction region core is likely Sindbis nt. 7598, corresponding to the subgenomic mRNA initiation point. Changes of this nucleotide in the tandemly inserted junction region core of plasmid pKSSINdIJRsjrc are not described here.

Substitution of the permissive nucleotides in toto in the synthetic minimal -19->+5 junction region core, is accomplished with the following oligonucleotide pair, synthesized *in vitro,* and flanked with Apa I and Xho I recognition sequences as shown:
oligonucleotide 1:
   CCCTTGTACGGCTAACCTAAAGGAC
oligonucleotide 2:
   TCGAGTCCTTTAGGTTAGCCGTACAAGGGGGCC

The oligonucleotides above are mixed together in the presence of to mM Mg, heated 100°C for 5 min. and cooled slowly to room temperature. The annealed oligonucleotides are ligated at a 25:1 molar ratio of insert to the pKSSINBVdIJR vector, prepared accordingly: complete digestion with Xho I, followed by digestion with Apa I under partial conditions, resulting in one Apa I induced cleavage per molecule (of two cleavages possible), gel purification of the 10,655 bp fragment, and treatment with CIAP. This vector is known as pKSSINdIJRsjrPc.

Each of the 13 (nt. 7598 not changed) nonpermissive nucleotides in the junction region core are changed individually, using the following rules, resulting in the most drastic transversional substitution:
A -> C
T -> G
G -> T
C -> A

For example, nt. 7582 is changed from T -> G, using the following oligonuelcotide pair, synthesized *in vitro*, and flanked with Apa I and Xho I recognition sequences as shown:
oligonucteotide 1:
   CATCGCTACGGTGGTCCTAAATAGTC
oligonucleotide 2:
   TCGAGACTATTTAGGACCACCGTAGCGATGGGCC

(Nucleotides effecting transversion in nonpermissive junction region sites shown in boldface type)
The oligonucleotides above are mixed together in the presence of 10 mM Mg²⁺, heated to 100°C for 5 min. and cooled slowly to room temperature. The annealed oligonucleotides are ligated at a 25:1 molar ratio of insert to the pKSSINBVdIJR vector, prepared accordingly: complete digestion with Xho 1, followed by digestion with Apa 1 under partial conditions, resulting in one Apa I induced cleavage per molecule (of two cleavages possible), gel purification of the 10,655 bp fragment, and treatment with CIAP. This vector is known pKSSINdIJRsjrNP7582.

Using the transversion change rules shown above, changes in each of the 12 remaining nonpermissive sites in the junction region core are made with 12 separate oligonucleotide pairs, flanked with Apa I and Xho I recognition sites, as described above. These vectors are known as:
pKSSINdIJRsjrNP7584
pKSSINdIJRsjrNP7585
pKSSINdIJRsjrNP7586
pKSSINdIJRsjrNP7587
pKSSINdIJRsjrNP7588
pKSSINdIJRsjrNP7593
pKSSINdIJRsjrNP7594
pKSSINdIJRsjrNP7595
pKSSINdIJRsjrNP7596
pKSSINdIJRsjrNP7597
pKSSINdIJRsjrNP7599
pKSSINdIJRsjrNP7601

In order to test the relative levels of subgenomic mRNA synthesis, the luciferase reporter gene is inserted into the modified tandem junction region vectors. This construction is accomplished by digesting with Xho I and Sac I and treating with CIAP the tandemly inserted synthetic junction region core vectors and gel purifying the resulting approximate 10,200 bp fragment. The thus-treated vector fragment is ligated together with the 2854 bp small fragment resulting from digestion of pKSII3'SIN-luc with Xho I and Sac I. These constructions contain the entire Sindbis nonstructural gene coding region terminating in an inactivated junction region at Sindbis nt. 7597, the tandemly inserted synthetic junction region core (modified or unmodified), the firefly luciferase gene, and 3' viral elements necessary for genome replication. The names of these vectors follows:

| Sindbis-luciferase vector | Tandemly Inserted Junction Region Modification |
|---|---|
| pKSSINdIJRsjrc-luc | not modified |
| pKSSINdIJRsjrPc-luc | permissive changes |
| pKSSINdIJRsjrNP7582-luc | nonpermissive change |
| pKSSrNdIJRsjrNP7584-luc | " |
| pKSSINdIJRsjrNP7585-luc | " |
| pKSSINdIJRsjrNP7586-luc | " |
| pKSSINdIJRsjrNP7587-luc | " |
| pKSSINdIJRsjrNP7588-luc | " |
| pKSSINdIJRsjrNP7593-luc | " |
| pKSSrNdIJRsjrNP7594-luc | " |
| pKSSINdIJRsjrNP7595-luc | " |
| pKSSINdIJRsjrNP7596-luc | " |
| pKSSINdIJRsjrNP7597-luc | " |
| pKSSINdIJRsjrNP7599-luc | " |
| pKSSINdIJRsjrNP7601-luc | " |

Assuming that the translation efficiencies are equivalent in all of the luciferase vectors shown immediately above, the relative levels of subgenomic synthesis are determined by comparing the levels of luciferase production at 16 h. post transfection of BHK-21 cells. The relative levels of subgenomic transcription are determined by comparing luciferase production by the vectors pKSSINBV-luc and pKSSINdIJRsjrc-luc with all of the modified junction region luciferase vectors shown above.

It is expected that the vectors containing the tandemly inserted synthetic junction region core (pKSSINdIJRsjrc, and derivatives thereof) will have a lower level of subgenomic mRNA expression, relative to the pKSSINBV construct. In certain embodiments, it may be necessary to increase the level of subgenomic mRNA expression observed from the pKSSINdIJRsjrc vector. This is accomplished by extension at the 5' and 3' synthetic junction region core termini with 11 additional flanking Sindbis nucleotides, according to the authentic viral sequence.

The synthetic oligonucleotide pair shown below is synthesized *in vitro.* and contains 46 Sindbis nts., including all 24 nts (shown in boldface type) of the minimal junction region core. The Sindbis nts. are flanked with the Apa I and Xho I recognition sequences as shown:
oligonucleotide 1:
   CGGAAATAAAGCATCTCTACGGTGGTCCTAAATAGTCAGCATAGT
oligonucleotide 2:
   TCGAGGTACTATGCTGACTATTTAGGACCACCGTAGAGATGCTTTA TTTC-CGGGC

The oligonucleotides above are mixed together in the presence of 10 mM Mg, heated to 100°C for 5 min. and cooled slowly to room temperature. The annealed oligonucleotides are ligated at a 25:1 molar ratio of insert to the pKSSINBVdIJR vector, prepared accordingly: complete digestion with Xho I, followed by digestion with Apa I under partial conditions, resulting in one Apa I induced cleavage per molecule (of two cleavages possible), get purification of the 10,655 bp fragment, and treatment with CIAP. This vector containing the entire NSP coding region which terminates in an inactivated junction region core, attached to an extended synthetic junction region, and followed by 3' viral elements required for replication, and contained in the pKSII+ plasmid, is known pKSSINdIJRsexjr.

In order to test the relative levels of subgenomic mRNA synthesis, the luciferase reporter gene is inserted into the extended tandem junction region pKSSINdIJRsexjr vector. This construction is accomplished by digesting with Xho I and Sac I and treating with CIAP the pKSSINdIJRsexjr plasmid and gel purifying the resulting approximate 10,200 bp fragment. The thus-treated vector fragment is ligated together with the 2854 bp small fragment resulting from digestion of pKSII3'SIN-luc with Xho I and Sac I. This construction contains the entire Sindbis nonstructural gene coding region terminating in an inactivated junction region at Sindbis nt. 7597, the tandemly inserted extended synthetic junction region, the firefly luciferase gene, and 3' viral elements necessary for genome replication. The name of this vector is pKSSINdIJRsexjr-luc.

The relative levels of subgenomic transcription are determined by comparing luciferase production by the pKSSINdIJRsexjr-luc vector with the pKSSINBV-luc and pKSSINdIJRsjrc-luc vectors.

### EXAMPLE 4

### A. INSERTION OF ADENOVIRUS EARTHY REGION E3 GENE INTO SINDBIS VECTORS

In order to inhibit the host CTL directed response against viral specific proteins expressed in vector infected cells in applications where repeated administration of the therapeutic is desired, the Adenovirus type 2 (Ad 2) E3/19K gene ATCC No. VR-846 is cloned into the pKSSINdIJRsjrc plasmid, immediately downstream from the junction region core.

Briefly, Ad 2 is propagated in a permissive cell line, for example HeLa or Vero cells, and after evidence of cytopathologic effects, virions are purified from the cell lysate, and the Ad 2 DNA is purified from the virus.

The Ad 2 DNA E3/19K gene, including the amino terminal signal sequence, followed by the intraluminal domain and carboxy terminal cytoplasmic tail which allows the E3 19K protein to embed itself in the endoplasmic reticulum, is located between viral nucleotides 28,812 and 29,288. Isolation of the Ad 2 E3 19K gene from the viral genomic DNA is accomplished by PCR amplification, with the primer pair shown below:
Ad 2 E3 Forward primer (Ad 2 nucleotides 28,812-28,835):
   5'-TAT ATC TCC AGA TGA GGT ACA TGA TTT TAG GCT TG-3' (SEQ. ID NO. 14)
Ad 2 E3 Reverse primer (Ad 2 nucleotides 29,241-29,213):
   5'-TAT ATA TCG ATT CAA GGC ATT TTC TTT TCA TCA ATA AAA C-3' (SEQ. ID NO. 15)

In addition to the Ad 2 complementary sequence, both primers contain a five nucleotide 'buffer sequence' at their 5' ends for efficient enzyme digestion of the PCR amplicon products. This sequence in the forward primer is followed by the Xho I recognition site, and in the reverse primer this sequence is followed by the Cla I recognition site. Thus, in the 5' to 3' direction, the E3/19K gene is flanked by Xho I and Cla I recognition sites. Amplification of the E3/19K gene from Ad 2 DNA is accomplished with the following PCR cycle protocol:

| Temperature (°C) | Time (Min.) | No. Cycles |
|---|---|---|
| 94 | 2 | 1 |
| 94 | 0.5 | |
| 55 | 0.17 | 5 |
| 72 | 3.5 | |
| 94 | 0.5 | 30 |
| 70 | 3.5 | |
| 72 | 10 | 10 |

Following amplification, the 451 bp amplicon is purified on a 1.5% agarose gel, and subsequently digested with the Xho I and Cla I enzymes and ligated into the CIAP treated pKSSINdIJRsjrc plasmid, previously digested with Xho I and Cla I. This clone is designated pKSSINdIJRsjrcAdE3. Using the same cloning strategy, the Ad 2 E3/19K gene is inserted into all of the modified synthetic junction region vectors described in Example 2.

### B. INSERTION OF THE HUMAN CYTOMEGALOVIRUS H301 GENE INTO SINDBIS VECTORS

In order to inhibit the host CTL directed response against viral specific proteins expressed in vector infected cells in applications where repeated administration of the therapeutic is desired, the human cytomegalovirus (HCMV) H301 gene is cloned into the pKSSINdIJRsjrc plasmid, immediately downstream from the junction region core.

Briefly, HCMV strain AD169 (ATCC No. VR-538), is propagated in a permissive cell line, for example primary human foreskin fibroblasts (HFF) (GIBCO/BRL, Gaithersburg, MD), and after evidence of cytopathologic effects, virions are purified from the cell lysate, and subsequently the HCMV DNA is purified from the virons.

The HCMV H301 gene is located between viral nucleotides 23,637 and 24,742. Isolation of the HCMV H301 gene from the viral genomic DNA is accomplished by PCR amplification, with the primer pair shown below:
HCMV H301 Forward primer (buffer sequence/Xho I site/ HCMV nucleotides 23,637-23,660):
   5'-TAT ATC TCC AGA TGA TGA CAA TGT GGT GTC TGA CG-3' (SEQ. ID NO. 16)
HCMV H301 Reverse primer (buffer sequence/Cla I site/HCMV nucleotides 24,744-24,722):
   5'-TAT ATA TCG ATT CAT GAC GAC CGG ACC TTG CG-3' (SEQ. ID NO. 17)

In addition to the HCMV H301 gene complementary sequences, both primers contain a five nucleotide 'buffer sequence' at their 5' ends for efficient enzyme digestion of the PCR amplicon products. This sequence in the forward primer is followed by the Xho I recognition site, and in the reverse primer this sequence is followed by the Cla I recognition site. Thus, in the 5' to 3' direction, the HCMV H301 gene is flanked by Xho I and Cla I recognition sites. Amplification of the HCMV H301 gene from HCMV DNA is accomplished with the following PCR cycle protocol:

| Temperature (°C) | Time (Min.) | No. Cycles |
|---|---|---|
| 94 | 2 | 1 |
| 94 | 0.5 | |
| 55 | 0.17 | 5 |
| 72 | 3.5 | |
| 94 | 0.5 | 30 |
| 70 | 3.5 | |
| 72 | 10 | 10 |

Following amplification, the 1,129 bp amplicon product is purified on a 1.0% agarose gel, and subsequently digested with the Xho I and Cla I enzymes and ligated into the CIAP treated pKSSINdIJRsjrc plasmid, previously digested with Xho I and Cla I. This clone is designated pKSSINdIJRsjrcH301. Using the same cloning strategy, the HCMV H301 gene is inserted into all of the modified synthetic junction region vectors described in Example 3.

### EXAMPLE 5

### EXPRESSION OF MULTIPLE HETEROLOGOUS GENES FROM SINDBIS VECTORS

The plasmid pBS-ECAT (Jang et al., J. Virol 63:1651, 1989) includes the 5' nontranslated region of Encephalomycarditis virus (EMCV) from nts 260-848 of the viral genome, which contains the internal ribosome entry site (IRES). EMCV nucleotides 260-827 are amplified from pBS-ECAT by PCR, using the following primer pair:
EMCV IRES Forward primer A (For insertion next to disabled junction region in vector pKSSINBVdIJR at Apa I site):
   5'-TAT ATG GGC CCC CCC CCC CCC CCC AAC G-3' (SEQ. ID NO. 18)
EMCV IRES Forward primer B (For insertion between heterologous genes terminating with Cla I sites and initiating with Nco I sites):
   5'-TAT ATA TCG ATC CCC CCC CCC CCC CCA ACG-3' (SEQ. ID NO. 19)
EMCV IRES Reverse Primer (To be used with either primers A or B):
   5'-TAT ATC CAT GGC TTA CAA TCG TGG TTT TCA AAG G-3' (SEQ. ID NO. 20)

The amplicon resulting from amplification with the forward primer A and the reverse primer is flanked by Apa I and Nco I recognition sites, inside a 5 bp 'buffer sequence'.

The amplicon resulting from amplification with the forward primer B and the reverse primer is flanked by Cla I and Nco I recognition sites, inside a 5 bp 'buffer sequence'.

Amplification of the EMCV IRES sequence from the pBS-ECAT plasmid is accomplished with the following PCR cycle protocol:

| Temperature (°C) | Time (Min.) | No. Cycles |
|---|---|---|
| 94 | 2 | 1 |
| 94 | 0.5 | |
| 55 | 0.17 | 5 |
| 72 | 3.5 | |
| 94 | 0.5 | 30 |
| 70 | 3.5 | |
| 72 | 10 | 10 |

For insertion into the pKSSINBVdIJR vector, the 589 bp amplicon is digested with Apa I and Nco I, purified on a 1% agarose gel, and ligated into the CIAP treated vector digested with Apa I and Nco I. The ATG corresponding to the start codon of the heterologous gene to be inserted immediately downstream of the EMCV IRES insert is modified to contain an Nco I site (CCATGG).

For insertion into the pKSSINBV or pKSSrNBVdIJRsjrc vectors between heterologous genes, the 589 bp amplicon is digested with Cla I and Nco I, purified on a 1% agarose gel, and ligated into the bicistronic heterologous gene vector digested with Cla I and Nco I and treated with CIAP. In a bicistronic heterologous gene configuration, the 3' end of the upstream heterologous gene is modified to terminate in a Cla I recognition site. The ATG corresponding to the start codon of the second downstream heterologous gene to be inserted immediately downstream of the EMCV IRES insert is modified to contain an Nco I site (CCATGG). Thus, from 5' to 3', the order of components is: pKSSINBV or pKSSINBVdIJRsjrc-gene #1-Cla/Nco EMCV IRES gene #2-3' SIN. Insertion into all of the modified junction region vectors described in Example 2 follows the strategy given here for the pKSSINBV or pKSSINBVdIJRsjrc vectors.

The pKSSINBVdIJR vector containing a bicistronic heterologous configuration is constructed with each of the EMCV IRES amplicons described above. The first EMCV IRES amplicon is flanked by Apa I and Nco I sites and is inserted immediately downstream of the disabled junction region at the Apa I site, as described above. This EMCV IRES sequence is followed by the first heterologous gene, which terminates in a Cla I recognition site. The first heterologous gene is followed by the second EMCV IRES sequence, using the amplicon flanked by Cla I and Nco I recognition sites. The second heterologous gene follows the second EMCV IRES sequence. Thus, from 5' to 3', the order of components is: SINBVdIJR-Apa/Nco EMCV IRES gene #1-Cla/Nco EMCV IRES #2-3' SIN..

The plasmid pP2-5' (Pelletier et al., Mol. Cell Biol. 8:1103. 1988) includes the 5' nontranslated region of the poliovirus P2/Lansing strain from nucleotides 1-1,872 of the viral genome, which contains the polio IRES. Poliovirus nucleotides 320-631 are amplified from pP2-5' by PCR, using the following primer pair:
Polio IRES Forward primer A (For insertion next to disabled junction region in vector pKSSINBVdIJR at Apa I site):
   5'-TAT ATG GGC CCT CGA TGA GTC TGG ACG TTC CTC-3' (SEQ. ID NO. 21)
Polio IRES Forward primer B (For insertion between heterologous genes terminating with Cla I sites and initiating with Nco I sites):
   5'-TAT ATA TCG ATT CGA TGA GTC TGG ACG TTC CTC-3' (SEQ. ID NO. 22)
Polio IRES Reverse Primer (To be used with either primers A or B):
   5'-TAT ATC CAT GGA TCC AAT TTG CTT TAT GAT AAC AAT C-3' (SEQ. ID NO. 23)

The amplicon resulting from PCR with the Polio IRES forward primer A/reverse primer pair shown above is flanked by Apa I and Nco I recognition sites, inside a 5 bp 'buffer sequence'.

The amplicon resulting from PCR with the Polio IRES forward primer B/reverse primer pair is shown above is flanked by Cla I and Nco I recognition sites, inside a 5 bp 'buffer sequence'.

Amplification of the polio IRES sequence from the pP2-5' plasmid is accomplished with the PCR protocol shown in Example 5:
For insertion into the pKSSINBVdIJR vector, the 333 bp amplicon is digested with Apa I and Nco I, purified on a 1.5% agarose gel, and ligated into the vector digested with Apa I and Nco I and treated with CIAP. The ATG corresponding to the start codon of the heterologous gene to be inserted immediately downstream of the polio IRES insert is modified to contain an Nco I site (CCATGG).

For insertion into the pKSSINBV or pKSSINBVdIJRsjrc vectors between heterologous genes, the 333 bp amplicon is digested with Cla I and Nco I. purified on a 1.5% agarose gel, and ligated into the bicistronic heterologous gene vector digested with Cla I and Nco I and treated with CIAP. In a biscistronic heterologous gene configuration, the 3' end of the upstream heterologous gene is modified to terminate in a Cla I recognition site. The ATG corresponding to the start codon of the second downstream heterologous gene to be inserted immediately downstream of the polio IRES insert is modified to contain an Nco I site (CCATGG). Thus, from 5' to 3', the order of components is: pKSSINBV or pKSSINBVdIJRsjrc-gene #1-Cla/Nco polio IRES gene #2-3' SIN. Insertion into all of the modified junction region vectors described in Example 3 follows the strategy given here for the pKSSINBV or pKSSINBVdIJRsjrc vectors.

The pKSSINBVdIJR vector containing a bicistronic heterologous configuration is constructed with each of the polio IRES amplicons described above. The first polio IRES amplicon is flanked by Apa I and Nco I sites and is inserted immediately downstream of the disabled junction region at the Apa I site, as described above. This polio IRES sequence is followed by the first heterologous gene, which terminates in a Cla I recognition site. The first heterologous gene is followed by the second polio IRES sequence, using the amplicon flanked by Cla I and Nco I recognition sites. The second heterologous gene follows the second polio IRES sequence. Thus, from 5' to 3', the order of components is: SINBVdIJR-Apa/Nco polio IRES gene #1-Cla/Nco EMCV IRES #2-3' SIN.

The 220 bp BiP cDNA, corresponding to the 5' leader region of the human immunoglobulin heavy-chain binding protein mRNA, is amplified from the clone pGEM5ZBiP5', using PCR. The sequence corresponding to BiP cDNA was determined originally in the bacteriophage lambda hu28-1 clone of the human GRP78 gene (Ting and Lee, DNA 7:275-286, 1988). The forward primer to be used in the PCR reaction varies, depending on the Sindbis vector into which the BiP cDNA is inserted. The reverse primer for the PCR reaction is the same for all Sindbis vectors. Amplification of the BiP cDNA sequence from pGEM5ZBiP5' from the plasmid for insertion into the Sindbis vector pKSSINBVdIJR, immediately downstream of the disabled junction region, is accomplished by amplification with the following forward primer:
5'-TAT ATG GGC CCG GTC GAC GCC GGC CAA GAC-3' (SEQ. ID NO. 24)

In addition to the BiP cDNA complementary sequences, beginning at nucleotide 12, the primer contains a five nucleotide 'buffer sequence' at its 5' end for efficient enzyme digestion of the PCR amplicon products. This sequence is followed by the Apa I recognition site.

Amplification of the BiP cDNA sequence from the pGEM5ZBiP5' plasmid for insertion into the Sindbis vectors pKSSINBV, or pKSSrNBVdIJRsjrc, is accomplished by amplification with the following forward primer shown below. For these vectors, the BiP cDNA is inserted between two heterologous genes, which are placed in the region corresponding to the Sindbis structural genes.
5'-TAT ATA TCG ATG GTC GAC GCC GGC CAA GAC-3' (SEQ. ID NO. 25)

In addition to the BiP cDNA complementary sequences, beginning at nucleotide 12, the primer contains a five nucleotide 'buffer sequence' at its 5' end for efficient enzyme digestion of the PCR amplicon products. This sequence is followed by the Cla I recognition site.

The reverse primer for amplification of the BiP cDNA sequence from the pGEM5ZBiP5' plasmid for insertion into the Sindbis vectors pKSSINBVdIJR, pKSSINBV, or pKSSINBVdIJRsjrc, is:
5'-TAT ATC CAT GGT GCC AGC CAG TTG GGC AGC AG-3' (SEQ. ID NO. 26)

In addition to the BiP cDNA complementary sequences, beginning at nucleotide 12, the reverse primer contains a five nucleotide 'buffer sequence' at its 5' end for efficient enzyme digestion of the PCR amplicon products. This sequence is followed by the Nco I recognition site.
Amplification of the BiP cDNA from the pGEM5ZBiP5' is accomplished with PCR protocol that are described above:

For insertion into the pKSSINBVdIJR vector, the 242 bp amplicon is digested with Apa I and Nco I, purified on a 2% agarose gel, and ligated into the vector digested with Apa I and Nco I and treated with CIAP. The ATG corresponding to the start codon of the heterologous gene to be inserted immediately downstream of the BiP cDNA insert is modified to contain an Nco I site (CCATGG).

For insertion into the pKSSINBV or pKSSINBVdIJRsjrc vectors between heterologous genes, the 242 bp amplicon is digested with Cla I and Nco I, purified on a 2% agarose gel, and ligated into the bicistronic heterologous gene vector digested with Cla I and Nco I and treated with CIAP. In a biscistronic heterologous gene configuration, the 3' end of the upstream heterologous gene is modified to terminate in a Cla I recognition site. The ATG corresponding to the start codon of the second downstream heterologous gene to be inserted immediately downstream of the BiP cDNA insert is modified to contain an Nco I site (CCATGG). Thus, from 5' to 3', the order of components is: pKSSINBV or pKSSINBVdIJRsjrc-gene #1-Cla/Nco BiP-gene #2-3' SIN. Insertion into all of the modified junction region vectors described in Example 2 follows the strategy given here for the pKSSINBV or pKSSINBVdIJRsjrc vectors.

The pKSSINBVdIJR vector containing a bicistronic heterologous configuration is constructed with each of the BiP cDNA amplicons described above. The first BiP cDNA amplicon is flanked by Apa I and Nco I sites and is inserted immediately downstream of the disabled junction region at the Apa I site, as described above. This BiP sequence is followed by the first heterologous gene, which terminates in a Cla I recognition site. The first heterologous gene is followed by the second BiP cDNA sequence, using the amplicon flanked by Cla I and Nco I recognition sites. The second heterologous gene follows the second BiP sequence. Thus, from 5' to 3', the order of components is: SINBVdIJR-Apa/Nco BiP-gene #1-Cla/Nco BiP-gene #2-3' SIN.

Sequences which promote ribosomal readthrough are placed immediately downstream of the disabled junction region in the pKSSINBVdIJR vector, which allows ribosomal scanning in genomic mRNA from non-structural gene termination to the heterologous genes. The heterologous proteins are expressed from genomic length mRNA by ribosomal scanning. This should extend the life of the infected target cell because no subgenomic transcription occurs in cells infected with this vector. Further, these same ribosomal scanning sequences are placed between heterologous genes contained in polycistronic subgenomic mRNAs. The ribosomal spanning sequence to be used in the pKSDINBVdIJR vector and between heterologous genes in the polycistronic mRNA region is:
5'-TTA ATT AAC GGC CGC CAC CAT GG-3' (SEQ. ID NO. 27)

The boldfaced codons refer to the ochre stop codon and AUG start codon, respectively. The bases underlined surrounding the stop codon refer to the Pac I recognition site and the bases underlined surrounding the start codon refer to the Nco I recognition site. The intercistronic distance of 15 bp between the start and stop codons allows efficient ribosomal readthrough, as shown previously (Levine et al., Gene 108:167-174, 1991). The sequences surrounding the ATG start codon from bases -9 to +1 conform to the Kozak consensus sequence for efficient translational initiation (Kozak, Cell 44:283-292, 1986). Where possible, the 3' terminal nucleotide corresponding to the carboxy terminal amino acid is changed to T, by site-directed mutagenesis. Also, the 5' terminal nucleotide corresponding to the amino terminal amino acid in the downstream cistron is changed to G, by site-directed mutagenesis.

Insertion of the intercistronic sequence between heterologous genes, or downstream of the disabled junction region in vector pKSDINBVdIJR, modified as described above, is accomplished by insertion of the double-stranded oligonucleotide pair shown below, into compatible Pac I/Nco I ends:
Read through sense Oligonucleotide:
   5'-TAA CGG CCG CCA C-3' (SEQ. ID NO. 28)
Read through antisense Oligonucleotide:
   5'-CCA TGG TGG CGG CCG TTA AT-3' (SEQ. ID NO. 29)

The oligonucleotides above are mixed in equal molar quantities in the presence of 10 mM Mg Cl₂ heated at 95°C for 5 min, then allowed to cool slowly to room temperature, yielding the desired intercistronic sequence flanked by Pac I and Nco I sites. The intercistronic sequence is then ligated into the appropriate vector containing Pac I and Nco I compatible sites.

### EXAMPLE 6

### EXPRESSION OF MULTIPLE HETEROLOGOUS GENES BY COPACKAGING

As noted above within one aspect of the disclosure, Sindbis non-structural protein genes and structural protein genes can be copackaged as separate positive-sense RNA molecules which maintain their replication-competence, if each of the RNAs contain cis-acting sequences required for replication and packaging. Therefore, within this aspect all copackaged Aura virus RNA fragments should also contain a 5' sequence which is capable of initiating transcription of Aura RNA, an Aura virus RNA polymerase recognition sequence, and at least one copy of the RNA packaging sequence. At least one of the co-packaged RNA molecules must contain the sequences which encode Aura virus nonstructural proteins. Within preferred embodiments of the disclosure, one or more of the RNA fragments to be copackaged also will contain a viral junction region followed by a heterologous gene.

### A. CONSTRUCTION OF COPACKAGED EXPRESSION CASSETTES FOR EXPRESSION OF MULTIPLE

### 1. Heterologous Genes

In order to demonstrate the feasibility of copackaging to allow for the expression of multiple heterologous genes, two vector constructs are created. The first construct consists of a 5' sequence that is capable of initiating transcription of Sindbis virus RNA, Sindbis RNA sequences required for packaging, sequences encoding the synthesis of nonstructural proteins 1-4, a Sindbis junction region, the luciferase gene, and Sindbis 3' sequences required for synthesis of the minus strand RNA. The second construct consists of a 5' sequence that is capable of initiating transcription of a Sindbis virus, a Sindbis Junction region, Sindbis sequences required for packaging, Sequences encoding the LacZ gene and Sindbis 3' sequences required for synthesis of the minus strand RNA. RNA transcripts of these constructs transfected into a packaging cell line are copackaged to produce a vector particle capable of transferring expression of both luciferase and β-galactosidase into the same eukaryotic cell.

The β-galactosidase reporter gene- is inserted into the Sindbis Basic Vector (pKSSINBV) followed by deletion of a portion of the Sindbis non-structural proteins from the vector. RNA from this construct is cotransfected with RNA from Sindbis Luciferase Vector (pKSSINBV-luc) and is copackaged by one of the methods described below. Infection of fresh BHK-21 cells with vector particles containing the copackaged RNA expression cassettes should result in the expression of both luciferase and β-galactosidase in the same cell.

### B CONSTRUCTION OF β-GALACTOSIDASE EXPRESSION CASSETTE

The lacZ gene is obtained by digestion of pSV-β-galactosidase vector DNA (Promega Corp., Madison, WI) with the enzymes HindIII and SAlI. The 3749 bp fragment containing the lacZ gene is purified in a 1% agarose gel. Subsequently, this fragment is ligated into pSP72 plasmid (Promega Corp.) that is also digested with HindIII and SalI and purified using Geneclean (Bio101 Corp., San Diego, CA). This construct is known as pSP72-lacZ. Plasmid pSP72 is digested with the enzymes XhoI and XbaI, and the 3767bp lacZ-containing fragment is purified in a 1% agarose gel. This fragment is then ligated into pKSSINBV that also is digested with XhoI and XbaI and purified using Geneclean. This Sindbis construct, containing lacZ, is known as pKSSINBV-lacZ. Plasmid pKSSINBV-lacZ is subsequently digested with the enzyme AgeI, which cuts at nucleotides 3172 and 6922 of the Sindbis nonstructural protein gene sequences. The remaining vector fragment is purified by Geneclean and its ends re-ligated. The plasmid now has a 3750 bp deletion in the Sindbis nonstructural protein genes, which functionally inactivates them. This construct is known as pKSSINBVdINSP-lacZ.

SP6 transcripts of pKSSINBVdINSP-lacZ and pKSSINBV-luc are prepared as described above. These RNA transcripts are cotransfected into packaging cells that express the Sindbis structural proteins by one of the mechanisms described previously. Each RNA transcript contains a 5' sequence that is capable of initiating transcription of a Sindbis virus, RNA sequences required for packaging, a Sindbis junction region, a reporter gene, and Sindbis 3' sequences required for synthesis of the minus strand RNA. The pKSSINBV-luc transcript also contains the Sindbis non-structural proteins. In cotransfected cells, both RNA transcripts are replicated and a some of viral particles will contain both RNA transcripts copackaged into the same particle. Infection of fresh cells with the copackaged RNA particles will result in cell that express both luciferase and β-galactosidase.

### C. COPACKAGING OF MULTIPLE EXPRESSION CASSETTES TO INCREASE PACKAGING CAPACITY

Large genes such as Factor VIII might benefit from copackaging. Insertion of the cDNA coding for Factor VIII into the Sindbis Basic Vector (pKSSINBV) results in an RNA transcript approaching 16 kb in length. Because of the increased length, this RNA might not be replicated or packaged efficiently. Using approaches described above, the Sindbis nonstructural proteins and the Factor VIII gene could be divided onto separate RNA molecules of approximately 8 kb and 9 kb in length, and copackaged into the same particles.

### D. CONSTRUCTION OF A FACTOR VIII EXPRESSION CASSETTE

The pKSSINBV construct is digested with the enzyme SacI, which cleaves immediately after the Sindbis 3'-end and poly A sequence. The protruding 3'-ends are made blunt by the addition of the enzyme T4 DNA Polymerase and dNTPs and incubation for 10 minutes at 16°C. The digested fragment is purified using Geneclean and ligated to a 12 nucleotide self-complementary linker (5'-GTCACCGGTGAC-3') (SEQ. ID NO. 30) containing the SgrAI recognition site. This step is necessary because the Factor VIII gene contains several SacI sites, and the SgrAI recognition site is substituted for the Sac I site in order to create a site for linearization of the plasmid prior to SP6 transcription. This construct is known as pKSSINBV-SgrAI. The pKSSINBV-SgrAI construct is digested with the enzymes XbaI and NotI, and purified by using Geneclean. The Factor VIII cDNA sequence is obtained by digestion with the enzymes XbaI and NotI. The 8kb fragment encoding Factor VIII is purified in a 1% agarose gel and subsequently ligated to the Xba I/ Not I digested pKSSINBV-SgrA I. This construct is known as pKSSINBV-Factor VIII.

The pKSSINBV-Factor VIII construct is digested with Age I, which cuts in the Sindbis nonstructural proteins at nucleotides 3172 and 6922, purified by using Geneclean, and religated to itself. The construct now has a 3750 bp deletion in the Sindbis nonstructural proteins which functionally inactivate them. This construct is known as pKSSINBVdINSP-Factor VIII. SP6 transcripts of pKSSINBVdINSP-Factor VIII and of pKSSINBV are prepared as described above. These RNA transcripts are cotransfected into packaging cells that express the Sindbis structural proteins by one of the mechanisms described above. Both RNA transcripts contain a 5' sequence that is capable of initiating transcription of Sindbis RNA, sequences required for RNA packaging, a Sindbis Junction region, and the Sindbis 3' sequences required for synthesis of the minus strand RNA. In addition, the pKSSINBV transcript contains the Sindbis nonstructural protein genes, and the pKSSINBV-Factor VIII construct contains the Factor VIII gene, but not the Sindbis nonstructural protein genes. In cotransfected cells, both RNA transcripts are replicated and some viral particles will contain both RNA transcripts copackaged into the same vector particle. Infection of fresh BHK-21 cells with the copackaged RNA will result in Factor VIII expression only if both RNA molecules are present in the same cell.

### E. CONSTRUCTION OF AN AURA VIRUS COPACKAGING VECTOR

To develop Aura virus expression systems analagous to those described for Sindbis, standard techniques known in the art, as well as specific approaches described within this patent, will be utilized for constructions. Virus will be obtained from ATCC, propagated cultured cells, its virion RNA extracted, and cDNA spanning the entire genome synthesized and cloned using conventional techniques. This cDNA then will be used to construct gene transfer vector systems similar in principal to those described for Sindbis patent, including, but not limited to, a replicon capable of carrying the heterologous gene(s), packaging cell lines that express the structural protein genes, and unique to this system, a separate packaging-competent subgenomic vector capable of carrying the additional heterologous gene(s). Since Aura virus subgenomic RNA contains a packaging signal, preliminary experiments must be performed to identify this sequence, in order to prevent its inactivation during replacements with heterologous the gene(s). After identification of the packaging sequence, the individual elements of this Aura-based system will be generated.

A basic replicon vector will be constructed to contain the following minimum requirements: Aura 5' sequences necessary for replication, nonstructural protein coding regions, a modified or unmodified junction region for subgenomic mRNA synthesis, a multiple cloning site for insertion of heterologous gene(s), one or more copies of the packaging signal, and 3' Aura sequences necessary for replication, including a polyadenylate sequence. An upstream bacteriophage RNA polymerase promoter will be utilized for *in vitro* transcription of replicon RNA; alternatively, a eukaryotic RNA polymerase promoter will be utilized for transcription directly from cDNA.

A packaging-competent subgenomic vector will be constructed to contain the following minimum requirements: a modified or unmodified junction region, a multiple cloning site for insertion of heterologous gene(s), one or more copies of the packaging signal, and 3' Aura sequences necessary for replication/minus-strand synthesis, including a polyadenylate sequence. The subgenomic vector may, in some cases, be constructed with the Aura 5' replication sequences positioned upstream of the junction region, such that the vector will function as an amplicon. Transcription of subgenomic vector RNA will be accomplished *in vitro* using a bacteriophage RNA polymerase promoter, or cDNA *in vivo* using a eukaryotic RNA polymerase promoter. Further, the initial transcript may be of the sense-configuration or of the antisense-configuration.

Packaging cell lines will be constructed as described previously for Sindbis vectors, such that mRNA for one or more of the structural proteins will be transcribed from the junction region and be inducible by the Aura replicon. In other cases, one or more of the structural proteins will be expressed under the control of an inducible or constitutive eukaryotic promoter. In each case, specific inactivating mutations will be made in any packaging sequences present in the structural protein genes, in order to prevent encapsidation of these sequences with the replicon. These mutations will be silent changes, usually at the third position of the codon, which do not affect the amino acid encoded.

The ability to package multiple heterologous genes will be exploited for many therapeutic applications, which include, but are not limited to, expression of multiple cytokines, multiple CTL epitopes, combinations of cytokines and CTL epitopes to enhance immune presentation, multiple subunits of a therapeutic protein, combinations of therapeutic proteins and antisense RNAs, etc. In addition to its utility for the expression of multiple heterologous genes, the packaging of subgenomic mRNAs into virions also will enable this vector system for the transfer of extremely long heterologous sequences, which might be impossible in other alphavirus systems. Furthermore, this multipartite approach may be useful in the development of producer cell lines, whereby replicase proteins and structural proteins are being stably expressed, and any heterologous gene contained within a subgenomic vector could then be readily introduced as a stable integrant.

### EXAMPLE 7

### CONSTRUCTION OF SINDBIS VIRUS PACKAGING CELL LINES

One further embodiment of the Sindbis gene transfer system prelates to the development of Sindbis packaging cell lines. Since the normal Sindbis replication cycle takes place entirely in the cytoplasm, one approach for creating a Sindbis packaging cell line system is to model the system after it's natural replication cycle. Using this approach, the Sindbis packaging cell line system is designed whereby the viral structural proteins, supplied in *trans* from one or more stably integrated expression vectors, are able to encapsidate transfected or transduced vector RNA transcripts in the cytoplasm and release infectious packaged vector particles through the cell membrane, thus creating a Sindbis vector producer cell line. The Sindbis RNA vector molecules, capable of replicating in the cytoplasm of the cell, are initially produced by a T7 RNA polymerase system used to transcribe *in vitro* a cDNA vector clone encoding the gene of interest and the Sindbis nonstructural proteins (described previously). Vector RNA transcripts are then transfected into the Sindbis packaging cell line, such that the vector RNA replicates to high levels, and is subsequently packaged by the viral structural proteins, yielding infectious vector particles. Because of the extended length of the Sindbis cDNA molecule, the *in vitro* transcription process is inefficient. Further, only a fraction of the cells contained in a monolayer are typically transfected by most procedures. In an effort to optimize vector producer cell line performance and titer, two successive cycles of gene transfer are performed. Rather than directly transfecting the Sindbis RNA vector molecules into the producer cell line, the vector is first transfected into a primary Sindbis packaging cell line. The transfected cell line secretes infectious vector particles into the culture supernatants and these infectious supernatants are then used to transduce a fresh monolayer of Sindbis packaging cells. Transduction of the Sindbis vector into the packaging cell line is preferred over transfection because of its higher RNA transfer efficiency into cells, and optimized biological placement of the vector in the cell. This leads to higher expression and higher titer of packaged infectious recombinant Sindbis vector.

In the instance that the produced Sindbis vector particles fail to transduce the same packaging cell line because the cell line produces extracellular envelope proteins which block cellular receptors for Sindbis vector attachment, a second type of Sindbis viral particle must be created which is able to transduce the Sindbis packaging cells. This second type of viral particle must be produced by a packaging cell line known as a "hopping cell line", which produces transient vector particles as the result of being transfected with *in vitro* transcribed Sindbis RNA vector transcripts. The hopping cell line is engineered to redirect the receptor tropism of the transiently produced vector particles by providing alternative viral envelope proteins which redirect Sindbis vectors to different cellular receptor in a process termed pseudotyping. Currently two approaches have been devised for Sindbis vector particle pseudotyping. The first approach consists of a Sindbis packaging cell line co-expressing the vesicular stomatitis virus G protein (VSV-G). In our hands, VSV-G pseudotyping previously has worked well for redirecting the receptor tropism of retroviral vectors and has been demonstrated to infect a wide variety of cell types. The second approach for producing a pseudotyped Sindbis vector particle is to use currently available retroviral packaging cell lines containing retroviral gag/pol and env sequences which would be capable of packaging a Sindbis RNA vector containing a retroviral packaging sequence.

Modeling the Sindbis packaging cell line after the natural replication cycle of Sindbis virus should result in a cell line yielding high levels of gene expression based on the number of RNA molecules available for translation that are generated from the replicating RNA molecule. On the other hand, positive-stranded RNA viruses tend to produce defective-interfering RNAs which may tend to alter the RNA vector, thus decreasing the overall effectiveness of the vector particles, and which may also decrease the high levels of expression during extended culture periods. Therefore, a second approach has been devised in which a stably integrated DNA expression vector is used to produce the Sindbis vector RNA molecule, which, as in the first approach, maintains the ability to self replicate. This approach allows for continuous vector expression over long periods of culturing because the integrated DNA vector expression system is maintained through a drug selection marker and the DNA system will constitutively express unaltered RNA vectors which cannot be diluted out by defective RNA copies. In this producer cell configuration, the DNA-based Sindbis vector is introduced initially into the packaging cell line by transfection, since size restrictions could prevent packaging of the expression vector into a viral vector particle for transduction. Also, for this configuration, the T7 RNA polymerase recognition site of the plasmid, previously used to transcribe vector RNA. is replaced with another appropriate promoter sequence defined by the parent cell line used. This plasmid sequence also would contain a selection marker different from those used to create the packaging cell line.

The expression of Sindbis proteins and/or replicon RNA at certain levels may result in cytotoxic effects in packaging cell lines. Therefore, in some instances, it may be desirable for these elements to be expressed only after the cells have been propagated to a certain critical density. For this purpose, additional modifications are made whereby the structural proteins necessary for packaging are synthesized only after induction by the RNA vector itself or some other stimulus. Also, some modifications allow for the individual expression of these proteins under the control of separate inducible elements, by utilizing expression vectors which unlink the genes encoding these proteins. In addition, expression of the integrated vector molecule itself may be controlled by yet another inducible system. This configuration results in a cascade of events following induction, that ultimately leads to the production of packaged vector particles.

### A. CHOOSING A PARENT CELL LINE FOR SINDBIS PACKAGING CELL LINE DEVELOPMENT

### 1. Persistently or chronically infectable cells

One important criteria for selecting potential parent cell lines to create Sindbis packaging cell lines, is the choice of cell lines that are not lysed during Sindbis vector replication and production. This criteria is essential for the development of a Sindbis vector producing cell line which can be propagated for long periods of time and used as a stable source of vector. It is known that Sindbis infection of most mammalian cells results in lysis of the cell; however, the utilization of various insect cell lines should circumvent this problem. As an example, infection of mosquito Aedes albopictus cells with Sindbis virus results in persistent or chronic noncytopathic virus growth in which infected cells remain viable and continually shed virus. Other insect cell lines such as Aedes aegypti, Spodoptera frugiperda, and Drosophila melanogaster cell lines also should exhibit the same persistent infection. Therefore, the first Sindbis packaging cell line configuration uses an insect parent cell line, such as the Aedes albopictus or Drosophila cell line, containing a stably transfected expression vector which expresses the Sindbis structural proteins under the control of inducible or non-inducible promoters active in these cell types, and co-expressing a selectable marker.

Recently, a Sindbis virus-induced protein of cellular origin, which has been associated with the down-regulation of Sindbis virus production in infected Aedes albopictus cells, has been identified and purified (*Virology 194*:44)*.* The protein is a small hydrophobic peptide of approximately 3200 Da., which can induce the antiviral state and inhibit both 49S and 26S viral RNA synthesis. Cells treated with the antiviral peptide usually demonstrate quiescent arrest of cellular division for 96 hours in uninfected cells, and then normal growth rates are restored. Cells that have been exposed to this peptide prior to infection are unable to replicate Sindbis virus and appear to maintain this phenotype by constitutively producing the antiviral protein through 10 months of continuous passage.

It is recognized that this cellular response to Sindbis infection in Aedes albopictus cells might decrease the optimal efficiency of a recombinant Sindbis vector producing system. To improve the efficiency of Sindbis vector production, two methods have been devised to inactivate the virus-induced cellular antiviral protein, thus preventing any reduction of vector particle titers. The first method entails purification of this cellular protein described above, and determination of a portion of the primary amino acid sequence from its carboxy terminus using established techniques known in the art. The resulting amino acid sequence is then used to derive possible corresponding genomic sequences, enabling one to design a degenerate PCR primer pair which can be used to amplify the specific cellular sequence. This amplified sequence is then cloned using standard techniques know in the art, to obtain a discreet region of the gene encoding this inhibitory protein. Determination of the nucleotide sequence of this clone then enables one to design a vector which will integrate specifically within this Sindbis inhibitory gene by homologous recombination, and "knock out" its capacity to express a functional protein. Clones which contain the knock out sequence may be selected by insertion of a selectable marker into the discreet cloned region of the inhibitory protein, prior to transfecting cells with the vector.

A second method for disabling this Sindbis virus inhibitory protein involves mutagenesis of Aedes albopictus cells with, for example, BUDR (5-bromodeoxyuridine). This mutagenized packaging cell line population is then infected with a Sindbis vector, which is able to express the neomycin resistance marker. Under high concentrations of the G418 drug, only those cells producing large amounts of Sindbis vector, and thus unable to express the Sindbis inhibitory gene, will be able to survive. After selection, resistant colonies are pooled, dilution cloned, and tested for high titer Sindbis production

### 2. Modification of cells to decrease susceptiblity to Sindbis expression:Suppression of apoptosis

Although most mammalian cell lines typically are lysed during Sindbis virus infection, recent experiments have demonstrated the conversion of lytic to persistent Sindbis infection in a rat prostatic adenocarcinoma (AT-3) cell line. This conversion was performed by constituitively expressing the bcl-2 oncogene product in the cell line prior to Sindbis infection. Sindbis infection of AT-3 cells expressing the bcl-2 oncogene results in production of virus without obvious cytopathology (Nature 361:739). This cell line modification could prove useful for converting cell lines such as canine cell lines D-17 and Cf2; human cell lines HT1080 and 293; quail cell line QT-6; baby hamster kidney cell line BHK-21; mouse neuroblastoma cell line N18; and the rat prostatic adenocarcinoma AT-3 to the persistently infectable state for use as potential Sindbis packaging and producer cell lines, similar to those of retrovector producer lines.

A bcl-2 oncogene retroviral expression vector has been described previously (Nature 361:739). A new bcl-2 expression vector is constructed by using standard recombinant DNA techniques known in the art to insert the 910 base pair EcoRI cDNA fragment derived from the plasmid p84 (Nature 336:259) into any commercially available expression vector containing a constitutive promoter and encoding a selectable marker. Careful consideration must be taken to avoid any type of homology between Sindbis nucleic acid sequences and other transduced vectors. This precaution should be taken in order to prevent recombination events which may lead to undesirable packaging of selectable markers or the bcl-2 oncogene in recombinant Sindbis particles. This is an important point to make since the Sindbis vector system described herein is designed for use as a biological therapeutic. Once the bcl-2 expression vector is constructed, the parent mammalian cell line (i.e., BHK-21 cells) is transfected using any standard technique and selected for the appropriate marker. The resistant colonies are then pooled, followed by dilution cloning. Individual clones are then propagated and screened for bcl-2 expression. Once expression is verified, persistent Sindbis infection is tested, followed by its use as a parent cell line for Sindbis packaging cell line development.

Sindbis infection of BHK cells results in morphological changes and DNA fragmentation patterns diagnostic of apoptosis, and the conversion of lytic to persistent infection by the bcl-2 oncongene may be mediated by a suppresion of this programmed cell death (Nature 361:739). In addition, infection of mammalian cells with other viruses including adenovirus, Polyomavirus, SV40, and HIV has resulted in cytotoxicity due to apoptosis. Therefore, other gene products, in addition to the bcl-2 oncogene, which suppress apoptosis would be desirable for expression in a Sindbis packaging or producer cell line. Initially, three viral genes, show to suppress apoptosis, will be expressed in Sindbis packaging cell lines: the adenovirus E1B gene encoding the 19-kD protein (Rao et al., PNAS 89:7742-7746. 1992), the herpes simplex virus type 1 g₁ 34.5 gene (Chou and Roizman. PNAS 89:3266-3270, 1992), and the AcMNPV baculovirus p35 gene (Clem et al., Science 254.1:1388-1390, 1991). The individual genes are inserted into plasmid expression vectors, under the control of constitutive eukaryotic transcriptional promoters and containing a selectable marker, using standard techniques known in the art. These expression vectors are subsequently transfected into cell lines as described previously, and the appropriate selection is applied. Selection for stable integration of these genes and constitutive expression their products should allow for more extended vector production in cell lines known to be susceptible to Sindbis-induced apoptotic events. In addition, it is feasible that each gene product may inhibit apoptosis by its own unique mechanism. Therefore, the genes also will be introduced into packaging cell lines in various combinations to obtain a stronger suppressive effect. Finally, other gene products having similar effects on apoptosis can be readily incorporated into packaging cell lines as they are discovered.

SV40 and Py transformed cell lines are established, and the kinetics and level of Sindbis production and cytopathology after viral infection determined. If apoptic events characteristic of Sindbis proliferation in hamster cells are diminished, each prototype Sindbis packaging cell line subsequently is transformed with Py or SV40 in order to increase the yield of packaged vector from these cells.

### 3. Modification of cells to decrease susceptiblity to Sindbis expression:Production of activation-dependent vector particles

The Sindbis E2 glycoprotein is synthesized as a precursor, PE2. This PE2 precursor and the second viral glycoprotein, E1, associate in the endoplasmic reticulum and are processed and transported to the infected cell membrane as a heterodimer for virion incorporation. At some point during this processing, PE2 is cleaved into E3 and mature E2. E3 is the 64 amino-terminal residues of PE2 and is lost in the extracellular void during maturation. The larger cleavage product, E2, is associated with E1 and anchored in what becomes the viral envelope. A host cell protease is responsible for processing of the PE2 precursor, cleaving at a site that immediately follows a highly conserved canonical four amino acid (aa) residue motif, basic-X-basic-basic aa's. A mutant cell line derived from the CHO-K1 strain, designated RPE.40 (Watson et. al., (1991) J. Virol 65:2332-2339), is defective in the production of Sindbis virus strain AR339, through its inability to process the PE2 precursor into the E3 and mature E2 forms. The envelopes of Sindbis virions produced in the RPE.40 cell line therefore contain the PE2/E1 heterodimer. RPE.40 cells are at least 100-fold more resistant to Sindbis virus infection than the parental CHO-K1 cells, suggesting an inefficiency in the ability of PE2 containing virions to infect cells. These defective virions produced by the RPE.40 cell line can be converted into a fully infectious form by treatment with trypsin.
In packaging or producer cell lines, any wild-type virus produced by recombination will re-infect cells and be rapidly amplified: thus, significantly contaminating packaged vector preparations. Producer cells developed from the RPE.40 line would be a significant improvement over other lines permissive for Sindbis virus infection, due to the inefficient amplification of any wild-type virus generated during vector production and packaging. Thus, vector preparations are not significantly contaminated with wild-type virus. Furthermore, this system can be extended to other cell lines by developing "knock-out" mutants in the analagous cellular protease.

### 4. Hipping cell line development

Sindbis hopping cell lines, as discussed previously, are used to transiently produce infectious RNA vector particles which have been pseudotyped for a different cellular receptor tropism. Once the hopping cell line produces vector particles, it is no longer required because only the infectious culture supernatants are needed to transduce the original Sindbis packaging cell lines discussed above.
Therefore, the hopping cell line need not exhibit persistent infection by Sindbis in order to transiently produce vector particles. In this instance, the parent cell line can be either an insect cell line that exhibits persistent infection, or a mammalian cell line which is likely to lyse with in 72 hours after a productive Sindbis infection. The only criteria is that the cell lines are able to express and process the Sindbis structural proteins while co-expressing either VSV-G protein or retroviral gag-pol and env protein without affecting cell growth prior to transfection with the Sindbis RNA vector. Therefore, the Sindbis hopping cell line can be any of the aforementioned parent cell lines able to support either Sindbis or retroviral replication, without additional cell modifications, as previously discussed, such as bcl-2 oncogene expression.

Creation of a VSV-G pseudotyped Sindbis vector requires co-transfection of Sindbis vector RNA transcribed *in vitro* or DNA, together with the vector pMLP-G, which expresses the VSV-G envelope protein into a Sindbis packaging cell line. Cell growth conditions and transfection procedures are described under the heading "assembly of the components" and any of the packaging cell configurations described are used. Supernatants, containing the VSV-G pseudotyped Sindbis vector are harvested at 24 hours post-transfection and then used to transduce fresh monolayers of the identical Sindbis packaging cell line to overcome the reduction in transduction efficiency.

For the pseudotyping of Sindbis vectors in retroviral packaging cell lines, any cell line referenced in the literature, which expresses retroviral gag-pol and env sequences, may be used to package a Sindbis RNA vector that has been engineered to contain a retroviral packaging sequence. The retrovirus psi packaging sequence is inserted between the inactivated junction region and a synthetic junction region tandem repeat, such that only genomic-length vector, and not subgenomic RNA, is packaged by the retroviral envelope proteins. Retroviral particles containing a Sindbis vector RNA are produced by transfecting *in vitro* transcribed Sindbis vector RNA using procedures that have been described previously. Supernatants with pseudotyped retroviral particles containing Sindbis RNA vector are harvested at 24 hours post-transfection, and these supernatants can then be used to transduce a Sindbis packaging cell line.

### B. STRUCTURAL PROTEIN EXPRESSION CONSTRUCTS

### 1. Inducible and constitutive structural protein vector constructs

The development of Sindbis packaging cell lines is dependent on the ability to synthesize high intracellular levels of the necessary structural proteins: capsid, E2, and E1. Unfortunately, high level expression of these proteins, in particular, the envelope glycoproteins E2 and E1, may lead to concomitant cytopathology and eventual cell death. Therefore structural protein expression cassettes have been designed with inducible regulatory elements which control the levels of gene expression, in addition to others which maintain constituitive levels of expression.

In the first configuration, expression of the Sindbis structural proteins are under control of the RSV LTR, in conjunction with the inducible lac operon sequences. This is achieved by insertion of Sindbis cDNA corresponding to the viral structural protein genes into the pOP13 and pOPRSV1 vectors (Stratagene). These vectors, used separately, are co-transfected with the p3'SS vector (Stratagene), which expresses the lac repressor "i" protein. In the absence of inducer, for example Isopropyl-B-D-thiogalactopyranoside (IPTG), the basal, or constitutive, level of expression of a luciferase reporter gene has been reported to be 10-20 copies per cell. Addition of IPTG, results in a conformational change of the repressor protein, which results in decreased affinity of the lac i protein for lac-operator sequences, permitting high level expression of the heterologous gene. Induction levels in the presence of IPTG of 95-fold have been reported for heterologous genes contained in the pOP13 vector.

Specifically, the Sindbis structural protein gene (SP) cDNA is inserted into the pOP13 and pOPRSV1 vectors as follows. The SP coding region is amplified in toto with a primer pair whose 5' ends map, respectively, to the authentic AUG translational start and UGA translational stop sites, including the surrounding nucleotides corresponding to the Kozak consensus sequence for efficient translational initiation at Sindbis nt. 7638. The forward primer is complementary to Sindbis nts 7638-7661. and the reverse primer is complementary to Sindbis nts. 11,384-11,364. PCR amplification of Sindbis cDNA corresponding to the structural protein genes is accomplished by a standard two-temperature cycling protocol, using the following oligonucleotide pair:
Forward primer (7638F):
   5'-TATATGCGGCCGCACCACCACCATGAATAGAGGATTCTTTAACATGC-3'. (SEQ. ID. No. 38)
Reverse primer (11384R):
   5'-TATATGCGGCCGCTCATCTTCGTGTGCTAGTCAG-3' (SEQ. ID. No. 39)

In addition to their respective complementarities to the indicated Sindbis nts., a 5 nucleotide "buffer sequence" followed by the Not I recognition sequence is attached to the 5' ends of each primer. Following PCR amplification, the 3,763 bp fragment is purified in a 1% agarose gel, then subsequently digested with the Not I enzyme. The resulting 3,749 bp fragment is then ligated, separately, into the pOP13 and pOPRSV1 vectors, which are digested with Not I and treated with calf intestine alkaline phosphatase. These expression cassette vectors, which contain the entire coding capacity of the Sindbis structural proteins are known as pOP13-SINSP and pOPRSV1-SINSP.

Variations of the lac operon-Sindbis structural protein gene expression cassettes also can be constructed using other viral, cellular or insect based promoters. Using common molecular biology techniques known in the art, the lac operon and the RSV LTR, promoter, or just the RSV LTR promoter, sequences can be switched out of the Stratagene pOP13 and pOPRSV1 vectors and replaced by other promoter sequences, such as the cytomegalovirus major immediate promoter (pOPCMV-SINSP); the adenovirus major late promoter (pOPAMLP-SINSP); or insect promoter sequences, which include the Drosophila metallothionein inducible promoter (pMET-SINSP), Drosophila actin 5C distal promoter (pOPASC-SINSP), heat shock promoters HSP65 or HSP70 (pHSP-SINSP), or the baculovirus polyhedrin promoter (pPHED-SFNSP).

### 2. Modification of cassettes to increase protein expression levels

Sindbis structural protein expression can be increased if the level of mRNA transcripts is increased. Increasing the level of mRNA transcripts can be accomplished by modifying the expression cassette such that Sindbis nonstructural proteins recognize these transcripts, and in turn, replicate the message to higher levels. This modification is performed by adding the wild-type minimal junction region core (nucleotides 7579 to 7602) to the extreme 5'-end of the Sindbis structural protein coding region, in between the first authentic ATG start site for translation and the promoter sequences of the expression cassette. This can be accomplished by following the same PCR amplification technique described above for placing the Sindbis structural protein cDNA into the pOP13 and pOPRSV1 expression vectors. The only modification to this procedure is the replacement of the 7638F forward primer with a similar primer that includes junction region core nucleotides 7579-7602 between the Not I restriction enzyme site and the first ATG of the coding region as follows:
Forward primer (JUN7638F):

Following PCR amplification, the resulting 3,787 bp fragment is purified in a 1% agarose gel, then subsequently digested with the Not I enzyme. The resulting 3,773 bp fragment is then ligated, separately, into the pOP13 and pOPRSV1 vectors which are digested with Not I and treated with calf intestine alkaline phosphatase. The resulting expression cassette vectors are known as pOP13-JUNSINSP and pOPRSV1-JUNSINSP. However, it must be stated that the introduction of junction region sequences into the structural protein expression cassettes will introduce sequences which may possibly lead to undesirable recombination events, leading to the generation of wild-type Sindbis virus.

### 3. Inducible expression of structural proteins via Sindbis vector.

Because of potential cytotoxic effects from structural protein expression, the establishment of inducible packaging cell lines which express even modest basal levels of these proteins may not be the best method. Therefore, packaging cell line expression cassettes are constructed which contain regulatory elements for the high level induction of structural protein synthesis via nonstructural proteins supplied in *trans* by the Sindbis vector, but with no basal level of synthesis until appropriately stimulated.

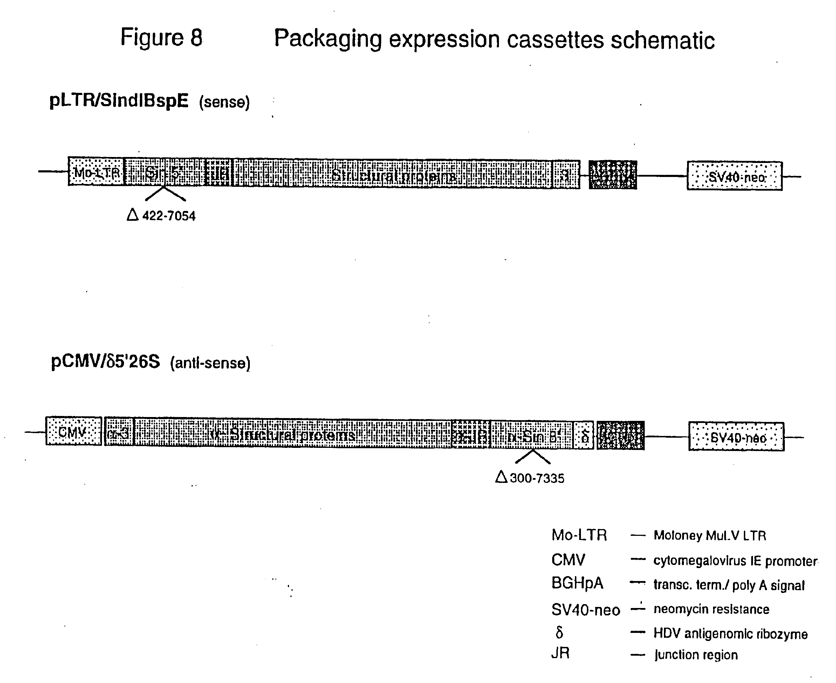

In this configuration, a structural protein gene cassette is constructed, whereby transcription of the structural protein genes occurs from an adjacent Sindbis junction region sequence. The primary features of this cassette are: an RNA polymerase II promoter positioned immediately adjacent to Sindbis nucleotide 1. such that transcription inititation begins with authentic Sindbis nucleotide 1, the 5'-end Sindbis sequences required for transcriptase recognition, the Sindbis junction region sequence for expression of the structural protein gene mRNA. the Sindbis structural protein gene sequences, the 3'-end Sindbis sequences required for replication, and a transcription termination/polyadenylation sequence. Because of an upstream open-reading frame which ends in translation termination codons prior to the AUG start site of the structural protein genes, expression of the Sindbis structural proteins can occur only after the synthesis of minus-strand RNA by vector-supplied nonstructural proteins and the subsequent transcription of a structural protein gene mRNA from the junction region. Therefore, the inducibility of this system is dependent entirely on the presence of nonstructural proteins, supplied by the Sindbis vector itself, introduced as either RNA transcribed *in vitro,* or cDNA positioned downstream of an appropriate promoter element. In addition, the 5'- and 3'-end Sindbis sequences allow for this RNA transcript of the structural protein gene cassette to be amplified by the same vector-supplied nonstructural proteins (see figure 8).

Specifically, the construction of a positive-sense, vector-inducible packaging cassette is accomplished as follows. The pVGELVIS vector described previously is digested with the enzyme BspEI to remove nucleotides 422 to 7054, including most of the nonstructural gene coding sequences, and the remaining 9925 bp fragment is purified in a 0.8% agarose gel, and subsequently re-ligated to itself to generate the construct known as pLTR/SindIBspE. This deletion leaves the 5'-end authentic translation start codon at nts. 60-62 intact, and creates in-frame downstream UAA and UGA stop codons at nts. 7130-7132 and 7190-7192 (original numbering), respectively, thus preventing translation of the downstream structural protein gene open-reading frame. The pLTR/SindIBspE packaging cassette construct is subsequently transfected into BHK cells (ATCC #CCL 10) and positive transfectants are selected using the G418 drug at 400 µg/ml as previously described. The data shown in figure 4 demonstrate that transfection of Sin-luc vector RNA into these LTR/SindIBspE packaging cells results in the production of infectious Sindbis particles containing the Sin-luc RNA, as the recovered supernatants are shown to transfer Sin-luc vector RNA to fresh monolayers of BHK cells.

A similar packaging construct also is made using the pVG-ELVISd clone (described previously) as initial material for creation of the BspEI deletion. In this clone, the Sindbis 3'-end sequence is followed by a catalytic ribozyme sequence to allow more precise processing of the primary transcript adjacent to the 3'-end sequences of Sindbis. In addition, variations of these packaging cassette constructions can be made using standard techniques known in the art, which include: the substitution of other RNA polymerase promoters for the current MuLV LTR, the addition of 1 or more nucleotides between the RNA polymerase promoter and the first Sindbis nucleotide, or the substitution of a non-Sindbis-encoded open reading frame upstream of the structural protein gene sequences, which may or may not retain the 5'-end Sindbis sequences required for transcriptase recognition.

In another vector-inducible packaging configuration, expression cassettes contain a cDNA copy of the Sindbis structural protein gene sequences flanked by their natural junction and 3'-untranslated regions, and inserted into an expression vector in an orientation, such that primary transcription from the promoter produces antisense structural protein gene RNA molecules. Additionally, these constructs also contain, adjacent to the junction region, Sindbis 5'-end sequences necessary for recognition by the viral transcriptase, and a catalytic ribozyme sequence positioned immediately adjacent to Sindbis nucleotide 1 of the 5'-end sequence. As such, this ribozyme cleaves the primary RNA transcript precisely after the first Sindbis nucleotide. In this anti-sense orientation, the structural protein genes cannot be translated, and are dependent entirely on the presence of Sindbis virus nonstructural proteins for transcription into postive-strand mRNA, prior to their expression. These nonstructural proteins are provided by the Sindbis vector itself. In addition, because this configuration contains the precise Sindbis genome 5'- and 3'-end sequences, the structural protein gene transcripts undergo amplification by utilizing the same nonstructural proteins provided by the Sindbis vector.

Specifically, the Sindbis structural protein gene cDNA is removed from the genomic clone pVGSP6GEN and inserted into the pcDNA3 (Invitrogen Corp., San Diego, CA) expression vector as follows. First, plasmid pVGSP6GEN is digested with the enzymes ApaI and BamHI to remove all Sindbis sequences through nucleotide 7335, including the genes encoding nonstructural proteins 1, 2, 3, and most of 4. The remaining 7285 bp vector fragment, which contains the Sindbis structural protein genes, is purified in a 0.8% agarose gel, and subsequently ligated with a polylinker sequence, called SinMCS, that is obtained by annealing two synthetic oligonucleotides. The oligonucleotides, SinMCSI and SinMCSII, contain the recognition sites for ClaI, BgIII, and SpeI. and have ApaI and BamHI ends after annealing. Their sequences are as follows:
SinMCSI:
   5'-CTCATCGATCAGATCTGACTAGTTG-3' (SEQ. ID. No. 31)
SinMCSII:
   5'-GATCCAACTAGTCAGATCTGATCGATGAGGGCC-3' (SEQ. ID. No. 32)

The resulting construct, known as pMCS-26s, is then modified to contain the 5'-end 299 nucleotides of Sindbis, fused to an 84 nucleotide ribozyme sequence from the antigenomic strand of hepatitis delta virus (HDV)(Nature 350:434)*,* using overlapping PCR amplification. Two primer pairs are used initially in separate reactions, followed by their overlapping synthesis in a second round of PCR. In reaction #1, the forward primer (HDV49-XC) is complementary to HDV genome nucleotides 823-859, and the reverse primer (HDV17-68) is complementary to HDV genome nucleotides 839-887, with sequences as follows:
Forward primer (HDV49-XC):
Reverse primer (HDV 17-68):

   In addition to their respective complementarities, primer HDV49-XC contains flanking XbaI and ClaI recognition sequences at the 5'-end. PCR amplification of HDV sequences is accomplished by a standard two-temperature cycling protocol with these primers and Vent polymerase. In reaction #2, the forward primer (SIN-HDV), which joins precisely the HDV and Sindbis sequences, is complementary to nucleotides 1-21 of Sindbis, and genomic nucleotides 871-903 of HDV, and overlaps the sequence of primer HDV 17-68 (from above) by 20 nucleotides, and the reverse primer (SIN276-SPE) is complementary to Sindbis nucleotides 299-276, with sequences as follows:
Forward primer (SIN-HDV):
Reverse primer (SIN276-SPE):
   5'-CTGGACTAGTTAATACTGGTGCTCGGAAAACATTCT-3' (SEQ. ID. No. 36)

In addition to their respective complementarities, primer SIN276-SPE contains a flanking UAA translation termination codon and SpeI recognition sequence at its 5' end. PCR amplification of the fragment containing Sindbis 5'-end sequences fused to HDV ribozyme sequences is accomplished by a standard two-temperature cycling protocol, using Vent polymerase, these primers, and pVGSP6GEN plasmid as template. After the first round of PCR amplification, 1/20th of the total amounts from each of reaction #1 and reaction #2 is combined and used as template in a second round of PCR amplification with additional input of primers HDV49-XC and SIN276-SPE and a standard two-temperature cycling protocol. Following the second round of PCR, the 414 bp amplicon is purified with the Mermaid Kit (Bio101, La Jolla, CA), and digested with the enzymes ClaI and SpeI. The digested amplicon is purified in a 1% agarose gel, and subsequently ligated into plasmid pMCS-26s, which also is digested with ClaI and SpeI and purified in a 1% agarose gel. The resulting construct, containing the expression cassette elements HDV antigenomic ribozyme/Sindbis 5'-end 299 nts./Sindbis junction region/Sindbis structural protein genes/Sindbis 3'-end untranslated region, is known as pδ5'26s.

Insertion of the structural protein gene cassette from pδ5'26s into the pcDNA3 vector is performed as follows. Plasmid pδ5'26s is digested with the enzyme XbaI and the 3'-recessed ends are made blunt by the addition of Klenow enzyme and dNTPs: The entire 4798 bp structural protein gene cassette is purified in a 1% agarose gel. Plamid pcDNA3 is digested with the enzymes HindIII and ApaI and the ends are made blunt by the addition of T4 DNA polymerase enzyme and dNTPs, and the 5342 bp vector is purified in a 1% agarose gels. The two purified, blunt-end DNA fragments are subsequently ligated, and the resulting structural protein gene expression cassette vector is known as pCMV-δ5'26s (see figure 8). Transfection of this DNA into cells and selection for G418 resistance is performed as previously described.

Modifications of the CMV promoter/antisense-Sindbis structural protein vector also can be constructed using other viral, cellular, or insect-based promoters. Using common molecular biology techniques know in the art, the CMV promoter can be switched out of the Invitrogen pcDNA3 vector and replaced by promoters such as those listed previously. Other variation of this antisense packaging cassette may include, but are not limited to: the addition of 1 or more nucleotides between the first Sindbis nucleotide and the catalytic ribozyme, the use of other catalytic ribozyme sequences for transcript processing, the substitution of a precise transcription termination signal for the catalytic ribozyme sequence, or the antisense expression of structural protein gene cassettes using any downstream sequence recognized by an RNA polymerase which results in transcription of a structural protein gene mRNA.

Further, it should be noted that each of the vector-inducible constructs described contains sequences homologous to the Sindbis vector itself. Therefore, the potential exists for the generation of wild-type virus by recombination between the two RNA molecules. Additional modifications are made to eliminate this possibility as described below.

### 4. Separation of structural protein genes to prevent recombination

After demonstrating the utility of the approaches described here, additional packaging cell lines are generated based on these principles. These additional lines segregate the integration and expression of the stuctural protein genes, allowing for their transcription as non-overlapping, independent RNA molecules. The expression of capsid protein independently of glycoproteins E2 and E1, or each of the three proteins independent of each other, eliminates the possibility of recombination with vector RNA and subsequent generation of contaminating wild-type virus.

Specifically, capsid protein is expressed independently from an inducible expression vector, such that sequences which might result in recombination with vector RNA are eliminated. As and example, the capsid protein gene is amplified from plasmid pVGSP6GEN with a primer pair complementary to nucleotides 7632-7655 (forward primer) and 8415-8439 (reverse primer), with sequences as follows:
Forward primer:
   5'-GTCAAGCTTGCTAGCTACAACACCACCACCATGAATAGAG-3' (SEQ. ID. No. 37)
Reverse primer:
   5'-CAGTCTCGAGTTACTACCACTCTTCTGTCCCTTCCGGGGT-3' (SEQ. ID. No. 41)

In addition to their respective complementarities, the forward primer contains Nhel and HindIII recognition sequences at its 5'-end, and the reverse primer contains both UAG and UAA translation stop codons and a XhoI recognition sequence at its 5'-end. Amplification is accomplished using a standard two-temperature cycling protocol, and the resulting amplicon is digested with the enzymes NheI and XhoI, and purified in a 1% agarose gel. Expression plasmid pMAM (Clontech), which contains a dexamethasone-inducible MMTV LTR promoter sequence, is digested with the enzymes NheI and XhoI and the plasmid DNA purified in a 1% agarose gel. The capsid protein gene fragment is ligated into the pMAM vector, and the resulting construct is known as pMAM-SinC. Plamsid pMAM-SinC is transfected into the appropriate cell line as described previously and selection for stable transfectants is accomplished by using HAT (hypoxanthine, aminopterin, thymidine) media as described by the manufacturer.

The glycoprotein genes, E1 and E2, are expressed together using one of the inducible systems previously described. For example, the E1 and E2 genes are amplified from plasmid pVGSP6GEN using a primer pair complementary to Sindbis nucleotides 8440-8459 (forward primer) and Sindbis nts. 11,384-11,364 (reverse primer). PCR amplification is performed using a standard two-temperature cycling protocol and the following oligonucleotide pair:
Reverse primer (11384R):
   5'-TATATGCGGCCGCTCATCTTCGTGTGCTAGTCAG-3' (SEQ. ID. No. 39)
Forward primer (8440F):
   5'-TATATGCGGCCGCACCACCATGTCCGCAGCACCACTGGTCACG-3' (SEQ. ID. No. 42)

In addition to their respective complementarities, the forward primer contains an "in-frame" AUG translation initiation codon, and both primers contain a NotI recognition sequence at their 5'-ends. Following PCR amplification, the amplicon is digested with the NotI enzyme and purified in a 1% agarose gel. The resulting fragment is then ligated separately into the pOP13 and pOPRSV1 vectors (Stratagene), digested with Not I and treated with calf intestinal alkaline phosphatase, as described previously. These glycoprotein expression vectors are used to transfect cells that have been previously transfected with the capsid protein expression construct.

### 5. Assembling the components to create the Sindbis packaging cell line

For example purposes, the Aedes albopictus cell line will be used to demonstrate assembly of the components. However, other possible parent cell lines can be used to create Sindbis packaging cell lines and have been discussed previously. Aedes albopictus mosquito cells (ATCC No. CRL 1660) are grown at 28°C in 5% CO₂ in minimum essential medium (Eagle) with nonessential amino acids, 2 mM L-glutamine, Earle's balanced salt solution, 0.11% sodium bicarbonate, and 10% fetal bovine serum (optimal media). Approximately 5 x 10⁵ mosquito cells, grown in a 35 mM petri dish, are transfected with 5 ug p3'SS using 5 ul of the Transfectam (Promega) cationic lipid reagent, in serum-free media conditions, as suggested by the supplier. However any method of transfection can be performed, i.e., by electroporation, calcium phosphate precipitation, or by using any of the readily available cationic liposome formulations and procedures commonly known in the art. At 24 hrs. post transfection, the cells are overlaid with 4 ml of optimal media, as described above, supplemented with 200 ug/ml of the antibiotic hygromyin and selected over a period of 10 to 14 days. Expanded clones are then isolated and tested for expression of the Lac repressor by Northern blot hybridization. An individual clone expressing satisfactory levels of the Lac repressor mRNA is then retransfected with a lac operon vector construct expressing the Sindbis structural proteins (i.e.. pOP13-SINSP or pOPRSV1-SINSP), followed by drug selection with 200-800 ug/ml of geneticin and continued hygromycin selection. Colonies displaying resistance to both antibiotics are then pooled, dilution cloned, and propagated. Individual clones are then induced with 5mM of IPTG for 12 hours and screened for high levels of Sindbis structural protein expression. Expression can be tested by western blot analysis using specific antibodies (available in the literature), or by quantification of Sindbis specific RNA in RNase protection assays, using ³²P end-labeled RNA probes complementary to the RNA of the Sindbis structural protein gene region. These assay procedures reveal clones with the highest levels of structural protein expression in response to induction with IPTG. Several of the highest expressing mosquito cell clones (refereed to as Albopictus SINpak cells) are then tested for functional activity. Functional activity is tested by demonstrating the cell line's ability to package luciferase-expressing vector, and the subsequent ability of the media supernatant to re-infect BHK-21 cells and transfer luciferase activity.

Specifically. Albopictus SINpak cells grown in 35 mm petri dishes with the selection media described above, containing hygromycin and geneticin, are transfected with RNA synthesized by *in vitro* transcription (Promega) of the pSKSINBV-luc cDNA described previously. At 1 hr. post-transfection, the media is supplemented with 5 mM IPTG. The supernatant is harvested at 24 hrs. post-transfection and used to infect. BHK-21 cells directly and a fresh monolayer of Albopictus SINpak cells induced as above with mM IPTG for at least 12 hrs. Supernatants from the infection are harvested at 24 hrs. post-infection and then used to infect a second monolayer of BHK-21 cells, grown in 35 mm petri dishes. At 16 hrs. post-infection, the BHK-21 cells are lysed and tested for luciferase activity as described (Promega). Comparison of the luciferase activities obtained from BHK-21 cells infected after the first round of vector production against BHK-21 cells infected after the second round of vector production should demonstrate at least a ten-fold difference in expression levels. If differences in levels are lower than ten-fold, the reduction in efficiency of transduction following transfer may be due to occupied cellular receptors on the identical Sindbis packaging cell line from which transient vector was produced. This may indicate the need for the generation of pseudotyped Sindbis vectors to improve transduction efficiency into envelope-related packaging cell lines.

### C. INDUCIBLE VECTOR AND STRUCTURAL PROTEIN EXPRESSION FOR SINDBIS PRODUCER CELL LINES

### 1. Use of viral promoters

The challenge of developing a Sindbis vector producer cell line lies in the question of whether a virus whose infection of mammalian cells results almost exclusively in productive lytic cell death can be somehow converted to establish persistent infection in these same cells. One possibility is to generate Sindbis vector producer lines from mosquito cells, where viral persistence results after infection. However, the titer of infectious virus in produced in persistently infected mosquito cells is only about 1 x 10⁴ PFU/ml, at least five orders of magnitude less than that observed after Sindbis lytic infection of BHK cells. Thus, it may not be commercially feasible to develop mosquito derived Sindbis vector producer cell lines.

Many strategies have been described for inducible. Sindbis vector producer cell lines, containing both vector and viral structural gene cassettes, such that productive cytolytic infection occurs only after the correct stimulus. Because these approaches operate on a "feed forward" level, any leakiness in the system will result in initiation of the Sindbis life cyle and cell death.

The hallmark of development is the differentiation state dependent pattern of gene expression. Gene expression patterns differ widely between undifferentiated and terminally differentiated states. Thus, it is possible that a cell whose differentiation state can be controlled is an ideal host in which to derive a Sindbis vector producer cell line. In such a configuration, the vector and structural components are coupled to terminal differentiation state inducible promoters, according to the described ELVIS strategy, and used to transform stably an undifferentiated host cell. Terminal differentiation of the host producer cell after induction with the appropriate stimuli coincidently results in the induction of Sindbis replication cycle and production of packaged vector. Other strategies described herein, including antisense structural genes and heterologous viral expression systems, would be coupled with cellular differentiation state dependent promoters described below.

In this approach, three examples are described, using either a viral or cellular promoter which are active in only terminally differentiated cells.

It has been shown that mouse Polyomavirus (Py), SV40, and Moloney murine leukemia virus (M-MuLV), are all able to infect and enter undifferentiated mouse embryonal carcinoma (EC) cells, but the expression of their genes (and heterologous genes) and establishment of productive infection is blocked (Swartzendruber and Lehman, J. Cell. Physiol. 85:179-188, 1975; Peries et al., J. Natl. Cancer Inst. 59:463-465, 1977). These viral growth properties have been demonstrated in two cell lines, PCC4 and F9, which are derived from the malignant stem cells of mouse teratorcarcinomas. The block to viral propagation occurs at the level of transcription and replication, and maps to the enhancers, contained within the viral noncoding control regions (Linney et al., Nature 308:470-472, 1984; Fujimura et al., Cell 23:809-814, 1981; Katinka and Yaniv, Cell 20:393-399, 1980).

When M-MuLV infects undifferentiated EC cells, the viral DNA integrates into the genome. However, as stated above, expression of viral genes or of heterologous genes is blocked. This block of viral expression is released upon terminal differentiation of EC cells by addition of retinoic acid to the growth medium.

To test the RNA expression properties of the pVGELVIS construct in EC cells, plasmid DNA is complexed with Lipofectamine (GIBCO-BRL, Gaithersburg, MD) according to the conditions suggested by the supplier (ca. 5 µg DNA/8 mg lipid reagent) and added to 35 mm wells containing undifferentiated PCC4 or F9 cells (Fujimura et. al. 1981. Cell 23:809-814) at approximately 75% confluency. The development of cytopathic effects (CPE), and the level of Sindbis productive infection, quantitated by plaque assay of media supernatant, is determined at regular intervals over 5 days in undifferentiated and differentiated transfected PCC4 or F9 cells. Differentiation of F9 and PCC4 cells is accomplished by addition of retinoic acid (Sigma Chemical Co., St. Louis, Mo), at a final concentration of 1 µM.

It has been proposed that the hierarchy of relative expression of heterologous genes observed in undifferentiated EC cells infected with M-MuLV vectors may be in part be insertional dependent (Linney et. al. 1987. J. virol. 61:3248-3253). Thus, undifferentiated EC cells transfected with pVGELVIS will likely produce different results, in terms of transcription of the Sindbis genomic cDNA and, in turn, initiation of the viral life cycle. In this event, following G418 selection of pVGELVIS transfected undifferentiated EC cells, remaining cells are cloned and expanded. The cell clones are then tested for the production of Sindbis virus after differentiation by addition of retinoic acid (Sigma Chemical Co., St. Louis, Mo), at a final concentration of 1 µM.

To isolate vector packaging cell lines, whose production of structural proteins in the presence of Sindbis NSP is cell differentiation state dependent, undifferentiated F9 or PCC4 cells are transfected with pLTR/SINdIBspE and G418 selected as described above. Differentiation state-sensitive clones are then selected by infection at high multiplicity with packaged SIN-luc vector. Clones which are resistant to cell lysis or do not produce packaged SIN-luc vector particles, are candidate vector packaging clones. These candidate clones are tested for SIN-luc vector particle production following terminal differentiation with retinoic acid, as described.

The murine wild type polyomavirus (Py) is unable to replicate in the teratocarcinoma cell lines PCC4 or F9. This block of replication in undifferentiated cells occurs at the level of transcription of early region (i.e. T antigen) genes, and is released by induction of terminal differentiation with vitamin A. Py mutants which are able to establish productive infection in undifferentiated PCC4 and F9 cells map to the viral enhancer region. The genesis of an embryonic tissue specific transcriptional enhancer element has resulted in these mutants. In order to exploit this property of inhibition of Py replication in undifferentiated teratocarcinoma cell lines, the viral regulatory noncoding region, including the enhancer, is coupled to the genomic cDNA of Sindbis virus, according to the ELVIS strategy. The precise transcriptional start site of the Py early region has been determined (see Tooze, DNA Tumor Viruses). The PCC4 and F9 cell lines are stably transformed with the Py-Sindbis vectors. In this model Sindbis productive infection occurs after addition of retinoic acid to the culture medium and induction of terminal differentiation.

The Py noncoding region from bases 5021-152, which includes the sequences corresponding to the viral enhancers, 21 bp repeats, replication origin, CAAT and TATA boxes, and the early mRNA transcription 5' cap site, is positioned at the 5' viral end such that *n vivo,* only a single capped C residue is added to the Sindbis 5' end. Juxtaposition of the Py noncoding region and the Sindbis 5' end is accomplished by overlapping PCR as described in the following detail. Amplification of the Py noncoding region in the first primary PCR reaction is accomplished in a reaction containing the pBR322/Py, strain A2 plasmid (ATCC number 45017-p53.A6.6 (pPy-1)) and the following primer pair:
Forward primer: Pybg15021F (buffer sequence/Bgl II recoginition sequence/Py nts 5021-5043):
   5'-TATATAGATCTCTTGATCAGCTTCAGAAGATGGC (SEQ. ID NO. 43)
Reverse primer: SINPy152R (SIN nts 5-1/Py nts 152-134):
   5'-TCAATGGCGGGAAGAGGCGGTTGG (SEQ. ID NO. 44)

PCR amplification of the Py noncoding region with the primer pair shown above is performed using the Thermalase thermostable DNA polymerase (Ameresco Inc., Solon, Ohio) and the buffer containing 1.5 mM MgCl₂, provided by the supplier. Additionally, the reaction contains 5% DMSO, and the Hot Start Wax beads (Perkin-Elmer), using the following PCR amplification protocol shown below:

| Temperature (°C) | Time (Min.) | No. Cycles |
|---|---|---|
| 94 | 2 | 1 |
| 94 | 0.5 | |
| 55 | 0.5 | 35 |
| 72 | 0.5 | |
| 72 | 10 | 1 |

Amplification of the Sindbis 5' end in the second primary PCR reaction is accomplished in a reaction containing the pVGSP6GEN clone and the following primer pair /
Forward primer: (Py nts 138-152/SIN nts 1-16):
   5'-CCGCCTCTTCCCGCCATTGACGGCGTAGTAC (SEQ. ID NO. 45)
Reverse primer: (SIN nts 3182-3160):
   5'-CTGGCAACCGGTAAGTACGATAC (SEQ. ID NO. 46)

PCR amplification of Sindbis 5' end region with the primer pair shown above is with the reaction conditions described above, using the following PCR amplification protocol shown below:

| Temperature (°C) | Time (Min.) | No. Cycles |
|---|---|---|
| 94 | 2 | 1 |
| 94 | 0.5 | |
| 55 | 0.5 | 35 |
| 72 | 3.0 | |
| 72 | 10 | |

The 442 bp and 3202 bp products from the primary PCR reactions are purified with Gene Clean (BIO 101), and used together in a PCR reaction with the following primer pair:
Forward primer: Pybg15021F (buffer sequenceBgl II recoginition sequence/Py nts 5021-5043):
   5'-TATATAGATCTCTTGATCAGCTTCAGAAGATGGC (SEQ. ID NO. 47)
Reverse primer: (SIN nts 2300-2278):
   5'-GGTAACAAGATCTCGTGCCGTG (SEQ. ID NO. 48)

PCR amplification of the of the primer PCR amplicon products with the primer pair shown above is with the reaction conditions described above, using the following PCR amplification protocol shown below:

| Temperature (°C) | Time (Min.) | No. Cycles |
|---|---|---|
| 94 | 2 | 1 |
| 94 | 0.5 | |
| 55 | 0.5 | 35 |
| 72 | 3.0 | |

the fetal liver and the δ- and b-globin genes in adult bone marrow (Collins and Weissman, 1984, Prog. Nucleic Acid Res. Mol. Bio.31:315).

At least two mouse erythroleukemia lines, MEL and Friend, serve as models for terminal differentiation dependent expression of β-globin. Expression of β-globin is observed in these lines only after induction of terminal differentiation by addition of 2% DMSO to the growth medium.

The entire β-globin locus is regulated by the locus control region (LCR). Within the LCR is the dominant control region (DCR) residing within the DNase I hypersenitive region, which is 5' of the coding region The DCR contains five DNase I hypersensitive (HS1- HS5) sites. The DCR directs high level siteof integration independent, copy number dependent expression on a linked human β-globin gene in transgenic mice and stably transfected mouse erythroleudemia (MEL) cells (Grosveld et. al., 1993, CSHSQB 58:7-12). In a recent study (Ellis et. al. 1993 EMBO 12:127-134), concatamers of a synthetic core coinciding to sequences within HS2 were shown to function as a locus control region.

In order to accomplish the differentiation state dependent expression of Sindbis vectors, the viral genomic cDNA is juxtaposed with a promoter containing a tandem synthetic core corresponding to the the LCR HS2 site. Alternatively, the desired Sindbis vector construct can be inserted downstream of the LCR in the endogenous β-globin gene by homologous recombination. In such a strategy, the β-globin transcription initiation site after terminal differentiation would be first determined, in order that the Sindbis vector could be placed precisely at the start site.

The strategy proposed herein in which initiation of a lytic viral life cycle is controlled by the differentiation state of the host cell should find application in other systems, where the control of viral induced cytopathology is desired.

### 3. Insertion of vector constructs into differentiation state

### controller inducible promoters.

Generation of clones whose expression of heterologous genes from Sindbis vectors positioned in the ELVIS configuration as described in Example 3 is differentiation state dependent, is accomplished as desribed above for the pVGELVIS, pLTR/Sind1BspE plasmids. Generation of clones whose production of vector particles is differentiation state dependent, is accomplished by transfecting the isolated differentiation dependent vector packaging clones described above with ELVIS heterologous gene expression vectors. Clones having the desired phenotype or vector production after retinoic acid induced differentiation are isolated as described above.

### D. Structural protein expression from a heterologous Astrovirus junction region.

Among the critical properties of a vector packaging system are a cell line which expresses the structural components necessary to generate an infectious particle, without the creation of wild-type virus through recombination between vector and structural gene components. These two desired properties of the packaging cell line are accomplished in the retrovirus based systems through the constitutive expression of the gag/pol and env genes on individual heterologous RNA polymerase II expression cassettes.

Another important aspect of vector packaging cell lines is to derive a system which mimics as closely as possible the normal replication strategy of the wild type virus. This issue is important in terms of the observed titer level of packaged recombinant vector. Synthesis of the viral structural proteins during Sindbis infection is accomplished after transcription of high levels of subgenomic mRNA from the junction region promoter, followed by efficient translation into the structural proteins. The junction region promoter is functional only in the antisense orientation and synthesis of the antigenomic RNA occurs after tranlation of the nonstructural proteins, thus delaying the expression of the structural proteins. It follows that, with regard to Sindbis, it would be desirable to construct a packaging cell line in which synthesis of the structural proteins is initiated from the junction region promoter, which in turn is activated by nonstructural proteins expressed from the recombinant vector molecule.

It is known that a relatively high frequency of recombination occurs between RNA genomic molecules occurs during infection with Sindbis virus via a copy choice mechanism (PNAS 1991 88:3253-3257). Recombination between vector and junction region/structural gene cassettes would result in the generation of wild-type Sindbis virus, perhaps at a level of I wild-type virus per million of packaged vector particles (Liljestrom Bio/Technology 1991 9:1356-1361). One way to mitigate the generation of wild-type virus is to separate the structural genes onto separate expression cassettes, an approach which has been used quite successfully with retrovirus packaging cell lines, and has been discussed previously in example 7.

An additional approach to diminish the level of wild-type virus production in Sindbis vector packaging cell lines would be to express the structural proteins under the control of Astrovirus genetic elements. A schematic for this configuration is depicted in figure 10. Similar to Sindbis virus, the expression of Astrovirus structural proteins incorporates a junction region strategy, in which high levels of structural proteins are synthesized from a subgenomic message. The Astrovirus expression cassette could consist of one of the two following ordered elements: (1) Inducible promoter/Astrovirus 5' end/Astrovirus junction region/Sindbis structural gene/Astrovirus 3' end, or (2) antisense Astrovirus 3' end/antisense Sindbis structural gene/antisense Astrovirus junction region/antsense Astrovirus 5' end/ Hepatitis Delta virus ribozyme, or other configurations described in example 7. In both configurations, the expression unit is amplified by the Astrovirus nonstructural proteins through the same mechanism that occurs during viral replication. Since multiple rounds of subgenomic mRNA synthesis initiated from the junction region occur from each expression unit, amplification of the expression unit by the Astrovirus nonstructural proteins will result in the production of very high levels of Sindbis structural proteins. The second configuration of the Sindbis structural protein expression cassette described above may function better than the first, because the primary transcript of the toxic Sindbis structural gene is antisense. Although expression of the structural genes in the first configuration should not occur until synthesis of the negative strand followed by synthesis of the positive subgenomic RNA from the junction region, the antisense nature of the primary transcript in the second configuration represents an additional level of control to prevent cytotoxic protein expression.

It is likely that no wild-type virus would be generated in a packaging cell line in which the Sindbis virus structural proteins are synthesized individually from Astrovirus junction region expression cassettes. Recombination between the nonstructural protein region of the vector and an Astrovirus structural protein expression cassette would result in a molecule in which Astrovirus cis elements were coupled with Sindbis virus genes, a nonviable combination. Correct coupling of Sindbis cis and trans elements would require two precise recombination events between the vector and the Astrovirus expression cassette, between the Astrovirus junction region and structural gene ATG, and between the structural gene termination codon and the Astrovirus 3' end. In order to generate wild type virus, this dual recombination event would have to occur three times on the same molecule (six total events), to incorporate the three separated Sindbis structural genes.

In order to diminish any possible toxicity of the Astrovirus proteins, synthesis of the Astrovirus expression cassettes are controlled by inducible promoters. One possibility is to use the lac operon, according to the "lac-switch" system described previously in example 7 (Stratagene). The constitutive level of expression of the lac operon controlled gene in the absense of the gratuitous inducer IPTG is about 10 copies of RNA per cell. The inducible promoter corresponding to the Astrovirus/Sindbis structural gene expression cassette may be the lac operon or other suitable promoters which have very low level of constitutive expression. Construction of packaging cell lines of these configurations, in which the control of Sindbis proteins is directed by a heterologous virus should result in the generation of high titer wild-type virus free packaged vector particles.

### EXAMPLE 8

### ALTERNATIVE VIRAL VECTOR PACKAGING TECHNIQUES

Various alternative systems can be used to produce recombinant Sindbis viruses carrying the vector construct. Each of these systems takes advantage of the fact that the baculovirus, and the mammalian viruses, vaccinia and adenovirus, have been adapted recently to make large amounts of any given protein for which the gene has been cloned. (Smith et al., Mol. Cell. Biol. 3:12, 1983; Piccini et al., Meth. Enzymology 153:545, 1987; and Mansour et al., Proc. Natl. Acad Sci. USA 82:1359, 1985.)

These viral vectors can be used to produce proteins in tissue culture cells by insertion of appropriate genes into the viral vector and can be adapted to make Sindbis vector particles.

Adenovirus vectors are derived from nuclear replicating viruses and can be defective. Genes can be inserted into vectors and used to express proteins in mammalian cells either by *in vitro* construction (Ballay et al.; EMBO J. 4:3861, 1985), or by recombination in cells (Thummel et al., J. Mol. Appl. Genetics 1:435, 1982).

One preferred method is to construct plasmids using the adenovirus major late promoter (MLP) driving: (1) Sindbis non-structural proteins, and (2) a modified Sindbis vector construct. A modified Sindbis vector in this configuration would still contain a modified junction region, which would enable the RNA vector transcribed, to be self replicating as it would in a natural setting.

These plasmids can then be used to make adenovirus genomes *in vitro* (Ballay et al.. Embo. J. 4:3861, 1985). These adenoviral genomes, which are replication defective, are transfected in 293 cells (a human cell line making adenovirus E1A protein), to yield pure stocks of Sindbis structural proteins and Sindbis vector carried separately in defective adenovirus vectors. Since the titres of such vectors are typically 10⁷-10¹¹/ml, these stocks can be used to infect tissue culture cells simultaneously at high multiplicity of infection. The cells then produce Sindbis proteins and Sindbis vector genomes at high levels. Since the adenovirus vectors are defective, no large amounts of direct cell lysis will occur and Sindbis vectors can be harvested from the cell supernatants.

Other viral vectors such as those derived from unrelated Sindbis vectors (*e.g*., RSV, MMTV or HIV) may also be used in the same manner to generate vectors from primary cells. In one embodiment, these adenoviral vectors are used in conjunction with primary cells, giving rise to Sindbis vector preparations.

Alternative expression system has also been described in which chimeric HIV/poliovirus genomes, result in the generation of chimeric minireplicons (J. Virol. 65:2875, 1991), capable of expressing fusion proteins. These chimeric polio virus minireplicons were later demonstrated to be encapsidated and produce infectious particles by using a recombinant vaccinia virus (VV-P1) expressing the substituted polio virus capsid precursor P1 protein which is defective in the chimeric minireplicon (J. Virol. 67:3712. 1993). In the study, HIV-1 *gag-pol* sequences were substituted for the VP2 and VP3 capsid genes of the P1 capsid of poliovirus. In a similar fashion, the Sindbis vector genome can be substituted for the P1 capsid sequences and used in this system as a means for providing polio pseudo-typed Sindbis vectors after transfecting *in vitro* transcribed Sindbis RNA transcripts into the cell line. Conversely, Sindbis structural proteins can also be substituted for the VP2 and VP3 sequences, subsequently providing an alternative packaging cell line system for Sindbis based vectors.

In an alternative system, the following components are used:
1. Sindbis structural proteins made in the baculovirus system in a similar manner as described in Smith et al. (*supra*) (or in other protein production systems, such as yeast or *E*. *coli*)*;*
2. viral vector RNA made in the known T7 or SP6 or other *in vitro* RNA-generating system (Flamant et al., J. Virol. 62:1827, 1988);
3. tRNA made as in (2) or purified from yeast or mammalian tissue culture cells;
4. liposomes (with embedded *env* protein); and
5. cell extract or purified necessary components when identified (typically from mouse cells) to provide RNA processing, and any or other necessary cell-derived functions.

Within this procedure (1), (2) and (3) are mixed, and then env associated Sindbis proteins, cell extract and pre-liposome mix (lipid in a suitable solvent) are added. It may be necessary to embed the Sindbis *env* proteins in the liposomes prior to adding the resulting liposome-embedded env to the mixture of (1), (2), and (3). The mix is treated (*e.g.*, by sonication, temperature manipulation, or rotary dialysis) to allow encapsidation of the nascent viral particles with lipid plus embedded Sindbis *env* protein in a manner similar to that for liposome encapsidation of pharmaceuticals (Gould-Fogerite et al., Anal. Biochem. 148:15, 1985). This procedure produces high titres of replication incompetent Sindbis virus vectors without the requirement of establishing intermediate packaging cell lines.

### EXAMPLE 9

### CELL LINE OR TISSUE SPECIFIC SINDBIS VECTORS-"HYBRID ENVELOPES"

The tissue and cell-type specificity of Sindbis virus is determined primarily by the virus-encoded envelope proteins, E1 and E2. These virion structural proteins are transmembrane glycoproteins embedded in a host cell-derived lipid envelope that is obtained when the viral particle buds from the surface of the infected cell. The envelope surrounds an icosahedral nucleocapsid, comprised of genomic RNA complexed with multiple, highly ordered copies of a single capsid protein. The E1 and the E2 envelope glycoproteins are complexed as heterodimers that appear to assemble into trimeric structures, forming the characteristic "spikes" on the virion surface. In addition, the cytoplasmic tails of these proteins interact with the nucleocapsids, initiating the assembly of new viral particles (Virology 193:424, 1993). Properties ascribed to the individual Sindbis glycoproteins include, receptor binding by glycoprotein E2 (Virology 181:694, 1991), and glycoprotein E1 mediated fusion of the virion envelope and the endosomal membrane, resulting in delivery of the nucleocapsid particle into the cytoplasm (New Aspects of Positive-Stranded RNA Virus, pp. 166-172, 1990).

The present disclosure recognizes that by disrupting glycoprotein activity (in particular, but not limited to. E2) and co-expressing an intact heterologous glycoprotein, or by creating hybrid envelope gene products (i.e., specifically, a Sindbis envelope glycoprotein having its natural cytoplasmic and membrane-spanning regions and exogenous binding domains not found naturally in the same protein molecule), or by replacing the E2 and/or E1 glycoproteins with those of other alphaviruses or their derivatives which differ from Sindbis in their tissue tropism, the host range specificity may be altered without disrupting the cytoplasmic functions required for virion assembly. Thus, recombinant Sindbis vector particles can be produced which will bind specifically to pre-selected target cells, depending on the tropism of the protein molecule or domain introduced.

In the first configuration, substitution of the analagous envelope glycoproteins E1 and/or E2 from other alphaviruses or their variants is used to alter tissue tropism. For example, Venezuelan equine encephalitis virus (VEE) is an alphavirus which exhibits tropism for cells of lymphoid origin, unlike its Sindbis virus counterpart. Therefore, Sindbis vectors packaged in cells line expressing the VEE structural proteins will display the same lymphotropic properties as the parental VEE virus from which the packaging cell structural protein gene cassette was obtained.

Specifically, the Trinidad donkey strain of VEE virus (ATCC #VR-69) is propagated in BHK cells, and virion RNA is extracted using procedures similar to those described for the cloning of Sindbis. The entire structural protein coding region is amplified with a primer pair whose 5'-ends map, respectively, to the authentic AUG translational start site, including the surrounding Kozak consensus sequence, and UGA translational stop site. The forward primer is complementary to VEE nucleotides 7553-7579, and the reverse primer is complementary to VEE nucleotides 11206-11186 (sequence from Virology 170:19) PCR amplification of VEE cDNA corresponding to the structural protein genes is accomplished using a two-step reverse transcriptase-PCR protocol as described for Sindbis, VEE genome RNA as template, and the following oligonucleotide pair:
Forward primer (VEE 7553F):
   5'-TATATGCGGCCGCACCGCCAAGATGTTCCCGTTCCAGCCA-3' (SEQ. ID NO. 49)
Reverse primer (VEE 11206R):
   5'-TATATGCGGCCGCTCAATTATGTTTCTGGTTGGT-3' (SEQ. ID NO. 50)

In addition to their respective complementarities to the indicated VEE nucleotides, each primer includes a NotI recognition sequence at their 5' ends. Following PCR amplification, the 3800 bp fragment is purified in a 1% agarose gel, then subsequently digested with the enzyme NotI. The resulting fragment is then ligated separately into the pOP 13 and pOPRSV1 vectors (Stratagene) described previously, which are digested with Not I and treated with calf intestinal alkaline phosphatase. These resulting vectors, which contain the entire VEE structural protein coding sequence are known as pOP13-VEESP and pOPRSV1-VEESP. The use of these clones in the development of packaging cell lines follows that described for Sindbis packaging lines. In addition, variations of the lac operon-VEE structural protein gene expression vectors also are constructed using the systems outlined for Sindbis in this patent, and standard techniques known in the art. Furthermore, variants of VEE, and other alphaviruses and their variants differing in tissue tropism, are useful when following this approach.

In the second configuration, a heterologous glycoprotein or cellular ligand is expressed in the lipid bilayer of a packaging cell line which is able to produce enveloped Sindbis vector particles. This configuration is similar to one described in Example 6, for the production of VSV-G pseudo-typed Sindbis vectors; except that in this configuration, the E2 receptor-binding function is inactivated by insertional, deletion, or base-specific sequence mutagenesis. The receptor binding function of E2 is inactivated to restrict vector particle tropism to that which is supplied by the heterologous glycoprotein or cellular ligand. In addition to the example of VSV-G pseudo-typing, other viral glycoproteins which target specific cellular receptors (such as the retroviral HIV gp120 protein for CD4 cell targeting) are utilized when expressed from standard vectors stably transfected into Sindbis packaging cell lines.

In the third configuration, chimeric glycoproteins also are prepared, which allow for targeting of Sindbis viral vectors into particular cell lines *in vitro* or tissue types *in vivo.* To construct such a chimeric glycoprotein, specific oligo primers containing the ligand binding domain of the desired receptor, plus homologous Sindbis sequences (which include a unique specific restriction endonuclease site), are used to amplify an insert sequence that can be substituted into the Sindbis structural protein expression vector. Alternatively, limited Bal-31 digestions from a convenient restriction enzyme site are performed, in order to digest back to a permissive insertion site, followed by blunt end ligation of a fragment encoding a small receptor binding domain, or an entire viral glycoprotein or cell surface ligand. As an example, peptides corresponding to the principal neutralizing domain of the HIV gp120 envelope protein (Virology 185:820, 1991) can be used to disrupt normal E2 tropism and provide CD4 cell targeting.

While the HIV gp120 example illustrates one hybrid protein, the possibilities are not limited to viral glycoproteins. For example, the receptor binding portion of human interleukin-2 is combined with the envelope protein(s) of Sindbis to target vectors into cells with IL-2 receptors. Furthermore, the foregoing technique is used to create a recombinant Sindbis vector particle with envelope proteins that recognize Fc portions of antibodies. Monoclonal antibodies which recognize only preselected target cells are then bound to such Fc receptor-bearing Sindbis vector particles, such that the vector particles bind to and infect only those preselected target cells (for example, tumor cells). Alternatively, a hybrid envelope with the binding domain of avidin is used to target cells that have been coated with biotinylated antibodies or other ligands. The patient is first flooded with antibodies, and then allowed time to clear unbound and nonspecifically-bound antibody before administering the vector. The high affinity (10-15) of the avidin binding site for biotin will allow accurate and efficient targeting to the original tissue identified by the monoclonal "image."

### EXAMPLE 10

### DETERMINATION OF VECTOR UNITS IN A PREPARATION BY INFECTION OF A β-GALACTOSIDASE EXPRESSING CELL LINE UNDER THE CONTROL OF THE SINDBIS JUNCTION REGION

### DETERMINATION OF VECTOR UNITS IN A PREPARATION BY INFECTION OF A β-GALACTOSIDASE EXPRESSING REPORTER CELL LINE

In order to administer the proper therapeutic dose of vector to individuals, it is desirable to derive a method by which the vector infectious units contained in a preparation can be determined easily. This is accomplished by the generation of a cell line which expresses β-galactocidase or another reporter gene only when functional Sindbis nonstructural proteins are present in the cell. The cell line can be infected with increasing dilutions of a Sindbis vector preparation such that individual cells are not infected with more than one vector particle, allowing the titer, or vector units, to be determined. Thus, the cell line is an assay of functional particles present in a vector preparation.

### A. Generation of a cell line which expresses functional β-galactosidase protein under the control of Sindbis nonstructural proteins.

In one configuration, a eukaryotic expression cassette is constructed which contains a 5'-end sequence capable of initiating transcription of Sindbis RNA, a Sindbis junction region, a reporter gene, and a 3'-end Sindbis RNA polymerase recognition sequence for minus-strand synthesis. This cassette is positioned in an antisense orientation, adjacent to a eukaryotic transcriptional promoter. Additionally, these constructs also may contain a catalytic ribozyme sequence immediately adjacent to Sindbis nucleotide 1 of the 5'-end sequence which will result in cleavage of the primary RNA transcript precisely after this Sindbis nucleotide. In this antisense orientation, the reporter gene cannot be translated and is dependent entirely on the presence of Sindbis nonstructural proteins for transcription into positive stranded mRNA prior to reporter gene expression. These non-structural proteins will be provided by the Sindbis vector preparation being titered. In addition, this configuration, if designed to contain the precise Sindbis genome 5'- and 3'-end sequences, will allow for the reporter gene transcripts to undergo amplification by utilizing the same nonstructural proteins provided by the Sindbis vector.

An example of this antisense titering construction is as follows. Plasmid pSKSINB V-lacZ is digested with the enzyme SacI, which cleaves immediately after the Sindbis 3'-end and poly A sequence. The protruding 3'-end is made blunt by the addition of the enzyme T4 DNA Polymerase and dNTPs, followed by incubation for 10 minutes at 16°C. The T4 DNA Polymerase is heat inactiveated by incubation at 75°C for 15 minutes. The linearized plasmid subsequently is digested with the enzyme Bam HI, which cuts at nucleotide 7335, upstream of the Sindbis junction region. The resulting fragment is purified in a 1% agarose gel and ligated with pMCS-26s (described in Example 7) that has been digested with the enzyme Xba I, blunt-ended as described above, digested with the Bam HI, purified in a 1% agarose gel, and dephosphorylated with calf intestinal alkaine phosphatase.

The resulting construct, known as pMCS-LacZ, is then modified to contain the 5'-end 299 nucleotides of Sindbis, fused to an 84 nucleotide ribozyme sequence from the antigenomic strand of hepatitis delta virus (HDV), using overlapping PCR amplification (described in detail, Example 7). Two primer pairs are used initially in separate PCR reactions, followed by their overlapping synthesis in a second round of PCR. Reaction #1 contains primers HDV17-68 and HDV49-XC and reaction #2 contains primers SIN-HDV and SIN276-SPE and plasmid pVGSP6GEN as template. PCR amplification is accomplished by a standard two-temperature cycling protocol. After the first round of PCR amplification. 1/20th of the total amounts from each of reaction #1 and reaction #2 is combined and used as template in a second round of PCR amplification with primers HDV49-XC and SIN276-SPE and a standard two-temperature cycling protocol. Following the second round of PCR, the 414 bp amplicon is purified with the Mermaid Kit (Bio101, La Jolla, CA), and digested with the enzymes ClaI and SpeI. The digested amplicon is purified in a 1% agarose gel, and subsequently ligated into plasmid pMCS-LacZ, which also is digested with ClaI and SpeI and purified in a 1% agarose gel. The resulting construct, containing the expression cassette elements HDV antigenomic ribozyme/Sindbis 5'-end 299 nts./junction region/lacZ gene/3'-end untranslated region, is known as pd5'LacZ.

The LacZ expression cassette from plasmid pd5'LacZ subsequently is inserted into pcDNA3 (Invitrogen Corp., San Diego, CA). Plasmid pd5'LacZ is digested with the enzyme Not I, blunt-ended as described above, and then digested with the enzyme Xba I. The fragment is purified in a 1% agarose gel and ligated with pcDNA3 that is digested with the enzyme Hind III, blunt-ended, then digested with Xba I. The resulting construct, containing a CMV promoter which transcribes an antisense reporter cassette RNA of the configuration Sindbis 3'-end sequence/LacZ gene/junction region/Sindbis 5'-end sequence/HDV ribozyme, is known as pSINjraβ-gal.

BHKSINjraβ-gal cells are derived by transfection of 5 x 10⁵ BHK-21 cells, grown in a 60 mm petri dish, with 5 ug of the pSINjraβ-gal vector complexed with the polycation reagent Transfectam (Promega, Madison WI). At 24 hr. post-transfection, the media is supplemented with 400 ug/ml of G418 (GibcoBRL, Gaithersburg, MD). After all non-transfected cells have died and G418 resistant colonies have begun dividing, the cells are removed from the plate by trypsinization, pooled, then cloned by limiting dilution. Several clones are tested for the production of functional β-galactocidase by infection with a known titer of a wild-type stock of Sindbis virus. Production of functional b-galactocidase in candidate BHKSINjraβ-gal clones is determined at 6 hr. post-infection by first fixing PBS-rinsed cells with a solution containing 2% formaldehyde (37% stock solution)/0.2% glutaraldhehyde, then staining the cells with a solution containing 0.5 mM potassium ferricyanide/0.5 mM potassium ferrocyanide/2 mM MgCl₂/l mg/ml X.gal. Blue cells are clearly visible within 3 hr. Provided that the Sindbis virus stock does not contain a high level of defective interfering (DI) particles, the virus titer as determined by plaque assay on BHK-21 cells should be similar to the titer observed by X-gal staining on BHKSINjraβ-gal cells.

The titer of various Sindbis vector preparations, in vector units, produced from packaging cell lines such as those described in Example 7, is determined by infection of confluent monolayers of BHKSINjraβ-gal cells with several dilutions of vector. The titer of the vector preparation is determined at 6 hr. post-infection by visualization of cells producing β-galactocidase protein, as described above. Since the Sindbis vectors described do not contain the viral region corresponding to the structural genes, it is not possible to determine the titer of a vector preparation by plaque assay in BHK-21 cells.

Alternatively, a titering cell line is produced by using a different reporter cassette configuration, which consists of a eukaryotic promoter/5'-end Sindbis sequence recognized by the viral transcriptase/Sindbis junction region/reporter gene/Sindbis RNA polymerase recognition sequence for minus-strand synthesis, and is expressed in a sense-orientation. This reporter expression cassette requires synthesis, by vector-supplied Sindbis nonstructural proteins, into an antisense RNA molecule, prior to transcription of the subgenomic message encoding the reporter gene.

Specifically, the sense-orientation packaging construct is created as follows. Plasmid pVGELVIS is digested with the enzyme Apa I, which cleaves at nucleotide 11737, just downstream of the Sindbis 3'-end. The Apa I-digested DNA is blunt-ended by the addition of T4 DNA polymerse and dNTPs and incubation at 16°C for 10 minutes. After heat inactivation of the polymerase, the DNA fragment is digested with the enzyme Sfi I, and the 10041 bp fragment is purified in a 1% agarose gel. Plasmid pSKSINBV-lacZ is digested with the enzyme Sac I and blunt-ended as described above. The fragment is then digested with Sfi I, and the 7 kbp fragment is purified in a 1% agarose gel. The 7kb pSKSINBV-lacZ fragment then is ligated into the purified pVGELVIS fragment to create the plasmid pELVIS-bgal. This plasmid contains the complete Sindbis nonstructural proteins, Sindbis junction region, LacZ gene and Sindbis 3'-end replicase recognition sequence under the control of the MuLV LTR promoter. Plasmid pELVIS-bgal is digested with BspE I, purified by Geneclean (Bio 101 corp., San Diego, CA) and religated to itself. BspE I removes the Sindbis nonstructural protein gene sequences between nts. 422-7054. The re-ligated construct contains a 5' sequence that is capable of initiating transcription of Sindbis RNA, Sindbis junction region, sequences encoding the LacZ gene, and Sindbis 3'-end sequences required for synthesis of the minus-strand RNA, all downstream, and under the transcriptional control of a MuLV-LTR promoter. This construct is known as pELVISdINSP-bgal.

Plasmid pELVISdINSP-bgal is transfected into BHK cells and tested as described previously. The BHK pELVISdINSP-bgal cells produces an RNA transcript with a 5'-end sequence that is recognized by the Sindbis transcriptase, a Sindbis junction region, sequences encoding the LacZ gene, and Sindbis 3'-end sequences required for synthesis of the minus-strand RNA. β-galactosidase expression from the primary transcript is prevented because of an upstream open-reading frame and stop codons created by the BspEI deletion. The addition of Sindbis nonstructural proteins, provided by the Sindbis vector being titered, will result in transcription of active LacZ transcripts from the Sindbis junction region, after initial synthesis of an antisense intermediate. Furthermore, this configuration, if designed to contain the precise Sindbis genome 5'- and 3'-end sequences, will allow for the reporter gene transcripts to undergo amplification by utilizing the same nonstructural proteins provided by the Sindbis vector.

In another configuration, a titering cell line is produced using an expression cassette containing an antisense reporter gene followed by the 3'-end Sindbis replicase recognition sequences, positioned in the sense-orientation. This construct, under the control of a eukaryotic promoter, produces an RNA transcript that is recognized and transcribed by Sindbis nonstructural proteins provided by the vector to be titered. The Sindbis nonstructural proteins recognize sequences in the primary reporter transcript, and in turn, synthesize a sense reporter transcript. This construct does not benefit from amplification of the reporter gene transcript, but should still provide sufficient transcripts to allow for vector titering.

Construction of this type of titering cassette is as follows. pSV-B-galactosidase vector (Promega Corp., Madison, WI) is digested with the enzyme Hind III and blunt-ended as described above. The plasmid is further digested with the enzymes BamHI and Xmn I to remove the LacZ gene, and reduce the size of the remaining fragment. The 3737 nt. fragment, containing the LacZ gene, is purified in a 1% agarose gel and ligated into pcDNA3 (Invitrogen, San Diego, CA) that has been digested with the enzymes Bam HI and EcoRV. The new plasmid construct is known as pcDNAaLacZ. This plasmid is digested with the enzyme Apa I, blunt-ended as above, and further digested with the enzyme Xho I. Plasmid pSKSINBV (described previously) is digested with Sac I, blunt-ended as before, and then digested with Xho I. The resulting 146 nt. fragment containing the Sindbis 3' replicase recognition sequence is purified in a 1.2% agarose gel, ligated into the digested pcDNAaLacZ vector. The re-ligated construct contains an antisense LacZ gene and a 3' Sindbis replicase protein recognition sequence downstream from a CMV promoter. The resulting construct is known as pcDNAaLacZ-3'Sin. The construct is transfected into BHK cells and utilized as described previously.

### EXAMPLE 11

### GENERATION OF VECTOR CONSTRUCTS WHICH EXPRESS HBV ANTIGENS FOR THE INDUCTION OF AN IMMUNE RESPONSE

### A. ISOLATION OF HBV E/CORE SEQUENCE

A 1.8 Kb BamH I fragment containing the entire precore/core coding region of hepatitis B is obtained from plasmid pAM6 (ATCC No 45020) and ligated into the BamH I site of KS II⁺ (Stratagene, La Jolla, CA). This plasmid is designated KS II⁺ HBpc/c. Xho I linkers are added to the Stu I site of precore/core in KS II⁺ HBpc/c (at nucleotide sequence 1,704), followed by cleavage with Hinc II (at nucleotide sequence 2,592). The resulting 877 base pair Xho I-Hinc II precore/core fragment is cloned into the Xho I/Hinc II site of SK II⁺. This plasmid is designated SK⁺HBe.

### B. PREPARATION OF SEQUENCES UTILIZING PCR

### 1. Site-Directed Mutagenesis of HBV e/core Sequence Utilizing PCR

The precore/core gene in plasmid KS II⁺ HB pc/c is sequenced to determine if the precore/core coding region is correct. This sequence was found to have a single base-pair deletion which causes a frame shift at codon 79 that results in two consecutive in-frame TAG stop codons at codons 84 and 85. This deletion is corrected by PCR overlap extension (Ho et al., Gene 77:51, 1989) of the precore/core coding region in plasmid SK⁺ HBe. Four oligonucleotide primers are used for the 3 PCR reactions performed to correct the deletion.

The first reaction utilizes two primers. The sense primer sequence corresponds to the nucleotide sequence 1,805 to 1,827 of the *adw* strain and contains two Xho I restriction sites at the 5' end. The nucleotide sequence numbering is obtained from Genbank (Intelligenics, Inc., Mountain View, CA).
5' CTC *GAG* CTC *GAG* GCA CCA GCA CCA TGC AAC TTT TT-3' (SEQ. ID NO. 51)

The second primer sequence corresponds to the anti-sense nucleotide sequence 2,158 to 2,130 of the *adw* strain of hepatitis B virus, and includes codons 79, 84 and 85.
5'-CTA CTA GAT CCC TAG ATG CTG GAT CTT CC-3' (SEQ. ID NO. 52)

The second reaction also utilizes two primers. The sense primer corresponds to nucleotide sequence 2,130 to 2,158 of the *adw* strain, and includes codons 79, 84 and 85.
5'-GGA AGA TCC AGC ATC TAG GGA TCT AGT AG-3' (SEQ. ID NO. 53)

The second primer corresponds to the anti-sense nucleotide sequence from SK⁺ plasmid polylinker and contains a Cla I site 135 bp downstream of the stop codon of the HBV precore/core coding region.
5'-GGG CGA TAT CAA GCT TAT CGA TAC CG-3' (SEQ. ID NO. 54)

The third reaction also utilizes two primers. The sense primer corresponds to nucleotide sequence 5 to 27 of the *adw* strain, and contains two Xho I restriction sites at the 5' end.
5'- CTC *GAG* CTC *GAG* GCA CCA GCA CCA TGC AAC TTT TT (SEQ. ID NO. 55)

The second primer sequence corresponds to the anti-sense nucleotide sequence from the SK⁺ plasmid polylinker and contains a Cla I site 135 bp downstream of the stop codon of the HBV precore/core coding region.
5'-GGG CGA TAT CAA GCT TAT CGA TAC CG-3' (SEQ. ID NO. 56)

The first PCR reaction corrects the deletion in the antisense strand and the second reaction corrects the deletion in the sense strands. PCR reactions one and two correct the mutation from CC to CCA which occurs in codon 79 and a base pair substitution from TCA to TCT in codon 81. Primer 1 contains two consecutive Xho I sites 10 bp upstream of the ATG codon of HBV e coding region and primer 4 contains a Cla I site 135 bp downstream of the stop codon of HBV precore/core coding region. The products of the first and second PCR reactions are extended in a third PCR reaction to generate one complete HBV precore/core coding region with the correct sequence.

The PCR reactions are performed using the following cycling conditions: The sample is initially heated to 94°C for 2 minutes. This step, called the melting step, separates the double-stranded DNA into single strands for synthesis. The sample is then heated at 56°C for 30 seconds. This step, called the annealing step, permits the primers to anneal to the single stranded DNA produced in the first step. The sample is then heated at 72°C for 30 seconds. This step, called the extension step, synthesizes the complementary strand of the single stranded DNA produced in the first step. A second melting step is performed at 94°C for 30 seconds, followed by an annealing step at 56°C for 30 seconds which is followed by an extension step at 72°C for 30 seconds. This procedure is then repeated for 35 cycles resulting in the amplification of the desired DNA product.

The PCR reaction product is purified by 1.5% agarose gel electrophoresis and transferred onto NA 45 paper (Schleicher and Schuell, Keene, New Hampshire). The desired 787 bp DNA fragment is eluted from the NA 45 paper by incubating for 30 minutes at 65°C in 400 µl high salt buffer (1.5 M NaCl, 20mM Tris, pH 8.0, and 0.1mM EDTA). Following elution, 500 µl of phenol:chloroform:isoamyl alcohol (25:24:1) is added to the solution. The mixture is vortexed and then centrifuged 14,000 rpm for 5 minutes in a Brinkmann Eppendorf centrifuge (5415L). The aqueous phase, containing the desired DNA fragment, is transferred to a fresh 1.5 ml microfuge tube and 1.0 ml of 100% EtOH is added. This solution is incubated on dry ice for 5 minutes, and then centrifuged for 20 minutes at 10,000 rpm. The supernatant is decanted, and the pellet is rinsed with 500 µl of 70% EtOH. The pellet is dried by centrifugation at 10,000 rpm under vacuum, in a Savant Speed-Vac concentrator, and then resuspended in 10 µl deionized H₂O. One microliter of the PCR product is analyzed by 1.5% agarose gel electrophoresis. The 787 Xho I-Cla I precore/core PCR amplified fragment is cloned into the Xho I-Cla I site of SK⁺ plasmid. This plasmid is designated SK⁺HBe-c. *E. coli* (DH5 alpha, Bethesda Research Labs, Gaithersburg, MD) is transformed with the SK⁺HBe-c plasmid and propagated to generate plasmid DNA. The plasmid is then isolated and purified, essentially as described by Bimboim et al. (Nuc. Acid Res. 7:1513, 1979; *see also* Molecular Cloning: A Laboratory Manual, Sambrook et al. (eds.), Cold Spring Harbor Press, 1989). The SK+HBe-c plasmid is analyzed to confirm the sequence of the precore/core gene (Figure 4).

### 2. Isolation of HBV Core Sequence

The single base pair deletion in plasmid SK⁺ HBe is corrected by PCR overlap extension as described in Example 9B. Four oligonucleotide primers are used for the PCR reactions performed to correct the mutation.

The first reaction utilizes two primers. The sense primer corresponds to the nucleotide sequence for the T-7 promoter of SK⁺HBe plasmid.
5'-AAT ACG ACT CAC TAT AGG G-3' (SEQ. ID NO. 57)

The second primer corresponds to the anti-sense sequence 2,158 to 2,130 of the *adw* strain, and includes codons 79, 84 and 85.
5'-CTA CTA GAT CCC TAG ATG CTG GAT CTT CC-3' (SEQ. ID NO. 58)

The second reaction utilizes two primers. The anti-sense primer corresponds to the nucleotide sequence for the T-3 promoter present in SK⁺HBe plasmid.
5'-3': ATT AAC CCT CAC TAA AG (SEQ. ID NO. 59)

The second primer corresponds to the sense nucleotide sequence 2,130 to 2,158 of the *adw* strain, and includes codons 79, 84 and 85.
5'-GGA AGA TCC AGC ATC TAG GGA TCT AGT AG-3' (SEQ. ID NO. 60)

The third reaction utilizes two primers. The anti-sense primer corresponds to the nucleotide sequence for the T-3 promoter present in SK⁺HBe plasmid.
5'-ATT AAC CCT CAC TAA AG-3' (SEQ. ID NO. 61)

The second primer corresponds to the sense sequence of the T-7 promoter present in the SK⁺HBe plasmid.
5'-AAT ACG ACT CAC TAT AGG G-3' (SEQ. ID NO. 62)

The PCR product from the third reaction yields the correct sequence for HBV precore/core coding region.

To isolate HBV core coding region, a primer is designed to introduce the Xho I restriction site upstream of the ATG start codon of the core coding region, and eliminate the 29 amino acid leader sequence of the HBV precore coding region. In a fourth reaction, the HBV core coding region is produced using the PCR product from the third reaction and the following two primers.

The sense primer corresponds to the nucleotide sequence 1,885 to 1,905 of the *adw* strain and contains two Xho I sites at the 5' end.
5'-CCT CGA GCT CGA GCT TGG GTG GCT TTG GGG CAT G-3' (SEQ. ID NO. 63)

The second primer corresponds to the anti-sense nucleotide sequence for the T-3 promoter present in the SK⁺ HBe plasmid. The approximately 600 bp PCR product from the fourth PCR reaction contains the HBV core coding region and novel Xho I restriction sites at the 5' end and Cla I restriction sites at the 3' end that was present in the multicloning site of SK⁺ HBe plasmid.
5'-ATT ACC CCT CAC TAA AG-3' (SEQ. ID NO. 64)

Following the fourth PCR reaction, the solution is transferred into a fresh 1.5 ml microfuge tube. Fifty microliters of 3 M sodium acetate is added to this solution followed by 500 µl of chloroform:isoamyl alcohol (24:1). The mixture is vortexed and then centrifuged at 14,000 rpm for 5 minutes. The aqueous phase is transferred to a fresh microfuge tube and 1.0 ml 100% EtOH is added. This solution is incubated at -20°C for 4.5 hours, and then centrifuged at 10,000 rpm for 20 minutes.
The supernatant is decanted, and the pellet rinsed with 500 µl of 70% EtOH. The pellet is dried by centrifugation at 10,000 rpm under vacuum and then resuspended in 10 µl deionized H₂O. One microliter of the PCR product is analyzed by 1.5% agarose gel electrophoresis.

### 3. Isolation of HBV X Antigen

A 642 bp NCO I - Taq I fragment containing the hepatitis B virus X open reading frame is obtained from the pAM6 plasmid (adw) (ATCC 45020), blunted by Klenow fragment, and ligated into the Hinc II site of SK⁺ (Stratagene, La Jolla, California). E. *coli* (DH5 alpha, Bethesda Research Laboratories, Gaithersburg, MD) is transformed with the ligation reaction and propagated. Miniprep DNA is then isolated and purified, essentially as described by Birnboim et al. (Nuc. Acid Res. 7:15134, 1979; Molecular Cloning A Laboratory Manual, Sambrook et al. (eds.), Cold Spring Harbor Press, 1989).

Since this fragment can be inserted in either orientation, clones are selected that have the sense orientation with respect to the Xho I and Cla I sites in the SK⁺ multicloning site. More specifically, miniprep DNAs are digested with the diagnostic restriction enzyme, Bam HI. Inserts in the correct orientation yield two fragments of 3.0 Kb and 0.6 Kb in size. Inserts in the incorrect orientation yield two fragments of 3.6 Kb and 0.74 Kb. A clone in the correct orientation is selected and designated SK-X Ag.

### 4. Construction of Sindbis Vectors Expressing HBVe-c, HBV Core and HBV X

Construction of a Sindbis vector expressing the HBVe-c sequence is accomplished by digesting the SK⁺HB e-c plasmid with Xho I and Cla I restriction enzyme sites to release the cDNA fragment encoding HBVe-c sequences. The fragment is then isolated by agarose gel electrophoresis, purified Gene Clean™ (BIO101, San Diego, CA), and inserted into the desired Sindbis vector backbone, prepared by digestion with Xho I and Cla I, and treated with CIAP. The Sindbis vectors described in Example 2, are suitable for the insertion of the HBV antigen sequences. Such Sindbis vectors include pKSSINBV, pKSSINdIJRsjrc, pKSSINdIJRsjrPC, pKSSINdIJRsjrNP(7582-7601) and pKSSINdIJRsexjr.

Construction of a Sindbis vector expressing the HBV core sequence is accomplished by Gene Clean™ treatment of the PCR product described above. The amplified product is then digested with Xho I and Cla I restriction enzyme sites, isolated with agarose gel electrophoresis, purified by Gene Clean™ and ligated into the same Sindbis vectors described above pre-treated with Xho I and Cla I enzymes.

[ Construction of a Sindbis vector expressing the HBV-X antigen sequence is accomplished by digesting the plasmid SK-X Ag with Xho I and Cla I restriction enzyme sites to release the cDNA fragment encoding HBV-X sequences.
The fragment is isolated by agarose gel electrophoresis, purified using Gene Clean™, and inserted into the desired Sindbis vector backbones, described above, pre-treated with Xho I and Cla I enzymes.
The above Sindbis HBV expressing vectors may also be modified to coexpress a selectable drug resistance marker dependent on the requirements of the experiment or treatment of the vector infected cells. Any of the above Sindbis HBV expression vectors described may also be designed to coexpress for G418 resistance. This is accomplished by incorporating an internal ribozyme entry site (Example 5) followed by the bacterial neomycin phosphotransferase gene placed 3' of the HBV coding sequences and 5' of the terminal 3' end of the vector using the multiple cloning site of the vector. These G418 resistant vector constructs can be used for selecting vector infected cells for the generation of HBV specific CTL targets in the following sections. ]

### D. EXPRESSION OF INFECTED CELLS WITH SINDBIS VECTORS

### 1. ELISA

Cell lysates from cells infected by any of the HBV expressing vectors are made by washing 1.0 x 10⁷ cultured cells with PBS, resuspending the cells to a total volume of 600 µl in PBS, and sonicating for two 5-second periods at a setting of 30 in a Branson sonicator, Model 350 (Fisher, Pittsburgh, PA) or by freeze thawing three times. Lysates are clarified by centrifugation at 10,000 rpm for 5 minutes.

Core antigen and precore antigen in cell lysates and secreted e antigen in culture supernatant are assayed using the Abbott HBe, rDNA EIA kit (Abbott Laboratories Diagnostic Division, Chicago, IL). Another sensitive EIA assay for precore antigen in cell lysates and secreted e antigen in culture supernatant is performed using the Incstar ETI-EB kit (Incstar Corporation, Stillwater, MN). A standard curve is generated from dilutions of recombinant hepatitis B core and e antigen obtained from Biogen (Geneva, Switzerland).

Using these procedures approximately 10 ng/ml e antigen is expressed in transduced cell lines.

### 2. Immunoprecipitation/Western Blot

Characterization of the precore/core and e antigens expressed by vector infected cells is performed by immunoprecipitation followed by Western blot analysis. Specifically, 0.5-1.0 ml of cell lysate in PBS or culture supernatant is mixed with polyclonal rabbit anti-hepatitis B core antigen (DAKO Corporation, Carpinteria, California) bound to G-Sepharose (Pharmacia LKB, Uppsala, Sweden) and incubated overnight at 4°C. Samples are washed twice in 20 mM Tris-HCl, pH 8.0, 100 mM NaCl, 10 mM EDTA and boiled in sample loading buffer with 0.5% β 2-mercaptoethanol. Proteins are first resolved by SDS polyacrylamide gel electrophoresis, and then transferred to Immobilon (Millipore Corp., Bedford, ME) and probed with the DAKO polyclonal rabbit anti-hepatitis core antigen, followed by ¹²⁵I-protein A.

### E. TESTING IMMUNE RESPONSE

### 1. Cytotoxicity Assays

### (a) Inbred Mice

Six- to eight-week-old female Balb/C, C57B1/6 and C3H mice (Harlan Sprague-Dawley, Indianapolis, IN) are injected twice intraperitoneally (i.p.) at 1 week intervals with 1 x 10⁷ irradiated (10,000 rads at room temperature) Sindbis vector infected L-M(TK-) cells (ATCC CLL 1.3). Animals are sacrificed 7 days later and the splenocytes (3 x 10⁶/ml) cultured *in vitro* with their respective irradiated Sindbis vector infected cells (6 x 10⁴/ml) in T-25 flasks (Corning, Coming, NY). Culture medium consists of RPMI 1640, 5% heat-inactivated fetal bovine serum, 1 mM sodium pyruvate, 50 µg/ml gentamycin and 10⁻⁵M β 2-mercaptoethanol (Sigma, St. Louis, MO). Effector cells are harvested 4-7 days later and tested using various effector:target cell ratios in 96 well microtiter plates (Coming, Coming, NY) in a standard chromium release assay. Targets are the vector infected and non-vector infected L-M(TK⁻) cells whereas the non-transduced cell lines are used as negative controls. Specifically, Na₂⁵¹CrO₄-labeled (Amersham, Arlington Heights, IL)(100 uCi, 1 hour at 37°C) target cells (1 x 10⁴ cells/well) are mixed with effector cells at various effector to target cell ratios in a final volume of 200 µl. Following incubation, 100 µl of culture medium is removed and analyzed in a Beckman gamma spectrometer (Beckman, Dallas, TX). Spontaneous release (SR) is determined as CPM from targets plus medium and maximum release (MR) is determined as CPM from targets plus 1M HCl. Percent target cell lysis is calculated as: [(Effector cell + target CPM) - (SR)/(MR) - (SR)] x 100. Spontaneous release values of targets are typically 10%-20% of the MR. For certain CTL assays, the effectors may be *in vitro* stimulated multiple times, for example, on day 8-12 after the primary *in vitro* stimulation. More specifically, 10⁷ effector cells are mixed with 6 x 10⁵ irradiated (10,000 rads) stimulator cells, and 2 x 10⁷ irradiated (3,000 rads) "filler" cells (prepared as described below) in 10 ml of "complete" RPMI medium. (RPMI containing: 5% heat inactivated Fetal Bovine Serum. 2 mM L-glutamine, I mM sodium pyruvate, 1X non essential amino acids, and 5 x 10⁵ M β2-mercaptoethanol). Stimulator cells for *in vitro* stimulation of effector cells are generated from infecting L-M (TK-) cells with a Sindbis HBV vector co-expressing G418 resistance. Select vector infected cells for marker selection using 800 µg/mL of G418 for two weeks. The G418 resistant cells are then irradiated at 10,000 rads and then used to restimulate effector cells *in vitro* as described. "Filler" cells are prepared from naive syngeneic mouse spleen cells resuspended in RPMI, irradiated with 3,000 rads at room temperature. Splenocytes are washed with RPMI, centrifuged at 3,000 rpm for 5 minutes at room temperature, and the pellet is resuspended in RPMI. The resuspended cells are treated with 1.0 ml tris-ammonium chloride (100 ml of 0.17 M tris base, pH 7.65, plus 900 ml of 0.155 M NH₄Cl; final solution is adjusted to a pH of 7.2) at 37°C for 3-5 minutes. The secondary *in vitro* restimulation is then cultured for 5-7 days before testing in a CTL assay. Any subsequent restimulations are cultured as described above with the addition of 2-10 U of recombinant human IL-2 (200 U/ml, catalog #799068, Boehringer Mannheim, W. Germany).

Using these procedures, it can be shown that CTLs to HBV e antigen can be induced.

### (b) HLA A2.1 Transgenic Mice

Six- to eight-week-old female HLA A2.1 transgenic mice (V. Engelhard, Charlottesville, VA) are injected twice intraperitoneally (i.p.) at one week intervals with 1.0 x 10⁷ irradiated (10,000 rads at room temperature) vector transduced EL4 A2/K^{b} cells (ATCC No. TIB-39). Animals are sacrificed 7 days later and the splenocytes (3 x 10⁶/ml) cultured *in vitro* with irradiated (10,000 rads) transduced Jurkat A2/K^{b} cells or with peptide coated Jurkat A2/K^{b} cells (6 x 10⁴/ml) in flasks (T-25, Coming, Corning, NY). The remainder of the chromium release assay is performed as described in Example 9E 1.a, where the targets are transduced and non-transduced EL4 A2/K^{b} and Jurkat A2/K^{b} cells. Non-transduced cell lines are utilized as negative controls. The targets may also be peptide coated EL4 A2/K^{b} cells.

### (c) Transduction of Human Cells With Vector Construct

Lymphoblastoid cell lines (LCL) are established for each patient by infecting (transforming) their B-cells with fresh Epstein-Barr virus (EBV) taken from the supernatant of a 3-week-old culture of B95-8, EBV transformed marmoset leukocytes (ATCC CRL 1612). Three weeks after EBV-transformation, the LCL are infected with Sindbis vector expressing HBV core or e antigen and G418 resistance. Vector infection of LCL is accomplished by co-culturing 1.0 x 10⁶ LCL cells with 1.0 x 10⁶ irradiated (10,000 rads) Sindbis vector producer cells in a 6 cm plate containing 4.0 ml of medium or by adding fectious vector supernatant. The culture medium consists of RPMI 1640, 20% heat inactivated fetal bovine serum (Hyclone, Logan, UT). 5.0 mM sodium pyruvate and 5.0 mM non-essential amino acids. After overnight co-culture at 37°C and 5% CO₂, the LCL suspension cells are removed from the irradiated (10,000 rads) Sindbis vector producer cells. Infected LCL cells are selected by adding 800 µg/ml G418. The Jurkat A2/K^{b} cells (L. Sherman, Scripps Institute, San Diego, CA) are infected essentially as described for the infection of LCL cells.

### (d) Human CTL assays

Human PBMC are separated by Ficoll (Sigma, St. Louis, MO) gradient centrifugation. Specifically, cells are centrifuged at 3,000 rpm at room temperature for 5 minutes. The PBMCs are restimulated *in vitro* with their autologous transduced LCL, Example 9E 1.c, at an effector:target ratio of 10:1 for 10 days. Culture medium consists of RPMI 1640 with prescreened lots of 5% heat-inactivated fetal bovine serum, 1 mM sodium pyruvate and 50 µg/ml gentamycin. The resulting stimulated CTL effectors are tested for CTL activity using infected autologous LCL or HLA-matched cells as targets in the standard chromium release assay, Example 9E 1.a. Since most patients have immunity to EBV, the non-transduced EBV-transformed B-cells (LCL) used as negative controls, will also be recognized as targets by EBV-specific CTL along with the transduced LCL. In order to reduce the high background due to killing of labeled target cells by EBV-specific CTL, it is necessary to add unlabeled non-transduced LCL to labeled target cells at a ratio of 50:1.

### 2. Detection of Humoral Immune Response

Humoral immune responses in mice specific for HBV core and e antigens are detected by ELISA. The ELISA protocol utilizes 100 µg/well of recombinant HBV core and recombinant HBV e antigen (Biogen, Geneva, Switzerland) to coat 96-well plates. Sera from mice immunized with cells or direct vector expressing HBV core or HBV e antigen are then serially diluted in the antigen-coated wells and incubated for 1 to 2 hours at room temperature. After incubation, a mixture of rabbit anti-mouse IgGI, IgG2a, IgG2b, and IgG3 with equivalent titers is added to the wells. Horseradish peroxidase ("HRP")-conjugated goat anti-rabbit anti-serum is added to each well and the samples are incubated for 1 to 2 hours at room temperature. After incubation, reactivity is visualized by adding the appropriate substrate. Color will develop in wells that contain IgG antibodies specific for HBV core or HBV e antigen.

### 3. T Cell Proliferation

Antigen induced T-helper activity resulting from two or three injections of direct vector preparations expressing HBV core or e antigen, is measured *in vitro.* Specifically, splenocytes from immunized mice are restimulated *in vitro* at a predetermined ratio with cells expressing HBV core or e antigen or with cells not expressing HBV core or e antigen as a negative control. After five days at 37°C and 5% CO₂ in RPMI 1640 culture medium containing 5% FBS, 1.0 mM sodium pyruvate and 10⁻⁵ β 2-mercaptoethanol, the supernatant is tested for IL-2 activity. IL-2 is secreted specifically by T-helper cells stimulated by HBV core or e antigen, and its activity is measured using the CTL clone, CTLL-2 (ATCC TIB 214). Briefly, the CTLL-2 clone is dependent on IL-2 for growth and will not proliferate in the absence of IL-2. CTLL-2 cells are added to serial dilutions of supernatant test samples in a 96-well plate and incubated at 37°C and 5%, CO₂ for 3 days. Subsequently, 0.5 µ Ci ³H-thymidine is added to the CTLL-2 cells. 0.5µCi ³H-thymidine is incorporated only if the CTLL-2 cells proliferate. After an overnight incubation, cells are harvested using a PHD cell harvester (Cambridge Technology Inc., Watertown, MA) and counted in a Beckman beta counter. The amount of IL-2 in a sample is determined from a standard curve generated from a recombinant IL-2 standard obtained from Boehringer Mannheim (Indianapolis, IN).

### F. ADMINISTRATION PROTOCOLS

### 1. Mice

### (a) Direct Vector Administration

The mouse system may also be used to evaluate the induction of humoral and cell-mediated immune responses with direct administration of vector encoding HBV core or e antigen. Briefly, six- to eight-week-old female Balb/C, C57B16 or C3H mice are injected intramuscularly (i.m.) with 0.1 ml of reconstituted (with sterile deionized, distilled water) lyophilized HBV core or HBV e expressing Sindbis vector. Two injections are given one week apart. Seven days after the second injection, the animals are sacrificed. Chromium release CTL assays are then performed essentially as described in Example 9E 1.a.

### 2. Chimpanzee Administration Protocol

The data generated in the mouse system described above is used to determine the protocol of administration of vector in chimpanzees chronically infected with hepatitis B virus. Based on the induction of HBV-specific CTLs in mice, the subjects in chimpanzee trials will receive three doses of vector encoding core or e antigen at 28 day intervals given in two successively escalating dosage groups. Control subjects will receive a placebo comprised of HBV-IT (V) formulation media. The dosage will be either 10⁶ or 10⁷ HBV-IT (V) cfu given in four 0.5 ml injections i.m. on each injection day. Blood samples will be drawn on days 4, 12, 24, 36, 52, 70 and 84 and months 6, 12, 18, 24, 30, and 36 in order to measure serum alanine aminotransferase (ALT) levels, the presence of hepatitis B e antigen, the presence of antibodies directed against the hepatitis B e antigen and to assess safety and tolerability of the treatment. The hepatitis B e antigen and antibodies to HB e antigen is detected by Abbott HB e rDNA EIA kit (Abbott Laboratories Diagnostic Division, Chicago, IL). Efficacy of the induction of CTLs against hepatitis B core or e antigen can be determined as in Example 9E 1.c.

Based on the safety and efficacy results from the chimpanzee studies, the dosage and inoculation schedule is determined for administration of the vector to subjects in human trials. These subjects are monitored for serum ALT levels, presence of HBV e antigen and the presence of antibodies directed against the HBV e antigen, essentially as described above. Induction of human CTLs against hepatitis B core or e antigen is determined as in Example 9E 1.c.

### EXAMPLE 12

### SINDBIS VECTORS EXPRESSING VIRAL PROTEINS FOR INDUCTION OF THE IMMUNE RESPONSE OR FOR BLOCKING VIRUS HOST CELL INTERACTIONS

The following example describes procedures for constructing Sindbis vectors capable of generating an immune response by expressing an HIV viral antigen. Methods are also given to test expression and induction of an immune response.

### SINDBIS VECTORS USED TO ELICIT AN IMMUNE RESPONSE

### A. HIV IIIB ENV EXPRESSION VECTOR

A 2.7 Kb Kpn I-Xho I DNA fragment was isolated from the HIV proviral clone BH10-R3 (for sequence, *see* Ratner et al., Nature 313:277, 1985) and a ~400 bp Sal I-Kpn I DNA fragment from IIIexE7deltaenv (a Bal31 deletion to nt. 5496) was ligated into the Sal I site in the plasmid SK⁺. From this clone, a 3.1 kb *env* DNA fragment (Xho I-Cla I) was purified and ligated into the previously described Sindbis vectors predigested with Xho I and Cla I.

### B. CREATION OF A PRODUCER CELL LINE WHICH EXPRESSES HIV SPECIFIC ANTIGENS

To construct a vector producing cell line that expresses the HIV IIIB *env* derived from the vector described above, *in vitro* transcribed RNA transcripts are transfected in a Sindbis packaging cell line (Example 7). Specifically, the Sindbis RNA vector molecules are initially produced by using a SP6 *in vitro* transcribed RNA polymerase system used to transcribe from a cDNA Sindbis vector clone encoding the HIV specific sequences. The generated *in vitro* RNA vector products, are then transfected into a Sindbis packaging or hopping cell line which leads to the production of transient infectious vector particles within 24 hours. These vector particles are then collected from the supernatants of the cell line cultures and then filtered through a 0.45 micron filter to avoid cellular contamination. The filtered supernatants are then used to infect a fresh monolayer of Sindbis packaging cells. Within 24 hours of infection, Sindbis vector particles are produced containing positive stranded Sindbis recombinant RNA encoding Sindbis non-structural proteins and HIV specific sequences.

An alternative configuration of a Sindbis HIV IIIB *env* vector is a promoter driven cDNA Sindbis construct containing a selectable marker. In this configuration the above described Xho I to Cla I fragment containing the specific HIV IIIB *env* sequence is placed in a similar cDNA Sindbis vector driven by a constitutive promoter in place of a bacteriophage polymerase recognition sequence. Using this configuration, the expression vector plasmids are transfected into the packaging cell line and selected for the drug resistance gene 24 to 48 hours post transfection. Resistant colonies are then pooled 14 days later (dependent on the selection marker used) and dilutioned cloned. Several dilution clones are then propagated, and assayed for highest vector titer. The highest titer clones are then expanded and stored frozen. The stored clones are tested for HIV specific protein production and immune response induction.

### C. TESTING FOR HIV SPECIFIC PROTEIN PRODUCTION AND AN IMMUNE RESPONSE

Cell lysates from the Sindbis HIV producer cell line are tested for HIV specific protein production by Western blot analysis. To test the ability of the vector to transfer expression *in vitro,* BHK-21 cells are infected with filtered supernatant containing viral vector and assayed by Western blot analysis 24 hours post infection. Once protein expression has been verified *in vivo* mouse and primate studies can be performed to demonstrate the ability of syngeneic cells expressing a foreign antigen after vector treatment to: (a) elicit a CTL response in mice by injecting either infected syngeneic cells or preparations of infectious vector; (b) elicit CTL responses in a human *in vitro* culture system; (c) to infect human, chimpanzee and macaque cells, including primary cells, so that these can be used to elicit CTL responses and can serve as targets in CTL assays; (d) map immune response epitopes; and (e) elicit and measure CTL responses to other non-HIV antigens such as mouse CMV(MCMV).

### 1. Immune Response to Sindbis Viral Vector-Encoded Antigens

To test the immune response elicited from a cell line transduced with a Sindbis HIV IIIB *env* vector, a murine tumor cell line (B/C10ME) (H-2^{d}) (Patek et al., Cell. Immunol. 72:113, 1982) is infected with a recombinant Sindbis virus carrying the HIV IIIB vector. The HIV *env* expressing cell line (B/C10ME-IIIB) was then utilized to stimulate HIV *env*-specific CTL in syngeneic (*i.e.*, MHC identical) Balb/c (H-2^{d}) mice. Mice are immunized by intraperitoneal injection with B/C10ME-IIIB cells (1 x 10⁷ cells) and boosted on day 7-14. (Boosting may not be required.) Responder spleen cell suspensions are prepared from these immunized mice and the cells cultured *in vitro* for 4 days in the presence of either B/C10ME-IIIB (BCenv) or B/C10ME (BC) mitomycin-C-treated cells at a stimulator:responder cell ratio of 1:50. The effector cells are harvested from these cultures, counted, and mixed with radiolabeled (⁵¹Cr) target cells (*i.e.*, B/C10MEenv-29 or B/C10ME) at various effector:target (E:T) cell ratios in a standard 4-5 hour ⁵¹Cr-release assay. Following incubation, the microtitre plates are centrifuged, 100 µl of culture supernate is removed, and the amount of radiolabel released from lysed cells quantitated in a Beckman gamma spectrometer. Target cell lysis was calculated as: % Target Lysis = Exp CPM - SR CPM/MR CPM - SR CPM x 100, where experimental counts per minute (Exp CPM) represents effectors plus targets; spontaneous release (SR) CPM represents targets alone; and maximum release (MR) CPM represents targets in the presence of 1M HCl.

### 2. Stimulation of an Immune Response in Mice by Direct Injection of Recombinant Sindbis Vector

Experiments are performed to evaluate the ability of recombinant Sindbis viral vectors to induce expression of HIV envelope proteins following direct injection in mice. Approximately 10⁴ to 10⁵ (pfu) of recombinant Sindbis virus carrying the HIV IIIB *env* vector construct are injected twice (2x) at 3-week intervals either by the intraperitoneal (i.p.) or intramuscular (i.m.) route. This amount of Sindbis virus is determined to be less than the amount considered to stimulate an immune response. Spleen cells are prepared for CTL approximately 7 to 14 days after the second injection of vector.

### D. BLOCKING AGENTS DERIVED FROM VIRAL PROTEIN ANALOGS EXPRESSED FROM RECOMBINANT SINDBIS VECTORS

Many infectious diseases, cancers, autoimmune diseases, and other diseases involve the interaction of viral particles with cells, cells with cells, or cells with factors. In viral infections, viruses commonly enter cells via receptors on the surface of susceptible cells. In cancers, cells may respond inappropriately or not at all to signals from other cells or factors. In autoimmune disease, there is inappropriate recognition of "self" markers. These interactions may be blocked by producing an analogue to either of the partners in an interaction, *in vivo.*

This blocking action may occur intracellularly, on the cell membrane, or extracellularly. The blocking action of a viral or, in particular, a Sindbis vector carrying a gene for a blocking agent, can be mediated either from inside a susceptible cell or by secreting a version of the blocking protein to locally block the pathogenic interaction.

In the case of HIV, the two agents of interaction are the gp 120/gp 41 envelope protein and the CD4 receptor molecule. Thus, an appropriate blocker would be a vector construct expressing either an HIV *env* analogue that blocks HIV entry without causing pathogenic effects, or a CD4 receptor analogue. The CD4 analogue would be secreted and would function to protect neighboring cells, while the gp 120/gp 41 is secreted or produced only intracellularly so as to protect only the vector-containing cell. It may be advantageous to add human immunoglobulin heavy chains or other components to CD4 in order to enhance stability or complement lysis. Delivery of a retroviral vector encoding such a hybrid-soluble CD4 to a host results in a continuous supply of a stable hybrid molecule.

Vector particles leading to expression of HIV *env* analogs may also be constructed as described above. It will be evident to one skilled in the art which portions are capable of blocking virus adsorption without overt pathogenic side effects (Willey et al., J. Virol. 62:139, 1988; Fisher et al., Science 233:655, 1986).

### EXAMPLE 13

### REPLACEMENT GENE THERAPY USING RECOMBINANT SINDBIS VECTORS FOR SYSTEMIC PROTEIN PRODUCTION. A TREATMENT FOR GAUCHER DISEASE

### A. CREATION OF A GLUCOCEREBROSIDASE SINDBIS VECTOR

A glucocerebrosidase (GC) cDNA clone containing an Xho I restriction enzyme site 5' of the cDNA coding sequence and a Cla I restriction enzyme site 3' of the cDNA coding sequence is first generated. The clone can be generated by digesting pMFG-GC (Ohashi et al., PNAS 89:11332, 1992) with Nco I, blunted with Vent DNA polymerase (New England Biolabs, Beverly, MA), then litigated to Xho I linkers. The plasmid is then digested with Bam HI, blunted with Vent DNA polymerase, then litigated to Cla I linkers. The fragment is then digested with Xho I and Cla I and litigated into the Xho I-Cla I site of the desired Sindbis vector.

### B. CONSTRUCTION OF A SINDBIS GC VECTOR PRODUCING CELL LINE

Two approaches are presented to create the producer cell line, depending on the Sindbis cDNA vector clone used. One type of Sindbis vector uses the SP6 RNA polymerase recognition sequence for the production of replication competent *in vitro* RNA transcripts. This type of vector is used to create a vector producer cell line based on a non-integrating vector which would persistently replicate in the packaging cell line. The second type of vector uses a heterologous promoter (Example 7) in a plasmid construct which are allowed stable integration in the packaging cell line. Replication competent vector transcripts are then transcribed in the transfected packaging cell line.

If the Sindbis GC viral vector uses a SP6 RNA recognition sequence, the cDNA must first be transcribed *in vitro* using any of the *in vitro* transcription systems available commercially (Megascript™ transcription kit; Ambion Inc., Austin, TX) followed by transfection of the full length RNA transcript by liposome or calcium phosphate precipitation into the Sindbis packaging cell line. As described previously, filtered supernatants containing infectious vector particles from the transfected packaging cell line are then used to reinfect a fresh monolayer of Sindbis packaging cells which then serves as the source of viral vector. Approximately 10ml of infectious supernatant should be used to infect 5 x 10⁶ cells in a 10cm dish.

If the Sindbis GC viral vector uses a heterologous promoter in an expression plasmid configuration with a selectable marker (neomycin resistance gene), then the cDNA vector must be transfected into the packaging cell line followed by drug resistance selection. Resistant colonies are then pooled and dilution cloned. Individual clones are then propagated and frozen. The clones are individually screened for high titer, expression of GC by Western blot analysis, and for the functional activity of the protein.

For those vectors which do not have a selectable marker, selection of stably or persistently transfected clones must be performed by dilution cloning the DA transduced cells one to two days after transfecting the cells with the Sindbis vector. The dilution clones are then screened for the presence of GC by using reverse transcription of messenger RNA, followed by amplification of the cDNA message by the polymerase chain reaction technique. This procedure is known as the RT-PCR technique and a commercial kit is available to perform this assay through Invitrogen Corp. (San Diego, CA). RT-PCR is performed on clones which have been propagated for at least 10 days. Approximately 50 to 100 clones are screened in order to find a reasonable number of stable, persistently infected clones. In order to perform RT-PCR, specific primers are required for the desired message to be screened by amplification of the RNA product. Primers designed to amplify a 521 base pair product for GC screening are:

### Primers for GC screening:

GC PCR primer # 1: 5'-TTT CTG GCT CCA GCC AAA GCC ACC CTA GGG *GAG*-3' (SEQ. ID NO. 69)

GC PCR primer #2: 5'-AAT GGA GTA GCC AGG TGA GAT TGT CTC CAG GAA-3' (SEQ. ID NO. 70)

Those clones demonstrating highest expression and titer are then tested for transfer of expression onto BHK-2 cells followed by Western blot analysis and a functional assay for GC activity. A specific monoclonal antibody (8E4) against human GC is used for Western blot analysis to determine the presence of the protein (Ohashi et al., PNAS 89:11332, 1992; Barneveld et al. Eur. J. Biochem. 134:310, 1983. ) GC enzymatic activity is tested as described by Correll et al. (Blood 80:331, 1992). Animal studies are conducted once the appropriate clone has been identified.

### EXAMPLE 14

### ADMINISTRATION OF RECOMBINANT SINDBIS PARTICLES

A therapeutic Sindbis vector used for the treatment of Gaucher disease (Example 13) may be administered by transducing autologous CD34⁺ cells in an *ex vivo* protocol or by direct injection of the vector into the patient's bone marrow. In order to achieve the longest therapeutic expression of GC from the recombinant multivalent vector, the best mode of administration is to transduce long lived cell precursors of the clinically affected cell type, for example monocytes or macrophages.
By transducing the earliest precursors of the effected cell type, the cell precursors are able to self renew and repopulate the peripheral blood with maturing GC positive cells.
The earliest pluripotent hematopoietic stem cell studied to date are the CD34⁺ cells which make up 1%-4% of a healthy bone marrow population or 0.1 % in the peripheral blood population. Being able to transduce CD34⁺ cells is important in sustaining long term expression not only for the monocyte/macrophage lineage but any hematopoietic cell targeted for a therapeutic protein. Two approaches for transducing CD34⁺ cells include an *ex vivo* and an *in vivo* protocol. The *in vivo* protocol focuses on transducing an indiscriminate population of bone marrow cells by direct injection of the vector into the bone marrow of patients. The *ex vivo* protocol focuses on isolating CD34⁺ positive stem cells, from the patient's bone marrow, or an infant patient's umbilical cord blood, transducing the cells with vector, then subsequently injecting the autologous cells back into the patient. Both approaches are feasible, but the *ex vivo* protocol enables the vector to be used most efficiently by transducing a specific cultured population of CD34⁺ cells. Details of an *ex vivo* method are provided in the following section.

### EX VIVO ADMINISTRATION OF A MULTIVALENT GC SINDBIS VECTOR

CD34⁺ cells are collected from the patient's bone marrow by a syringe evacuation performed by a physician familiar with the technique. Alternatively, CD34⁺ cells may also be obtained from an infant's umbilical cord blood if the patient is diagnosed before birth. Generally, if the bone marrow is the source of the CD34⁺ cells, 20 bone marrow aspirations are obtained by puncturing femoral shafts or from the posterior iliac crest under local or general anesthesia. Bone marrow aspirations are then pooled, suspended in Herpes-buffered Hanks' balanced salt solution containing heparin at 100 units per ml and deoxyribonuclease I at 100 ug/ml and then subjected to a Ficoll gradient separation. The buffy coated marrow cells are then collected and washed according to CellPro's CEPRATE™ LC (CellPro. Bothell, WA) (CD34) Separation system. The washed buffy coated cells are then stained sequentially with anti-CD34 monoclonal antibody, washed then stained with biotinylated secondary antibody supplied with CEPRATE™ system. The cell mixture is then loaded onto the CEPRATE™ avidin column. The biotin-labeled cells are adsorbed onto the column while unlabeled cells passed through. The column is then rinsed according to the CEPRATE™ system directions and CD34⁺ cells eluted by agitation of the column by manually squeezing the gel bed. Once the CD34⁺ cells are purified, the purified stem cells are counted and plated at a concentration of 1 x 10⁵ cells/ml in Iscove's modified Dulbecco's medium (*JMDM*; Irvine Scientific, Santa Ana, CA) containing 20% pooled non-heat inactivated human AB serum (hAB serum).

After purification, [?] several methods of transducing purified stem cells may be performed. One approach involves immediate transduction of the purified stem cell population with vector containing supernatant cultures derived from vector producing cells. A second approach involves co-cultivation of an irradiated monolayer of vector producing cells with the purified population of nonadherent CD34⁺ cells. A third and preferred approach involves a similar co-cultivation approach, however, the purified CD34⁺ cells are prestimulated with various cytokines and cultured 48 hours prior to the co-cultivation with the irradiated vector producing cells. Recent publications have demonstrated that prestimulating the stem cells prior to transducing the cells with retroviral vectors increases the level of gene transfer into these cell types (Nolta et al., Exp. Hematol. 20:1065, 1992). The increased level of transduction is attributed to increased proliferation of the stem cells necessary for efficient retroviral transduction. Since Sindbis vectors are able to infect nonreplicating cells, prestimulation of these cells may not be required, however prestimulation of these cultures causing proliferation will provide increased cell populations for reinfusion into the patient. Prestimulation of the CD34⁺ cells is performed by incubating the cells with a combination of cytokines and growth factors which include IL-1, IL-3, IL-6 and mast cell growth factor (MGF). Prestimulation is performed by culturing 1-2 x 10⁵ CD34⁺ cells/ml of medium in T25 tissue culture flasks containing bone marrow stimulation medium for 48 hours. The bone marrow stimulation medium consists of IMDM containing 30% non-heat inactivated hAB serum, 2mM L-glutamine, 0.1 mM β 2-mercaptoethanol, 1 µM hydrocortisone, and 1% deionized bovine serum albumin. Ail reagents used in the bone marrow cultures should be screened for their ability to support maximal numbers of granulocyte, erythrocyte, macrophage, megakaryocyte, colony-forming units from normal marrow. Purified recombinant human cytokines and growth factors (Immunex Corp., Seattle. WA) for prestimulation should be used at the following concentrations: *E. coli*-derived IL-1α (100 U/ml), yeast-derived IL-3 (5 ng/ml), IL-6 (50 U/ml), and MGF (50 ng/ml) (Anderson et al., Cell Growth Differ. 2:373,1991).

After prestimulation of the CD34⁺ cells, they are then infected by co-cultivation with the irradiated Sindbis producer cell line (expressing the GC therapeutic vector) in the continued presence of the stimulation medium. The DA vector producing cell line is first trypsinized, irradiated (10,000 Rads) and replated at 1-2 x 10⁵ cells/ml of bone marrow stimulation medium. The following day, 1-2 x 10⁵ prestimulated CD34⁺ cells/ml is added to the Sindbis vector producing cell line monolayer. Co-cultivation of the cells is performed for 48 hours. After co-cultivation, the CD34⁺ cells are collected from the adherent Sindbis vector producing cell monolayer by vigorous washing with medium and plated for 2 hours to allow adherence of any dislodged vector producing cells. The cells are then collected and expanded for an additional 72 hours. The cells are then collected and frozen in liquid nitrogen using a cryo-protectant in aliquots of 1 x 10⁷ cells per vial. Once the transformed CD34⁺ cells have been tested for the absence of adventitious agents, frozen transformed CD34⁺ cells may be thawed, plated to a concentration of 1 x 10⁵ cells/ml and cultured for an additional 48 hours in bone marrow stimulation medium. Transformed cells are then collected, washed twice and resuspended in normal saline. The number of transduced cells used to infuse back into the patient per infusion is projected to be at a minimum of 1-10 x 10⁷ cells per patient per injection site requiring up to four injection sites. Infusion may be performed directly back into the patient's bone marrow or directly into the peripheral blood stream. Patients receiving autologous transduced bone marrow cells may be either partially or whole body irradiated, to deplete existing bone marrow populations. Treatment may be assessed at various time points post infusion to determine GC activity and for length of expression in differentiated cell types. If at some point during the course of follow-up procedures expression decreases or is nonexistent, transduced autologous cells may be reinjected into the patient.

### EXAMPLE 15

### TISSUE SPECIFIC EXPRESSION BY ACTIVATION OF DISABLED SINDBIS VECTORS USING TISSUE SPECIFIC CELLULAR RNA

### CONSTRUCTION OF SINDBIS TUMOR SPECIFIC EXPRESSION VECTORS FOR THE TREATMENT OF COLORECTAL CANCER

### A. CONSTRUCTION OF A RECOMBINANT SINDBIS VECTOR (SIN-CEA) DEPENDANT ON THE EXPRESSION OF THE CEA TUMOR MARKER

To produce a disabled Sindbis vector particle, in a Sindbis packaging cell line, the Sindbis vector must be driven by a DNA polymerase promoter. The option of using *in vitro* transcription of the vector RNA, followed by transfixing the RNA into the Sindbis packaging cell line, is not recommended since the tertiary structure generated by the disabled RNA transcript may prevent full genomic transcripts, limit vector replication and titers in the packaging cell line. For this reason a starting Sindbis vector containing the CMV MIEP promoter (pCMV-SIN) or the Drosophila metallothionein promoter (pMET-CMV) is used depending on the packaging cell line (*i.e.,* insect or mammalian).

As described previously, the disabled junction loop out model is constructed with the junction region of the vector flanked by inverted repeat sequences which are homologous to the RNA of choice. In this example, sequences from the CEA tumor antigen cDNA (Beauchemin et al., Molec. and Cell. Biol. 7:3221, 1987) are used in the inverted repeats. To construct a CEA RNA responsive Sindbis vector, the junction region is preceded by two CEA anti-sense sequence domains (A¹ and B¹) separated by a six base pair hinge domain. A single twenty base pair CEA sense sequence (A2), which is complementary to A1, is placed at the 3' end of the junction region. In choosing the correct A1 and B1 antisense sequences, the only two requirements are that they be specific for the targeted RNA sequence and that the anti-sense sequences hybridize to two RNA sequence domains separated by three nucleotides. This three nucleotide gap will serve as a hinge domain for the polymerase to hop and switch reading strands bridging the non-structural protein domain of the vector to the junction region of the vector (Figure 5). To construct such a configuration, two oligonucleotides are synthesized complementing each other to create a fragment insert contain convenient restriction enzyme sites at the extreme 5' and 3' ends. The oligonucleotide fragment insert is then ligated into the Sindbis vector between the disabled junction region and the multiple cloning sites of the Sindbis vector. The sense oligonucleotide strand, from 5' to 3', should contain an Apa I restriction site, followed by the A1 anti-sense domain, a six bp hinge domain, a B1 anti-sense domain, a synthetic junction region domain, and the A2 sense domain, followed by a Xho I restriction enzyme site. The following oligonucleotide sequence is used to design a CEA RNA responsive Sindbis vector. The nucleotide number sequence is obtained from Beauchemin et al., Molec. and Cell Biol. 7:3221, 1987.

### 5'-3' CEA sense strand:

The 5'-3' CEA anti-sense strand is complementary to the above oligonucleotide. After both oligonucleotides are synthesized, the oligonucleotides are mixed together in the presence of 10mM Mg, heated to 1000°C for 5 min. and cooled slowly to room temperature. The oligonucleotide pair is then digested with the Apa I and Xho I restriction enzymes, mixed and ligated at a 25:1 molar ratio of insert to plasmid, pCMV-SIN or pMET-SIN predigested with the same enzymes. These constructs are designated pCMV/SIN-CEA and pMET/SIN-CEA, respectively.

### Construction of a SIN-CEA vector and producer cell line expressing gamma interferon (SIN-CEA/gIFN)

The human g-IFN cDNA is derived from RNA isolated from PHA-stimulated Jurkat T cells by guanidinium thiocyanate extraction followed by ultracentrifugation through a CsCl gradient. The RNA (Sigma, St. Louis, MO) is then reverse-transcribed *in vitro* and a gene-specific oligonucleotide pair is used to amplify g-IFN cDNA by polymerase chain reaction using Tac polymerase. The PCR DNA was repaired with T4 DNA polymerase and Klenow and cloned into the Hinc II site of SK⁺ plasmid (Stratagene, San Diego, CA) treated with CIAP. In the sense orientation, the 5' end of the cDNA is adjacent to the Xho I site of the SK⁺ polylinker and the 3' end adjacent to the Cla I site. The 512 base pair fragment encoding the human γ-IFN molecule is placed into the Xho I / Cla I site of either the pCMV/SIN-CEA or pMET/SIN-CEA vectors. These new plasmids are designated pCMV/SIN-CEA/γIFN or pMET/SIN-CEA/γIFN, respectively.

### B. CONSTRUCTION OF A SIN-CEA VECTOR AND PRODUCER CELL LINE EXPRESSING THYMIDINE KINASE (SIN-CEA/TK)

A PCR amplified product containing the cDNA clone of the herpes simplex thymidine kinase ("HSVTK"), flanked with 5' Xho I and 3' Cla I restriction enzyme sites is obtained using the pHSITK3KB (Mcknight et al., Nuc. Acids Res. 8:5949, 1980) clone as target DNA. The sequences for the primers used for the PCR amplification are obtained from published sequences (Wagner et al., PNAS 78:1442, 1981). The 1,260 base pair amplified product is then digested with Xho I and Cla I ligated into the Xho I / Cla I site of either the pCMV/SIN-CEA or pMET/SIN-CEA vectors. These new plasmids are designated pCMV/SIN-CEA/HSVTK or pMET/SIN-CEA/HSVTK, respectively.

### C. CREATION OF CEA RNA DEPENDENT SINDBIS VECTOR PRODUCER CELL LINES

Unlike the previous examples of creating producer cell lines (Example 7), it may be that only a single round of gene transfer into the packaging cell line is possible by vector transfection. Since these vectors will be disabled and prevented in the synthesis of full genomic vectors, re-infection of a fresh layer of Sindbis packaging cell lines will end in an aborted infection since these vectors are now dependent on the presence of the CEA RNA to become active. Higher titers may be achieved by dilution cloning transfected producer cell lines using the RT-PCR technique described previously in Example 11.

### EXAMPLE 16

### REPLACEMENT GENE THERAPY USING RECOMBINANT SINDBIS VECTORS FOR SYSTEMIC PROTEIN PRODUCTION. A TREATMENT FOR FACTOR VIII DEFICIENT HEMOPHILIA A DISEASE

Hemophilia A disease is characterized by the absence of factor VIII, a blood plasma coagulating factor. Approximately I in 20,000 males have hemophilia A in which the disease state is presented as a bleeding disorder, due to the inability of affected individuals to complete the blood clotting cascade.

The treatment of individuals with hemophilia A is replacement with the factor VIII protein. The only source for human factor VIII is human plasma. In order to process human plasma for factor VIII purification, human donor samples are pooled in lots of over 1000 donors. Due to the instability of the factor VIII protein, the resulting pharmaceutical products are highly impure and with an estimated purity by weight of approximately 0.04%. In addition, there is the serious threat of such infectious diseases as hepatitis B virus and the Human Immunodeficiency Virus, among others, which contaminate the blood supply and can thus be potentially co-purified with the factor VIII protein.

For the reasons presented above, a recombinant production source of factor VIII would mark a seminal progress towards treatment of hemophilia A.

The ideal treatment for hemophilia A would be gene replacement therapy; that is, the replacement in affected individuals of a normal factor VIII gene, which would permit the normal clotting cascade to ensue.

The discussion presented above is well known to those skilled in the art of possible treatment of the hemophilia A disorder. Retroviruses are one possible gene therapy vector which have been developed for the expression of factor VIII. However, for reasons that are poorly understood the level of recombinant factor VIII expression with this and other methods is relatively low.

One possibility for the low expression of recombinant factor VIII in cells transduced by retrovirus and other vectors, is the relative low efficiency of processing and transport of mature factor VIII RNA from the nucleus to the cytoplasm. Known to those in the area, the deletion of the B domain of factor VIII increases the level at which this clotting factor is produced and secreted. To avoid the problems associated with low levels of factor VIII protein production, due to low RNA expression levels and inefficient processing, the insertion of factor VIII cDNA into Sindbis vectors is described. Because the Sindbis life cycle occurs entirely in the cytoplasm of the infected cells, the problems associated with factor VIII expressed from the nucleus are circumvented. Further, given the efficiency with which Sindbis self-replicates, in theory, the level of factor VIII production in Sindbis/factor VIII infected cells could be as high as 1 x 10⁸ factor VIII protein molecules/cell.

One potential problem associated with inserting factor VIII into Sindbis vectors is the resulting physical size of the vector/heterologous gene construction, and the possible constraint of packaging the factor VIII vector construct into a functional infectious Sindbis particle. The genomic size of wild-type Sindbis is 11,703 nucleotides, plus a poly A tail. The size of the Sindbis basic vector construct (pKSSINBV, *see* Example 2) is 7,830 nucleotides, including a 25-mer poly A tail. Thus, the allowable size of heterologous genetic material to be inserted into pKSSINBV which results in a genomic complement the same physical size as wild-type virus, is 3,898 nucleotides.

However, one key piece of evidence suggests that the capacity of heterologous genetic material which can be inserted into pKSSINBV and packaged into a functional infectious Sindbis particle is considerably larger than 3,898 bps. In a work published in the literature (Geigenmuller-Gnirke et al., PNAS 88:3253-3257, 1991), the packaging of two molecules, each containing the cis-required packaging signal, into single functional Sindbis particles is described. The total physical size of the two packaged genomes in this study is 14,600 bps. This result strongly indicates that the capacity of Sindbis vectors for heterologous genetic material is much greater than previously envisioned. The potential of insertion of large cellular genes into Sindbis vectors extends substantially the utility of this system.

The factor VIII cDNA clone is approximately 8,000 bps. Insertion of the factor VIII cDNA into pKSSINBV yields a vector/heterologous genomic size of approximately 15,830 bps. If the packaging of this particle is inefficient, the size of the insert can be decreased further by eliminating the "B-domain" of the factor VIII insert. It has been shown that the Factor VIII B-domain region can be removed from the cDNA without affecting the functionality of the subsequently expressed protein. The source of factor VIII cDNA is clone pSP64-VIII, an ATCC clone under the accession number 39812 having a cDNA encoding the full-length human protein. pSP64-VIII is digested with SaII, the ends are blunted with T4 DNA polymerase and 50 uM of each dNTP. and the ca. 7700 bp. fragment is electrophoresed on a 1% agarose/TBE gel and purified with Gene Clean™. The factor VIII cDNA containing blunt ends is then ligated into pKSII3'SIN (Example 2), prepared by digestion with Hinc II, treated with CIAP, and purified with Gene Clean™. This plasmid is known as pF83'SIN.

For insertion of factor VIII into the various Sindbis vectors described in Example 2, plasmid pF83'SIN is digested with Xho I and Sac II, and the resulting 7,850 bp fragment is isolated on a 1% agarose/TBE gel and purified by Gene Clean™. This factor VIII-3'SIN fragment is inserted into each of the vectors listed below. Prior to insertion of this fragment the plasmids are prepared by digestion with Xho I and Sac II, treated with CIAP, isolated by 1% agarose/TBE gel electrophoresis, and purified with Gene Clean™:

| Vector | Functional Junction Region (+/-) |
|---|---|
| pKSSINBV | + |
| pKSSINdIJRsjrc | + |
| pKSSINdIJRsjrPC | + |
| pKSSINdIJRsjrNP(7,582-7,601) | + |
| pKSSINdIJRsexjr | + |

Following insertion of the factor VIII cDNA, these vectors are designated:
pKSSINBVF8
pKSSINdIJRsjrcF8
pKSSINdIJRsjrPCF8
pKSSINdIJRsjrNP(7,582-7,601)F8
pKSSINdIJRsexjrF8
respectively.

Packaging of the Factor VIII cDNA containing vectors is accomplished by either infecting cells transfected with vector/factor VIII: *in vitro* transcribed RNA with wild-type virus, or alternatively by transfection with *in vitro* transcribed vector/factor VIII RNA of the packaging cell lines described in Example 7. The efficiency of packaging is determined by measuring the level of factor VIII expression in infected cells and comparison with the expression levels observed in the same experiments performed with the pKSSIN-luc vector described in Example 3.

### EXAMPLE 17

### A. CREATING A GLUCOCEREBROSIDASE SINDBIS VECTOR

A glucocerebrosidase (GC) cDNA clone containing an Xho I restriction enzyme site 5' of the cDNA coding sequence and a Cla I restriction enzyme site 3' of the cDNA coding sequence is first generated. The clone can be generated by digesting pMFG-GC (PNAS 89:11332; 1992) with Nco I, blunted with Vent DNA polymerase, then ligated with Xho I linkers. The plasmid is then digested with Bam HI, blunted with Vent DNA polymerase, then ligated to Cla I linkers. For insertion of factor VIII into the various Sindbis vectors described in Example 2, the fragment is then digested with Xho I and Cla I and gel isolated on a 1% agrose/TBE gel and purified by Gene Clean. The GC fragment is inserted into each of the vectors below, prepared by digestion with Xho I and Cla I, treatment with CIAP, isolation by 1% agrose/TBE gel electrophoresis, and purification with Gene Clean:

| Vector | Functional Junction Region (+/-) |
|---|---|
| | |
| pKSSINBV | + |
| pKSSINdIFRsjrc | + |
| pKSSINdIFRsjrPC | + |
| pKSSINdIFRsjrNP(7582-7601) | + |
| pKSSINdIFRsexjr | + |

Following insertion of the factor VIIIcDNA, these vectors are known as shown below:
pKSSINBV-GC
pKSSINdIFRsjrc-GC
pKSSINdIFRsjrPC-GC
pKSSINdIFRsjrNP(7582-7601)-GC
pKSSINdIFRsexjr-GC

The glucocerebrosidase(GC) Sindbis vector will be used to express the GC cDNA in animal studies and eventually used to treat humans by direct injection. If multiple injections are required to sustain GC expression in the treated individual it would be ideal if the Sindbis vector were designed to be minimally antigenic or designed in such a fashion as not to induce an immune response to the vector. Examples of Sindbis vectors which are designed in such a manner and are capable of expressing multiple proteins are described in Examples 3 (Adenovirus Early Region E3); Example 4 (HCMV H301); Example 5 (Expression of multiple genes) and Example 17 (Ribozyme expression of interferon A). Using the above examples, a Sindbis vector can be designed to express the GC cDNA in such a configuration were by the interferon A hairpin ribozyme is followed by the AD E3 or CMV H301 gene, followed by a an internal ribozyme entry site followed by the GC cDNA or other therapeutic palliative, using the same Xho I to Cla I cloning sites in the vector's multiple cloning site.

### B. CONSTRUCTION OF A SINDBIS GLUCOCEREBROSIDASE VECTOR PRODUCING CELL LINE

Two approaches are presented to create the producer cell line depending on the type of Sindbis cDNA vector clone used, described in Example 7. One type of Sindbis vector uses the SP6 RNA polymerase recognition sequence for the synthesis of *in vitro* RNA transcripts. This type of vector is used to create a vector producer cell line based on a non-integrating vector which would persistently replicate in the packaging cell line. The second type of vector uses a heterologous promoter in place of the Sp6 sequence in a plasmid construct which would allow for stable integration in the packaging cell line (*see* Example 7 for review). Replication competent Sindbis vector transcripts are then expressed from the DNA level and would be transfected into the Sindbis packaging cell line.

If the Sindbis GC viral vector uses a T7 RNA recognition sequence, the cDNA must be first *in vitro* transcribed using any of the *in vitro* transcription systems available commercially (Megascript transcription kit; Ambion Inc., Austin, Texas) followed by transfection of the full length RNA transcript by liposome or calcium phosphate precipitation into the Sindbis packaging cell line. As described: previously (Example 7), filtered supernatants containing infectious vector particles from the transfected packaging cell line are then used to re-infect a fresh monolayer of Sindbis packaging cells which then serves as the source of viral vector. Approximately 10ml of infectious supernatant should be used to infect 5 x10⁶ cells in a 10cm dish.

If the Sindbis GC viral vector uses a heterologous promoter in an expression plasmid configuration with a selectable marker (neomycin resistance gene), then the cDNA vector must be transfected into the packaging cell line followed by drug resistance selection. Resistant colonies are then pooled and dilution cloned. Individual clones are then propagated and frozen. The individual clones are then screened for high titer as described in Example 9, for expression of glucocerebrosidase by western blot analysis, and then tested for functional activity of the protein (Correll et al.; Blood 80:331, 1992).

For those vectors which do not have a selectable marker, selection of stably or persistently transfected clones must be performed by dilution cloning the packaging cell line one to two days after transfecting the cells with the Sindbis vector. The dilution clones are then screened for the presence of glucocerebrosidase by using reverse transcription of messenger RNA, followed by amplification of the cDNA message by the polymerase chain reaction technique. This procedure is known as the RT-PCR technique and a commercial kit is available to perform this assay through Invitrogen Corp. (San Diego, CA). RT-PCR is performed on clones which have been propagated for at least 10 days. Approximately 50 to 100 clones are screened in order to find a reasonable number of stable, persistently infected, clones. In order to perform RT-PCR, specific primers are required for the desired message to be screened by amplification of the RNA product. Primers designed to amplify a 521 base pair product for glucocerebrosidase screening are:

### Primers for glucocerebrosidase screening:

Those clones demonstrating highest expression and titer are then tested for transfer of expression by infecting BHK-2 cells followed by Western blot analysis and a functional assay for glucocerebrosidase (GC) activity in the BHK-2 cells. A specific monoclonal antibody (8E4) against human GC is used for western blots (PNAS 89:11332, 1992) and described in Barneveld, R.A. et al (Eur. J. Biochem. 134:310, 1983). Once the appropriate clone has been identified, supernatants from the growth cultures are used to obtain vector, filtered through 0.45uC filter used to begin animal studies via direct injection into the animals. After animal studies are conducted, scaled up direct injection protocols, derived from the animal studies, can be used for treatment of human patients with Gaucher Disease. Alternative administrative protocols for this vector are given in the following example.

### EXAMPLE 18

### INHIBITION OF HUMAN PAPILLOMA VIRUS PATHOGENICITY BY SEQUENCE-SPECIFIC ANTISENSE OR RIBOZYME MOLECULES EXPRESSED FROM SINDBIS VIRUS VECTORS

To date, more than sixty types of human papilloma viruses (HPV), which have a pronounced tropism for cells of epithelial origin, have been isolated and
characterized. Among the HPV group are a substantial number of types which infect the human anogenital tract. This group of HPVs can be further subdivided into types which are associated with benign or with malignant proliferation of the anogenital tract.

There are between 13,000 and 20,000 cervical cancer deaths per year in the U.S. In developing countries, cervical cancer is the most frequent malignancy, and in developed countries cervical cancer ranks behind breast, lung, uterus, and ovarian cancers. One statistic which especially supports the notion that anogenital proliferation is a growing health problem is that medical consultations for genital warts increased from 169,000 in 1966 to greater than 2 million in 1988.

Several lines of evidence exist which link HPV to the pathogenesis of cervical proliferative disease. A distinct subset of types, so called 'low risk HPVs', are associated with benign proliferative states of the cervix (e.g., HPV 6, 11, 43, 44), while another subset of types, the 'high risk HPVs', are associated with lesions which may progress to the malignant state (e.g., HPV 16, 18, 31, 33, 35, etc.). Approximately 95% of cervical tumors contain HPV, with HPV type 16 or 18 DNA being found in about 70% of them.

The frequency of HPV in the young sexually active female population appears to be quite high. Indeed, in a recent study of 454 college women, 213, or 46% were HPV positive. Among the HPV positive group, 3% were HPV 6/11 positive, and 14% were HPV 16/18 positive. Of these 454 women, 33 (7.3%) had abnormal cervical proliferation, as determined by cytology.

With regard to the design of antisense and ribozyme therapeutic agents targeted to HPV, there are important parameters to consider relating to the HPV types to target (i.e., types associated with condyloma acuminatum or types associated with malignant cervical proliferation) and HPV expressed genes to target, including but not limited to, HPV genes E2, E6, or E7. In general, the expression of HPV genes is defined temporally in two phases, early (E) genes expressed prior to viral DNA replication, and late (L) genes expressed after viral DNA replication. There are 7 early enzymatic HPV genes, and 2 late structural HPV genes.

Based on the discussion presented above, antisense/riboryme therapeutics directed towards the HPV 6/11 groups may be constructed which target the viral E2 gene. It seems possible that the E2 gene target may be precarious with regard to the HPV 16/18 group, by a mechanism of driving integration of the virus through inhibition of E2 protein expression. Thus, it seems that the E6/E7 genes in HPV types 16/18 should be targeted directly. Described below is the construction of antisense and ribozyme therapeutics into Sindbis virus vectors (described in Example 2) specific for HPV type 16 E6 and E7 RNA. Insertion of the HPV antisense and ribozyme moieties is between the Cla I and Xba I sites of the Sindbis vector.

### A. CONSTRUCTION OF AN HPV 16 E6/E7 ANTISENSE THERAPEUTIC

The HPV 16 viral genomic clone, pHPV-16 (ATCC number 45113) is used as a template in a PCR reaction for the amplification of specific sequences from the viral E6/E7 genes. The HPV 16 antisense moiety is first inserted into the plasmid vector pKSII⁺; removal of the antisense therapeutic from the plasmid vector and insertion into the various Sindbis vector backbones is accomplished via the unique antisense moiety terminal Cla, I and Xba I restriction endonuclease sites. Amplification of a portion of the HPV 16 E6/E7 genes is accomplished with the primer pair shown below:
Forward primer (buffer sequence/Xba I site/HPV 16 nucleotides 201-222):
   TATATTCTAGAGCAAGCAACAGTTACTGCGACG (SEQ. ID NO. 76)
Reverse primer (buffer sequence/Cla I site/HPV 16 nucleotides 759-738):
   TATATATCGATCCGAAGCGTAGAGTCACACTTG (SEQ. ID NO. 77)

The HPV 16 E6/E7 580 bp amplicon product is first purified with Gene Clean (Bio 101, San Diego, CA), the digested with the restriction enzymes Cla I and Xba I, and electrophoresed on a 1% agarose/TBE gel. The 568 bp band is then excised from the gel, the DNA purified with Gene Clean, and ligated into the pKSII⁺ plasmid prepared by digestion with Cla I and Xba I, treatment with CIAP, and treatment with Gene Clean. This plasmid is known as pKSaHPV16E6/E7.

In addition to the HPV 16 E6/E7 complementary sequences, both primers contain a five nucleotide 'buffer sequences' at their 5' ends for efficient enzyme digestion of the PCR amplicon products. Generation of the HPV 16 amplicon with the primers shown above is accomplished with the PCR protocol described in Example 4. It has been shown previously that the E6/E7 mRNA in infected cervical epithelia is present in three forms, unspliced and two spliced alternatives (E6* and E6**), one in which nucleotides 226-525 of E6 are not present in the mature message (Smotkin et al., J. Virol 63:1441-1447, 1989). The region of complementary between the antisense moiety described here and the HPV 16 genome is viral nucleotides 201-759. Thus the antisense moiety will be able to bind to and inhibit the translation of the E6/E7 unspliced message and the spliced E6* and E6** spliced messages.

### B. CONSTRUCTION OF HPV 16 E6/E7 HAIRPIN RIBOZYME THERAPEUTICS

In order to efficiently inhibit the expression of HPV 16 E6 and E7 proteins, a hairpin ribozyme (HRBZ) with target specificities to E6 mRNA is constructed. The HPV 16 ribozyme moiety is first inserted into the plasmid vector pKSII⁺; removal of the ribozyme therapeutic from the plasmid vector and insertion into the various Sindbis vector backbones is accomplished via the unique ribozyme moiety terminal Cla I and Xba I restriction endonuclease sites.

The HRBZ is homologous to the HPV 16 E6 RNA (nts. 414-431) shown below:
TT AACTGTCAAAAGCCAC (SEQ. ID NO. 78)

The HRBZ is designed to cleave after the T residue in the TCTC hairpin ribozyme loop 5 substrate motif, shown underlined above. Following cleavage, the HRBZ is recycled and able to hybridize to, and cleave, another unspliced E6/E7 mRNA or the E6* spliced mRNA molecule.

Double-stranded HRBZ as defined previously (Hampel et al., Nucleic Acids Research 18:299-304, 1990), containing a 4 base 'tetraloop' 3 and an extended helix 4, with specificity for the HPV 16 E6 RNA shown above, is chemically synthesized and includes both the 5' and 3' ends, respectively, Cla I and Xba I sites. The sequence of the chemically synthesized HPV 16 E6 HRBZ strands are shown below:
HPV 16 E6 HRBZ. top strand (5'->3'):
HPV 16 E6 HBRZ, bottom strand (5'->3'):

In order to form the double-stranded HPV 16 E6 specific HRBZ with Cla I and Xba I cohesive ends, equal amounts of the oligonucleotides are mixed together in 10 mM Mg²⁺, heated at 95°C for 5 min, then cooled slowly to room temperature to allow the strands to anneal.

The double-stranded HPV 16 E6 HRBZ with Cla I and Xba I cohesive ends is first ligated into the pKSII⁺ plasmid vector, prepared by digestion with Cla I and Xba I, treatment with CIAP, and treatment with Gene Clean. This plasmid is known as pKSHPV16E6HRBZ.

The HPV 16 antisense and hairpin ribozyme moieties are liberated from their plasmid vectors, pKSaHPV16E6/E7 and pKSHPV16E6HRBZ, respectively, by digestion with Cla I and Xba I, purification by agarose electrophoresis and Gene Clean, and insertion into the desired vector backbone, prepared by digestion with Cla I and Xba I, and treatment with CIAP. Several possible Sindbis vectors some of which are shown below, and whose detailed construction is described in Example 2, are suitable for the insertion of the HPV 16 antisense and ribozyme therapeutic moieties:

| Vector | Functional Junction Region (+/-) |
|---|---|
| pKSSINBV | + |
| pKSSINBVdIJR | - |
| pKSSINdIJRsjrc | + |
| pKSSINdIJRsjrPC | + |
| pKSSINdIJRsjrNP(7582-7601 | + |
| pKSSrNdIJRsexjr | + |

Since the antisense and ribozyme therapeutic operate at the level of RNA, it is not necessary that the vectors containing these moieties contain a functional junction region. That is, translation of the region corresponding to the Sindbis structural proteins occurs only from subgenomic RNA. However, because translation of the antisense and hairpin ribozyme therapeutic is not an issue, these moieties will exert their affect from the level of positive stranded Sindbis genomic vector RNA.

On the other hand, it may be desired to administer repeated doses to an individual; thus the antisense and hairpin palliative would be inserted downstream of the adenovirus E3 or human cytomegalovirus H301 genes, which down-regulate the expression of MHC class I molecules in infected cells. Insertion of the antisense and hairpin palliatives is accomplished in the vectors from Examples 3 and 4 shown below, between the Cla I and Xba I sites:

| Vector | Functional Junction Region (+/-) |
|---|---|
| pKSSINdIJRsjrcAdE3 | + |
| pKSSINdIJRsjrcH301 | + |

Subgenomic mRNA is synthesized in these vectors, which serves as a translational template for the Ad E3 and CMV H301 genes. Thus, in these constructions, functional HPV 16 antisense and hairpin ribozyme palliatives will be present on the levels of both subgenomic and positive stranded genomic Sindbis vector

RNA.

Further, the HPV 16 antisense and hairpin ribozyme palliatives can be inserted downstream of a heterologous gene inserted into the described Sindbis vectors. For example, one could insert the HPV 16 antisense and hairpin ribozyme palliatives downstream of a heterologous gene coding for an immunogenic epitope of HPV 16 from, for example, the E6/E7 or L1 proteins. In these vectors, it would not be desired to include the immunoregulatory Ad E3 or CMV H301 genes.

Expression of the E6/E7 genes during infection with both the high- and low-risk HPV groups is required for proliferation of the cervical epithelium. The HPV E7 protein from all HPV types tested forms a complex with the retinoblastoma protein, and the E6 protein from HPV types 16 and 18 associates with and degrades the cellular p53 protein. The p53 and retinoblastoma cellular gene products are involved in the growth control of the cell, and altering the expression or function of these proteins can release the growth control in affected cells. Thus, an antisense or ribozyme therapeutic agent to both HPV groups should either directly or ultimately diminish the expression of one or both of these genes. Expression of the E6/E7 genes is trans-activated by the viral E2 protein. However, by utilizing an alternative splicing strategy, the E2 protein can also act as a trans-repressor. Integration of the oncogenic HPV types occurs in the viral E2 region and abrogates the expression of the E2 protein. Integration by the oncogenic HPV types appears to be a pivotal event in the frank induction and/or maintenance of cervical carcinoma. This event results in the constitutive expression of the E6/E7 genes. In the integrated state, expression of the E6/E7 genes is trans-activated by factors present in infected keratinocytes. The inactivation of the viral E2 control mechanism in response to the cellular keratinocyte factor activation of E6/E7 expression might be a critical event in viral integration.

Described below is the construction of antisense and ribozyme, therapeutics into Sindbis virus vectors (described in Example 2) specific for HPV type 16 E6 and E7 RNA. Insertion of the HPV antisense and ribozyme moieties is between the Cla I and Xba I sites of the Sindbis vector.

### C. CONSTRUCTION OF AN HPV 16 E6/E7 ANTISENSE THERAPEUTIC

The HPV 16 viral genomic clone, pHPV-16 (ATCC number 45113) is used as a template in a PCR reaction for the amplification of specific sequences from the viral E6/E7 genes. The HPV 16 antisense moiety is first inserted into the plasmid vector pKSII⁺; removal of the antisense therapeutic from the plasmid vector and insertion into the various Sindbis vector backbones is accomplished via the unique antisense moiety terminal Cla I and Xba I restriction endonuclease sites. Amplification of a portion of the HPV 16 E6/E7 genes is accomplished with the primer pair below:
HPV 16 Forward primer (buffer sequence/Xba I site/HPV 16 nucleotides 201-222):
   5'-TAT ATT CTA *GAG* CAA GCA ACA GTT ACT GCG ACG-3' (SEQ. ID NO. 81)
HPV 16 Reverse primer (buffer sequence/Cla I site/HPV 16 nucleotides 759-738):
   5'-TAT ATA TCG ATC CGA AGC GTA *GAG* TCA CAC TTG-3' (SEQ. ID NO. 82)

The HPV 16 E6/E7 580 bp amplicon product is first purified with Gene Clean (Bio 101, San Diego CA), digested with the restriction enzymes Cla I and Xba I, and isolated by 1% agarose/FBE gel electrophoresis. The 568 bp band is then excised from the gel, purified with Gene Clean™, and ligated into the pKSII⁺ plasmid prepared by digestion with Cla I and Xba I, treated with CIAP, and purified with Gene Clean™. This plasmid is designated pKSαHPV16E6/E7.

In addition to the HPV 16 E6/E7 complementary sequences, both primers contain a five nucleotide "buffer sequences" at their 5' ends for efficient enzyme digestion of the PCR amplicon products. Generation of the HPV 16 amplicon with the primers above is accomplished using the PCR protocol described in Example 4. It is known that the E6/E7 mRNA in infected cervical epithelia is present in three forms, unspliced and two spliced alternatives (E6* and E6**), in which nucleotides 226-525 of E6 are not present in the mature message (Smotkin et al., J. Virol 63:1441, 1989). The region of complementary between the antisense and the HPV 16 genome is viral nucleotides 201-759. Thus the antisense moiety will be able to bind and inhibit the translation of the E6/E7 unspliced message and the spliced E6* and E6** messages.

### D. CONSTRUCTION OF HPV 16 E6/E7 HAIRPIN RIBOZYME THERAPEUTICS

In order to efficiently inhibit the expression of HPV 16 E6 and E7 proteins, a hairpin ribozyme (HRBZ) with target specificities to E6 mRNA is constructed. The HPV 16 ribozyme moiety is first inserted into the plasmid vector pKSII⁺; removal of the ribozyme therapeutic from the plasmid vector and insertion into the various Sindbis vector backbones is accomplished via the unique ribozyme moiety terminal Cla I and Xba I restriction endonuclease sites.

The HRBZ is homologous to the HPV 16 E6 RNA nucleotide sequence 414-431 shown below:
5'-TTA ACT GTC AAA AGC CAC-3' (SEQ. ID NO. 83)

The HRBZ is designed to cleave after the first T residue in the TGTC hairpin ribozyme loop 5 substrate motif, shown underlined above. Following cleavage, the HRBZ is recycled and able to hybridize to, and cleave, another unspliced E6/E7 mRNA or the E6* spliced mRNA molecule.

Double-stranded HRBZ (Hampel et al., Nucleic Acids Research 18:299, 1990), containing a 4 base "tetraloop" 3 and an extended helix 4, with specificity for the HPV 16 E6 RNA shown above, is chemically synthesized and includes Cla I and Xba I sites at the 5' and 3' ends, respectively. The sequence of the chemically synthesized HPV 16 E6 HRBZ strands are shown below:
HPV 16 E6 HRBZ, sense strand:
HPV 16 E6 HBRZ, antisense strand:

In order to form the double-stranded HPV 16 E6 specific HRBZ with Cla I and Xba I cohesive ends, equal amounts of the oligonucleotides are mixed in 10 mM MHCl₂, heated at 95°C for 5 min, then cooled slowly to room temperature to allow the strands to anneal.

The double-stranded HPV 16 E6 HRBZ with Cla I and Xba I cohesive ends is ligated into the pKSII⁺ plasmid vector. The pKSII⁺ vector is first digested with Cla I and Xba I, treated with CIAP, and purified with Gene Clean™ prior to ligation. This plasmid is designated pKSHPV16E6HRBZ.

The HPV 16 antisense and hairpin ribozyme moieties are liberated from their plasmid vectors, pKSaHPV16E6/E7 and pKSHPV16E6HRBZ, respectively, by digestion with Cla I and Xba I, isolation by agarose gel electrophoresis, and purified using Gene Clean™. They are then ligated into the desired vector backbone. The vector backbone is prepared by digestion with Cla I and Xba I, and treated with CIAP. Several possible Sindbis vectors are suitable for the insertion of the HPV 16 antisense and ribozyme therapeutic moieties. Some of these vectors from Example 2 are presented below:

| Vector | Functional Junction Region (+/-) |
|---|---|
| pKSSINBV | + |
| pKSSINBVdIJR | - |
| pKSNdIJRsjrc | + |
| pKSNdIJRsjrPC | + |
| pKSSINdIJRsjrNP(7.582-7.601) | + |
| pKSSINdIJRsexjr | + |

Since the antisense and ribozyme therapeutic operate at the RNA level, it is not necessary that the vectors containing these moieties also contain a functional junction region. Specifically, translation of the region corresponding to the Sindbis structural proteins occurs only from subgenomic RNA. However, because translation of the antisense and hairpin ribozyme therapeutic is not an issue, these moieties will exert their affect from the level of positive stranded Sindbis genomic vector RNA.

On the other hand, it may be desired to administer repeated doses to an individual; thus the antisense and hairpin palliative would be inserted downstream of the adenovirus E3 or human cytomegalovirus H301 genes. (E3 and H301 down-regulate the expression of MHC class I molecules in infected cells.) Insertion of the antisense and hairpin palliatives is accomplished in the vectors from Examples 3 and 4, between the Cla I and Xba I restriction sites:

| Vector | Functional Junction Region (+/-) |
|---|---|
| pKSSINd1JRsjrcAdE3 | + |
| pKSSINd1JRsjrcH301 | + |

Subgenomic mRNA is synthesized in these vectors, which serves as a translational template for the Ad E3 and CMV H301 genes. Thus, in these constructions, functional HPV 16 antisense and hairpin ribozyme palliatives will be present at the levels of both subgenomic and positive stranded genomic Sindbis vector RNA.

Further, the HPV 16 antisense and hairpin ribozyme palliatives can be inserted downstream of a heterologous gene contained in the described Sindbis vectors. For example, the HPV 16 antisense and hairpin ribozyme palliatives can be inserted downstream of a heterologous gene coding for an immunogenic epitope of HPV 16 E6/E7 or L1 proteins. In these vectors, it would not be desired to include the immunoregulatory Ad E3 or CMV H301 genes.

### EXAMPLE 19

### INHIBITION OF HUMAN INTERFERON A EXPRESSION IN INFECTED CELLS BY SEQUENCE-SPECIFIC RIBOZYME MOLECULES EXPRESSED FROM SINDBIS VIRUS VECTORS

Interferons (IFNs) comprise a family of small proteins which effect a wide range of biological activities in the mammalian cell, including the expression of MHC antigens, the expression of several genes which modulate cell growth control, and the resistance to viral infections (Pestka et al., Ann. Rev. Biochem. 56:727-777, 1987). Of the three classes of IFNs, a, b, and g, IFN-a, or leukocyte interferon, is responsible for the activity which limits viral replication in the infected cell.

The antiviral effects of IFN-a are associated with the induction of two cellular enzymes which inhibit the viral lifecycle in the infected cell. One enzyme is a double-stranded RNA dependent 68-kDa protein kinase that catalyzes the phosphorylation of the a subunit of the protein synthesis initiation factor eIF-2. The second enzyme induced by IFN-a is 2',5'-oligoadenylate synthetase (2',5'-OAS), which in the presence of double-stranded RNA activates the latent endonuclease, RNase L, which is responsible for degradation of viral and cellular RNAs (Johnston and Torrence, Interferons 3:189-298, Friedman (ed.), Elsevier Science Publishers, B.V., Amsterdam, 1984).

Because their replication strategy includes a double-stranded RNA intermediate, the RNA viruses in particular are strong inducers of interferon. With regard to Sindbis virus, double-stranded RNA molecules are present during the replication of both positive- and negative-stranded genome length molecules, and during the transcription of subgenomic mRNA. It has been demonstrated that infection of cells with Sindbis virus results in the induction of interferon (Saito, J. Interferon Res. 9:23-24, 1989).

In applications where extended expression of the therapeutic palliative is desired, expression of IFN in the infected cell is inhibited by inclusion of a hairpin ribozyme with specificity for IFN-a mRNA in the Sindbis vector. Inhibition of IFN-a expression thus mitigates induction of the cascade of cellular proteins, including the eIF-2 protein kinase and 2'.5'-OAS, which inhibit the extent to which virus can replicate in the infected cell. Prolonged expression of the therapeutic palliative without induction of an immune response targeted towards the vector infected cell is desired in all applications other than antigen presentation and includes, for example, systemic protein production, antisense and ribozyme, and accessory molecules.

### A. CONSTRUCTION OF A HAIRPIN RIBOZYME WITH TARGETED SPECIFICITY FOR INTERFERON A MRNA

In order to efficiently inhibit the expression of interferon A protein in cells infected with Sindbis vectors, a hairpin ribozyme (HRBZ) with target specificity for interferon A mRNA is constructed. The IFN-a ribozyme moiety is first inserted into the plasmid vector pKSII⁺ (Stratagene, La Jolla, CA); removal of the ribozyme therapeutic from the plasmid vector and insertion into the various Sindbis vector backbones is accomplished via the unique ribozyme moiety terminal Cla I and Xba I restriction endonuclease sites.

The HRBZ is homologous to nucleotides 1026-1041 of the human interferon alpha gene IFN-alpha 4b shown below, and to all IFN-a genes sequenced, including 5, 6, 7, 8, and 14, but not gene 16 (Henco et al.. J. Mol. Biol. 185:227-260, 1985):
TCT CTG TCC TCC ATG A (SEQ. ID NO. 86)

The HRBZ is designed to cleave after the T residue in the TGTC hairpin ribozyme loop 5 substrate motif, shown underlined above. Following cleavage, the HRBZ is recycled and able to hybridize to, and cleave, another IFN-a mRNA molecule.

Double-stranded HRBZ as defined previously (Hampel et al., Nucleic Acids Research 18:299-304, 1990), containing a 4 base tetraloop 3 and an extended helix 4, with specificity for the IFN-a mRNA shown above, is chemically synthesized and includes at the 5' and 3' ends, respectively, Cla I and Xba I sites. The sequence of the chemically synthesized IFN-a HRBZ strands are shown below:
IFN-a HRBZ, sense strand (5' to 3'):
IFN-a HBRZ, antisense strand (5' to 3'):

In order to form the double-stranded IFN-a specific HRBZ with Cla I and Xba I cohesive ends, equal amounts of the oligonucleotides are mixed together in 10 mM Mg²⁺, heated at 95°C for 5 min, then cooled slowly to room temperature to allow the strands to anneal.

The double-stranded IFN-a HRBZ with Cla I and Xba I cohesive ends is first ligated into the pKSII⁺ plasmid vector, prepared by digestion with Cla I and Xba I, treatment with CIAP, and treatment with Gene Clean. This plasmid is known as pKSIFNaHRBZ.

The IFN-a hairpin ribozyme moiety is liberated from the pKSIFNaHRBZ plasmid by digestion with Cla I and Xba I, purification by 2% NuSieve/1% agarose electrophoresis and Gene Clean, and insertion into the desired vector backbone, prepared by digestion with Cla I and Xba I, and treatment with CIAP. Several possible Sindbis vectors some of which are shown below, and whose detailed construction is described in Examples 2, 3, and 4 are suitable for the insertion of the IFN-a hairpin ribozyme moiety:

| **Vector** | **Functional Junction Region (+/-)** |
|---|---|
| pKSSINBV | + |
| pKSSINBVdIJR | - |
| pKSSINdIJRsjrc | + |
| pKSSINdIJRsjrPC | + |
| pKSSINdIJRsjrNP(7582-7601) | + |
| pKSSINdIJRsexjr | + |
| pKSSINdIJRsjrcAdE3 | + |
| pKSSINdIJRsjrcH301 | + |

Since the ribozyme activity operates at the level of RNA, it is not necessary that this region is expressed as a portion subgenomic mRNA. However, when placed downstream of a functional junction region, the level of ribozyme synthesized is much greater and perhaps more effective in cleaving the IFN-a RNA target.

Further, in some applications, for examples systemic expression of protein, multiple dose administration to an individual is required. In these applications, prolonged expression of the therapeutic palliative without induction of an immune response targeted towards the vector infected cell is desired. In this configuration, the IFN-aHRBZ moiety could be inserted upstream of the adenovirus E3 or human cytomegalovirus H301 genes, which down-regulate the expression of MHC class I molecules in infected cells. Following the gene which modulates MHC class I expression is, consecutively, an IRES element selected from among the group described in Example 5, and the therapeutic palliative. Ordered insertion of the hairpin ribozyme, Ad E3 or CMV H301, IRES, and heterologous gene of interest components along the multiple cloning sequence located in the vector between the vector junction region and 3' end is accomplished by modification with the appropriate restriction enzyme recognition sites of the component 5' and 3' ends. In these constructions, functional INF-a hairpin ribozyme palliatives will be present at the level of both subgenomic and positive stranded genomic Sindbis vector RNA.

### EXAMPLE 20

### LACTOSE FORMULATION OF A RECOMBINANT ALPHAVIRUS VECTOR

Crude recombinant alphavirus vector is obtained from a Celligan bioreactor (New Brunswick, NJ) containing packaging cells transfected or transduced with the recombinant alphavirus vector, and bound to the beads of the bioreactor matrix. The cells release the recombinant alphavirus vector into the growth media that is passed over the cells in a continuous flow process. The media exiting the bioreactor is collected and passed initially through a 0.8 micron filter then through a 0.65 micron filter to clarify the crude recombinant alphavirus vector. The filtrate is concentrated utilizing a cross flow concentrating system (Filtron, Boston, MA). Approximately 50 units of DNase (Intergen, New York, NY) per ml of concentrate is added to digest exogenous DNA. The digest is diafiltrated using the same cross flow system to 150 mM NaCl, 25 mM tromethamine, pH 7.2. The diafiltrate is loaded onto a Sephadex S-500 gel column (Pharmacia, Piscataway, NJ), equilibrated in 50 mM NaCl, 25 mM tromethamine, pH 7.4. The purified recombinant alphavirus vector is eluted from the Sephadex S-500 gel column in 50 mM NaCl, 25 mM tromethamine, pH 7.4.

The formulation buffer containing lactose was prepared as a 2X concentrated stock solution. The formulation buffer contains 25 mM tromethamine, 70 mM NaCl, 2 mg/ml arginine, 10 mg/ml human serum albumin (HSA), and 100 mg/ml lactose in a final volume of 100 mls at a pH 7.4.

The purified recombinant alphavirus vector is formulated by adding one part 2X lactose formulation buffer to one part S-500 purified recombinant alphavirus vector. The formulated recombinant alphavirus vector can be stored at -70°C to -80°C or dried.

The formulated alphavirus vector is lyophilized in an Edwards Refrigerated Chamber (3 Shelf RC3S unit) attached to a Supermodulyo 12K freeze dryer (Edwards High Vacuum, Tonawanda. NY). When the freeze drying cycle is completed, the vials are stoppered under a vacuum following a slight nitrogen gas bleeding. Upon removal, vials are crimped with aluminum seals. The lyophilized recombinant retrovirus is reconstituted with 1.0 ml water.

From the foregoing, it will be appreciated that, although specific embodiments of the invention have been described herein for purposes of illustration, various modifications may be made. Accordingly, the invention is not limited except as by the appended claims.

## Claims

1. A DNA alphavirus structural protein expression cassette which comprises a promoter which directs the synthesis of an RNA molecule in a eukaryotic cell, said RNA molecule comprising the coding sequence for an alphavirus capsid protein, but not coding sequences for alphavirus glycoproteins E1 and E2.

2. A DNA alphavirus structural protein expression cassette which comprises a promoter which directs the synthesis of an RNA molecule in a eukaryotic cell, said RNA molecule comprising coding sequences for alphavirus glycoproteins E1 and E2, but not a coding sequence for an alphavirus capsid protein.

3. An expression cassette as claimed in claim 1 or claim 2 further comprising a poly-A tail.

4. An expression cassette as claimed in any one of claims 1 to 3 which is capable of directing expression of one or more alphavirus structural proteins derived from an alphavirus selected from Venezuelan equine encephalitis virus, Ross River virus or Semliki Forest virus.

5. An expression cassette as claimed in any one of claims 1 to 3 which is capable of directing expression of one or more structural proteins derived from Sindbis virus.

6. An expression cassette as claimed in any one of claims 1 to 5, wherein the promoter is an inducible eukaryotic promoter.

7. An expression cassette as claimed in any one of claims 1 to 5, wherein the promoter is an constitutive eukaryotic promoter.

## Patentansprüche

1. Expressionskassette für ein DNA-Alphavirus-Strukturprotein, die einen Promotor umfaßt, der die Synthese eines RNA-Moleküls in einer eukaryotischen Zelle steuert, wobei das RNA-Molekül die kodierende Sequenz für ein Alphavirus-Capsidprotein, aber nicht kodierende Sequenzen für die Alphavirus-Glykoproteine E1 und E2, umfaßt.

2. Expressionskassette für ein DNA-Alphavirus-Strukurprotein, die einen Promotor umfaßt, der die Synthese eines RNA-Moleküls in einer eukaryotischen Zelle steuert, wobei das RNA-Molekül kodierende Sequenzen für die Alphavirus-Glykoproteine E1 und E2, aber nicht eine kodierende Sequenz für ein Alphavirus-Capsidprotein, umfaßt.

3. Expressionskassette wie In Anspruch 1 oder Anspruch 2 beansprucht, weiter umfassend einen poly-A-Schwanz.

4. Expressionskassette wie in einem der Ansprüche 1 bis 3 beansprucht, die befähigt ist, die Expression eines oder mehrerer Alphavirus-Strukturproteine zu steuern, die von einem Alphavirus abgeleitet sind, ausgewählt aus venezolanischem Pferdeenzephalomyelitis-Virus, Ross-River-Virus oder Semliki-Forest-Virus.

5. Expressionskassette wie in einem der Ansprüche 1 bis 3 beansprucht, die befähigt ist, die Expression eines oder mehrerer von Sindbis-Virus abgeleiteter Strukturproteine zu steuern.

6. Expressionskassette wie in einem der Ansprüche 1 bis 5 beansprucht, wobei der Promotor ein induzierbarer eukaryotischer Promotor ist.

7. Expressionskassette wie in einem der Ansprüche 1 bis 5 beansprucht, wobei der Promotor ein konstitutiver eurkaryotischer Promotor ist.

## Revendications

1. Cassette d'expression d'une protéine structurale d'alphavirus à ADN qui comprend un promoteur qui dirige la synthèse d'une molécule d'ARN dans une cellule eucaryote, ladite molécule d'ARN comprenant la séquence codante pour une protéine de capside d'alphavirus, mais pas de séquence codante pour les glycoprotéines E1 et E2 d'alphavirus.

2. Cassette d'expression d'une protéine structurale d'alphavirus à ADN qui comprend un promoteur qui dirige la synthèse d'une molécule d'ARN dans une cellule eucaryote, ladite molécule d'ARN comprenant des séquences codantes pour les glycoprotéines E1 et E2 d'alphavirus, mais pas de séquence codante pour une protéine de capside d'alphavirus.

3. Cassette d'expression selon la revendication 1 ou la revendication 2, comprenant en outre une queue poly-A.

4. Cassette d'expression selon l'une quelconque des revendications 1 à 3, qui est capable de diriger l'expression d'une ou de plusieurs protéines structurales d'alphavirus dérivées d'un alphavirus choisi parmi le virus de l'encéphalite équine du Venezuela, le virus de la rivière Ross et le virus de la forêt de Semliki.

5. Cassette d'expression selon l'une quelconque des revendications 1 à 3, qui est capable de diriger l'expression d'une ou de plusieurs protéines structurales dérivées du virus de Sindbis.

6. Cassette d'expression selon l'une quelconque des revendications 1 à 5, dans laquelle le promoteur est un promoteur eucaryote inductible.

7. Cassette d'expression selon l'une quelconque des revendications 1 à 5, dans laquelle le promoteur est un promoteur eucaryote constitutif.


### SEQUENCE LISTING

(1) GENERAL INFORMATION:
   (i) APPLICANT:
      (A) NAME: CHIRON CORPORATION
      (B) STREET: 4560 Horton Street
      (C) CITY: Emeryville
      (D) STATE: California
      (E) COUNTRY: US
      (F) POSTAL CODE (ZIP): 92121
   (ii) TITLE OF INVENTION: RECOMBINANT ALPHAVIRUS VECTORS
   (iii) NUMBER OF SEQUENCES: 89
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: WO US95/07994
(2) INFORMATION FOR SEQ ID NO: 1:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO: 1:
      ATCTCTACGG TGGTCCTAAA TAGT 24
(2) INFORMATION FOR SEQ ID NO: 2:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO: 2:
      TATATTCTAG ATTTTTTTTT TTTTTTGAAA 42
(2) INFORMATION FOR SEQ ID NO:3:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (xi) SEQUENCE DESCRIPTION: SEQ ID NO:3:
      TATATGGGCC CGATTTAGGT GACACTATAG ATTGACGGCG TAGTACAC 48
(2) INFORMATION FOR SEQ ID NO:4:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:4:
      CTGGCAACCG GTAAGTACGA TAC 23
(2) INFORMATION FOR SEQ ID NO:5:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:5:
      ATACTAGCCA CGGCCGGTAT C 21
(2) INFORMATION FOR SEQ ID NO:6:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:6:
      TCCTCTTTCG ACGTGTCGAG C 21
(2) INFORMATION FOR SEQ ID NO:7:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:7:
      ACCTTGGAGC GCAATGTCCT G 21
(2) INFORMATION FOR SEQ ID NO:8:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:8:
      CCTTTTCAGG GGATCCGCCA C 21
(2) INFORMATION FOR SEQ ID NO:9:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:9:
      GTGGCGGATC CCCTGAAAAG G 21
(2) INFORMATION FOR SEQ ID NO:10:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO:10:
      TGGGCCGTGT GGTCGCATG 19
(2) INFORMATION FOR SEQ ID NO:11:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:11:
      TGGGTCTTCA ACTCACCGGA C 21
(2) INFORMATION FOR SEQ ID NO:12:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:12:
      CAATTCGACG TACGCCTCAC TC 22
(2) INFORMATION FOR SEQ ID NO:13:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:13:
      GAGTGAGGCG TACGTCGAAT TG 22
(2) INFORMATION FOR SEQ ID NO:14:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:14:
      TATATCTCCA GATGAGGTAC ATGATTTTAG GCTTG 35
(2) INFORMATION FOR SEQ ID NO:15:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO:15:
      TATATATCGA TTCAAGGCAT TTTCTTTTCA TCAATAAAAC 40
(2) INFORMATION FOR SEQ ID NO:16:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:16:
      TATATCTCCA GATGATGACA ATGTGGTGTC TGACG 35
(2) INFORMATION FOR SEQ ID NO:17:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:17:
      TATATATCGA TTCATGACGA CCGGACCTTG CG 32
(2) INFORMATION FOR SEQ ID NO:18:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:18:
      TATATGGGCC CCCCCCCCCC CCCCAACG 28
(2) INFORMATION FOR SEQ ID NO:19:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:19:
      TATATATCGA TCCCCCCCCC CCCCCCAACG 30
(2) INFORMATION FOR SEQ ID NO:20:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:20:
      TATATCCATG GCTTACAATC GTGGTTTTCA AAGG 34
(2) INFORMATION FOR SEQ ID NO:21:
   (i) SEQUENCE-CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:21:
      TATATGGGCC CTCGATGAGT CTGGACGTTC CTC 33
(2) INFORMATION FOR SEQ ID NO:22:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:22:
      TATATATCGA TTCGATGAGT CTGGACGTTC CTC 33
(2) INFORMATION FOR SEQ ID NO:23:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (xi) SEQUENCE DESCRIPTION: SEQ ID NO:23:
      TATATCCATG GATCCAATTT GCTTTATGAT AACAATC 37
(2) INFORMATION FOR SEQ ID NO:24:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:24:
      TATATGGGCC CGGTCGACGC CGGCCAAGAC 30
(2) INFORMATION FOR SEQ ID NO:25:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:25:
      TATATATCGA TGGTCGACGC CGGCCAAGAC 30
(2) INFORMATION FOR SEQ ID NO:26:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:26:
      TATATCCATG GTGCCAGCCA GTTGGGCAGC AG 32
(2) INFORMATION FOR SEQ ID NO:27:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:27:
      TTAATTAACG GCCGCCACCA TGG 23
(2) INFORMATION FOR SEQ ID NO:28:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (xi) SEQUENCE DESCRIPTION: SEQ ID NO:28:
      TAACGGCCGC CAC 13
(2) INFORMATION FOR SEQ ID NO:29:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:29:
      CCATGGTGGC GGCCGTTAAT 20
(2) INFORMATION FOR SEQ ID NO:30:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (xi) SEQUENCE DESCRIPTION: SEQ ID NO:30:
      GTCACCGGTG AC 12
(2) INFORMATION FOR SEQ ID NO:31:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:31:
      CTCATCGATC AGATCTGACT AGTTG 25
(2) INFORMATION FOR SEQ ID NO:32:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:32:
      GATCCAACTA GTCAGATCTG ATCGATGAGG GCC 33
(2) INFORMATION FOR SEQ ID NO:33:
   (i) SEQUENCE CHARACTERISTICS:
      (A) LENGTH: 56 base pairs
      (B) TYPE: nucleic acid
      (C) STRANDEDNESS: single
      (D) TOPOLOGY: linear
   (xi) SEQUENCE DESCRIPTION: SEQ ID NO:33:
      ACTTATCGAT GGTTCTAGAC TCCCTTAGCC ATCCGAGTGG ACGTGCGTCC TCCTTC 56
(2) INFORMATION FOR SEQ ID NO:34:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:34:
      TCCACCTCCT CGCGGTCCGA CCTGGGCATC CGAAGGAGGA CGCACGTCCA CT 52
(2) INFORMATION FOR SEQ ID NO:35:
   (i) SEQUENCE CHARACTERISTICS:
      (A) LENGTH: 57 base pairs
      (B) TYPE: nucleic acid
      (C) STRANDEDNESS: single
      (D) TOPOLOGY: linear
   (xi) SEQUENCE DESCRIPTION: SEQ ID NO:35:
      TCGGACCGCG AGGAGGTGGA GATGCCATGC CGACCCATTG ACGGCGTAGT ACACACT 57
(2) INFORMATION FOR SEQ ID NO:36:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:36:
      CTGGACTAGT TAATACTGGT GCTCGGAAAA CATTCT 36
(2) INFORMATION FOR SEQ ID NO:37:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO:37:
      GTCAAGCTTG CTAGCTACAA CACCACCACC ATGAATAGAG 40
(2) INFORMATION FOR SEQ ID NO:38:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
         (xi) SEQUENCE DESCRIPTION: SEQ ID NO:38:
         TATATGCGGC CGCACCACCA CCATGAATAG AGGATTCTTT AACATGC 47
(2) INFORMATION FOR SEQ ID NO:39:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:39:
      TATATGCGGC CGCTCATCTT CGTGTGCTAG TCAG 34
(2) INFORMATION FOR SEQ ID NO:40-.
   (i) SEQUENCE CHARACTERISTICS:
      (A) LENGTH: 61 base pairs
      (B) TYPE: nucleic acid
      (C) STRANDEDNESS: single
      (D) TOPOLOGY: linear
   (xi) SEQUENCE DESCRIPTION: SEQ ID NO:40:
   TATATGCGGC CGCATCTCTA CGGTGGTCCT AAATAGTACC ACCACCATGA ATAGAGGATT 60
   C 61
(2) INFORMATION FOR SEQ ID NO:41:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO:41:
      CAGTCTCGAG TTACTACCAC TCTTCTGTCC CTTCCGGGGT 40
(2) INFORMATION FOR SEQ ID NO:42:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (xi) SEQUENCE DESCRIPTION: SEQ ID NO:42:
      TATATGCGGC CGCACCACCA TGTCCGCAGC ACCACTGGTC ACG 43
(2) INFORMATION FOR SEQ ID NO:43:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:43:
      TATATAGATC TCTTGATCAG CTTCAGAAGA TGGC 34
(2) INFORMATION FOR SEQ ID NO:44:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:44:
      TCAATGGCGG GAAGAGGCGG TTGG 24
(2) INFORMATION FOR SEQ ID NO:45:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:45:
      CCGCCTCTTC CCGCCATTGA CGGCGTAGTA C 31
(2) INFORMATION FOR SEQ ID NO:46:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:46:
      CTGGCAACCG GTAAGTACGA TAC 23
(2) INFORMATION FOR SEQ ID NO:47:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:47:
      TATATAGATC TCTTGATCAG CTTCAGAAGA TGGC 34
(2) INFORMATION FOR SEQ ID NO:48:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:48:
      GGTAACAAGA TCTCGTGCCG TG 22
(2) INFORMATION FOR SEQ ID NO:49:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO:49:
      TATATGCGGC CGCACCGCCA AGATGTTCCC GTTCCAGCCA 40
(2) INFORMATION FOR SEQ ID NO:50:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:50:
      TATATGCGGC CGCTCAATTA TGTTTCTGGT TGGT 34
(2) INFORMATION FOR SEQ ID NO:51:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:51:
      CTCGAGCTCG AGGCACCAGC ACCATGCAAC TTTTT 35
(2) INFORMATION FOR SEQ ID NO:52:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:52:
      CTACTAGATC CCTAGATGCT GGATCTTCC 29
(2) INFORMATION FOR SEQ ID NO:53:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:53:
      GGAAGATCCA GCATCTAGGG ATCTAGTAG 29
(2) INFORMATION FOR SEQ ID NO:54:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (xi) SEQUENCE DESCRIPTION: SEQ ID NO:54:
      GGGCGATATC AAGCTTATCG ATACCG 26
(2) INFORMATION FOR SEQ ID NO:55:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:55:
      CTCGAGCTCG AGGCACCAGC ACCATGCAAC TTTTT 35
(2) INFORMATION FOR SEQ ID NO:56:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (xi) SEQUENCE DESCRIPTION: SEQ ID NO:56:
      GGGCGATATC AAGCTTATCG ATACCG 26
(2) INFORMATION FOR SEQ ID NO:57:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO:57:
      AATACGACTC ACTATAGGG 19
(2) INFORMATION FOR SEQ ID NO:58:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:58:
      CTACTAGATC CCTAGATGCT GGATCTTCC 29
(2) INFORMATION FOR SEQ ID NO:59:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (xi) SEQUENCE DESCRIPTION: SEQ ID NO:59:
      ATTAACCCTC ACTAAAG 17
(2) INFORMATION FOR SEQ ID NO:60:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:60:
      GGAAGATCCA GCATCTAGGG ATCTAGTAG 29
(2) INFORMATION FOR SEQ ID NO:61:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (xi) SEQUENCE DESCRIPTION: SEQ ID NO:61:
      ATTAACCCTC ACTAAAG 17
(2) INFORMATION FOR SEQ ID NO:62:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO:62:
      AATACGACTC ACTATAGGG 19
(2) INFORMATION FOR SEQ ID NO:63:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
         (xi) SEQUENCE DESCRIPTION: SEQ ID NO:63:
         CCTCGAGCTC GAGCTTGGGT GGCTTTGGGG CATG 34
(2) INFORMATION FOR SEQ ID NO:64:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
         (xi) SEQUENCE DESCRIPTION: SEQ ID NO:64:
         ATTACCCCTC ACTAAAG 17
(2) INFORMATION FOR SEQ ID NO:65:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:65:
      TCCACCTCCT CGCGGTCCGA CCTGGGCATC CGAAGGAGGA CGCACGTCCA CT 52
(2) INFORMATION FOR SEQ ID NO:66:
   (i) SEQUENCE CHARACTERISTICS:
      (A) LENGTH: 56 base pairs
      (B) TYPE: nucleic acid
      (C) STRANDEDNESS: single
      (D) TOPOLOGY: linear
   (xi) SEQUENCE DESCRIPTION: SEQ ID NO:66:
      ACTTATCGAT-GGTTCTAGAC TCCCTTAGCC ATCCGAGTGG ACGTGCGTCC TCCTTC 56
(2) INFORMATION FOR SEQ ID NO:67:
   (i) SEQUENCE CHARACTERISTICS:
      (A) LENGTH: 57 base pairs
      (B) TYPE: nucleic acid
      (C) STRANDEDNESS: single
      (D) TOPOLOGY: linear
   (xi) SEQUENCE DESCRIPTION: SEQ ID NO:67:
      TCGGACCGCG AGGAGGTGGA GATGCCATGC CGACCCATTG ACGGCGTAGT ACACACT 57
(2) INFORMATION FOR SEQ ID NO:68:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:68:
      CTGGACTAGT TAATACTGGT GCTCGGAAAA CATTCT 36
(2) INFORMATION FOR SEQ ID NO:69:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:69:
      TTTCTGGCTC CAGCCAAAGC CACCCTAGGG GAG 33
(2) INFORMATION FOR SEQ ID NO:70:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:70:
      AATGGAGTAG CCAGGTGAGA TTGTCTCCAG GAA 33
(2) INFORMATION FOR SEQ ID NO:71:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (xi) SEQUENCE DESCRIPTION: SEQ ID NO:71:
      CGCGCGGGCC CTGTGACATT GAATAGAGTG AGGGTCCTGT TGGG 44
(2) INFORMATION FOR SEQ ID NO:72:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:72:
      AAAGGTTTCA CATTTGTAGC TTGCTGTGTC ATTGCGATCT CTACG 45
(2) INFORMATION FOR SEQ ID NO:73:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION. SEQ ID NO:73:
      GTGGTCCTAA ATAGTTCACT CTATTCAATG TCACACTCGA GCCGG 45
(2) INFORMATION FOR SEQ ID NO:74:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:74:
      TTTCTGGCTC CAGCCAAAGC CACCCTAGGG GAG 33
(2) INFORMATION FOR SEQ ID NO:75:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:75:
      AATGGAGTAG CCAGGTGAGA TTGTCTCCAG GAA 33
(2) INFORMATION FOR SEQ ID NO:76:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear.
   (xi) SEQUENCE DESCRIPTION: SEQ ID NO:76:
      TATATTCTAG AGCAAGCAAC AGTTACTGCG ACG 33
(2) INFORMATION FOR SEQ ID NO:77:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:77:
      TATATATCGA TCCGAAGCGT AGAGTCACAC TTG 33
(2) INFORMATION FOR SEQ ID NO:78:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:78:
      TTAACTGTCA AAAGCCAC 18
(2) INFORMATION FOR SEQ ID NO:79:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (xi) SEQUENCE DESCRIPTION: SEQ ID NO:79:
   CGATGTGGCT TTTAGATGTT AAACCAGAGA AACACACGGA CTTCGGTCCG TGGTATATTA 60
   GCTGGTAT 68
(2) INFORMATION FOR SEQ ID NO:80:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
   (xi) SEQUENCE DESCRIPTION: SEQ ID NO:80:
   CTAGATACCA GCTAATATAC CACGGACCGA AGTCCGTGTG TTTCTCTGGT TTAACATCTA 60
   AAAGCCACAT 70
(2) INFORMATION FOR SEQ ID NO:81:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear.
   (xi) SEQUENCE DESCRIPTION: SEQ ID NO:81:
      TATATTCTAG AGCAAGCAAC AGTTACTGCG ACG 33
(2) INFORMATION FOR SEQ ID NO:82:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:82:
      TATATATCGA TCCGAAGCGT AGAGTCACAC TTG 33
(2) INFORMATION FOR SEQ ID NO:83:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:83:
      TTAACTGTCA AAAGCCAC 18
(2) INFORMATION FOR SEQ ID NO:84:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (xi) SEQUENCE DESCRIPTION: SEQ ID NO:84:
   CGATGTGGCT TTTAGATGTT AAACCAGAGA AACACACGGA CTTCGGTCCG TGGTATATTA 60
   GCTGGTAT 68
(2) INFORMATION FOR SEQ ID NO:85:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
         (xi) SEQUENCE DESCRIPTION: SEQ ID NO:85:
   CTAGATACCA GCTAATATAC CACGGACCGA AGTCCGTGTG TTTCTCTGGT TTAACATCTA 60
   AAAGCCACAT 70
(2) INFORMATION FOR SEQ ID NO:86:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (xi) SEQUENCE DESCRIPTION: SEQ ID NO:86:
      TCTCTGTCCT CCATGA 16
(2) INFORMATION FOR SEQ ID NO:87:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (xi) SEQUENCE DESCRIPTION: SEQ ID NO:87:
   TCGAGTCATG GAGAGAGGAG AACCAGAGAA ACACACGGAC TTCGGTCCGT GGTATATTAC 60
   CTGGAT 66
(2) INFORMATION FOR SEQ ID NO:88:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (xi) SEQUENCE DESCRIPTION: SEQ ID NO:88:
   CGATCCAGGT AATATACCAC GGACCGAAGT CCGTGTGTTT CTCTGGTTCT CCTCTCTCCA 60
   TGAC 64
(2) INFORMATION FOR SEQ ID NO:89:
   (i) SEQUENCE CHARACTERISTICS:
      (A) LENGTH: 16656 base pairs
      (B) TYPE: nucleic acid
      (C) STRANDEDNESS: single
      (D) TOPOLOGY: linear
   (xi) SEQUENCE DESCRIPTION: SEQ ID NO:89:
   ATTGACGGCG TAGTACACAC TATTGAATCA AACAGCCGAC CAATCGCACT ACCATCACAA 60
   TGGAGAAGCC AGTAGTAAAC GTAGACGTAG ACCCCCAGAG TCCGTTTGTC GTGCAACTGC 120
   AAAAAAGCTT CCCGCAATTT GAGGTAGTAG CACAGCAGGT CACTCCAAAT GACCATGCTA 180
   ATGCCAGAGC ATTTTCGCAT CTGGCCAGTA AACTAATCGA GCTGGAGGTT CCTACCACAG 240
   CGACGATCTT GGACATAGGC AGCGCACCGG CTCGTAGAAT GTTTTCCGAG CACCAGTATC 300
   ATTGTGTCTG CCCCATGCGT AGTCCAGAAG ACCCGGACCG CATGATGAAA TATGCCAGTA 360
   AACTGGCGGA AAAAGCGTGC AAGATTACAA ACAAGAACTT GCATGAGAAG ATTAAGGATC 420
   TCCGGACCGT ACTTGATACG CCGGATGCTG AAACACCATC GCTCTGCTTT CACAACGATG 480
   TTACCTGCAA CATGCGTGCC GAATATTCCG TCATGCAGGA CGTGTATATC AACGCTCCCG 540
   GAACTATCTA TCATCAGGCT ATGAAAGGCG TGCGGACCCT GTACTGGATT GGCTTCGACA 600
   CCACCCAGTT CATGTTCTCG GCTATGGCAG GTTCGTACCC TGCGTACAAC ACCAACTGGG 660
   CCGACGAGAA AGTCCTTGAA GCGCGTAACA TCGGACTTTG CAGCACAAAG CTGAGTGAAG 720
   GTAGGACAGG AAAATTGTCG ATAATGAGGA AGAAGGAGTT GAAGCCCGGG TCGCGGGTTT 780
   ATTTCTCCGT AGGATCGACA CTTTATCCAG AACACAGAGC CAGCTTGCAG AGCTGGCATC 840
   TTCCATCGGT GTTCCACTTG AATGGAAAGC AGTCGTACAC TTGCCGCTGT GATACAGTGG 900
   TGAGTTGCGA AGGCTACGTA GTGAAGAAAA TCACCATCAG TCCCGGGATC ACGGGAGAAA 960
   CCGTGGGATA CGCGGTTACA CACAATAGCG AGGGCTTCTT GCTATGCAAA GTTACTGACA 1020
   CAGTAAAAGG AGAACGGGTA TCGTTCCCTG TGTGCACGTA CATCCCGGCC ACCATATGCG 1080
   ATCAGATGAC TGGTCTAATG GCCACGGATA TATCACCTGA CGATGCACAA AAACTTCTGG 1140
   TTGGGCTCAA CCAGCGAATT GTCATTAACG GTAGGACTAA CAGGAACACC AACACCATGC 1200
   AAAATTACCT TCTGCCGATC ATAGCACAAG GGTTCAGCAA ATGGGCTAAG GAGCGCAAGG 1260
   ATGATCTTGA TAACGAGAAA ATGCTGGGTA CTAGAGAACG CAAGCTTACG TATGGCTGCT 1320
   TGTGGGCGTT TCGCACTAAG AAAGTACATT CGTTTTATCG CCCACCTGGA ACGCAGACCT 1380
   GCGTAAAAGT CCCAGCCTCT TTTAGCGCTT TCCCCATGTC GTCCGTATGG ACGACCTCTT 1440
   TGCCCATGTC GCTGAGGCAG AAATTGAAAC TGGCATTGCA ACCAAAGAAG GAGGAAAAAC 1500
   TGCTGCAGGT CTCGGAGGAA TTAGTCATGG AGGCCAAGGC TGCTTTTGAG GATGCTCAGG 1560
   AGGAAGCCAG AGCGGAGAAG CTCCGAGAAG CACTTCCACC ATTAGTGGCA GACAAAGGCA 1620
   TCGAGGCAGC CGCAGAAGTT GTCTGCGAAG TGGAGGGGCT CCAGGCGGAC ATCGGAGCAG 1680
   CATTAGTTGA AACCCCGCGC GGTCACGTAA GGATAATACC TCAAGCAAAT GACCGTATGA 1740
   TCGGACAGTA TATCGTTGTC TCGCCAAACT CTGTGCTGAA GAATGCCAAA CTCGCACCAG 1800
   CGCACCCGCT AGCAGATCAG GTTAAGATCA TAACACACTC CGGAAGATCA GGAAGGTACG 1860
   CGGTCGAACC ATACGACGCT AAAGTACTGA TGCCAGCAGG AGGTGCCGTA CCATGGCCAG 1920
   AATTCCTAGC ACTGAGTGAG AGCGCCACGT TAGTGTACAA CGAAAGAGAG TTTGTGAACC 1980
   GCAAACTATA CCACATTGCC ATGCATGGCC CCGCCAAGAA TACAGAAGAG GGGCAGTACA 2040
   AGGTTACAAA GGCAGAGCTT GCAGAAACAG AGTACGTGTT TGACGTGGAC AAGAAGCGTT 2100
   GCGTTAAGAA GGAAGAAGCC TCAGGTCTGG TCCTCTCGGG AGAACTGACC AACCCTCCCT 2160
   ATCATGAGCT AGCTCTGGAG GGACTGAAGA CCCGACCTGC GGTCCCGTAC AAGGTCGAAA 2220
   CAATAGGAGT GATAGGCACA CCGGGGTCGG GCAAGTCCGC TATTATCAAG TCAACTGTCA 2280
   CGGCACGAGA TCTTGTTACC AGCGGAAAGA AAGAAAATTG TCGCGAAATT GAGGCCGACG 2340
   TGCTAAGACT GAGGGGTATG CAGATTACGT CGAAGACAGT AGATTCGGTT ATGCTCAACG 2400
   GATGCCACAA AGCCGTAGAA GTGCTGTACG TTGACGAAGC GTTCGCGTGC CACGCAGGAG 2460
   CACTACTTGC CTTGATTGCT ATCGTCAGGC CCCGCAAGAA GGTAGTACTA TGCGGAGACC 2520
   CCATGCAATG CGGATTCTTC AACATGATGC AACTAAAGGT ACATTTCAAT CACCCTGAAA 2580
   AAGACATATG CACCAAGACA TTCTACAAGT ATATCTCCCG GCGTTGCACA CAGCCAGTTA 2640
   CAGCTATTGT ATCGACACTG CATTACGATG GAAAGATGAA AACCACGAAC CCGTGCAAGA 2700
   AGAACATTGA AATCGATATT ACAGGGGCCA CAAAGCCGAA GCCAGGGGAT ATCATCCTGA 2760
   CATGTTTCCG CGGGTGGGTT AAGCAATTGC AAATCGACTA TCCCGGACAT GAAGTAATGA 2820
   CAGCCGCGGC CTCACAAGGG CTAACCAGAA AAGGAGTGTA TGCCGTCCGG CAGAAAGTCA 2880
   ATGAAAACCC ACTGTACGCG ATCACATCAG AGCATGTGAA CGTGTTGCTC ACCCGCACTG 2940
   AGGACAGGCT AGTGTGGAAA ACCTTGCAGG GCGACCCATG GATTAAGCAG CTCACTAACA 3000
   TACCTAAAGG AAACTTTCAG GCTACTATAG AGGACTGGGA AGCTGAACAC AAGGGAATAA 3060
   TTGCTGCAAT AAACAGCCCC ACTCCCCGTG CCAATCCGTT CAGCTGCAAG ACCAACGTTT 3120
   GCTGGGCGAA AGCATTGGAA CCGATACTAG CCACGGCCGG TATCGTACTT ACCGGTTGCC 3180
   AGTGGAGCGA ACTGTTCCCA CAGTTTGCGG ATGACAAACC ACATTCGGCC ATTTACGCCT 3240
   TAGACGTAAT TTGCATTAAG TTTTTCGGCA TGGACTTGAC AAGCGGACTG TTTTCTAAAC 3300
   AGAGCATCCC ACTAACGTAC CATCCCGCCG ATTCAGCGAG GCCGGTAGCT CATTGGGACA 3360
   ACAGCCCAGG AACCCGCAAG TATGGGTACG ATCACGCCAT TGCCGCCGAA CTCTCCCGTA 3420
   GATTTCCGGT GTTCCAGCTA GCTGGGAAGG GCACACAACT TGATTTGCAG ACGGGGAGAA 3480
   CCAGAGTTAT CTCTGCACAG CATAACCTGG TCCCGGTGAA CCGCAATCTT CCTCACGCCT 3540
   TAGCCCCCGA GTACAAGGAG AAGCAACCCG GCCCGGTCGA AAAATTCTTG AACCAGTTCA 3600
   AACACCACTC AGTACTTGTG GTATCAGAGG AAAAAATTGA AGCTCCCCGT AAGAGAATCG 3660
   AATGGATCGC CCCGATTGGC ATAGCCGGTG CAGATAAGAA CTACAACCTG GCTTTCGGGT 3720
   TTCCGCCGCA GGCACGGTAC GACCTGGTGT TCATCAACAT TGGAACTAAA TACAGAAACC 3780
   ACCACTTTCA GCAGTGCGAA GACCATGCGG CGACCTTAAA AGCCCTTTCG CGTTCGGCCC 3840
   TGAATTGCCT CAACCCAGGA GGCACCCTCG TGGTGAAGTC CTATGGCTAC GCCGACCGCA 3900
   ACAGTGAGGA CGTAGTCACC GCTCTTGCCA GAAAGTTTGT CAGGGTGTCT GCAGCGAGAC 3960
   CAGATTGTGT CTCAAGCAAT ACAGAAATGT ACCTGATTTT CCGACAACTA GACAACAGCC 4020
   GTACACGGCA ATTCACCCCG CACCATCTGA ATTGCGTGAT TTCGTCCGTG TATGAGGGTA 4080
   CAAGAGATGG AGTTGGAGCC GCGCCGTCAT ACCGCACCAA AAGGGAGAAT ATTGCTGACT 4140
   GTCAAGAGGA AGCAGTTGTC AACGCAGCCA ATCCGCTGGG TAGACCAGGC GAAGGAGTCT 4200
   GCCGTGCCAT CTATAAACGT TGGCCGACCA GTTTTACCGA TTCAGCCACG GAGACAGGCA 4260
   CCGCAAGAAT GACTGTGTGC CTAGGAAAGA AAGTGATCCA CGCGGTCGGC CCTGATTTCC 4320
   GGAAGCACCC AGAAGCAGAA GCCTTGAAAT TGCTACAAAA CGCCTACCAT GCAGTGGCAG 4380
   ACTTAGTAAA TGAACATAAC ATCAAGTCTG TCGCCATTCC ACTGCTATCT ACAGGCATTT 4440
   ACGCAGCCGG AAAAGACCGC CTTGAAGTAT CACTTAACTG CTTGACAACC GCGCTAGACA 4500
   GAACTGACGC GGACGTAACC ATCTATTGCC TGGATAAGAA GTGGAAGGAA AGAATCGACG 4560
   CGGCACTCCA ACTTAAGGAG TCTGTAACAG AGCTGAAGGA TGAAGATATG GAGATCGACG 4620
   ATGAGTTAGT ATGGATCCAT CCAGACAGTT GCTTGAAGGG AAGAAAGGGA TTCAGTACTA 4680
   CAAAAGGAAA ATTGTATTCG TACTTCGAAG GCACCAAATT CCATCAAGCA GCAAAAGACA 4740
   TGGCGGAGAT AAAGGTCCTG TTCCCTAATG ACCAGGAAAG TAATGAACAA CTGTGTGCCT 4800
   ACATATTGGG TGAGACCATG GAAGCAATCC GCGAAAAGTG CCCGGTCGAC CATAACCCGT 4860
   CGTCTAGCCC GCCCAAAACG TTGCCGTGCC TTTGCATGTA TGCCATGACG CCAGAAAGGG 4920
   TCCACAGACT TAGAAGCAAT AACGTCAAAG AAGTTACAGT ATGCTCCTCC ACCCCCCTTC 4980
   CTAAGCACAA AATTAAGAAT GTTCAGAAGG TTCAGTGCAC GAAAGTAGTC CTGTTTAATC 5040
   CGCACACTCC CGCATTCGTT CCCGCCCGTA AGTACATAGA AGTGCCAGAA CAGCCTACCG 5100
   CTCCTCCTGC ACAGGCCGAG GAGGCCCCCG AAGTTGTAGC GACACCGTCA CCATCTACAG 5160
   CTGATAACAC CTCGCTTGAT GTCACAGACA TCTCACTGGA TATGGATGAC AGTAGCGAAG 5220
   GCTCACTTTT TTCGAGCTTT AGCGGATCGG ACAACTCTAT TACTAGTATG GACAGTTGGT 5280
   CGTCAGGACC TAGTTCACTA GAGATAGTAG ACCGAAGGCA GGTGGTGGTG GCTGACGTTC 5340
   ATGCCGTCCA TGAGCCTGCC CCTATTCCAC CGCCAAGGCT AAAGAAGATG GCCCGCCTGG 5400
   CAGCGGCAAG AAAAGAGCCC ACTCCACCGG CAAGCAATAG CTCTGAGTCC CTCCACCTCT 5460
   CTTTTGGTGG GGTATCCATG TCCCTCGGAT CAATTTTCGA CGGAGAGACG GCCCGCCAGG 5520
   CAGCGGTACA ACCCCTGGCA ACAGGCCCCA CGGATGTGCC TATGTCTTTC GGATCGTTTT 5580
   CCGACGGAGA GATTGATGAG CTGAGCCGCA GAGCAACTGA GTCCGAACCC GTCCTGTTTG 5640
   GATCATTTGA ACCGGGCGAA GTGAACTCAA TTATATCGTC CCGATCAGCC GTATCTTTTC 5700
   CACTACGCAA GCAGAGACGT AGACGCAGGA GCAGGAGGAC TGAATACTGA CTAACCGGGG 5760
   TAGGTGGGTA CATATTTTCG ACGGACACAG GCCCTGGGCA CTTGCAAAAG AAGTCCGTTC 5820
   TGCAGAACCA GCTTACAGAA CCGACCTTGG AGCGCAATGT CCTGGAAAGA ATTCATGCCC 5880
   CGGTGCTCGA CACGTCGAAA GAGGAACAAC TCAAACTCAG GTACCAGATG ATGCCCACCG 5940
   AAGCCAACAA AAGTAGGTAC CAGTCTCGTA AAGTAGAAAA TCAGAAAGCC ATAACCACTG 6000
   AGCGACTACT GTCAGGACTA CGACTGTATA ACTCTGCCAC AGATCAGCCA GAATGCTATA 6060
   AGATCACCTA TCCGAAACCA TTGTACTCCA GTAGCGTACC GGCGAACTAC TCCGATCCAC 6120
   AGTTCGCTGT AGCTGTCTGT AACAACTATC TGCATGAGAA CTATCCGACA GTAGCATCTT 6180
   ATCAGATTAC TGACGAGTAC GATGCTTACT TGGATATGGT AGACGGGACA GTCGCCTGCC 6240
   TGGATACTGC AACCTTCTGC CCCGCTAAGC TTAGAAGTTA CCCGAAAAAA CATGAGTATA 6300
   GAGCCCCGAA TATCCGCAGT GCGGTTCCAT CAGCGATGCA GAACACGCTA CAAAATGTGC 6360
   TCATTGCCGC AACTAAAAGA AATTGCAACG TCACGCAGAT GCGTGAACTG CCAACACTGG 6420
   ACTCAGCGAC ATTCAATGTC GAATGCTTTC GAAAATATGC ATGTAATGAC GAGTATTGGG 6480
   AGGAGTTCGC TCGGAAGCCA ATTAGGATTA CCACTGAGTT TGTCACCGCA TATGTAGCTA 6540
   GACTGAAAGG CCCTAAGGCC GCCACACTAT TTGCAAAGAC GTATAATTTG GTCCCATTGC 6600
   AAGAAGTGCC TATGGATAGA TTCGTCATGG ACATGAAAAG AGACGTGAAA GTTACACCAG 6660
   GCACGAAACA CACAGAAGAA AGACCGAAAG TACAAGTGAT ACAAGCCGCA GAACCCCTGG 6720
   CGACTGCTTA CTTATGCGGG ATTCACCGGG AATTAGTGCG TAGGCTTACG GCCGTCTTGC 6780
   TTCCAAACAT TCACACGCTT TTTGACATGT CGGCGGAGGA TTTTGATGCA ATCATAGCAG 6840
   AACACTTCAA GCAAGGCGAC CCGGTACTGG AGACGGATAT CGCATCATTC GACAAAAGCC 6900
   AAGACGACGC TATGGCGTTA ACCGGTCTGA TGATCTTGGA GGACCTGGGT GTGGATCAAC 6960
   CACTACTCGA CTTGATCGAG TGCGCCTTTG GAGAAATATC ATCCACCCAT CTACCTACGG 7020
   GTACTCGTTT TAAATTCGGG GCGATGATGA AATCCGGAAT GTTCCTCACA CTTTTTGTCA 7080
   ACACAGTTTT GAATGTCGTT ATCGCCAGCA GAGTACTAGA AGAGCGGCTT AAAACGTCCA 7140
   GATGTGCAGC GTTCATTGGC GACGACAACA TCATACATGG AGTAGTATCT GACAAAGAAA 7200
   TGGCTGAGAG GTGCGCCACC TGGCTCAACA TGGAGGTTAA GATCATCGAC GCAGTCATCG 7260
   GTGAGAGACC ACCTTACTTC TGCGGCGGAT TTATCTTGCA AGATTCGGTT ACTTCCACAG 7320
   CGTGCCGCGT GGCGGATCCC CTGAAAAGGC TGTTTAAGTT GGGTAAACCG CTCCCAGCCG 7380
   ACGACGAGCA AGACGAAGAC AGAAGACGCG CTCTGCTAGA TGAAACAAAG GCGTGGTTTA 7440
   GAGTAGGTAT AACAGGCACT TTAGCAGTGG CCGTGACGAC CCGGTATGAG GTAGACAATA 7500
   TTACACCTGT CCTACTGGCA TTGAGAACTT TTGCCCAGAG CAAAAGAGCA TTCCAAGCCA 7560
   TCAGAGGGGA AATAAAGCAT CTCTACGGTG GTCCTAAATA GTCAGCATAG TACATTTCAT 7620
   CTGACTAATA CTACAACACC ACCACCATGA ATAGAGGATT CTTTAACATG CTCGGCCGCC 7680
   GCCCCTTCCC GGCCCCCACT GCCATGTGGA GGCCGCGGAG AAGGAGGCAG GCGGCCCCGA 7740
   TGCCTGCCCG CAACGGGCTG GCTTCTCAAA TCCAGCAACT GACCACAGCC GTCAGTGCCC 7800
   TAGTCATTGG ACAGGCAACT AGACCTCAAC CCCCACGTCC ACGCCCGCCA CCGCGCCAGA 7860
   AGAAGCAGGC GCCCAAGCAA CCACCGAAGC CGAAGAAACC AAAAACGCAG GAGAAGAAGA 7920
   AGAAGCAACC TGCAAAACCC AAACCCGGAA AGAGACAGCG CATGGCACTT AAGTTGGAGG 7980
   CCGACAGATT GTTCGACGTC AAGAACGAGG ACGGAGATGT CATCGGGCAC GCACTGGCCA 8040
   TGGAAGGAAA GGTAATGAAA CCTCTGCACG TGAAAGGAAC CATCGACCAC CCTGTGCTAT 8100
   CAAAGCTCAA ATTTACCAAG TCGTCAGCAT ACGACATGGA GTTCGCACAG TTGCCAGTCA 8160
   ACATGAGAAG TGAGGCATTC ACCTACACCA GTGAACACCC CGAAGGATTC TATAACTGGC 8220
   ACCACGGAGC GGTGCAGTAT AGTGGAGGTA GATTTACCAT CCCTCGCGGA GTAGGAGGCA 8280
   GAGGAGACAG CGGTCGTCCG ATCATGGATA ACTCCGGTCG GGTTGTCGCG ATAGTCCTCG 8340
   GTGGCGCTGA TGAAGGAACA CGAACTGCCC TTTCGGTCGT CACCTGGAAT AGTAAAGGGA 8400
   AGACAATTAA GACGACCCCG GAAGGGACAG AAGAGTGGTC CGCAGCACCA CTGGTCACGG 8460
   CAATGTGTTT GCTCGGAAAT GTGAGCTTCC CATGCGACCG CCCGCCCACA TGCTATACCC 8520
   GCGAACCTTC CAGAGCCCTC GACATCCTTG AAGAGAACGT GAACCATGAG GCCTACGATA 8580
   CCCTGCTCAA TGCCATATTG CGGTGCGGAT CGTCTGGCAG AAGCAAAAGA AGCGTCGTTG 8640
   ACGACTTTAC CCTGACCAGC CCCTACTTGG GCACATGCTC GTACTGCCAC CATACTGAAC 8700
   CGTGCTTCAG CCCTGTTAAG ATCGAGCAGG TCTGGGACGA AGCGGACGAT AACACCATAC 8760
   GCATACAGAC TTCCGCCCAG TTTGGATACG ACCAAAGCGG AGCAGCAAGC GCAAACAAGT 8820
   ACCGCTACAT GTCGCTTAAG CAGGATCACA CCGTTAAAGA AGGCACCATG GATGACATCA 8880
   AGATTAGCAC CTCAGGACCG TGTAGAAGGC TTAGCTACAA AGGATACTTT CTCCTCGCAA 8940
   AATGCCCTCC AGGGGACAGC GTAACGGTTA GCATAGTGAG TAGCAACTCA GCAACGTCAT 9000
   GTACACTGGC CCGCAAGATA AAACCAAAAT TCGTGGGACG GGAAAAATAT GATCTACCTC 9060
   CCGTTCACGG TAAAAGAATT CCTTGCACAG TGTACGACCG TCTGAAAACA ACTGCAGGCT 9120
   ACATCACTAT GCACAGGCCG GGACCGCACG CTTATACATC CTACCTGGAA GAATCATCAG 9180
   GGAAAGTTTA CGCAAAGCCG CCATCTGGGA AGAACATTAC GTATGAGTGC AAGTGCGGCG 9240
   ACTACAAGAC CGGAACCGTT TCGACCCGCA CCGAAATCAC TGGTTGCACC GCCATCAAGC 9300
   AGTGCGTCGC CTATAAGAGC GACCAAACGA AGTGGGTCTT CAACTCACCG GACTTGATCA 9360
   GACATGACGA CCACACGGCC CAAGGGAAAT TGCATTTGCC TTTCAAGTTG ATCCCGGGTG 9420
   CCTGCATGGT CCCTGTTGCC CACGCGCCGA ATGTAATACA TGGCTTTAAA CACATCAGCC 9480
   TCCAATTAGA TACAGACCAC TTGACATTGC TCACCACCAG GAGACTAGGG GCAAACCCGG 9540
   AACCAACCAC TGAATGGATC GTCGGAAAGA CGGTCAGAAA CTTCACCGTC GACCGAGATG 9600
   GCCTGGAATA CATATGGGGA AATCATGAGC CAGTGAGGGT CTATGCCCAA GAGTCAGCAC 9660
   CAGGAGACCC TCACGGATGG CCACACGAAA TAGTACAGCA TTACTACCAT CGCCATCCTG 9720
   TGTACACCAT CTTAGCCGTC GCATCAGCTA CCGTGGCGAT GATGATTGGC GTAACTGTTG 9780
   CAGTGTTATG TGCCTGTAAA GCGCGCCGTG AGTGCCTGAC GCCATACGCC CTGGCCCCAA 9840
   ACGCCGTAAT CCCAACTTCG CTGGCACTCT TGTGCTGCGT TAGGTCGGCC AATGCTGAAA 9900
   CGTTCACCGA GACCATGAGT TACTTGTGGT CGAACAGTCA GCCGTTCTTC TGGGTCCAGT 9960
   TGTGCATACC TTTGGCCGCG TTCATCGTTC TAATGCGCTA CTGCTCCTGC TGCCTGCCTT 10020
   TTTTAGTGGT TGCCGGCGCC TACCTGGCGA AGGTAGACGC CTACGAACAT GCGACCACTG 10080
   TTCCAAATGT GCCACAGATA CCGTATAAGG CACTTGTTGA AAGGGCAGGG TATGCCCCGC 10140
   TCAATTTGGA GATCACTGTC ATGTCCTCGG AGGTTTTGCC TTCCACCAAC CAAGAGTACA 10200
   TTACCTGCAA ATTCACCACT GTGGTCCCCT CCCCAAAAAT CAAATGCTGC GGCTCCTTGG 10260
   AATGTCAGCC GGCCGCTCAT GCAGACTATA CCTGCAAGGT CTTCGGAGGG GTCTACCCCT 10320
   TTATGTGGGG AGGAGCGCAA TGTTTTTGCG ACAGTGAGAA CAGCCAGATG AGTGAGGCGT 10380
   ACGTCGAATT GTCAGCAGAT TGCGCGTCTG ACCACGCGCA GGCGATTAAG GTGCACACTG 10440
   CCGCGATGAA AGTAGGACTG CGTATAGTGT ACGGGAACAC TACCAGTTTC CTAGATGTGT 10500
   ACGTGAACGG AGTCACACCA GGAACGTCTA AAGACTTGAA AGTCATAGCT GGACCAATTT 10560
   CAGCATCGTT TACGCCATTC GATCATAAGG TCGTTATCCA TCGCGGCCTG GTGTACAACT 10620
   ATGACTTCCC GGAATATGGA GCGATGAAAC CAGGAGCGTT CGGAGACATT CAAGCTACCT 10680
   CCTTGACTAG CAAGGATCTC ATCGCCAGCA CAGACATTAG GCTACTCAAG CCTTCCGCCA 10740
   AGAACGTGCA TGTCCCGTAC ACGCAGGCCG CATCAGGATT TGAGATGTGG AAAAACAACT 10800
   CAGGCCGCCC ACTGCAGGAA ACCGCACCTT TCGGGTGTAA GATTGCAGTA AATCCGCTCC 10860
   GAGCGGTGGA CTGTTCATAC GGGAACATTC CCATTTCTAT TGACATCCCG AACGCTGCCT 10920
   TTATCAGGAC ATCAGATGCA CCACTGGTCT CAACAGTCAA ATGTGAAGTC AGTGAGTGCA 10980
   CTTATTCAGC AGACTTCGGC GGGATGGCCA CCCTGCAGTA TGTATCCGAC CGCGAAGGTC 11040
   AATGCCCCGT ACATTCGCAT TCGAGCACAG CAACTCTCCA AGAGTCGACA GTACATGTCC 11100
   TGGAGAAAGG AGCGGTGACA GTACACTTTA GCACCGCGAG TCCACAGGCG AACTTTATCG 11160
   TATCGCTGTG TGGGAAGAAG ACAACATGCA ATGCAGAATG TAAACCACCA GCTGACCATA 11220
   TCGTGAGCAC CCCGCACAAA AATGACCAAG AATTTCAAGC CGCCATCTCA AAAACATCAT 11280
   GGAGTTGGCT GTTTGCCCTT TTCGGCGGCG CCTCGTCGCT ATTAATTATA GGACTTATGA 11340
   TTTTTGCTTG CAGCATGATG CTGACTAGCA CACGAAGATG ACCGCTACGC CCCAATGATC 11400
   CGACCAGCAA AACTCGATGT ACTTCCGAGG AACTGATGTG CATAATGCAT CAGGCTGGTA 11460
   CATTAGATCC CCGCTTACCG CGGGCAATAT AGCAACACTA AAAACTCGAT GTACTTCCGA 11520
   GGAAGCGCAG TGCATAATGC TGCGCAGTGT TGCCACATAA CCACTATATT AACCATTTAT 11580
   CTAGCGGACG CCAAAAACTC AATGTATTTC TGAGGAAGCG TGGTGCATAA TGCCACGCAG 11640
   CGTCTGCATA ACTTTTATTA TTTCTTTTAT TAATCAACAA AATTTTGTTT TTAACATTTC 11700
   AAAAAAAAAA AAAAAAAAAA AAAAATCTAG AGGGCCCTAT TCTATAGTGT CACCTAAATG 11760
   CTAGAGCTCG CTGATCAGCC TCGACTGTGC CTTCTAGTTG CCAGCCATCT GTTGTTTGCC 11820
   CCTCCCCCGT GCCTTCCTTG ACCCTGGAAG GTGCCACTCC CACTGTCCTT TCCTAATAAA 11880
   ATGAGGAAAT TGCATCGCAT TGTCTGAGTA GGTGTCATTC TATTCTGGGG GGTGGGGTGG 11940
   GGCAGGACAG CAAGGGGGAG GATTGGGAAG ACAATAGCAG GCATGCTGGG GATGCGGTGG 12000
   GCTCTATGGC TTCTGAGGCG GAAAGAACCA GCTGGGGCTC TAGGGGGTAT CCCCACGCGC 12060
   CCTGTAGCGG CGCATTAAGC GCGGCGGGTG TGGTGGTTAC GCGCAGCGTG ACCGCTACAC 12120
   TTGCCAGCGC CCTAGCGCCC GCTCCTTTCG CTTTCTTCCC TTCCTTTCTC GCCACGTTCG 12180
   CCGGCTTTCC CCGTCAAGCT CTAAATCGGG GCATCCCTTT AGGGTTCCGA TTTAGTGCTT 12240
   TACGGCACCT CGACCCCAAA AAACTTGATT AGGGTGATGG TTCACGTAGT GGGCCATCGC 12300
   CCTGATAGAC GGTTTTTCGC CCTTTGACGT TGGAGTCCAC GTTCTTTAAT AGTGGACTCT 12360
   TGTTCCAAAC TGGAACAACA CTCAACCCTA TCTCGGTCTA TTCTTTTGAT TTATAAGGGA 12420
   TTTTGGGGAT TTCGGCCTAT TGGTTAAAAA ATGAGCTGAT TTAACAAAAA TTTAACGCGA 12480
   ATTAATTCTG TGGAATGTGT GTCAGTTAGG GTGTGGAAAG TCCCCAGGCT CCCCAGGCAG 12540
   GCAGAAGTAT GCAAAGCATG CATCTCAATT AGTCAGCAAC CAGGTGTGGA AAGTCCCCAG 12600
   GCTCCCCAGC AGGCAGAAGT ATGCAAAGCA TGCATCTCAA TTAGTCAGCA ACCATAGTCC 12660
   CGCCCCTAAC TCCGCCCATC CCGCCCCTAA CTCCGCCCAG TTCCGCCCAT TCTCCGCCCC 12720
   ATGGCTGACT AATTTTTTTT ATTTATGCAG AGGCCGAGGC CGCCTCTGCC TCTGAGCTAT 12780
   TCCAGAAGTA GTGAGGAGGC TTTTTTGGAG GCCTAGGCTT TTGCAAAAAG CTCCCGGGAG 12840
   CTTGTATATC CATTTTCGGA TCTGATCAAG AGACAGGATG AGGATCGTTT CGCATGATTG 12900
   AACAAGATGG ATTGCACGCA GGTTCTCCGG CCGCTTGGGT GGAGAGGCTA TTCGGCTATG 12960
   ACTGGGCACA ACAGACAATC GGCTGCTCTG ATGCCGCCGT GTTCCGGCTG TCAGCGCAGG 13020
   GGCGCCCGGT TCTTTTTGTC AAGACCGACC TGTCCGGTGC CCTGAATGAA CTGCAGGACG 13080
   AGGCAGCGCG GCTATCGTGG CTGGCCACGA CGGGCGTTCC TTGCGCAGCT GTGCTCGACG 13140
   TTGTCACTGA AGCGGGAAGG GACTGGCTGC TATTGGGCGA AGTGCCGGGG CAGGATCTCC 13200
   TGTCATCTCA CCTTGCTCCT GCCGAGAAAG TATCCATCAT GGCTGATGCA ATGCGGCGGC 13260
   TGCATACGCT TGATCCGGCT ACCTGCCCAT TCGACCACCA AGCGAAACAT CGCATCGAGC 13320
   GAGCACGTAC TCGGATGGAA GCCGGTCTTG TCGATCAGGA TGATCTGGAC GAAGAGCATC 13380
   AGGGGCTCGC GCCAGCCGAA CTGTTCGCCA GGCTCAAGGC GCGCATGCCC GACGGCGAGG 13440
   ATCTCGTCGT GACCCATGGC GATGCCTGCT TGCCGAATAT CATGGTGGAA AATGGCCGCT 13500
   TTTCTGGATT CATCGACTGT GGCCGGCTGG GTGTGGCGGA CCGCTATCAG GACATAGCGT 13560
   TGGCTACCCG TGATATTGCT GAAGAGCTTG GCGGCGAATG GGCTGACCGC TTCCTCGTGC 13620
   TTTACGGTAT CGCCGCTCCC GATTCGCAGC GCATCGCCTT CTATCGCCTT CTTGACGAGT 13680
   TCTTCTGAGC GGGACTCTGG GGTTCGAAAT GACCGACCAA GCGACGCCCA ACCTGCCATC 13740
   ACGAGATTTC GATTCCACCG CCGCCTTCTA TGAAAGGTTG GGCTTCGGAA TCGTTTTCCG 13800
   GGACGCCGGC TGGATGATCC TCCAGCGCGG GGATCTCATG CTGGAGTTCT TCGCCCACCC 13860
   CAACTTGTTT ATTGCAGCTT ATAATGGTTA CAAATAAAGC AATAGCATCA CAAATTTCAC 13920
   AAATAAAGCA TTTTTTTCAC TGCATTCTAG TTGTGGTTTG TCCAAACTCA TCAATGTATC 13980
   TTATCATGTC TGTATACCGT CGACCTCTAG CTAGAGCTTG GCGTAATCAT GGTCATAGCT 14040
   GTTTCCTGTG TGAAATTGTT ATCCGCTCAC AATTCCACAC AACATACGAG CCGGAAGCAT 14100
   AAAGTGTAAA GCCTGGGGTG CCTAATGAGT GAGCTAACTC ACATTAATTG CGTTGCGCTC 14160
   ACTGCCCGCT TTCCAGTCGG GAAACCTGTC GTGCCAGCTG CATTAATGAA TCGGCCAACG 14220
   CGCGGGGAGA GGCGGTTTGC GTATTGGGCG CTCTTCCGCT TCCTCGCTCA CTGACTCGCT 14280
   GCGCTCGGTC GTTCGGCTGC GGCGAGCGGT ATCAGCTCAC TCAAAGGCGG TAATACGGTT 14340
   ATCCACAGAA TCAGGGGATA ACGCAGGAAA GAACATGTGA GCAAAAGGCC AGCAAAAGGC 14400
   CAGGAACCGT AAAAAGGCCG CGTTGCTGGC GTTTTTCCAT AGGCTCCGCC CCCCTGACGA 14460
   GCATCACAAA AATCGACGCT CAAGTCAGAG GTGGCGAAAC CCGACAGGAC TATAAAGATA 14520
   CCAGGCGTTT CCCCCTGGAA GCTCCCTCGT GCGCTCTCCT GTTCCGACCC TGCCGCTTAC 14580
   CGGATACCTG TCCGCCTTTC TCCCTTCGGG AAGCGTGGCG CTTTCTCAAT GCTCACGCTG 14640
   TAGGTATCTC AGTTCGGTGT AGGTCGTTCG CTCCAAGCTG GGCTGTGTGC ACGAACCCCC 14700
   CGTTCAGCCC GACCGCTGCG CCTTATCCGG TAACTATCGT CTTGAGTCCA ACCCGGTAAG 14760
   ACACGACTTA TCGCCACTGG CAGCAGCCAC TGGTAACAGG ATTAGCAGAG CGAGGTATGT 14820
   AGGCGGTGCT ACAGAGTTCT TGAAGTGGTG GCCTAACTAC GGCTACACTA GAAGGACAGT 14880
   ATTTGGTATC TGCGCTCTGC TGAAGCCAGT TACCTTCGGA AAAAGAGTTG GTAGCTCTTG 14940
   ATCCGGCAAA CAAACCACCG CTGGTAGCGG TGGTTTTTTT GTTTGCAAGC AGCAGATTAC 15000
   GCGCAGAAAA AAAGGATCTC AAGAAGATCC TTTGATCTTT TCTACGGGGT CTGACGCTCA 15060
   GTGGAACGAA AACTCACGTT AAGGGATTTT GGTCATGAGA TTATCAAAAA GGATCTTCAC 15120
   CTAGATCCTT TTAAATTAAA AATGAAGTTT TAAATCAATC TAAAGTATAT ATGAGTAAAC 15180
   TTGGTCTGAC AGTTACCAAT GCTTAATCAG TGAGGCACCT ATCTCAGCGA TCTGTCTATT 15240
   TCGTTCATCC ATAGTTGCCT GACTCCCCGT CGTGTAGATA ACTACGATAC GGGAGGGCTT 15300
   ACCATCTGGC CCCAGTGCTG CAATGATACC GCGAGACCCA CGCTCACCGG CTCCAGATTT 15360
   ATCAGCAATA AACCAGCCAG CCGGAAGGGC CGAGCGCAGA AGTGGTCCTG CAACTTTATC 15420
   CGCCTCCATC CAGTCTATTA ATTGTTGCCG GGAAGCTAGA GTAAGTAGTT CGCCAGTTAA 15480
   TAGTTTGCGC AACGTTGTTG CCATTGCTAC AGGCATCGTG GTGTCACGCT CGTCGTTTGG 15540
   TATGGCTTCA TTCAGCTCCG GTTCCCAACG ATCAAGGCGA GTTACATGAT CCCCCATGTT 15600
   GTGCAAAAAA GCGGTTAGCT CCTTCGGTCC TCCGATCGTT GTCAGAAGTA AGTTGGCCGC 15660
   AGTGTTATCA CTCATGGTTA TGGCAGCACT GCATAATTCT CTTACTGTCA TGCCATCCGT 15720
   AAGATGCTTT TCTGTGACTG GTGAGTACTC AACCAAGTCA TTCTGAGAAT AGTGTATGCG 15780
   GCGACCGAGT TGCTCTTGCC CGGCGTCAAT ACGGGATAAT ACCGCGCCAC ATAGCAGAAC 15840
   TTTAAAAGTG CTCATCATTG GAAAACGTTC TTCGGGGCGA AAACTCTCAA GGATCTTACC 15900
   GCTGTTGAGA TCCAGTTCGA TGTAACCCAC TCGTGCACCC AACTGATCTT CAGCATCTTT 15960
   TACTTTCACC AGCGTTTCTG GGTGAGCAAA AACAGGAAGG CAAAATGCCG CAAAAAAGGG 16020
   AATAAGGGCG ACACGGAAAT GTTGAATACT CATACTCTTC CTTTTTCAAT ATTATTGAAG 16080
   CATTTATCAG GGTTATTGTC TCATGAGCGG ATACATATTT GAATGTATTT AGAAAAATAA 16140
   ACAAATAGGG GTTCCGCGCA CATTTCCCCG AAAAGTGCCA CCTGACGTCG ACGGATCGGG 16200
   AGATCTAATG AAAGACCCCA CCTGTAGGTT TGGCAAGCTA GCTTAAGTAA CGCCATTTTG 16260
   CAAGGCATGG AAAAATACAT AACTGAGAAT AGAGAAGTTC AGATCAAGGT CAGGAACAGA 16320
   TGGAACAGCT GAATATGGGC CAAACAGGAT ATCTGTGGTA AGCAGTTCCT GCCCCGGCTC 16380
   AGGGCCAAGA ACAGATGGAA CAGCTGAATA TGGGCCAAAC AGGATATCTG TGGTAAGCAG 16440
   TTCCTGCCCC GGCTCAGGGC CAAGAACAGA TGGTCCCCAG ATGCGGTCCA GCCCTCAGCA 16500
   GTTTCTAGAG AACCATCAGA TGTTTCCAGG GTGCCCCAAG GACCTGAAAT GACCCTGTGC 16560
   CTTATTTGAA CTAACCAATC AGTTCGCTTC TCGCTTCTGT TCGCGCGCTT CTGCTCCCCG 16620
   AGCTCAATAA AAGAGCCCAC AACCCCTCAC TCGGGG 16656